# EUROPEAN PATENT APPLICATION

(11) **EP 4 477 665 A1**
(43) Date of publication of application: **18.12.2024**
(21) Application number: 23753222.1
(22) Date of filing: 10.02.2023
(51) Int. Cl.: C07K 14/81, A61K 47/64, A61P 37/00, C12N 15/86, C12N 15/88, A61K 38/00

(54) **STEFIN A PROTEIN VARIANTS SPECIFICALLY BINDING TO CD40L, AND USES THEREOF**

(30) Priority: 10.02.2022 US 202263308629 P
(71) Applicant: AffyXell Therapeutics Co., Ltd., Gimhae-si, Gyeongsangnam-do 50969 (KR)
(72) Inventor: STANLEY, Emma, Hitchin, Hertfordshire SG5 2HY (GB); JENKINS, Emma, Stansted, Essex CM24 8BB (GB); ADAM, Estelle, Hitchin, Hertfordshire SG5 1UE (GB); BASRAN, Amrik, Cambridge, Cambridgeshire CB1 8PY (GB); VINCENT, Matthew P., Amesbury, Massachusetts 01913 (US)
(74) Representative: Mewburn Ellis LLP
(86) International application number: PCT/KR2023/002022
(87) International publication number: WO 2023/153876

(57) **Abstract**

The present invention relates to a stefin A protein variant specifically binding to CD40L and the use thereof. The stefin A protein variant specifically binding to CD40L according to the present invention, and/or a fusion protein or conjugate comprising the same, is a non-immunogenic polypeptide that can bind to CD40L with high affinity and specificity, and thus is useful for targeting CD40L and various cells known to express CD40L. In addition, the stefin A protein variant specifically binding to CD40L according to the present invention exhibits excellent CD40L activity inhibitory ability, and thus may be useful for medical/pharmaceutical purposes for the prevention and treatment of various diseases related to CD40L.

## Description

### Technical Field

The present invention relates to a stephin A protein variant that specifically binds to CD40L and uses thereof.

### Background Art

The immune system is a biological network composed of various cells and organs designed to protect an organism from external invasion. The immune system operates based on the specific functions of the organs and cells that form it and the signaling and interaction between cells. The immune system maintains immune homeostasis by balancing between immune tolerance that regulates and modulates immunity and immune response that enhances immunity. Imbalance of immune tolerance and immune response may be caused by various factors, and this imbalance in immunity leads to the development of various diseases. For example, when the immune tolerance mechanism is strengthened, occurrence of cancer or invasion of external infectious agents is facilitated, thereby causing cancer, infectious diseases, and the like. Conversely, when the immune response mechanism is strengthened, inflammatory diseases such as autoimmune diseases and allergies may occur.

Recently, thorough research into immune synapses, which are signaling systems between immune cells constituting the immune system or between target cells and immune cells, is ongoing. Immune synapses are composed of various cytokines and signal transmitters secreted from cells, and various costimulatory molecules and receptors expressed on the cell surface. As various factors involved in maintaining homeostasis of the immune system have been reported, interest in immunotherapeutic agents for regulating immune responses by targeting such factors is increasing. Most immunotherapeutic agents developed to date are antibody drugs against cytokines or cell surface molecules, but the use thereof is very limited due to insufficient efficacy or side effects. Recently, in order to more effectively treat immune diseases, cell therapeutic agents using differentiated immune cells such as T cells, NK cells, etc. or stem cells capable of differentiating into various immune cells are being developed. Cell therapeutic agents based on differentiated immune cells such as CAR-T or CAR-NK are expensive due to the use of autologous cells and have limited targets, and mesenchymal stem cells have a low therapeutic effect relative to the cost of treatment and have limitations in that it is difficult to explain a clear mechanism of action (Blood Cancer J. 11, 69 (2021); World J. Stem Cells. 2019;11(4):212-221).

Meanwhile, a CD40 ligand (also called CD40L, or CD154) is a protein that binds to CD40 of antigen-presenting cells, and exhibits various effects depending on target cells (The Journal of Experimental Medicine. 175 (4): 1091-101). CD40L typically has three binding partners: CD40, α5β1 integrin, and αIIbβ3. It is reported that CD40L acts as a costimulatory molecule and is particularly important for a subset of T cells called T follicular helper cells (TFH cells) (Journal of Immunology. 149 (12): 3817-26). CD40L is mainly expressed in activated CD4+ T cells, but is also found in a soluble form, and is reported to be expressed not only in T cells, but also in platelets, mast cells, macrophages, basophils, NK cells, B cells, and non-hematopoietic cells such as endothelial cells and epithelial cells (Cellular and Molecular Life Sciences. 58 (1): 4-43). CD40L is classified as a member of the tumor necrosis factor (TNF) superfamily, and signaling of CD40/CD40L as a costimulatory factor plays an important role in T-cell activation and T-cell-mediated B-cell differentiation and activation. Moreover, stimulation of CD40/CD40L signaling plays an important role in regulating the expression and signaling mechanism of OX40/OX40L, which is a costimulatory factor of the same type, and is thus involved in the survival of T cells and the development of memory T cells.

In various immune homeostasis-related diseases, it is reported that CD40L plays an important role in the interaction between antigen-presenting cells (APCs) and T cells. In particular, the CD40L/CD40 interaction acts as a pathogenic factor in autoimmune diseases or inflammatory diseases in which the activation of T cells and B cells has a major influence on pathology. Specifically, it is a pathogenic factor of various diseases such as type 1 diabetes, thyroiditis, psoriasis, lupus (systemic lupus erythematosus; SLE), rheumatoid arthritis (RA), multiple sclerosis (MS), and the like. Various compounds or antibodies targeting CD40L have been developed for the treatment of these diseases (Semin Immunol. 2009;21(5):293-300; Advanced Drug Delivery Reviews Volume 141, 15 February 2019, Pages 92-103).

Affimer^{®}, developed by Avacta, is a small stable protein molecule engineered based on a stefin A protein, which is an in-vivo protein. Affimer^{®} includes two short peptide sequences having a random sequence and an N-terminal sequence, and is able to bind to a target material with high affinity and specificity in a manner similar to a monoclonal antibody. Affimer^{®} shows remarkably improved binding affinity and specificity compared to the free peptide library, and has a very small size and high stability compared to antibodies, and is therefore receiving great attention as a next-generation alternative pharmaceutical platform to replace antibodies (U.S. Patent Nos. 9447170, 8853131, etc.).

Against this background, the present inventors have made intensive efforts to develop a polypeptide that specifically binds to CD40L by engineering the natural stefin A protein. As a result, a stefin A protein mutant binding to CD40L with excellent affinity and specificity was invented. In particular, it was confirmed that the stefin A protein variant specifically binding to CD40L exhibits excellent inhibitory activity against CD40L.

### Summary of the Invention

It is an object of the present invention to provide a stefin A protein variant that specifically binds to CD40L and a use thereof.

It is another object of the present invention to provide a fusion protein comprising the stefin A protein variant that specifically binds to CD40L.

It is still another object of the present invention to provide a conjugate comprising the stefin A protein variant that specifically binds to CD40L.

It is yet another object of the present invention to provide nucleic acids encoding the stefin A protein variant that specifically binds to CD40L, or the fusion protein or conjugate comprising the same, and a vector containing the nucleic acids.

It is still yet another object of the present invention is to provide genetically engineered cells into which the nucleic acids and/or vectors are introduced and uses thereof.

In order to accomplish the objects of the present invention, the present invention provides a stefin A protein variant specifically binding to CD40L.

The present invention also provides a fusion protein comprising the stefin A protein variant specifically binding to CD40L.

The present invention also provides a conjugate comprising the stefin A protein variant specifically binding to CD40L.

The present invention also provides nucleic acids encoding the stefin A protein variant specifically binding to CD40L or the fusion protein containing the same.

The present invention also provides a delivery vehicle comprising the nucleic acids.

The present invention also provides a genetically engineered cell into which the nucleic acids have been introduced.

The present invention also provides the use of the genetically engineered cells for the preparation of stefin A protein variants, the fusion proteins or the conjugates.

The present invention also provides a method for producing the stefin A protein variant specifically binding to CD40L, the fusion protein or the conjugate, comprising the step of culturing the genetically engineered cells.

The present invention also provides a pharmaceutical composition comprising the stefin A protein variant specifically binding to CD40L, the fusion protein, the conjugate, the nucleic acid, and/or the delivery vehicle.

The present invention also provides the use of the stefin A protein variant specifically binding to CD40L, the fusion protein, the conjugate, the nucleic acid, and/or the delivery vehicle for manufacturing the pharmaceutical composition.

The present invention also provides a method for treatment comprising, administering the stefin A protein variant specifically binding to CD40L, the fusion protein, the conjugate, the nucleic acid, and/or the delivery vehicle to the subject.

The stefin A protein variant specifically binding to CD40L, and/or a fusion protein or conjugate comprising the same of the present invention, is a non-immunogenic polypeptide that can bind to CD40L with high affinity and specificity, thereby is useful for targeting CD40L and a variety of cells known to express CD40L. In addition, the stefin A protein variant specifically binding to CD40L of the present invention exhibits excellent ability to inhibit CD40L activity, and thus can be usefully used for medical/pharmaceutical purposes for the prevention and treatment of various CD40L-related diseases.

### Brief Description of Drawings

**FIG. 1** shows the results of a direct binding ELISA for monomeric clones to huCD40L.
**FIG. 2** shows a validation of hCD40L expression on HEK293 cells following monomeric clone binding determined by flow cytometry.
**FIG. 3** is a validation of hCD40L expression of hCD40L-HEK293 cells (top) compared to control HEK 293 cells (bottom).
**FIG.4** shows the binding of clone 230 (SEQ ID NO: 249) to hCD40L-HEK293 cells at different concentrations.
**FIG. 5** shows the dose effect of the stefin A protein variant specifically binding to CD40L in blocking the binding of hCD40-L to CD40 in a CD40L HEK-Blue reporter cell assay.
**FIG. 6** shows various in line fusion (ILF) formats used to increase avidity to CD40L. 1 µg (left) and 5 µg (right) concentrations were tested for each format. Formats are depicted above the gels (e.g., monomer, dimer, or trimer).
**FIG. 7** shows the binding of different clones, including ILF dimeric and trimeric structures, to hCD40L using a BIACORE^{™} assay.
**FIG. 8** shows the binding of different clones, including ILF dimeric and trimeric structures, to hCD40L using flow cytometry.
**FIG. 9** shows the dose effect of the stefin A protein variant having different formats (monomeric, dimeric, trimeric) in blockading the binding of hCD40L to CD40 in a CD40L HEK-Blue reporter cell assay.
**FIG. 10** shows the results of a competitive ELISA for the binding of clones having different formats (DT, trimer with a rigid linker; XT75, trimer with a rigid linker and an stefin A protein variant binding to HSA; DS, tetramer with a rigid linker; XT76, tetramer with a rigid linker and an stefin A protein variant binding to HSA) to hCD40L.
**FIG. 11** shows the dose effect of the different formats (DT, trimer with a rigid linker; XT75, trimer with a rigid linker and an stefin A protein variant binding to HSA; DS, tetramer with a rigid linker; XT76, tetramer with a rigid linker and an stefin A protein variant binding to HSA) in blockading the binding of hCD40L to CD40 in a CD40L HEK-Blue reporter cell assay.

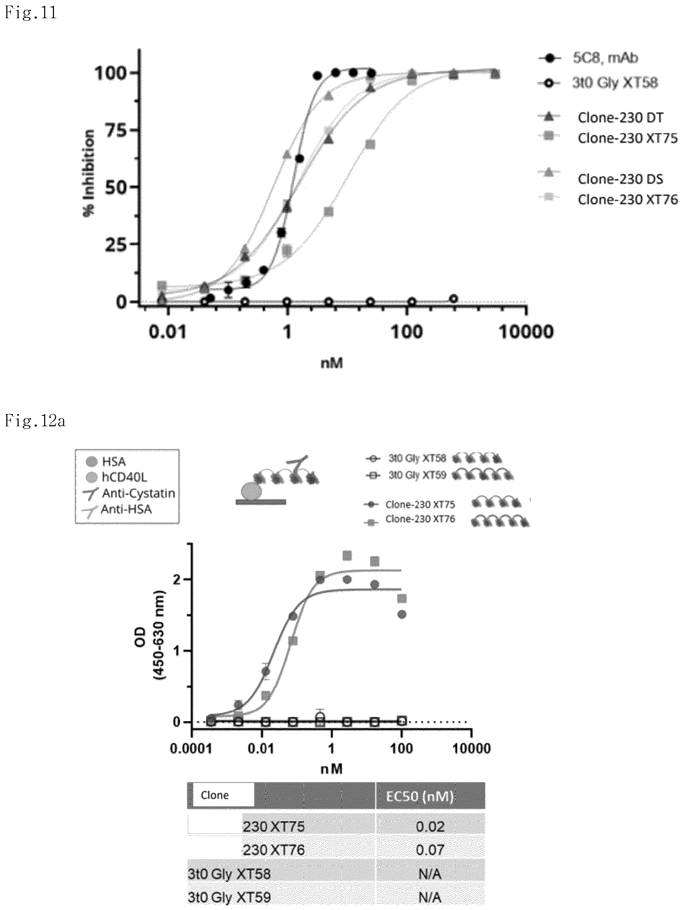

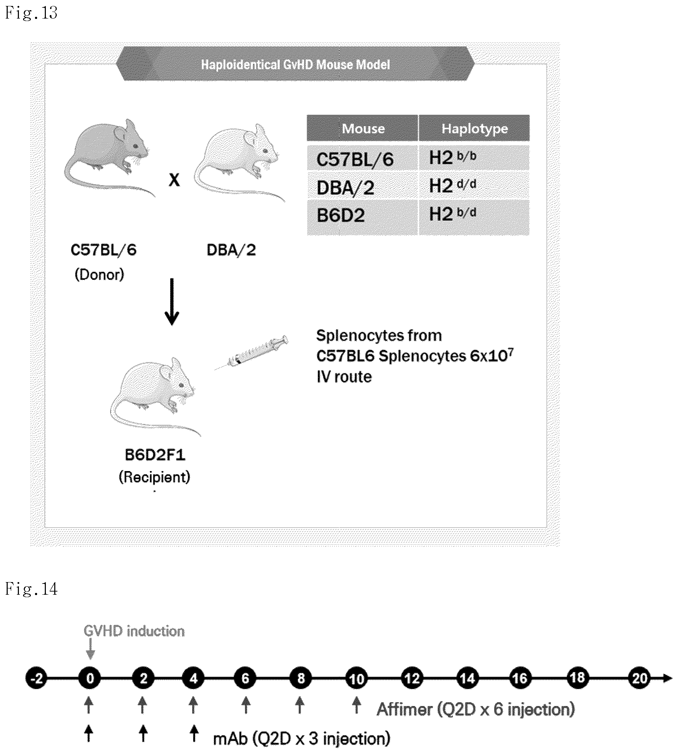

**FIGs. 12A-12C** show the results of a hCD40L/HSA bridging ELISAs using clone-230 XT75 (an ILF protein comprising three clone 230 monomers and an stefin A protein variant binding to HSA (HSA AFFIMER^{®}) polypeptide) and clone-230 XT76 (an ILF protein comprising four clone 230 monomers and an stefin A protein variant binding to HSA). 3t0 Gly XT58 is an ILF protein comprising a trimer of non-hCD40L-targeting protein and an stefin A protein variant binding to HSA polypeptide, joined together with a rigid linker, and 3t0 Gly XT59 is an ILF protein comprising two non-hCD40L-targeting proteins, an stefin A protein variant binding to HSA, and two non-hCD40L-targeting proteins joined together with a rigid linker, FIG. 13A shows AFFIMER^{®} polypeptide binding to bound hCD40L (measured with an anti-cystatin antibody). FIG. 13B shows stefin A protein variant binding to bound hCD40L (measured with an anti-cystatin antibody) in the presence of HSA. FIG. 13C shows stefin A protein variant binding to bound hCD40L and to HSA (measured by an anti-HSA antibody).
**FIG. 13** schematically shows a haploidentical GVHD mouse model.
**FIG. 14** shows the administration frequency and administration interval of the anti-CD40L stefin A protein variant (XT54 SEQ ID NO: 729, XT55 SEQ ID NO: 730) in the haploidentical GVHD mouse model.
**FIG. 15** shows the efficacy of the anti-CD40L stefin A protein variant (XT54 SEQ ID NO: 729, XT55 SEQ ID NO: 730) in the haploidentical GVHD mouse model depending on body weight change (top) and GVHD clinical score (bottom).

### Detailed Description

Unless otherwise defined, all technical and scientific terms used herein have the same meanings as those typically understood by those skilled in the art to which the present invention belongs. In general, the nomenclature used herein is well known in the art and is typical.

The term, "Protein" or "Polypeptides" and proteins) are polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing at least one analog of an amino acid (including, for example, unnatural amino acids), as well as other modifications known in the art.

For the most part, the amino acids and amino acids sequence used in the application are those naturally occurring amino acids found in proteins, or the naturally occurring anabolic or catabolic products of such amino acids which contain amino and carboxyl groups, and isomer thereof (e.g. D- or L- stereoisomers).

Amino acid residues further include analogs, derivatives and congeners of any specific amino acid referred to herein, as for instance, if the stefin A protein variant of the present inveniton generated by chemical synthesis can include an amino acid analog such as, for example, cyanoalanine, canavanine, djenkolic acid, norleucine, 3-phosphoserine, homoserine, dihydroxy-phenylalanine, 5-hydroxytryptophan, 1-methylhistidine, 3-methylhistidine, diaminiopimelic acid, ornithine, or diaminobutyric acid.

The terms "identical" or percent "identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. In the present invention, two nucleic acid or amino acid sequences can be "substantially identical", which means that the two sequences are at least 70%, 75%, 80%, 85%, 90%, 95%, 96%, 97%, 98%, or 99% identical. In some embodiments, identity may exist over a length of at least about 10, 20, 40-60, 60-80, 80-100 or more residues.

The protein or polypeptide described herein, for example, a stefin A protein variant, a fusion protein, or a fusion protein configuration, may include not only the amino acid sequence described in regard thereto, but also a protein or polypeptide in which a portion of the amino acid sequence is substituted through conservative substitution.

As used herein, "conservative substitution" refers to a modification of a polypeptide comprising substituting one or more amino acids with amino acids having similar biochemical properties that do not cause loss of biological or biochemical functions of the polypeptide.

A conservative amino acid substitution is one in which one amino acid residue is replaced with another amino acid residue having a similar side chain. Families of amino acid residues having similar side chains have been generally defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). For example, substitution of a phenylalanine for a tyrosine is a conservative substitution. Generally, conservative substitutions in the sequences of the polypeptides, proteins of the present invention do not cause functional loss, for example, a stefin A protein variant that specifically binds to CD40L does not abrogate its binding to CD40L by its conservative substitution. Methods of identifying amino acid conservative substitutions which do not eliminate binding are well-known in the art.

A polypeptide, soluble protein, antibody, polynucleotide, vector, cell, or composition which is "isolated" is a polypeptide, soluble protein, antibody, polynucleotide, vector, cell, or composition which is in a form not found in nature. Isolated polypeptides, soluble proteins, antibodies, polynucleotides, vectors, cells, or compositions include those which have been purified to a degree that they are no longer in a form in which they are found in nature. In some embodiments, a polypeptide, soluble protein, antibody, polynucleotide, vector, cell, or composition which is isolated is substantially pure.

"Cluster of Differentiation 40 ligand (CD40L)" is a protein that, in the case of humans, is encoded by the CD40L gene. CD40L is a protein that acts as a ligand to CD40/TNFRSF5 and costimulates T-cell proliferation and cytokine production. Its cross-linking on T-cells generates a costimulatory signal which enhances the production of IL4 and IL10 in conjunction with the TCR/CD3 ligation and CD28 costimulation. CD40L induces the activation of NF-kappa-B, induces the activation of kinases MAPK8 and PAK2 in T-cells, and induces tyrosine phosphorylation of isoform 3 of CD28. It also mediates B-cell proliferation in the absence of co-stimulus as well as IgE production in the presence of IL4. CD40 L is also involved in immunoglobulin class switching. Activation of endothelial cells by CD40L (e.g., from activated platelets) leads to reactive oxygen species production, as well as chemokine and cytokine production, and expression of adhesion molecules such as E-selectin, ICAM-1, and VCAM-1. This inflammatory reaction in endothelial cells promotes recruitment of leukocytes to lesions and may potentially promote atherogenesis. CD40L also has shown to be a potential biomarker for atherosclerotic instability. Although usually considered to be a membrane-bound protein, natural, proteolytically cleaved 15-18-kDa soluble forms of CD40L with full biological activity have also been described. CD40L binds to its receptor CD40 on B cells and dendritic cells and thereby provides a critical helper T cell signal needed for germinal centre formation, isotype class switching and production of immunoglobulin antibodies. Defects in CD40L are the cause of an X-linked immunodeficiency with hyper-IgM (HIGM1), an immunoglobulin switch defect characterized by elevated concentrations of IgM and decreased amounts of all other isotypes. CD40L knockout mice fail to mount secondary antibody responses to T cell-dependent antigens and undergo isotype class switching. CD40L may also transmit a signal back to T cells, resulting in short-term T-cell proliferation. A role for CD40L in proliferation of epithelial, fibroblast and smooth muscle cells has also been demonstrated.

In the present invention, a stefin A protein variant that binds to CD40L with excellent affinity and specificity was invented. In particular, the stefin A protein variant specifically binding to CD40L exhibits excellent inhibitory activity against CD40L.

Accordingly, one aspect of the present invention relates to a stefin A protein variant specifically binding to CD40L.

### Stefin A protein variant specifically binding to CD40L

As used herein, the term "stefin A protein variant (stefin A protein variant)" a scaffold based on a stefin A polypeptide, meaning that it has a sequence which is derived from a stefin A polypeptide, for example, a mammalian stefin A polypeptide, for example, a human stefin A polypeptide.. In the present invention, the "stefin A protein Variant" may be used interchangeably in substantially the same sense as "stefin A protein variant" or "AFFIMER^{®} protein". In the present invention, the "stefin A protein Variant" may be used interchangeably in substantially the same sense as "stefin A polypeptide variant" or "AFFIMER^{®} protein".

In one embodiment of the present invention, a stefin A protein variant that specifically binds to CD40L was developed. The "stefin A protein variant specifically binding to CD40L" may be used interchangeably with "anti-CD40L stefin A protein variant" and "anti-CD40L Affimer" in substantially the same meaning.

As used herein, the term "stefin A protein" or (stefin polypeptides)" encompass a subgroup of proteins in the cystatin superfamily, a family which encompasses proteins that contain multiple cystatin-like sequences. The stefin subgroup of the cystatin family includes relatively small (around 100 amino acids) single domain proteins. They receive no known post-translational modification, and lack disulfide bonds, suggesting that they will be able to fold identically in a wide range of extracellular and intracellular environments. stefin A itself is a monomeric, single chain, single domain protein of 98 amino acids. Avacta developed the Affimer^{®} platform using protein engineering techniques through mutation, based on the structure of stefin A. The only known biological activity of cystatins is the inhibition of cathepsin activity, which allowed for exhaustive testing for residual biological activity of the engineered proteins.

As used herein, the term "stefin A protein variant" refers to a small, highly stable protein that is an engineered variant of a stefin polypeptide. stefin A protein variantdisplay two peptide loops and an N-terminal sequence that can all be randomized to bind to desired target proteins with high affinity and specificity, in a similar manner to monoclonal antibodies. Stabilization of the two peptides by the stefin A protein scaffold constrains the possible conformations that the peptides can take, increasing the binding affinity and specificity compared to libraries of free peptides. These engineered non-antibody binding proteins are designed to mimic the molecular recognition characteristics of monoclonal antibodies in different applications. Variations to other parts of the stefin A polypeptide sequence can be carried out, with such variations improving the properties of these affinity reagents, such as increase stability, make them robust across a range of temperatures and pH and the like. In some embodiments, an stefin A protein variant includes a sequence derived from stefin A, sharing substantial identify with a stefin A wild type sequence, such as human stefin A. It will be apparent to a person skilled in the art that modifications may be made to the scaffold sequence without departing from the disclosure. In particular, an stefin A protein variant can have an amino acid sequences that is at least 25%, 35%, 45%, 55% or 60% identity to the corresponding sequences to human stefin A, for example, at least 70%, 80%, 85%, 90%, 92%, 94%, 95% identical, e.g., where the sequence variations do not adversely affect the ability of the scaffold to bind to the desired target (such as CD40L), and e.g., which do not restore or generate biological functions such as those which are possessed by wild type stefin A but which are abolished in mutational changes described herein. A target protein-specific binding platform using such a stefin A protein variant is disclosed in detail in US Patent No. 9447170 and No. 8853131.

In some embodiments, the stefin A protein variant is featured in that it can bind to CD40L, a target protein, with high affinity and specificity through engineering of the stefin A protein.

In some embodiments, the stefin A protein variant specifically binding to CD40L may bind to CD40L, thereby reducing or inhibiting the activity of CD40L.

As used herein, the term "CD40L" or "CD40 ligand" refers to a protein that binds to its receptor, CD40, and is also called "CD154". Aliases for CD40L include TNF-Related Activation Protein, TRAP, Tumor Necrosis Factor (Ligand) Superfamily Member, T-B Cell-Activating Molecule, CD40 Antigen Ligand, T- Cell Antigen Gp39, TNFSF5, HCD40L, CD154, Gp39, Tumor Necrosis Factor (Ligand) Superfamily, Member 5 (Hyper-IgM Syndrome), Tumor Necrosis Factor Ligand Superfamily Member, Hyper-IgM Syndrome, CD154 Antigen, CD40LG, HIGM1, T-BAM, IMD3, IGM, and CD40-L. The human amino acid and nucleic acid sequences can be found in a public database, such as GenBank, UniProt and Swiss-Prot. For example, the amino acid sequence of human CD40L can be found as UniProt/Swiss-Prot. Accession No. P29965 and the nucleotide sequence encoding of the human CD40L can be found at Accession No. NM_000074.2.

The CD40L includes any native, mature CD40L which results from processing of a CD40L precursor protein in a cell. The term encompasses CD40L from any vertebrate source, including mammals such as primates (e.g., humans and cynomolgus monkeys) and rodents (e.g., mice and rats), unless otherwise indicated but is not limited to. The term also includes any CD40L proteins comprising mutations, e.g., point mutations, fragments, insertions, deletions, and splice variants of full length wild-type CD40L, but is not limited to.

In some embodiments, the stefin A protein variant may comprise at least one of the solvent accessible loops from the wild-type stefin A protein having the ability to bind CD40L.

In some embodiments, the stefin A protein variant may bind to CD40L with Kd of 10⁻⁶ M or less.

In some embodiments, the stefin A protein variant may be variant derived from the stefin A polypeptide having a backbone sequence and in which one or both of loop 2 [designated (Xaa)ₙ] and loop 4 [designated (Xaa)ₘ] are replaced with alternative loop sequences (Xaa)ₙ and (Xaa)ₘ.

In some embodiments, the stefin A protein variant may comprise an amino acid sequence represented by Formula (I):

[Formula I] FR1-(Xaa)ₙ-FR2-(Xaa)ₘ-FR3

wherein
FR1 comprises a polypeptide sequence represented by MIPGGLSEAK PATPEIQEIV DKVKPQLEEK TGETYGKLEA VQYKTQVX (SEQ ID NO: 1) or a polypeptide sequence having at least 70% (e.g., at least 75%, at least 80%, at least 85%, at least 90%, at least 95%, or 100%) identity to the amino acid sequence of SEQ ID NO: 1, In some embodiments, wherein X may be V
FR2 comprises a polypeptide sequence comprising the amino acid sequence of GTNYYIKVRA GDNKYMHLKV FKSL (SEQ ID NO: 2) or a polypeptide sequence having at least 70% (e.g., at least 75%, at least 80%, at least 85%, at least 90%, at least 95%, or 100%) identity to the amino acid sequence of SEQ ID NO: 2;
FR3 comprises a polypeptide sequence comprising the amino acid sequence of EDLVLTGYQV DKNKDDELTG F (SEQ ID NO: 3) or a polypeptide sequence having at least 70%, at least 75%, at least 80%, at least 85%, at least 90%, at least 95%, or 100% identity to the amino acid sequence of SEQ ID NO: 3; and Xaa, individually for each occurrence, is an amino acid residue; and n and m are each, independently, an integer from 3 to 20.

In some embodiments, (Xaa)n and (Xaa)m may independently represents an any amino acid sequence of 3 to 20.

In some embodiments, FR1 has a polypeptide sequence having at least 80%, 85%, 90%, 95% or even 98% homology with SEQ ID NO: 1. In some embodiments, FR1 has a polypeptide sequence having at least 80%, 85%, 90%, 95% or even 98% identity with SEQ ID NO: 1; In some embodiments, FR2 has a polypeptide sequence having at least 80%, 85%, 90%, 95% or even 98% homology with SEQ ID NO: 2. In some embodiments, FR2 has a polypeptide sequence having at least 80%, 85%, 90%, 95% or even 98% identity with SEQ ID NO: 2; In some embodiments, FR3 has a polypeptide sequence having at least 80%, 85%, 90%, 95% or even 98% homology with SEQ ID NO: 3. In some embodiments, FR3 has a polypeptide sequence having at least 80%, 85%, 90%, 95% or even 98% identity with SEQ ID NO: 3.

In some embodiments, the stefin A protein variant comprises an amino acid sequence represented in the general Formula (II) (SEQ ID NO: 4): 
[Formula II]
MIP-Xaa1-GLSEAKPATPEIQEIVDKVKPQLEEKTGETYGKLEAVQYKTQVV-
(Xaa)n-Xaa2-TNYYIKVRAGDNKYMHLKVF-Xaa3-Xaa4-Xaa5-(Xaa)m-Xaa6-D-Xaa7-
VLTGYQVDKNKDDELTGF (SEQ ID NO: 4)
 wherein
Xaa, individually for each occurrence, is an amino acid residue, and
n and m are each, independently, an integer from 3-20.

In some embodiments, the stefin A protein variant comprises an amino acid sequence having at least 90%, at least 95%, at least 96%, at least 97%, at least 98%, at least 99%, or 100% identity to the amino acid sequence of: 
MIPGGLSEAKPATPEIQEIVDKVKPQLEEKTGETYGKLEAVQYKTQVD-(Xaa)n-
GTNYYIKVRAGDNKYMHLKVFKSL-(Xaa)m-EDLVLTGYQVDKNKDDELTGF (SEQ ID
NO: 5), wherein
Xaa, individually for each occurrence, is an amino acid residue, and
n and m are each, independently, an integer from 3-20.

In some embodiments, Xaa1 is Gly, Ala, Val, Arg, Lys, Asp, or Glu, more preferably Gly, Ala, Arg or Lys, and more even more preferably Gly or Arg; Xaa2 is Gly, Ala, Val, Ser or Thr, more preferably Gly or Ser; Xaa3 is Arg, Lys, Asn, Gln, Ser, Thr, more preferably Arg, Lys, Asn or Gln, and even more preferably Lys or Asn; Xaa4 is Gly, Ala, Val, Ser or Thr, more preferably Gly or Ser; Xaa5 is Ala, Val, Ile, Leu, Gly or Pro, more preferably Ile, Leu or Pro, and even more preferably Leu or Pro; Xaa6 is Gly, Ala, Val, Asp or Glu, more preferably Ala, Val, Asp or Glu, and even more preferably Ala or Glu; and Xaa7 is Ala, Val, Ile, Leu, Arg or Lys, more preferably Ile, Leu or Arg, and even more preferably Leu or Arg.

In some embodiments, n is 3 to 15, 3 to 12, 3 to 9, 3 to 7, 5 to 7, 5 to 9, 5 to 12, 5 to 15, 7 to 12 or 7 to 9.

In some embodiments, m is 3 to 15, 3 to 12, 3 to 9, 3 to 7, 5 to 7, 5 to 9, 5 to 12, 5 to 15, 7 to 12 or 7 to 9.

In some embodiments, Xaa, independently for each occurrence, is an amino acid that can be added to a polypeptide by recombinant expression in a prokaryotic or eukaryotic cell, and even more preferably one of the 20 naturally occurring amino acids.

In some embodiments of the above sequences and formulas, (Xaa)ₙ comprises an amino acid sequence selected from SEQ ID NOs: 6 to 125, or an amino acid sequence having at least 80%, 85%, 90%, 95% or even 98% homology with a sequence selected from SEQ ID NOs: 6 to 125. In some embodiments, (Xaa)ₙ comprises an amino acid sequence having at least 80%, 85%, 90%, 95% or even 98% identity with a sequence selected from SEQ ID NOs: 6 to 125.

**Table 1. Loop 2 Sequences**

| **Clone #** | **Loop 2** | **SEQ ID NO:** |
|---|---|---|
| 227 | HDVFYDQKA | 6 |
| 228 | PFKIITVWH | 7 |
| 229 | QYKIITWWS | 8 |
| 230 | HYYVHYNDQ | 9 |
| 231 | YYQIITWWQ | 10 |
| 232 | VHGPHLEQA | 11 |
| 233 | LQDQWPWRD | 12 |
| 234 | DA | 13 |
| 235 | SWVKPDNEL | 14 |
| 236 | NSLGVSEWV | 15 |
| 237 | VYRIVSWWR | 16 |
| 238 | YYRQLKFNT | 17 |
| 239 | GWIKVDNVL | 18 |
| 240 | TAYYIHVNA | 19 |
| 241 | VRVEPTPND | 20 |
| 242 | DLNSSVWYQ | 21 |
| 243 | FTYQISYTV | 22 |
| 244 | RENWYGEFA | 23 |
| 245 | TALAWNTQA | 24 |
| 246 | HIPALKVHA | 25 |
| 247 | HHAYAAVYV | 26 |
| 248 | YDELYHEKA | 27 |
| 249 | SWYYPKNVL | 28 |
| 250 | FLEWHFWEW | 29 |
| 251 | SWVRGTNQL | 30 |
| 252 | WRKLLSWWK | 31 |
| 253 | PKSHQTNEQ | 32 |
| 254 | NWNQIDNPL | 33 |
| 255 | FWVGLHNQL | 34 |
| 256 | FTQPFNEPI | 35 |
| 257 | PEYQPQPKA | 36 |
| 258 | NDPYFQSFS | 37 |
| 259 | HQDDVAWQS | 38 |
| 260 | KGYFHTVEA | 39 |
| 261 | VDQVWSENA | 40 |
| 262 | LSSIWQDVA | 41 |
| 263 | TASLYWNYA | 42 |
| 264 | SWKQWHNYL | 43 |
| 265 | QVHLDNSPL | 44 |
| 266 | QNNWYLSNY | 45 |
| 267 | WEWALLLEY | 46 |
| 268 | GDSYWEYVS | 47 |
| 269 | GQKWWQHKI | 48 |
| 270 | LDGLYHVNS | 49 |
| 271 | HAPAPVQAI | 50 |
| 272 | WEDHWNYQY | 51 |
| 273 | SWRYKSNQL | 52 |
| 274 | RVDWWLPEV | 53 |
| 275 | SWIEISNQL | 54 |
| 276 | SLGQAETWA | 55 |
| 278 | KIGQLPSRV | 56 |
| 279 | AWHTKNNSI | 57 |
| 280 | QTKLVTWWW | 58 |
| 281 | AVFDYADPS | 59 |
| 282 | HVKIVTWWK | 60 |
| 283 | HVLKVSSVS | 61 |
| 284 | YQDYDSATH | 62 |
| 286 | SWGKVSNEL | 63 |
| 287 | LEDEWWSIL | 64 |
| 288 | YYKLVTWWR | 65 |
| 289 | AWVKEHNLI | 66 |
| 290 | QEYLWKQYI | 67 |
| 291 | FNKDLFLGW | 68 |
| 292 | HFGIRYAHV | 69 |
| 293 | WYAWQEGWQ | 70 |
| 294 | GNAAWGSFA | 71 |
| 295 | REDLDLFYT | 72 |
| 296 | NENVYRVTQ | 73 |
| 297 | WHKIVTQWP | 74 |
| 298 | HYRIITHWE | 75 |
| 299 | LVFDASTSA | 76 |
| 300 | QYAEFLEHI | 77 |
| 301 | GNVQEDWHI | 78 |
| 302 | GWSASYPES | 79 |
| 303 | YQFEHISYG | 80 |
| 304 | AWITPVNVL | 81 |
| 305 | ESYAAIFWG | 82 |
| 306 | RVWPWTWDR | 83 |
| 307 | QVKKWWEYFV | 84 |
| 308 | IGQISEQQW | 85 |
| 309 | AFHKLGWIL | 86 |
| 310 | FKKWWWKYK | 87 |
| 311 | ASQRLWIIY | 88 |
| 312 | QRETHILWS | 89 |
| 313 | GQYWFWAPG | 90 |
| 314 | RDWAYYIYF | 91 |
| 315 | IGYKLNYIW | 92 |
| 316 | VSLTEQRWK | 93 |
| 317 | WVSQQHYSH | 94 |
| 318 | DDDNQHYGL | 95 |
| 319 | PTRWISIYW | 96 |
| 320 | TAFAPNKAS | 97 |
| 321 | HYTDFKWHK | 98 |
| 322 | NKFWSWAPG | 99 |
| 323 | ELDEPAWDW | 100 |
| 324 | HEKWGFWEL | 101 |
| 325 | VATADLIKA | 102 |
| 326 | YYKVISWWH | 103 |
| 327 | HWHHYDNII | 104 |
| 328 | VYKIITFWS | 105 |
| 329 | GWIQRHNLI | 106 |
| 330 | KTWEDTVVA | 107 |
| 331 | LDQHHVYQV | 108 |
| 332 | LEDVWGVWA | 109 |
| 333 | WLPWTVELI | 110 |
| 334 | QHFTWDWFW | 111 |
| 335 | LFEVLQGSS | 112 |
| 336 | PYSWQHEVV | 113 |
| 337 | WWQENAEWI | 114 |
| 338 | SYDPVYQDT | 115 |
| 339 | RVFDTPLAA | 116 |
| 340 | WWDIPWIWV | 117 |
| 341 | YQKVITWWQ | 118 |
| 342 | QWIKWGNIL | 119 |
| 343 | ATPEKKYVV | 120 |
| 344 | TSERLWILY | 121 |
| 345 | EHHSQPLAH | 122 |
| 346 | WTADWLWTV | 123 |
| 347 | SFQREHYSW | 124 |
| 348 | GKTWHKHNR | 125 |

In some embodiments of the above sequences and formulas, (Xaa)ₘ comprises an amino acid sequence selected from SEQ ID NOs: 126 to 245, or an amino acid sequence having at least 80%, 85%, 90%, 95% or even 98% homology with a sequence selected from SEQ ID NOs: 126 to 245. In some embodiments, (Xaa)ₘ comprises an amino acid sequence having at least 80%, 85%, 90%, 95% or even 98% identity with a sequence selected from SEQ ID NOs: 126 to 245.

**Table 2. Loop 4 Sequences**

| **Clone #** | **Loop 4** | **SEQ ID NO:** |
|---|---|---|
| 227 | KWWYRIGNP | 126 |
| 228 | LERFNFHGE | 127 |
| 229 | VYKNKRGGH | 128 |
| 230 | WGENLFAKW | 129 |
| 231 | GIRKKQQQR | 130 |
| 232 | GHAFYFAFV | 131 |
| 233 | TYGKKWYSW | 132 |
| 234 | YFYRHWEDR | 133 |
| 235 | WQVHDKQWL | 134 |
| 236 | PYWYRKNNL | 135 |
| 237 | ASDIYLRID | 136 |
| 238 | FLHRKPQDR | 137 |
| 239 | WQDHHNVKR | 138 |
| 240 | PYGYRAKAP | 139 |
| 241 | PLVQELWGF | 140 |
| 242 | YWLLEIEHR | 141 |
| 243 | RWRYPARKP | 142 |
| 244 | LYIYRHPNY | 143 |
| 245 | PWLYRQKSD | 144 |
| 246 | PYWYRHDQE | 145 |
| 247 | PYWYRKYPD | 146 |
| 248 | EYKFRNLE | 147 |
| 249 | EDFIAADDD | 148 |
| 250 | DSQEFPSHL | 149 |
| 251 | EPDWPDFLI | 150 |
| 252 | ILNPKSWNF | 151 |
| 253 | WNENHFSVW | 152 |
| 254 | NKDWEDQII | 153 |
| 255 | YYNGDNTQI | 154 |
| 256 | YWDDKVGYY | 155 |
| 257 | FHFRKPQDR | 156 |
| 258 | PYWYREPEN | 157 |
| 259 | EYGWYHLPY | 158 |
| 260 | TYHFRTDLN | 159 |
| 261 | PYLWRKHAV | 160 |
| 262 | LYQFRKEPV | 161 |
| 263 | FYYRGPQPV | 162 |
| 264 | YPDGDLILY | 163 |
| 265 | QFGEIIWWE | 164 |
| 266 | VEERWGLWV | 165 |
| 267 | HHPWQPAKQ | 166 |
| 268 | PYRWRPDHA | 167 |
| 269 | HSNAWNLQH | 168 |
| 270 | FYYYRSEPT | 169 |
| 271 | PYWYRPNRP | 170 |
| 272 | ELYSNQIVW | 171 |
| 273 | YKDWVVNPP | 172 |
| 274 | PYQYRKDPK | 173 |
| 275 | YYGNTSILI | 174 |
| 276 | FKYRHQQDK | 175 |
| 278 | YWYRNPADR | 176 |
| 279 | ETKLAHEDI | 177 |
| 280 | LGEQYIDVD | 178 |
| 281 | PYRYRTHHK | 179 |
| 282 | YNPKPGIDN | 180 |
| 283 | FYYRKRYDR | 181 |
| 284 | YQYRNPIDK | 182 |
| 286 | IEYQSPALY | 183 |
| 287 | PDAPAYSKS | 184 |
| 288 | PDLNYEVTL | 185 |
| 289 | YDQNQWSPH | 186 |
| 290 | RWQYRHPQT | 187 |
| 291 | RFSAGEEWE | 188 |
| 292 | DYPRHHQDQ | 189 |
| 293 | FRKKWAQLW | 190 |
| 294 | PFFYRKDNP | 191 |
| 295 | EFILQPWWY | 192 |
| 296 | KYRHRLTYE | 193 |
| 297 | YAQRGEVYF | 194 |
| 298 | VRAFLYYKD | 195 |
| 299 | PWEFRGDHK | 196 |
| 300 | GWSHYFQYL | 197 |
| 301 | FSEDLTLNG | 198 |
| 302 | TQWEFIEWP | 199 |
| 303 | FTYRLPQHR | 200 |
| 304 | DEIFHGAYN | 201 |
| 305 | WGEIQHWRQ | 202 |
| 306 | EQWLPNPYF | 203 |
| 307 | QLLSHQWTE | 204 |
| 308 | HRYWTRDVT | 205 |
| 309 | GDESHKWHK | 206 |
| 310 | RVDEHYIQF | 207 |
| 311 | SDWRHHGGW | 208 |
| 312 | WFDNLHNNF | 209 |
| 313 | PSSGGGPKW | 210 |
| 314 | RSDTLHYWN | 211 |
| 315 | IQYRWQDRV | 212 |
| 316 | FQPIKLANA | 213 |
| 317 | FAFRKYEDR | 214 |
| 318 | LHNWWKGNW | 215 |
| 319 | LNRYYHASI | 216 |
| 320 | RYWAFRYLW | 217 |
| 321 | PFWANEHGW | 218 |
| 322 | ADSGFNATP | 219 |
| 323 | RQHKKPHPN | 220 |
| 324 | HSHRFHEAH | 221 |
| 325 | PWHYPPNKW | 222 |
| 326 | YKTPFPQYT | 223 |
| 327 | YQLVDLDLD | 224 |
| 328 | EYPPVQQVL | 225 |
| 329 | YSEPPPVQN | 226 |
| 330 | EYLYRPLNP | 227 |
| 331 | ALLIHHDPW | 228 |
| 332 | EFQHRTFNH | 229 |
| 333 | GEIWSAKHK | 230 |
| 334 | PEYKPVDHT | 231 |
| 335 | GEIVWYQVE | 232 |
| 336 | IVFYLQEYYE | 233 |
| 337 | GYAVFHKYL | 234 |
| 338 | FFHRTSVVY | 235 |
| 339 | PWQYRNNPE | 236 |
| 340 | QNDELYPYK | 237 |
| 341 | YWENHRGHV | 238 |
| 342 | YEAIERWHG | 239 |
| 343 | WNANILRYW | 240 |
| 344 | TPRYVPLNP | 241 |
| 345 | PYHAFDWYW | 242 |
| 346 | YDPSEWFPR | 243 |
| 347 | FDIFYFNPI | 244 |
| 348 | NLYFFFYQT | 245 |

In some embodiments, the stefin A protein variant comprisesan amino acid sequence selected from SEQ ID NOs: 246 to 365. In some embodiments, the stefin A protein variant comprisesan amino acid sequence having at least 70%, 75% 80%, 85%, 90%, 95% or even 98% identity with a sequence selected from SEQ ID NOs: 246 to 365.

**Table 3. Exemplary stefin A protein variant specifically binding to CD40LSequences**

| **Clone #** | **Amino Acid Sequence** | **SEQ ID NO:** |
|---|---|---|
| 227 | MIPGGLSEAKPATPEIQEIVDKVKPQLEEKTGETYGKLEAVQYKTQVVHDVFYD QKAGTNYYIKVRAGDNKYMHLKVFKSLKWWYRIGNPEDLVLTGYQVDKNKD DELTGF | 246 |
| 228 | MIPGGLSEAKPATPEIQEIVDKVKPQLEEKTGETYGKLEAVQYKTQVVPFKIITV WHGTNYYIKVRAGDNKYMHLKVFKSLLERFNFHGEEDLVLTGYQVDKNKDDE LTGF | 247 |
| 229 | MIPGGLSEAKPATPEIQEIVDKVKPQLEEKTGETYGKLEAVQYKTQVVQYKIITW WSGTNYYIKVRAGDNKYMHLKVFKSLVYKNKRGGHEDLVLTGYQVDKNKDD ELTGF | 248 |
| 230 | MIPGGLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVDKNKD DELTGF | 249 |
| 231 | MIPGGLSEAKPATPEIQEIVDKVKPQLEEKTGETYGKLEAVQYKTQVVYYQIITW WQGTNYYIKVRAGDNKYMHLKVFKSLGIRKKQQQREDLVLTGYQVDKNKDDE LTGF | 250 |
| 232 | MIPGGLSEAKPATPEIQEIVDKVKPQLEEKTGETYGKLEAVQYKTQVVVHGPHL EQAGTNYYIKVRAGDNKYMHLKVFKSLGHAFYFAFVEDLVLTGYQVDKNKDD ELTGF | 251 |
| 233 | MIPGGLSEAKPATPEIQEIVDKVKPQLEEKTGETYGKLEAVQYKTQVVLQDQWP WRDGTNYYIKVRAGDNKYMHLKVFKSLTYGKKWYSWEDLVLTGYQVDKNKD DELTGF | 252 |
| 234 | MIPGGLSEAKPATPEIQEIVDKVKPQLEEKTGETYGKLEAVQYKTQVDAGTNYY IKVRAGDNKYMHLKVFKSLYFYRHWEDREDLVLTGYQVDKNKDDELTGF | 253 |
| 235 | MIPGGLSEAKPATPEIQEIVDKVKPQLEEKTGETYGKLEAVQYKTQVVSWVKPD NELGTNYYIKVRAGDNKYMHLKVFKSLWQVHDKQWLEDLVLTGYQVDKNKD DELTGF | 254 |
| 236 | MIPGGLSEAKPATPEIQEIVDKVKPQLEEKTGETYGKLEAVQYKTQVVNSLGVSE WVGTNYYIKVRAGDNKYMHLKVFKSLPYWYRKNNLEDLVLTGYQVDKNKDD ELTGF | 255 |
| 237 | MIPGGLSEAKPATPEIQEIVDKVKPQLEEKTGETYGKLEAVQYKTQVVVYRIVS WWRGTNYYIKVRAGDNKYMHLKVFKSLASDIYLRIDEDLVLTGYQVDKNKDD ELTGF | 256 |
| 238 | MIPGGLSEAKPATPEIQEIVDKVKPQLEEKTGETYGKLEAVQYKTQVVYYRQLK FNTGTNYYIKVRAGDNKYMHLKVFKSLFLHRKPQDREDLVLTGYQVDKNKDD ELTGF | 257 |
| 239 | MIPGGLSEAKPATPEIQEIVDKVKPQLEEKTGETYGKLEAVQYKTQVVGWIKVD NVLGTNYYIKVRAGDNKYMHLKVFKSLWQDHHNVKREDLVLTGYQVDKNKD DELTGF | 258 |
| 240 | MIPGGLSEAKPATPEIQEIVDKVKPQLEEKTGETYGKLEAVQYKTQVVTAYYIH VNAGTNYYIKVRAGDNKYMHLKVFKSLPYGYRAKAPEDLVLTGYQVDKNKDD ELTGF | 259 |
| 241 | MIPGGLSEAKPATPEIQEIVDKVKPQLEEKTGETYGKLEAVQYKTQVVVRVEPTP NDGTNYYIKVRAGDNKYMHLKVFKSLPLVQELWGFEDLVLTGYQVDKNKDDE LTGF | 260 |
| 242 | MIPGGLSEAKPATPEIQEIVDKVKPQLEEKTGETYGKLEAVQYKTQVVDLNSSV WYQGTNYYIKVRAGDNKYMHLKVFKSLYWLLEIEHREDLVLTGYQVDKNKDD ELTGF | 261 |
| 243 | MIPGGLSEAKPATPEIQEIVDKVKPQLEEKTGETYGKLEAVQYKTQVVFTYQISY TVGTNYYIKVRAGDNKYMHLKVFKSLRWRYPARKPEDLVLTGYQVDKNKDDE LTGF | 262 |
| 244 | MIPGGLSEAKPATPEIQEIVDKVKPQLEEKTGETYGKLEAVQYKTQVVRENWYG EFAGTNYYIKVRAGDNKYMHLKVFKSLLYIYRHPNYEDLVLTGYQVDKNKDDE LTGF | 263 |
| 245 | MIPGGLSEAKPATPEIQEIVDKVKPQLEEKTGETYGKLEAVQYKTQVVTALAWN TQAGTNYYIKVRAGDNKYMHLKVFKSLPWLYRQKSDEDLVLTGYQVDKNKDD ELTGF | 264 |
| 246 | MIPGGLSEAKPATPEIQEIVDKVKPQLEEKTGETYGKLEAVQYKTQVVHIPALKV HAGTNYYIKVRAGDNKYMHLKVFKSLPYWYRHDQEEDLVLTGYQVDKNKDD ELTGF | 265 |
| 247 | MIPGGLSEAKPATPEIQEIVDKVKPQLEEKTGETYGKLEAVQYKTQVVHHAYAA VYVGTNYYIKVRAGDNKYMHLKVFKSLPYWYRKYPDEDLVLTGYQVDKNKD DELTGF | 266 |
| 248 | MIPGGLSEAKPATPEIQEIVDKVKPQLEEKTGETYGKLEAVQYKTQVVYDELYH EKAGTNYYIKVRAGDNKYMHLKVFKSLEYKFRNLEEDLVLTGYQVDKNKDDE LTGF | 267 |
| 249 | MIPGGLSEAKPATPEIQEIVDKVKPQLEEKTGETYGKLEAVQYKTQVVSWYYPK NVLGTNYYIKVRAGDNKYMHLKVFKSLEDFIAADDDEDLVLTGYQVDKNKDD ELTGF | 268 |
| 250 | MIPGGLSEAKPATPEIQEIVDKVKPQLEEKTGETYGKLEAVQYKTQVVFLEWHF WEWGTNYYIKVRAGDNKYMHLKVFKSLDSQEFPSHLEDLVLTGYQVDKNKDD ELTGF | 269 |
| 251 | MIPGGLSEAKPATPEIQEIVDKVKPQLEEKTGETYGKLEAVQYKTQVVSWVRGT NQLGTNYYIKVRAGDNKYMHLKVFKSLEPDWPDFLIEDLVLTGYQVDKNKDDE LTGF | 270 |
| 252 | MIPGGLSEAKPATPEIQEIVDKVKPQLEEKTGETYGKLEAVQYKTQVVWRKLLS WWKGTNYYIKVRAGDNKYMHLKVFKSLILNPKSWNFEDLVLTGYQVDKNKD DELTGF | 271 |
| 253 | MIPGGLSEAKPATPEIQEIVDKVKPQLEEKTGETYGKLEAVQYKTQVVPKSHQT NEQGTNYYIKVRAGDNKYMHLKVFKSLWNENHFSVWEDLVLTGYQVDKNKD DELTGF | 272 |
| 254 | MIPGGLSEAKPATPEIQEIVDKVKPQLEEKTGETYGKLEAVQYKTQVVNWNQID NPLGTNYYIKVRAGDNKYMHLKVFKSLNKDWEDQIIEDLVLTGYQVDKNKDD ELTGF | 273 |
| 255 | MIPGGLSEAKPATPEIQEIVDKVKPQLEEKTGETYGKLEAVQYKTQVVFWVGLH NQLGTNYYIKVRAGDNKYMHLKVFKSLYYNGDNTQIEDLVLTGYQVDKNKDD ELTGF | 274 |
| 256 | MIPGGLSEAKPATPEIQEIVDKVKPQLEEKTGETYGKLEAVQYKTQVVFTQPFNE PIGTNYYIKVRAGDNKYMHLKVFKSLYWDDKVGYYEDLVLTGYQVDKNKDDE LTGF | 275 |
| 257 | MIPGGLSEAKPATPEIQEIVDKVKPQLEEKTGETYGKLEAVQYKTQVVPEYQPQP KAGTNYYIKVRAGDNKYMHLKVFKSLFHFRKPQDREDLVLTGYQVDKNKDDE LTGF | 276 |
| 258 | MIPGGLSEAKPATPEIQEIVDKVKPQLEEKTGETYGKLEAVQYKTQVVNDPYFQ SFSGTNYYIKVRAGDNKYMHLKVFKSLPYWYREPENEDLVLTGYQVDKNKDD ELTGF | 277 |
| 259 | MIPGGLSEAKPATPEIQEIVDKVKPQLEEKTGETYGKLEAVQYKTQVVHQDDVA WQSGTNYYIKVRAGDNKYMHLKVFKSLEYGWYHLPYEDLVLTGYQVDKNKD DELTGF | 278 |
| 260 | MIPGGLSEAKPATPEIQEIVDKVKPQLEEKTGETYGKLEAVQYKTQVVKGYFHT VEAGTNYYIKVRAGDNKYMHLKVFKSLTYHFRTDLNEDLVLTGYQVDKNKDD ELTGF | 279 |
| 261 | MIPGGLSEAKPATPEIQEIVDKVKPQLEEKTGETYGKLEAVQYKTQVVVDQVWS ENAGTNYYIKVRAGDNKYMHLKVFKSLPYLWRKHAVEDLVLTGYQVDKNKD DELTGF | 280 |
| 262 | MIPGGLSEAKPATPEIQEIVDKVKPQLEEKTGETYGKLEAVQYKTQVVLSSIWQD VAGTNYYIKVRAGDNKYMHLKVFKSLLYQFRKEPVEDLVLTGYQVDKNKDDE LTGF | 281 |
| 263 | MIPGGLSEAKPATPEIQEIVDKVKPQLEEKTGETYGKLEAVQYKTQVVTASLYW NYAGTNYYIKVRAGDNKYMHLKVFKSLFYYRGPQPVEDLVLTGYQVDKNKDD ELTGF | 282 |
| 264 | MIPGGLSEAKPATPEIQEIVDKVKPQLEEKTGETYGKLEAVQYKTQVVSWKQW HNYLGTNYYIKVRAGDNKYMHLKVFKSLYPDGDLILYEDLVLTGYQVDKNKD DELTGF | 283 |
| 265 | MIPGGLSEAKPATPEIQEIVDKVKPQLEEKTGETYGKLEAVQYKTQVVQVHLDN SPLGTNYYIKVRAGDNKYMHLKVFKSLQFGEIIWWEEDLVLTGYQVDKNKDDE LTGF | 284 |
| 266 | MIPGGLSEAKPATPEIQEIVDKVKPQLEEKTGETYGKLEAVQYKTQVVQNNWYL SNYGTNYYIKVRAGDNKYMHLKVFKSLVEERWGLWVEDLVLTGYQVDKNKD DELTGF | 285 |
| 267 | MIPGGLSEAKPATPEIQEIVDKVKPQLEEKTGETYGKLEAVQYKTQVVWEWALL LEYGTNYYIKVRAGDNKYMHLKVFKSLHHPWQPAKQEDLVLTGYQVDKNKD DELTGF | 286 |
| 268 | MIPGGLSEAKPATPEIQEIVDKVKPQLEEKTGETYGKLEAVQYKTQVVGDSYWE YVSGTNYYIKVRAGDNKYMHLKVFKSLPYRWRPDHAEDLVLTGYQVDKNKDD ELTGF | 287 |
| 269 | MIPGGLSEAKPATPEIQEIVDKVKPQLEEKTGETYGKLEAVQYKTQVVGQKWW QHKIGTNYYIKVRAGDNKYMHLKVFKSLHSNAWNLQHEDLVLTGYQVDKNKD DELTGF | 288 |
| 270 | MIPGGLSEAKPATPEIQEIVDKVKPQLEEKTGETYGKLEAVQYKTQVVLDGLYH VNSGTNYYIKVRAGDNKYMHLKVFKSLFYYYRSEPTEDLVLTGYQVDKNKDD ELTGF | 289 |
| 271 | MIPGGLSEAKPATPEIQEIVDKVKPQLEEKTGETYGKLEAVQYKTQVVHAPAPV QAIGTNYYIKVRAGDNKYMHLKVFKSLPYWYRPNRPEDLVLTGYQVDKNKDD ELTGF | 290 |
| 272 | MIPGGLSEAKPATPEIQEIVDKVKPQLEEKTGETYGKLEAVQYKTQVVWEDHW NYQYGTNYYIKVRAGDNKYMHLKVFKSLELYSNQIVWEDLVLTGYQVDKNKD DELTGF | 291 |
| 273 | MIPGGLSEAKPATPEIQEIVDKVKPQLEEKTGETYGKLEAVQYKTQVVSWRYKS NQLGTNYYIKVRAGDNKYMHLKVFKSLYKDWVVNPPEDLVLTGYQVDKNKD DELTGF | 292 |
| 274 | MIPGGLSEAKPATPEIQEIVDKVKPQLEEKTGETYGKLEAVQYKTQVVRVDWW LPEVGTNYYIKVRAGDNKYMHLKVFKSLPYQYRKDPKEDLVLTGYQVDKNKD DELTGF | 293 |
| 275 | MIPGGLSEAKPATPEIQEIVDKVKPQLEEKTGETYGKLEAVQYKTQVVSWIEISN QLGTNYYIKVRAGDNKYMHLKVFKSLYYGNTSILIEDLVLTGYQVDKNKDDEL TGF | 294 |
| 276 | MIPGGLSEAKPATPEIQEIVDKVKPQLEEKTGETYGKLEAVQYKTQVVSLGQAE TWAGTNYYIKVRAGDNKYMHLKVFKSLFKYRHQQDKEDLVLTGYQVDKNKD DELTGF | 295 |
| 278 | MIPGGLSEAKPATPEIQEIVDKVKPQLEEKTGETYGKLEAVQYKTQVVKIGQLPS RVGTNYYIKVRAGDNKYMHLKVFKSLYWYRNPADREDLVLTGYQVDKNKDD ELTGF | 296 |
| 279 | MIPGGLSEAKPATPEIQEIVDKVKPQLEEKTGETYGKLEAVQYKTQVVAWHTKN NSIGTNYYIKVRAGDNKYMHLKVFKSLETKLAHEDIEDLVLTGYQVDKNKDDE LTGF | 297 |
| 280 | MIPGGLSEAKPATPEIQEIVDKVKPQLEEKTGETYGKLEAVQYKTQVVQTKLVT WWWGTNYYIKVRAGDNKYMHLKVFKSLLGEQYIDVDEDLVLTGYQVDKNKD DELTGF | 298 |
| 281 | MIPGGLSEAKPATPEIQEIVDKVKPQLEEKTGETYGKLEAVQYKTQVVAVFDYA DPSGTNYYIKVRAGDNKYMHLKVFKSLPYRYRTHHKEDLVLTGYQVDKNKDD ELTGF | 299 |
| 282 | MIPGGLSEAKPATPEIQEIVDKVKPQLEEKTGETYGKLEAVQYKTQVVHVKIVT WWKGTNYYIKVRAGDNKYMHLKVFKSLYNPKPGIDNEDLVLTGYQVDKNKD DELTGF | 300 |
| 283 | MIPGGLSEAKPATPEIQEIVDKVKPQLEEKTGETYGKLEAVQYKTQVVHVLKVS SVSGTNYYIKVRAGDNKYMHLKVFKSLFYYRKRYDREDLVLTGYQVDKNKDD ELTGF | 301 |
| 284 | MIPGGLSEAKPATPEIQEIVDKVKPQLEEKTGETYGKLEAVQYKTQVVYQDYDS ATHGTNYYIKVRAGDNKYMHLKVFKSLYQYRNPIDKEDLVLTGYQVDKNKDD ELTGF | 302 |
| 286 | MIPGGLSEAKPATPEIQEIVDKVKPQLEEKTGETYGKLEAVQYKTQVVSWGKVS NELGTNYYIKVRAGDNKYMHLKVFKSLIEYQSPALYEDLVLTGYQVDKNKDDE LTGF | 303 |
| 287 | MIPGGLSEAKPATPEIQEIVDKVKPQLEEKTGETYGKLEAVQYKTQVVLEDEWW SILGTNYYIKVRAGDNKYMHLKVFKSLPDAPAYSKSEDLVLTGYQVDKNKDDE LTGF | 304 |
| 288 | MIPGGLSEAKPATPEIQEIVDKVKPQLEEKTGETYGKLEAVQYKTQVVYYKLVT WWRGTNYYIKVRAGDNKYMHLKVFKSLPDLNYEVTLEDLVLTGYQVDKNKD DELTGF | 305 |
| 289 | MIPGGLSEAKPATPEIQEIVDKVKPQLEEKTGETYGKLEAVQYKTQVVAWVKEH NLIGTNYYIKVRAGDNKYMHLKVFKSLYDQNQWSPHEDLVLTGYQVDKNKDD ELTGF | 306 |
| 290 | MIPGGLSEAKPATPEIQEIVDKVKPQLEEKTGETYGKLEAVQYKTQVVQEYLWK QYIGTNYYIKVRAGDNKYMHLKVFKSLRWQYRHPQTEDLVLTGYQVDKNKDD ELTGF | 307 |
| 291 | MIPGGLSEAKPATPEIQEIVDKVKPQLEEKTGETYGKLEAVQYKTQVVFNKDLFL GWGTNYYIKVRAGDNKYMHLKVFKSLRFSAGEEWEEDLVLTGYQVDKNKDDE LTGF | 308 |
| 292 | MIPGGLSEAKPATPEIQEIVDKVKPQLEEKTGETYGKLEAVQYKTQVVHFGIRYA HVGTNYYIKVRAGDNKYMHLKVFKSLDYPRHHQDQEDLVLTGYQVDKNKDD ELTGF | 309 |
| 293 | MIPGGLSEAKPATPEIQEIVDKVKPQLEEKTGETYGKLEAVQYKTQVVWYAWQ EGWQGTNYYIKVRAGDNKYMHLKVFKSLFRKKWAQLWEDLVLTGYQVDKNK DDELTGF | 310 |
| 294 | MIPGGLSEAKPATPEIQEIVDKVKPQLEEKTGETYGKLEAVQYKTQVVGNAAW GSFAGTNYYIKVRAGDNKYMHLKVFKSLPFFYRKDNPEDLVLTGYQVDKNKD DELTGF | 311 |
| 295 | MIPGGLSEAKPATPEIQEIVDKVKPQLEEKTGETYGKLEAVQYKTQVVREDLDLF YTGTNYYIKVRAGDNKYMHLKVFKSLEFILQPWWYEDLVLTGYQVDKNKDDE LTGF | 312 |
| 296 | MIPGGLSEAKPATPEIQEIVDKVKPQLEEKTGETYGKLEAVQYKTQVVNENVYR VTQGTNYYIKVRAGDNKYMHLKVFKSLKYRHRLTYEEDLVLTGYQVDKNKDD ELTGF | 313 |
| 297 | MIPGGLSEAKPATPEIQEIVDKVKPQLEEKTGETYGKLEAVQYKTQVVWHKIVT QWPGTNYYIKVRAGDNKYMHLKVFKSLYAQRGEVYFEDLVLTGYQVDKNKD DELTGF | 314 |
| 298 | MIPGGLSEAKPATPEIQEIVDKVKPQLEEKTGETYGKLEAVQYKTQVVHYRIITH WEGTNYYIKVRAGDNKYMHLKVFKSLVRAFLYYKDEDLVLTGYQVDKNKDD ELTGF | 315 |
| 299 | MIPGGLSEAKPATPEIQEIVDKVKPQLEEKTGETYGKLEAVQYKTQVVLVFDAST SAGTNYYIKVRAGDNKYMHLKVFKSLPWEFRGDHKEDLVLTGYQVDKNKDDE LTGF | 316 |
| 300 | MIPGGLSEAKPATPEIQEIVDKVKPQLEEKTGETYGKLEAVQYKTQVVQYAEFL EHIGTNYYIKVRAGDNKYMHLKVFKSLGWSHYFQYLEDLVLTGYQVDKNKDD ELTGF | 317 |
| 301 | MIPGGLSEAKPATPEIQEIVDKVKPQLEEKTGETYGKLEAVQYKTQVVGNVQED WHIGTNYYIKVRAGDNKYMHLKVFKSLFSEDLTLNGEDLVLTGYQVDKNKDD ELTGF | 318 |
| 302 | MIPGGLSEAKPATPEIQEIVDKVKPQLEEKTGETYGKLEAVQYKTQVVGWSASY PESGTNYYIKVRAGDNKYMHLKVFKSLTQWEFIEWPEDLVLTGYQVDKNKDDE LTGF | 319 |
| 303 | MIPGGLSEAKPATPEIQEIVDKVKPQLEEKTGETYGKLEAVQYKTQVVYQFEHIS YGGTNYYIKVRAGDNKYMHLKVFKSLFTYRLPQHREDLVLTGYQVDKNKDDE LTGF | 320 |
| 304 | MIPGGLSEAKPATPEIQEIVDKVKPQLEEKTGETYGKLEAVQYKTQVVAWITPV NVLGTNYYIKVRAGDNKYMHLKVFKSLDEIFHGAYNEDLVLTGYQVDKNKDD ELTGF | 321 |
| 305 | MIPGGLSEAKPATPEIQEIVDKVKPQLEEKTGETYGKLEAVQYKTQVVESYAAIF WGGTNYYIKVRAGDNKYMHLKVFKSLWGEIQHWRQEDLVLTGYQVDKNKDD ELTGF | 322 |
| 306 | MIPGGLSEAKPATPEIQEIVDKVKPQLEEKTGETYGKLEAVQYKTQVVRVWPWT WDRGTNYYIKVRAGDNKYMHLKVFKSLEQWLPNPYFEDLVLTGYQVDKNKD DELTGF | 323 |
| 307 | MIPGGLSEAKPATPEIQEIVDKVKPQLEEKTGETYGKLEAVQYKTQVVQVKKW WEYFVTNYYIKVRAGDNKYMHLKVFKSLQLLSHQWTEEDLVLTGYQVDKNKD DELTGF | 324 |
| 308 | MIPGGLSEAKPATPEIQEIVDKVKPQLEEKTGETYGKLEAVQYKTQVVIGQISEQ QWGTNYYIKVRAGDNKYMHLKVFKSLHRYWTRDVTEDLVLTGYQVDKNKDD ELTGF | 325 |
| 309 | MIPGGLSEAKPATPEIQEIVDKVKPQLEEKTGETYGKLEAVQYKTQVVAFHKLG WILGTNYYIKVRAGDNKYMHLKVFKSLGDESHKWHKEDLVLTGYQVDKNKD DELTGF | 326 |
| 310 | MIPGGLSEAKPATPEIQEIVDKVKPQLEEKTGETYGKLEAVQYKTQVVFKKWW WKYKGTNYYIKVRAGDNKYMHLKVFKSLRVDEHYIQFEDLVLTGYQVDKNKD DELTGF | 327 |
| 311 | MIPGGLSEAKPATPEIQEIVDKVKPQLEEKTGETYGKLEAVQYKTQVVASQRLW IIYGTNYYIKVRAGDNKYMHLKVFKSLSDWRHHGGWEDLVLTGYQVDKNKDD ELTGF | 328 |
| 312 | MIPGGLSEAKPATPEIQEIVDKVKPQLEEKTGETYGKLEAVQYKTQVVQRETHIL WSGTNYYIKVRAGDNKYMHLKVFKSLWFDNLHNNFEDLVLTGYQVDKNKDD ELTGF | 329 |
| 313 | MIPGGLSEAKPATPEIQEIVDKVKPQLEEKTGETYGKLEAVQYKTQVVGQYWF WAPGGTNYYIKVRAGDNKYMHLKVFKSLPSSGGGPKWEDLVLTGYQVDKNKD DELTGF | 330 |
| 314 | MIPGGLSEAKPATPEIQEIVDKVKPQLEEKTGETYGKLEAVQYKTQVVRDWAYY IYFGTNYYIKVRAGDNKYMHLKVFKSLRSDTLHYWNEDLVLTGYQVDKNKDD ELTGF | 331 |
| 315 | MIPGGLSEAKPATPEIQEIVDKVKPQLEEKTGETYGKLEAVQYKTQVVIGYKLN YIWGTNYYIKVRAGDNKYMHLKVFKSLIQYRWQDRVEDLVLTGYQVDKNKDD ELTGF | 332 |
| 316 | MIPGGLSEAKPATPEIQEIVDKVKPQLEEKTGETYGKLEAVQYKTQVVVSLTEQR WKGTNYYIKVRAGDNKYMHLKVFKSLFQPIKLANAEDLVLTGYQVDKNKDDE LTGF | 333 |
| 317 | MIPGGLSEAKPATPEIQEIVDKVKPQLEEKTGETYGKLEAVQYKTQVVWVSQQH YSHGTNYYIKVRAGDNKYMHLKVFKSLFAFRKYEDREDLVLTGYQVDKNKDD ELTGF | 334 |
| 318 | MIPGGLSEAKPATPEIQEIVDKVKPQLEEKTGETYGKLEAVQYKTQVVDDNQH YGLGTNYYIKVRAGDNKYMHLKVFKSLLHNWWKGNWEDLVLTGYQVDKNK DDELTGF | 335 |
| 319 | MIPGGLSEAKPATPEIQEIVDKVKPQLEEKTGETYGKLEAVQYKTQVVPTRWISI YWGTNYYIKVRAGDNKYMHLKVFKSLLNRYYHASIEDLVLTGYQVDKNKDDE LTGF | 336 |
| 320 | MIPGGLSEAKPATPEIQEIVDKVKPQLEEKTGETYGKLEAVQYKTQVVTAFAPN KASGTNYYIKVRAGDNKYMHLKVFKSLRYWAFRYLWEDLVLTGYQVDKNKD DELTGF | 337 |
| 321 | MIPGGLSEAKPATPEIQEIVDKVKPQLEEKTGETYGKLEAVQYKTQVVHYTDFK WHKGTNYYIKVRAGDNKYMHLKVFKSLPFWANEHGWEDLVLTGYQVDKNKD DELTGF | 338 |
| 322 | MIPGGLSEAKPATPEIQEIVDKVKPQLEEKTGETYGKLEAVQYKTQVVNKFWSW APGGTNYYIKVRAGDNKYMHLKVFKSLADSGFNATPEDLVLTGYQVDKNKDD ELTGF | 339 |
| 323 | MIPGGLSEAKPATPEIQEIVDKVKPQLEEKTGETYGKLEAVQYKTQVVELDEPA WDWGTNYYIKVRAGDNKYMHLKVFKSLRQHKKPHPNEDLVLTGYQVDKNKD DELTGF | 340 |
| 324 | MIPGGLSEAKPATPEIQEIVDKVKPQLEEKTGETYGKLEAVQYKTQVVHEKWGF WELGTNYYIKVRAGDNKYMHLKVFKSLHSHRFHEAHEDLVLTGYQVDKNKDD ELTGF | 341 |
| 325 | MIPGGLSEAKPATPEIQEIVDKVKPQLEEKTGETYGKLEAVQYKTQVVVATADLI KAGTNYYIKVRAGDNKYMHLKVFKSLPWHYPPNKWEDLVLTGYQVDKNKDD ELTGF | 342 |
| 326 | MIPGGLSEAKPATPEIQEIVDKVKPQLEEKTGETYGKLEAVQYKTQVVYYKVIS WWHGTNYYIKVRAGDNKYMHLKVFKSLYKTPFPQYTEDLVLTGYQVDKNKD DELTGF | 343 |
| 327 | MIPGGLSEAKPATPEIQEIVDKVKPQLEEKTGETYGKLEAVQYKTQVVHWHHY DNIIGTNYYIKVRAGDNKYMHLKVFKSLYQLVDLDLDEDLVLTGYQVDKNKDD ELTGF | 344 |
| 328 | MIPGGLSEAKPATPEIQEIVDKVKPQLEEKTGETYGKLEAVQYKTQVVVYKIITF WSGTNYYIKVRAGDNKYMHLKVFKSLEYPPVQQVLEDLVLTGYQVDKNKDDE LTGF | 345 |
| 329 | MIPGGLSEAKPATPEIQEIVDKVKPQLEEKTGETYGKLEAVQYKTQVVGWIQRH NLIGTNYYIKVRAGDNKYMHLKVFKSLYSEPPPVQNEDLVLTGYQVDKNKDDE LTGF | 346 |
| 330 | MIPGGLSEAKPATPEIQEIVDKVKPQLEEKTGETYGKLEAVQYKTQVVKTWEDT VVAGTNYYIKVRAGDNKYMHLKVFKSLEYLYRPLNPEDLVLTGYQVDKNKDD ELTGF | 347 |
| 331 | MIPGGLSEAKPATPEIQEIVDKVKPQLEEKTGETYGKLEAVQYKTQVVLDQHHV YQVGTNYYIKVRAGDNKYMHLKVFKSLALLIHHDPWEDLVLTGYQVDKNKDD ELTGF | 348 |
| 332 | MIPGGLSEAKPATPEIQEIVDKVKPQLEEKTGETYGKLEAVQYKTQVVLEDVWG VWAGTNYYIKVRAGDNKYMHLKVFKSLEFQHRTFNHEDLVLTGYQVDKNKDD ELTGF | 349 |
| 333 | MIPGGLSEAKPATPEIQEIVDKVKPQLEEKTGETYGKLEAVQYKTQVVWLPWTV ELIGTNYYIKVRAGDNKYMHLKVFKSLGEIWSAKHKEDLVLTGYQVDKNKDDE LTGF | 350 |
| 334 | MIPGGLSEAKPATPEIQEIVDKVKPQLEEKTGETYGKLEAVKYKTQVVQHFTWD WFWGTNYYIKVRAGDNKYMHLKVFKSLPEYKPVDHTEDLVLTGYQVDKNKD DELTGF | 351 |
| 335 | MIPGGLSEAKPATPEIQEIVDKVKPQLEEKTGETYGKLEAVQYKTQVVLFEVLQ GSSGTNYYIKVRAGDNKYMHLKVFKSLGEIVWYQVEEDLVLTGYQVDKNKDD ELTGF | 352 |
| 336 | MIPGGLSEAKPATPEIQEIVDKVKPQLEEKTGETYGKLEAVQYKTQVVPYSWQH EVVGTNYYIKVRAGDNKYMHLKVFKSLIVFYLQEYYEEDLVLTGYQVDKNKD DELTGF | 353 |
| 337 | MIPGGLSEAKPATPEIQEIVDKVKPQLEEKTGETYGKLEAVQYKTQVVWWQEN AEWIGTNYYIKVRAGDNKYMHLKVFKSLGYAVFHKYLEDLVLTGYQVDKNKD DELTGF | 354 |
| 338 | MIPGGLSEAKPATPEIQEIVDKVKPQLEEKTGETYGKLEAVQYKTQVVSYDPVY QDTGTNYYIKVRAGDNKYMHLKVFKSLFFHRTSVVYEDLVLTGYQVDKNKDD ELTGF | 355 |
| 339 | MIPGGLSEAKPATPEIQEIVDKVKPQLEEKTGETYGKLEAVQYKTQVVRVFDTPL AAGTNYYIKVRAGDNKYMHLKVFKSLPWQYRNNPEEDLVLTGYQVDKNKDDE LTGF | 356 |
| 340 | MIPGGLSEAKPATPEIQEIVDKVKPQLEEKTGETYGKLEAVQYKTQVVWWDIPW IWVGTNYYIKVRAGDNKYMHLKVFKSLQNDELYPYKEDLVLTGYQVDKNKDD ELTGF | 357 |
| 341 | MIPGGLSEAKPATPEIQEIVDKVKPQLEEKTGETYGKLEAVQYKTQVVYQKVIT WWQGTNYYIKVRAGDNKYMHLKVFKSLYWENHRGHVEDLVLTGYQVDKNK DDELTGF | 358 |
| 342 | MIPGGLSEAKPATPEIQEIVDKVKPQLEEKTGETYGKLEAVQYKTQVVQWIKWG NILGTNYYIKVRAGDNKYMHLKVFKSLYEAIERWHGEDLVLTGYQVDKNKDD ELTGF | 359 |
| 343 | MIPGGLSEAKPATPEIQEIVDKVKPQLEEKTGETYGKLEAVQYKTQVVATPEKK YVVGTNYYIKVRAGDNKYMHLKVFKSLWNANILRYWEDLVLTGYQVDKNKD DELTGF | 360 |
| 344 | MIPGGLSEAKPATPEIQEIVDKVKPQLEEKTGETYGKLEAVQYKTQVVTSERLWI LYGTNYYIKVRAGDNKYMHLKVFKSLTPRYVPLNPEDLVLTGYQVDKNKDDEL TGF | 361 |
| 345 | MIPGGLSEAKPATPEIQEIVDKVKPQLEEKTGETYGKLEAVQYKTQVVEHHSQPLA AHGTNYYIKVRAGDNKYMHLKVFKSLPYHAFDWYWEDLVLTGYQVDKNKDD ELTGF | 362 |
| 346 | MIPGGLSEAKPATPEIQEIVDKVKPQLEEKTGETYGKLEAVQYKTQVVWTADW LWTVGTNYYIKVRAGDNKYMHLKVFKSLYDPSEWFPREDLVLTGYQVDKNKD DELTGF | 363 |
| 347 | MIPGGLSEAKPATPEIQEIVDKVKPQLEEKTGETYGKLEAVQYKTQVVSFQREH YSWGTNYYIKVRAGDNKYMHLKVFKSLFDIFYFNPIEDLVLTGYQVDKNKDDE LTGF | 364 |
| 348 | MIPGGLSEAKPATPEIQEIVDKVKPQLEEKTGETYGKLEAVQYKTQVVGKTWHK HNRGTNYYIKVRAGDNKYMHLKVFKSLNLYFFFYQTEDLVLTGYQVDKNKDD ELTGF | 365 |

### Nucleic acids encoding the stefin A protein variant tspecifically binding to CD40L

As used herein, "Nuceleic acid" is a polynucleotide of any length and may comprise DNA, RNA or a combination of DNA and RNA. The nucleotides can be deoxyribonucleotides, ribonucleotides, modified nucleotides or bases, and/or their analogs, or any substrate that can be incorporated into a polymer by DNA or RNA polymerase. In the present invention, the nucleic acids is, for example but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, a-LNA having an a-L-ribo configuration (a diastereomer of LNA), 2'-amino-LNA having a 2 '-amino functionalization, and 2'-amino- a-LNA having a 2'-amino functionalization), ethylene nucleic acids (ENA), cyclohexenyl nucleic acids (CeNA) or hybrids or combinations thereof.

As used herein, "nucleic acid encoding ~" is nucleic acid sequence encoding a specific protein or polypeptide. As used herein, "nucleic acid encoding ~" is nucleic acid sequence encoding a specific protein or polypeptide. In the art, when the sequence of a specific protein or polypeptide has been known, methods for designing or deriving a nucleic acid encoding the same are well known.

Therefore, the nucleic acid encoding the stefin A protein variant specifically binding to CD40L of the present invention can be easily understood from the description of the "stefin A protein variant specifically binding to CD40L".

In some embodiments, the stefin A protein variant specifically binding to CD40L is encoded by a nucleic acid comprising a sequence at least 70%, 75% 80%, 85%, 90%, 95% or even 98% identical with a sequence selected from SEQ ID NOs: 366 to 485. In some embodiments, the stefin A protein variant specifically binding to CD40L is encoded by a nucleic acid that comprising a coding sequence that hybridizes to a sequence selected from SEQ ID NOs: 366 to 485 under stringent conditions such as in the presence of 6X sodium chloride/sodium citrate (SSC) at 45°C followed by a wash in 0.2X SSC at 65°C.

**Table 4. Exemplary Encoded the stefin A protein variant specifically binding to CD40L**

| **Clone #** | **Nucleic Acid Sequence** | **SEQ ID NO:** |
|---|---|---|
| 227 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATGATGTGTT TTACGATCAGAAAGCAGGTACGAACTACTACATCAAGGTTCGTGCGGGTGAC AACAAGTATATGCACCTGAAAGTGTTTAAGAGCCTGAAATGGTGGTACAGA ATCGGTAACCCAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 366 |
| 228 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCCATTCAAAAT CATCACCGTTTGGCATGGTACGAACTACTACATCAAGGTTCGTGCGGGTGAC AACAAGTATATGCACCTGAAAGTGTTTAAGAGCCTGCTGGAAAGATTCAACT TCCATGGTGAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 367 |
| 229 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGTACAAAA TCATCACCTGGTGGTCTGGTACGAACTACTACATCAAGGTTCGTGCGGGTGA CAACAAGTATATGCACCTGAAAGTGTTTAAGAGCCTGGTTTACAAAAACAAA AGAGGTGGTCATGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 368 |
| 230 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCACTACTACGT TCATTACAACGATCAGGGTACGAACTACTACATCAAGGTTCGTGCGGGTGAC AACAAGTATATGCACCTGAAAGTGTTTAAGAGCCTGTGGGGTGAAAACCTGT TCGCAAAATGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 369 |
| 231 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACTACCAGAT CATCACCTGGTGGCAGGGTACGAACTACTACATCAAGGTTCGTGCGGGTGAC AACAAGTATATGCACCTGAAAGTGTTTAAGAGCCTGGGTATCAGAAAAAAA CAGCAGCAGAGAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 370 |
| 232 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTCATGGTCC ACATCTGGAACAGGCAGGTACGAACTACTACATCAAGGTTCGTGCGGGTGAC AACAAGTATATGCACCTGAAAGTGTTTAAGAGCCTGGGTCATGCATTCTACT TCGCATTCGTTGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 371 |
| 233 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGCAGGATCA GTGGCCATGGCGTGATGGTACGAACTACTACATCAAGGTTCGTGCGGGTGAC AACAAGTATATGCACCTGAAAGTGTTTAAGAGCCTGACCTACGGTAAAAAAT GGTACTCTTGGGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 372 |
| 234 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGACGCGGGTACGA ACTACTACATCAAGGTTCGTGCGGGTGACAACAAGTATATGCACCTGAAAGT | 373 |
| | GTTTAAGAGCCTGTACTTCTACAGACATTGGGAAGATAGAGAAGATTTGGTG CTGACGGGCTACCAGGTTGACAAGAACAAAGATGACGAGCTGACGGGTTTC GCGGCCGCTGAACAGAAGCTGATCTCCGAAGAAGATCTGGCCGCGCATCAC CATCACCACCACTAATGA | |
| 235 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTGGGTTAA ACCAGATAACGAACTGGGTACGAACTACTACATCAAGGTTCGTGCGGGTGAC AACAAGTATATGCACCTGAAAGTGTTTAAGAGCCTGTGGCAGGTTCATGATA AACAGTGGCTGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 374 |
| 236 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAACTCTCTGGG TGTTTCTGAATGGGTTGGTACGAACTACTACATCAAGGTTCGTGCGGGTGAC AACAAGTATATGCACCTGAAAGTGTTTAAGAGCCTGCCATACTGGTACAGAA AAAACAACCTGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 375 |
| 237 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTTACCGTAT CGTTTCTTGGTGGCGTGGTACGAACTACTACATCAAGGTTCGTGCGGGTGAC AACAAGTATATGCACCTGAAAGTGTTTAAGAGCCTGGCATCTGATATCTACC TGAGAATCGATGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 376 |
| 238 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACTACCGTCA GCTGAAATTCAACACCGGTACGAACTACTACATCAAGGTTCGTGCGGGTGAC AACAAGTATATGCACCTGAAAGTGTTTAAGAGCCTGTTCCTGCATAGAAAAC CACAGGATAGAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 377 |
| 239 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTTGGATCAA AGTTGATAACGTTCTGGGTACGAACTACTACATCAAGGTTCGTGCGGGTGAC AACAAGTATATGCACCTGAAAGTGTTTAAGAGCCTGTGGCAGGATCATCATA ACGTTAAAAGAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 378 |
| 240 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCACCGCATACTA CATCCATGTTAACGCAGGTACGAACTACTACATCAAGGTTCGTGCGGGTGAC AACAAGTATATGCACCTGAAAGTGTTTAAGAGCCTGCCATACGGTTACAGAG CAAAAGCACCAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 379 |
| 241 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTCGTGTTGA ACCAACCCCAAACGATGGTACGAACTACTACATCAAGGTTCGTGCGGGTGAC AACAAGTATATGCACCTAAAAGTGTTTAAGAGCCTGCCACTGGTTCAGGAAC TGTGGGGTTTCGAAGATTTGGTGCTGACGGGCTACCAAGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 380 |
| 242 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGATCTGAACTC TTCTGTTTGGTACCAGGGTACGAACTACTACATCAAGGTTCGTGCGGGTGAC AACAAGTATATGCACCTGAAAGTGTTTAAGAGCCTGTACTGGCTGCTGGAAA TCGAACATAGAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 381 |
| 243 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCACCTACCA GATCTCTTACACCGTTGGTACGAACTACTACATCAAGGTTCGTGCGGGTGAC AACAAGTATATGCACCTGAAAGTGTTTAAGAGCCTGAGATGGAGATACCCA GCAAGAAAACCAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 382 |
| 244 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGAAAACT GGTACGGTGAATTCGCAGGTACGAACTACTACATCAAGGTTCGTGCGGGTGA CAACAAGTATATGCACCTGAAAGTGTTTAAGAGCCTGCTGTACATCTACAGA CATCCAAACTACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 383 |
| 245 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCACCGCACTGGC ATGGAACACCCAGGCAGGTACGAACTACTACATCAAGGTTCGTGCGGGTGA CAACAAGTATATGCACCTGAAAGTGTTTAAGAGCCTGCCATGGCTGTACAGA CAGAAATCTGATGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 384 |
| 246 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATATCCCAGC ACTGAAAGTTCATGCAGGTACGAACTACTACATCAAGGTTCGTGCGGGTGAC AACAAGTATATGCACCTGAAAGTGTTTAAGAGCCTGCCATACTGGTACAGAC ATGATCAGGAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 385 |
| 247 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATCATGCATA CGCAGCAGTTTACGTTGGTACGAACTACTACATCAAGGTTCGTGCGGGTGAC AACAAGTATATGCACCTGAAAGTGTTTAAGAGCCTGCCATACTGGTACAGAA AATACCCAGATGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 386 |
| 248 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACGATGAACT GTACCATGAAAAAGCAGGTACGAACTACTACATCAAGGTTCGTGCGGGTGA CAACAAGTATATGCACCTGAAAGTGTTTAAGAGCCTGGAATACAAATTCAGA AACCTGGAAGAAGATTTGGTGCTGACGGGCTACCAGGTTGACAAGAACAAA GATGACGAGCTGACGGGTTTCGCGGCCGCTGAACAGAAGCTGATCTCCGAA GAAGATCTGGCCGCGCATCACCATCACCACCACTAATGA | 387 |
| 249 | ATGATTCCCGGCGGACTTTCGGAAGCCAAGCCAGCCACTCCGGAGATTCAGG ΛΛΛTCGTGGΛCΛΛGGTCΛΛΛCCGCΛGCTGGΛΛGΛGΛΛΛCCGGCGΛΛΛCCT ACGGCAAACTGGAAGCCGTCCAGTATAAGACTCAAGTCGTCTCTTGGTACTA CCCAAAAACGTTCTGGGTACGAACTACTACATCAAGGTTCGTGCGGGTGAC | 388 |
| | AACAAGTATATGCACCTGAAAGTGTTTAAGAGCCTGGAAGATTTCATCGCAG CAGATGATGATGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | |
| 250 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCCTGGAATG GCACTTCTGGGAATGGGGTACGAACTACTACATCAAGGTTCGTGCGGGTGAC AACAAGTATATGCACCTGAAAGTGTTTAAGAGCCTGGATTCTCAGGAATTCC CATCTCATCTGGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 389 |
| 251 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTGGGTTCG TGGTACCAACCAGCTGGGTACGAACTACTACATCAAGGTTCGTGCGGGTGAC AACAAGTATATGCACCTGAAAGTGTTTAAGAGCCTGGAACCAGATTGGCCAG ATTTCCTGATCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 390 |
| 252 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGCGTAAACT GCTGTCTTGGTGGAAAGGTACGAACTACTACATCAAGGTTCGTGCGGGTGAC AACAAGTATATGCACCTGAAAGTGTTTAAGAGCCTGATCCTGAACCCAAAAT CTTGGAACTTCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 391 |
| 253 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCCAAAATCTCA TCAGACCAACGAACAGGGTACGAACTACTACATCAAGGTTCGTGCGGGTGA CAACAAGTATATGCACCTGAAAGTGTTTAAGAGCCTGTGGAACGAAAACCAT TTCTCTGTTTGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 392 |
| 254 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAACTGGAACC AGATCGATAACCCACTGGGTACGAACTACTACATCAAGGTTCGTGCGGGTGA CAACAAGTATATGCACCTGAAAGTGTTTAAGAGCCTGAACAAAGATTGGGA AGATCAGATCATCGAAGATTTGGTGCTGACGGGCTACCAGGTTGACAAGAAC AAAGATGACGAGCTGACGGGTTTCGCGGCCGCGGGTGGAAGAGCAGAACAG AAGCTGATCTCCGAAGAAGATCTGGCCGCGCATCACCATCACCACCACTAAT GA | 393 |
| 255 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCTGGGTTGG TCTGCATAACCAGCTGGGTACGAACTACTACATCAAGGTTCGTGCGGGTGAC AACAAGTATATGCACCTGAAAGTGTTTAAGAGCCTGTACTACAACGGTGATA ACACCCAGATCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 394 |
| 256 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCACCCAGCC ATTCAACGAACCAATCGGTACGAACTACTACATCAAGGTTCGTGCGGGTGAC AACAAGTATATGCACCTGAAAGTGTTTAAGAGCCTGTATTGGGATGATAAAG TTGGTTACTACGAAGATTTGGTGCTGACGGGCTACCAGGTTGACAAGAACAA | 395 |
| | AGATGACGAGCTGACGGGTTTCGCGGCCGCTGAACAGAAGCTGATCTCCGA AGAAGATCTGGCCGCGCATCACCATCACCACCACTAATGA | |
| 257 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCCAGAATACC AGCCACAGCCAAAAGCAGGTACGAACTACTACATCAAGGTTCGTGCGGGTG ACAACAAGTATATGCACCTGAAAGTGTTTAAGAGCCTGTTCCATTTCAGAAA ACCACAGGATAGAGAAGATTTGGTGCTGACGGGCTACCAGGTTGACAAGAA CAAAGATGACGAGCTGACGGGTTTCGCGGCCGCTGAACAGAAGCTGATCTCC GAAGAAGATCTGGCCGCGCATCACCATCACCACCACTAATGA | 396 |
| 258 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAACGATCCATA CTTCCAGTCTTTCTCTGGTACGAACTACTACATCAAGGTTCGTGCGGGTGACA ACAAGTATATGCACCTGAAAGTGTTTAAGAGCCTGCCATACTGGTACAGAGA ACCAGAAAACGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 397 |
| 259 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATCAGGATG ATGTTGCATGGCAGTCTGGTACGAACTACTACATCAAGGTTCGTGCGGGTGA CAACAAGTATATGCACCTGAAAGTGTTTAAGAGCCTGGAATACGGTTGGTAC CATCTGCCATACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 398 |
| 260 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAAAGGTTACTT CCATACCGTTGAAGCAGGTACGAACTACTACATCAAGGTTCGTGCGGGTGAC AACAAGTATATGCACCTGAAAGTGTTTAAGAGCCTGACCTACCATTTCAGAA CCGATCTGAACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 399 |
| 261 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTGATCAGGT TTGGTCTGAAAACGCAGGTACGAACTACTACATCAAGGTTCGTGCGGGTGAC AACAAGTATATGCACCTGAAAGTGTTTAAGAGCCTGCCATACCTGTGGAGAA AACATGCAGTTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 400 |
| 262 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGTCTTCTAT CTGGCAGGATGTTGCAGGTACGAACTACTACATCAAGGTTCGTGCGGGTGAC AACAAGTATATGCACCTGAAAGTGTTTAAGAGCCTGCTGTACCAGTTCAGAA AAGAACCAGTTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 401 |
| 263 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCACCGCATCTCT GTACTGGAACTACGCAGGTACGAACTACTACATCAAGGTTCGTGCGGGTGAC AACAAGTATATGCACCTGAAAGTGTTTAAGAGCCTGTTCTACTACAGAGGTC CACAGCCAGTTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 402 |
| 264 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 403 |
| | ACGGCAAACTGGAAGCCGTCCAGTATAAGACTCAAGTCGTCTCTTGGAAACA GTGGCATAACTACCTGGGTACGAACTACTACATCAAGGTTCGTGCGGGTGAC AACAAGTATATGCACCTGAAAGTGTTTAAGAGCCTGTACCCAGATGGTGATC TGATCCTGTACGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | |
| 265 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGGTTCATCT GGATAACTCTCCACTGGGTACGAACTACTACATCAAGGTTCGTGCGGGTGAC AACAAGTATATGCACCTGAAAGTGTTTAAGAGCCTGCAGTTCGGTGAAATCA TCTGGTGGGAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 404 |
| 266 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGAACAACT GGTACCTGTCTAACTACGGTACGAACTACTACATCAAGGTTCGTGCGGGTGA CAACAAGTATATGCACCTGAAAGTGTTTAAGAGCCTGGTTGAAGAAAGATG GGGTCTGTGGGTTGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 405 |
| 267 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGGAATGGG CACTGCTGCTGGAATACGGTACGAACTACTACATCAAGGTTCGTGCGGGTGA CAACAAGTATATGCACCTGAAAGTGTTTAAGAGCCTGCATCATCCATGGCAG CCAGCAAAACAGGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 406 |
| 268 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTGATTCTTA CTGGGAATACGTTTCTGGTACGAACTACTACATCAAGGTTCGTGCGGGTGAC AACAAGTATATGCACCTGAAAGTGTTTAAGAGCCTGCCATACAGATGGAGAC CAGATCATGCAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 407 |
| 269 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTCAGAAAT GGTGGCAGCATAAAATCGGTACGAACTACTACATCAAGGTTCGTGCGGGTGA CAACAAGTATATGCACCTGAAAGTGTTTAAGAGCCTGCATTCTAACGCATGG AACCTGCAGCATGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 408 |
| 270 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGGATGGTCT GTACCATGTTAACTCTGGTACGAACTACTACATCAAGGTTCGTGCGGGTGAC AACAAGTATATGCACCTGAAAGTGTTTAAGAGCCTGTTCTACTACTACAGAT CTGAACCAACCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 409 |
| 271 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATGCACCAGC ACCAGTTCAGGCAATCGGTACGAACTACTACATCAAGGTTCGTGCGGGTGAC AACAAGTATATGCACCTGAAAGTGTTTAAGAGCCTGCCATACTGGTACAGAC CAAACAGACCAGAAGATTTGGTGCTGACGGGCTACCAGGTTGACAAGAACA | 410 |
| | AAGATGACGAGCTGACGGGTTTCGCGGCCGCTGAACAGAAGCTGATCTCCG AAGAAGATCTGGCCGCGCATCACCATCACCACCACTAATGA | |
| 272 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGGAAGATC ATTGGAACTACCAGTACGGTACGAACTACTACATCAAGGTTCGTGCGGGTGA CAACAAGTATATGCACCTGAAAGTGTTTAAGAGCCTGGAACTGTACTCTAAC CAGATCGTTTGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 411 |
| 273 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTGGCGTTA CAAATCTAACCAGCTGGGTACGAACTACTACATCAAGGTTCGTGCGGGTGAC AACAAGTATATGCACCTGAAAGTGTTTAAGAGCCTGTACAAAGATTGGGTTG TTAACCCACCAGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 412 |
| 274 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGTTGATTG GTGGCTGCCAGAAGTTGGTACGAACTACTACATCAAGGTTCGTGCGGGTGAC AACAAGTATATGCACCTGAAAGTGTTTAAGAGCCTGCCATACCAGTACAGAA AAGATCCAAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 413 |
| 275 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTGGATCGA AATCTCTAACCAGCTGGGTACGAACTACTACATCAAGGTTCGTGCGGGTGAC AACAAGTATATGCACCTGAAAGTGTTTAAGAGCCTGTACTACGGTAACACCT CTATCCTGATCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 414 |
| 276 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTCTGGGTCA GGCAGAAACCTGGGCAGGTACGAACTACTACATCAAGGTTCGTGCGGGTGA CAACAAGTATATGCACCTGAAAGTGTTTAAGAGCCTGTTCAAATACAGACAT CAGCAGGATAAAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 415 |
| 278 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAAAATCGGTC AGCTGCCATCTCGTGTTGGTACGAACTACTACATCAAGGTTCGTGCGGGTGA CAACAAGTATATGCACCTGAAAGTGTTTAAGAGCCTGTACTGGTACAGAAAC CCAGCAGATAGAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 416 |
| 279 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCATGGCATAC CAAAAACAACTCTATCGGTACGAACTACTACATCAAGGTTCGTGCGGGTGAC AACAAGTATATGCACCTGAAAGTGTTTAAGAGCCTGGAAACCAAACTGGCA CATGAAGATATCGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 417 |
| 280 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 418 |
| | ACGGCAAACTGGAAGCCGTCCAGTATAAGACTCAAGTCGTCCAGACCAAAC TGGTTACCTGGTGGTGGGGTACGAACTACTACATCAAGGTTCGTGCGGGTGA CAACAAGTATATGCACCTGAAAGTGTTTAAGAGCCTGCTGGGTGAACAGTAC ATCGATGTTGATGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | |
| 281 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCAGTTTTCGA TTACGCAGATCCATCTGGTACGAACTACTACATCAAGGTTCGTGCGGGTGAC AACAAGTATATGCACCTGAAAGTGTTTAAGAGCCTGCCATACAGATACAGAA CCCATCATAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 419 |
| 282 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATGTTAAAAT CGTTACCTGGTGGAAAGGTACGAACTACTACATCAAGGTTCGTGCGGGTGAC AACAAGTATATGCACCTGAAAGTGTTTAAGAGCCTGTACAACCCAAAACCAG GTATCGATAACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 420 |
| 283 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATGTTCTGAA AGTTTCTTCTGTTTCTGGTACGAACTACTACATCAAGGTTCGTGCGGGTGACA ACAAGTATATGCACCTGAAAGTGTTTAAGAGCCTGTTCTACTACAGAAAAAG ATACGATAGAGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 421 |
| 284 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACCAGGATTA CGATTCTGCAACCCATGGTACGAACTACTACATCAAGGTTCGTGCGGGTGAC AACAAGTATATGCACCTGAAAGTGTTTAAGAGCCTGTACCAGTACAGAAACC CAATCGATAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 422 |
| 286 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTGGGGTAA AGTTTCTAACGAACTGGGTACGAACTACTACATCAAGGTTCGTGCGGGTGAC AACAAGTATATGCACCTGAAAGTGTTTAAGAGCCTGATCGAATACCAGTCTC CAGCACTGTACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 423 |
| 287 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGGAAGATG AATGGTGGTCTATCCTGGGTACGAACTACTACATCAAGGTTCGTGCGGGTGA CAACAAGTATATGCACCTGAAAGTGTTTAAGAGCCTGCCAGATGCACCAGCA TACTCTAAATCTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 424 |
| 288 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACTACAAACT GGTTACCTGGTGGCGTGGTACGAACTACTACATCAAGGTTCGTGCGGGTGAC AACAAGTATATGCACCTGAAAGTGTTTAAGAGCCTGCCAGATCTGAACTACG AAGTTACCCTGGAAGATTTGGTGCTGACGGGCTACCAGGTTGACAAGAACAA | 425 |
| | AGATGACGAGCTGACGGGTTTCGCGGCCGCTGAACAGAAGCTGATCTCCGA AGAAGATCTGGCCGCGCATCACCATCACCACCACTAATGA | |
| 289 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCATGGGTTAA AGAACATAACCTGATCGGTACGAACTACTACATCAAGGTTCGTGCGGGTGAC AACAAGTATATGCACCTGAAAGTGTTTAAGAGCCTGTACGATCAGAACCAGT GGTCTCCACATGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 426 |
| 290 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGGAATACCT GTGGAAACAGTACATCGGTACGAACTACTACATCAAGGTTCGTGCGGGTGAC AACAAGTATATGCACCTGAAAGTGTTTAAGAGCCTGAGATGGCAGTACAGA CATCCACAGACCGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 427 |
| 291 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCAACAAAG ATCTGTTCCTGGGTTGGGGTACGAACTACTACATCAAGGTTCGTGCGGGTGA CAACAAGTATATGCACCTGAAAGTGTTTAAGAGCCTGAGATTCTCTGCAGGT GAAGAATGGGAAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 428 |
| 292 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATTTCGGTAT CCGTTACGCACATGTTGGTACGAACTACTACATCAAGGTTCGTGCGGGTGAC AACAAGTATATGCACCTGAAAGTGTTTAAGAGCCTGGATTACCCAAGACATC ATCAGGATCAGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 429 |
| 293 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGTACGCATG GCAGGAAGGTTGGCAGGGTACGAACTACTACATCAAGGTTCGTGCGGGTGA CAACAAGTATATGCACCTGAAAGTGTTTAAGAGCCTGTTCAGAAAAAAATGG GCACAGCTGTGGGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 430 |
| 294 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTAACGCAG CATGGGGTTCTTTCGCAGGTACGAACTACTACATCAAGGTTCGTGCGGGTGA CAACAAGTATATGCACCTGAAAGTGTTTAAGAGCCTGCCATTCTTCTACAGA AAAGATAACCCAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 431 |
| 295 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGAAGATCT GGATCTGTTCTACACCGGTACGAACTACTACATCAAGGTTCGTGCGGGTGAC AACAAGTATATGCACCTGAAAGTGTTTAAGAGCCTGGAATTCATCCTGCAGC CATGGTGGTACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 432 |
| 296 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 433 |
| | ACGGCAAACTGGAAGCCGTCCAGTATAAGACTCAAGTCGTCAACGAAAACG TTTACCGTGTTACCCAGGGTACGAACTACTACATCAAGGTTCGTGCGGGTGA CAACAAGTATATGCACCTGAAAGTGTTTAAGAGCCTGAAATACAGACATAG ACTGACCTACGAAGAAGATTTGGTGCTGACGGGCTACCAAGTTGACAAGAA CAAAGATGACGAGCTGACGGGTTTCGCGGCCGCTGAACAGAAGCTGATCTCC GAAGAAGATCTGGCCGCGCATCACCATCACCACCACTAATGA | |
| 297 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGCATAAAAT CGTTACCCAGTGGCCAGGTACGAACTACTACATCAAGGTTCGTGCGGGTGAC AACAAGTATATGCACCTGAAAGTGTTTAAGAGCCTGTACGCACAGAGAGGT GAAGTTTACTTCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 434 |
| 298 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATTACCGTAT CATCACCCATTGGGAAGGTACGAACTACTACATCAAGGTTCGTGCGGGTGAC AACAAGTATATGCACCTGAAAGTGTTTAAGAGCCTGGTTAGAGCATTCCTGT ACTACAAAGATGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 435 |
| 299 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGGTTTTCGA TGCATCTACCTCTGCAGGTACGAACTACTACATCAAGGTTCGTGCGGGTGAC AACAAGTATATGCACCTGAAAGTGTTTAAGAGCCTGCCATGGGAATTCAGAG GTGATCATAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 436 |
| 300 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGTACGCAG AATTCCTGGAACATATCGGTACGAACTACTACATCAAGGTTCGTGCGGGTGA CAACAAGTATATGCACCTGAAAGTGTTTAAGAGCCTGGGTTGGTCTCATTAC TTCCAGTACCTGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 437 |
| 301 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTAACGTTCA GGAAGATTGGCATATCGGTACGAACTACTACATCAAGGTTCGTGCGGGTGAC AACAAGTATATGCACCTGAAAGTGTTTAAGAGCCTGTTCTCTGAAGATCTGA CCCTGAACGGTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 438 |
| 302 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTTGGTCTGC ATCTTACCCAGAATCTGGTACGAACTACTACATCAAGGTTCGTGCGGGTGAC AACAAGTATATGCACCTGAAAGTGTTTAAGAGCCTGACCCAGTGGGAATTCA TCGAATGGCCAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 439 |
| 303 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTACCAGTTCGA ACATATCTCCTACGGTGGTACGAACTACTACATCAAGGTTCGTGCGGGTGAC AACAAGTATATGCACCTGAAAGTGTTTAAGAGCCTGTTCACCTACAGACTGC CACAGCATAGAGAAGATTTGGTGCTGACGGGCTACCAGGTTGACAAGAACA | 440 |
| | AAGATGACGAGCTGACGGGTTTCGCGGCCGCTGAACAGAAGCTGATCTCCG AAGAAGATCTGGCCGCGCATCACCATCACCACCACTAATGA | |
| 304 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCATGGATCAC CCCAGTTAACGTTCTGGGTACGAACTACTACATCAAGGTTCGTGCGGGTGAC AACAAGTATATGCACCTGAAAGTGTTTAAGAGCCTGGATGAAATCTTCCATG GTGCATACAACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 441 |
| 305 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGAAAACTGGAAGCCGTCCAGTATAAGACTCAAGTCGTCGAATCTTACGC AGCAATCTTCTGGGGTGGTACGAACTACTACATCAAGGTTCGTGCGGGTGAC AACAAGTATATGCACCTGAAAGTGTTTAAGAGCCTGTGGGGTGAAATCCAGC ATTGGAGACAGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 442 |
| 306 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGTTTGGCC ATGGACCTGGGATCGTGGTACGAACTACTACATCAAGGTTCGTGCGGGTGAC AACAAGTATATGCACCTGAAAGTGTTTAAGAGCCTGGAGCAGTGGCTGCCAA ACCCATACTTCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 443 |
| 307 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGGTTAAAA AATGGTGGGAATACTTCGTTACGAACTACTACATCAAGGTTCGTGCGGGTGA CAACAAGTATATGCACCTGAAAGTGTTTAAGAGCCTGCAGCTGCTGTCTCAT CAGTGGACCGAAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 444 |
| 308 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCATCGGTCAGAT CTCTGAACAGCAGTGGGGTACGAACTACTACATCAAGGTTCGTGCGGGTGAC AACAAGTATATGCACCTGAAAGTGTTTAAGAGCCTGCATAGATACTGGACCA GAGATGTTACCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 445 |
| 309 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCATTCCATAA ACTGGGTTGGATCCTGGGTACGAACTACTACATCAAGGTTCGTGCGGGTGAC AACAAGTATATGCACCTGAAAGTGTTTAAGAGCCTGGGTGATGAATCTCATA AATGGCATAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 446 |
| 310 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTTCAAAAAATG GTGGTGGAAATACAAAGGTACGAACTACTACATCAAGGTTCGTGCGGGTGA CAACAAGTATATGCACCTGAAAGTGTTTAAGAGCCTGAGAGTTGATGAACAT TACATCCAGTTCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 447 |
| 311 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 448 |
| | ACGGCAAACTGGAAGCCGTCCAGTATAAGACTCAAGTCGTCGCATCTCAGCG TCTGTGGATCATCTACGGTACGAACTACTACATCAAGGTTCGTGCGGGTGAC AACAAGTATATGCACCTGAAAGTGTTTAAGAGCCTGTCTGATTGGAGACATC ATGGTGGTTGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | |
| 312 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGCGTGAAA CCCATATCCTGTGGTCTGGTACGAACTACTACATCAAGGTTCGTGCGGGTGA CAACAAGTATATGCACCTGAAAGTGTTTAAGAGCCTGTGGTTCGATAACCTG CATAACAACTTCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 449 |
| 313 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTCAGTACTG GTTCTGGGCACCAGGTGGTACGAACTACTACATCAAGGTTCGTGCGGGTGAC AACAAGTATATGCACCTGAAAGTGTTTAAGAGCCTGCCATCTTCTGGTGGTG GTCCAAAATGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 450 |
| 314 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGATTGGGC ATACTACATCTACTTCGGTACGAACTACTACATCAAGGTTCGTGCGGGTGAC AACAAGTATATGCACCTGAAAGTGTTTAAGAGCCTGAGATCTGATACCCTGC ATTACTGGAACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 451 |
| 315 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCATCGGTTACAA ACTGAACTACATCTGGGGTACGAACTACTACATCAAGGTTCGTGCGGGTGAC AACAAGTATATGCACCTGAAAGTGTTTAAGAGCCTGATCCAGTACAGATGGC AGGATAGAGTTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 452 |
| 316 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTTCTCTGAC CGAACAGCGTTGGAAAGGTACGAACTACTACATCAAGGTTCGTGCGGGTGA CAACAAGTATATGCACCTGAAAGTGTTTAAGAGCCTGTTCCAGCCAATCAAA CTGGCAAACGCAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 453 |
| 317 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGGTTTCTCA GCAGCATTACTCTCATGGTACGAACTACTACATCAAGGTTCGTGCGGGTGAC AACAAGTATATGCACCTGAAAGTGTTTAAGAGCCTGTTCGCATTCAGAAAAT ACGAAGATAGAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 454 |
| 318 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGATGATGATA ACCAGCATTACGGTCTGGGTACGAACTACTACATCAAGGTTCGTGCGGGTGA CAACAAGTATATGCACCTGAAAGTGTTTAAGAGCCTGCTGCATAACTGGTGG AAAGGTAACTGGGAAGATTTGGTGCTGACGGGCTACCAGGTTGACAAGAAC | 455 |
| | AAAGATGACGAGCTGACGGGTTTCGCGGCCGCTGAACAGAAGCTGATCTCC GAAGAAGATCTGGCCGCGCATCACCATCACCACCACTAATGA | |
| 319 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCCAACCCGTTG GATCTCTATCTACTGGGGTACGAACTACTACATCAAGGTTCGTGCGGGTGAC AACAAGTATATGCACCTGAAAGTGTTTAAGAGCCTGCTGAACAGATACTACC ATGCATCTATCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 456 |
| 320 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCACCGCATTCGC ACCAAACAAAGCATCTGGTACGAACTACTACATCAAGGTTCGTGCGGGTGAC AACAAGTATATGCACCTGAAAGTGTTTAAGAGCCTGAGATACTGGGCATTCA GATACCTGTGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 457 |
| 321 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATTACACCGA TTTCAAATGGCATAAAGGTACGAACTACTACATCAAGGTTCGTGCGGGTGAC AACAAGTATATGCACCTGAAAGTGTTTAAGAGCCTGCCATTCTGGGCAAACG AACATGGTTGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 458 |
| 322 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAACAAATTCTG GTCTTGGGCACCAGGTGGTACGAACTACTACATCAAGGTTCGTGCGGGTGAC AACAAGTATATGCACCTGAAAGTGTTTAAGAGCCTGGCAGATTCTGGTTTCA ACGCAACCCCAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 459 |
| 323 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGAACTGGATG AACCAGCATGGGATTGGGGTACGAACTACTACATCAAGGTTCGTGCGGGTGA CAACAAGTATATGCACCTGAAAGTGTTTAAGAGCCTGAGACAGCATAAAAA ACCACATCCAAACGAAGATTTGGTGCTGACGGGCTACCAGGTTGACAAGAA CAAAGATGACGAGCTGACGGGTTTCGCGGCCGCTGAACAGAAGCTGATCTCC GAAGAAGATCTGGCCGCGCATCACCATCACCACCACTAATGA | 460 |
| 324 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATGAAAAAT GGGGTTTCTGGGAACTGGGTACGAACTACTACATCAAGGTTCGTGCGGGTGA CAACAAGTATATGCACCTGAAAGTGTTTAAGAGCCTGCATTCTCATAGATTC CATGAAGCACATGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 461 |
| 325 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTGCAACCGC AGATCTGATCAAAGCAGGTACGAACTACTACATCAAGGTTCGTGCGGGTGAC AACAAGTATATGCACCTGAAAGTGTTTAAGAGCCTGCCATGGCATTACCCAC CAAACAAATGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 462 |
| 326 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 463 |
| | ACGGCAAACTGGAAGCCGTCCAGTATAAGACTCAAGTCGTCTACTACAAAGT TATCTCTTGGTGGCATGGTACGAACTACTACATCAAGGTTCGTGCGGGTGAC AACAAGTATATGCACCTGAAAGTGTTTAAGAGCCTGTACAAAACCCCATTCC CACAGTACACCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | |
| 327 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCATTGGCATCA CTACGATAACATCATCGGTACGAACTACTACATCAAGGTTCGTGCGGGTGAC AACAAGTATATGCACCTGAAAGTGTTTAAGAGCCTGTACCAGCTGGTTGATC TGGATCTGGATGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 464 |
| 328 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGTTTACAAAAT CATCACCTTCTGGTCTGGTACGAACTACTACATCAAGGTTCGTGCGGGTGAC AACAAGTATATGCACCTGAAAGTGTTTAAGAGCCTGGAATACCCACCAGTTC AGCAGGTTCTGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 465 |
| 329 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTTGGATCCA GCGTCATAACCTGATCGGTACGAACTACTACATCAAGGTTCGTGCGGGTGAC AACAAGTATATGCACCTGAAAGTGTTTAAGAGCCTGTACTCTGAACCACCAC CAGTTCAGAACGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 466 |
| 330 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCAAAACCTGGG AAGATACCGTTGTTGCAGGTACGAACTACTACATCAAGGTTCGTGCGGGTGA CAACAAGTATATGCACCTGAAAGTGTTTAAGAGCCTGGAATACCTGTACAGA CCACTGAACCCAGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 467 |
| 331 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGGATCAGCA TCATGTTTACCAGGTTGGTACGAACTACTACATCAAGGTTCGTGCGGGTGAC AACAAGTATATGCACCTGAAAGTGTTTAAGAGCCTGGCACTGCTGATCCATC ATGATCCATGGGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 468 |
| 332 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGGAAGATGT TTGGGGTGTTTGGGCAGGTACGAACTACTACATCAAGGTTCGTGCGGGTGAC AACAAGTATATGCACCTGAAAGTGTTTAAGAGCCTGGAATTCCAGCATAGAA CCTTCAACCATGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 469 |
| 333 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGCTGCCATG GACCGTTGAACTGATCGGTACGAACTACTACATCAAGGTTCGTGCGGGTGAC AACAAGTATATGCACCTGAAAGTGTTTAAGAGCCTGGGTGAAATCTGGTCTG CAAAACATAAAGAAGATTTGGTGCTGACGGGCTACCAGGTTGACAAGAACA | 470 |
| | AAGATGACGAGCTGACGGGTTTCGCGGCCGCTGAACAGAAGCTGATCTCCG AAGAAGATCTGGCCGCGCATCACCATCACCACCACTAATGA | |
| 334 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCAAGTATAAGACTCAAGTCGTCCAGCATTTCAC CTGGGATTGGTTCTGGGGTACGAACTACTACATCAAGGTTCGTGCGGGTGAC AACAAGTATATGCACCTGAAAGTGTTTAAGAGCCTGCCAGAATACAAACCA GTTGATCATACCGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 471 |
| 335 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCTGTTCGAAGT TCTGCAGGGGTCTTCTGGTACGAACTACTACATCAAGGTTCGTGCGGGTGAC AACAAGTATATGCACCTGAAAGTGTTTAAGAGCCTGGGTGAAATCGTTTGGT ACCAGGTTGAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 472 |
| 336 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCCATACTCTTG GCAGCATGAAGTTGTTGGTACGAACTACTACATCAAGGTTCGTGCGGGTGAC AACAAGTATATGCACCTGAAAGTGTTTAAGAGCCTGATCGTTTTCTACCTGC AGGAATACTACGAAGAAGATTTGGTGCTGACGGGCTACCAGGTTGACAAGA ACAAAGATGACGAGCTGACGGGTTTCGCGGCCGCTGAACAGAAGCTGATCT CCGAAGAAGATCTGGCCGCGCATCACCATCACCACCACTAATGA | 473 |
| 337 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGTGGCAGG AAAACGCAGAATGGATCGGTACGAACTACTACATCAAGGTTCGTGCGGGTG ACAACAAGTATATGCACCTGAAAGTGTTTAAGAGCCTGGGTTACGCAGTTTT CCATAAATACCTGGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 474 |
| 338 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTACGATCC AGTTTACCAGGATACCGGTACGAACTACTACATCAAGGTTCGTGCGGGTGAC AACAAGTATATGCACCTGAAAGTGTTTAAGAGCCTGTTCTTCCATAGGACCT CTGTTGTTTACGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 475 |
| 339 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCGTGTTTTCGA TACCCCACTGGCAGCAGGTACGAACTACTACATCAAGGTTCGTGCGGGTGAC AACAAGTATATGCACCTGAAAGTGTTTAAGAGCCTGCCATGGCAGTACAGAA ACAACCCAGAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 476 |
| 340 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGTGGGATAT CCCATGGATCTGGGTTGGTACGAACTACTACATCAAGGTTCGTGCGGGTGAC AACAAGTATATGCACCTGAAAGTGTTTAAGAGCCTGCAGAACGATGAACTGT ACCCATACAAAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 477 |
| 341 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT | 478 |
| | ACGGCAAACTGGAAGCCGTCCAGTATAAGACTCAAGTCGTCTACCAGAAAG TTATCACCTGGTGGCAGGGTACGAACTACTACATCAAGGTTCGTGCGGGTGA CAACAAGTATATGCACCTGAAAGTGTTTAAGAGCCTGTACTGGGAAAACCAT AGAGGTCATGTTGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | |
| 342 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCCAGTGGATCA AATGGGGTAACATCCTGGGTACGAACTACTACATCAAGGTTCGTGCGGGTGA CAACAAGTATATGCACCTGAAAGTGTTTAAGAGCCTGTACGAAGCAATCGAA AGATGGCATGGTGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 479 |
| 343 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGCAACCCCAG AAAAAAAATACGTTGTTGGTACGAACTACTACATCAAGGTTCGTGCGGGTGA CAACAAGTATATGCACCTGAAAGTGTTTAAGAGCCTGTGGAACGCAAACATC CTGAGATACTGGGAAGATTTGGTGCTGACGGGCTACCAGGTTGACAAGAAC AAAGATGACGAGCTGACGGGTTTCGCGGCCGCTGAACAGAAGCTGATCTCC GAAGAAGATCTGGCCGCGCATCACCATCACCACCACTAATGA | 480 |
| 344 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCACCTCTGAACG TCTGTGGATCCTGTACGGTACGAACTACTACATCAAGGTTCGTGCGGGTGAC AACAAGTATATGCACCTGAAAGTGTTTAAGAGCCTGACCCCAAGATACGTTC CACTGAACCCAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 481 |
| 345 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGAACATCATTC TCAGCCACTGGCACATGGTACGAACTACTACATCAAGGTTCGTGCGGGTGAC AACAAGTATATGCACCTGAAAGTGTTTAAGAGCCTGCCATACCATGCATTCG ATTGGTACTGGGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 482 |
| 346 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTGGACCGCAG ATTGGCTGTGGACCGTTGGTACGAACTACTACATCAAGGTTCGTGCGGGTGA CAACAAGTATATGCACCTGAAAGTGTTTAAGAGCCTGTACGATCCATCTGAA TGGTTCCCAAGAGAAGATTTGGTGCTGACGGGCTACCAGGTTGACAAGAACA AAGATGACGAGCTGACGGGTTTCGCGGCCGCTGAACAGAAGCTGATCTCCG AAGAAGATCTGGCCGCGCATCACCATCACCACCACTAATGA | 483 |
| 347 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCTCTTTCCAGCG TGAACATTACTCTTGGGGTACGAACTACTACATCAAGGTTCGTGCGGGTGAC AACAAGTATATGCACCTGAAAGTGTTTAAGAGCCTGTTCGATATCTTCTACTT CAACCCAATCGAAGATTTGGTGCTGACGGGCTACCAGGTTGACAAGAACAA AGATGACGAGCTGACGGGTTTCGCGGCCGCTGAACAGAAGCTGATCTCCGA AGAAGATCTGGCCGCGCATCACCATCACCACCACTAATGA | 484 |
| 348 | ATGATTCCCGGCGGACTTTCGGAAGCCAAGCCAGCCACTCCGGAGATTCAGG AAATCGTGGACAAGGTCAAACCGCAGCTGGAAGAGAAAACCGGCGAAACCT ACGGCAAACTGGAAGCCGTCCAGTATAAGACTCAAGTCGTCGGTAAAACCT GGCATAAACATAACCGTGGTACGAACTACTACATCAAGGTTCGTGCGGGTGA CAACAAGTATATGCACCTGAAAGTGTTTAAGAGCCTGAACCTGTACTTCTTC TTCTACCAGACCGAAGATTTGGTGCTGACGGGCTACCAGGTTGACAAGAACA | 485 |
| | AAGATGACGAGCTGACGGGTTTCGCGGCCGCTGAACAGAAGCTGATCTCCG AAGAAGATCTGGCCGCGCATCACCATCACCACCACTAATGA | |

In some embodiments, the stefin A protein variant may also include small deletions or additions - beyond the loop 2 and loop 4 inserts described above - to the stefin A protein or stefin A protein variant sequences disclosed herein, such as addition or deletion of up to 10 amino acids relative to stefin A protein.

In some embodiments, the stefin A protein variant can bind to human CD40L with a dissociation constant (K_{D}) of about 1 µM or less, about 100 nM or less, about 40 nM or less, about 20 nM or less, about 10 nM or less, about 1 nM or less, or about 0.1 nM or less.

In some embodiments, the stefin A protein variant can bind to human CD40L with an off-rate constant (K_{off}), such as measured by BIACORE^{™} assay, of about 10⁻³ s⁻¹ (e.g., unit of 1/second) or slower; of about 10⁻⁴ s⁻¹ or slower, of about 10⁻⁵ s⁻¹ or slower, or of about 10⁻⁶ s⁻¹ or slower.

In some embodiments, the stefin A protein variant can bind to human CD40L with an association constant (Kₒₙ), such as measured by BIACORE^{™} assay, of at least about 10³ M⁻¹s⁻¹ or faster; at least about 10⁴ M⁻¹s⁻¹ or faster; at least about 10⁵ M⁻¹s⁻¹ or faster; or even at least about 10⁶ M⁻¹s⁻¹ or faster.

In some embodiments, the stefin A protein variant can bind to human CD40L with an IC50 in a competitive binding assay with human CD40L of 1 µM or less, about 100 nM or less, about 40 nM or less, about 20 nM or less, about 10 nM or less, about 1 nM or less, or about 0.1 nM or less.

In some embodiments, the stefin A protein variant has a melting temperature (Tm, e.g., temperature at which both the folded and unfolded states are equally populated) of 65°C or higher, and preferably at least 70°C, 75°C, 80°C or even 85°C or higher. Melting temperature is a particularly useful indicator of protein stability. The relative proportions of folded and unfolded proteins can be determined by many techniques known to the skilled person, including differential scanning calorimetry, UV difference spectroscopy, fluorescence, circular dichroism (CD), and NMR (Pace et al. (1997) "Measuring the conformational stability of a protein" in Protein structure: A practical approach 2: 299-321).

In some embodiments, the stefin A protein variant can form a multimer.

In some embodiments, the stefin A protein variant can form a dimer, trimer, tetramer, pentamer or more multimer.

In some embodiments, the multimer may be formed by covalently or non-covalently linked by an interaction between amino acid residues of the stefin A protein variant.

In some embodiments, the multimer multimer may be a fusion protein formed through a fusion domain fused with a stefin A protein variant.

In some embodiments, the multimer may be formed by in-line fusion of a stefin A protein variant, and the in-line fusion protein in the form of such a multimer will be described in detail in the section on fusion proteins below.

### Fusion Protein

In another aspect, the present invention relates to a fusion protein comprising the stefin A protein variant specifically binding to the CD40L.

In another aspect, the present invention relates to a use for preparing a fusion protein of the stefin A protein variant specifically binding to the CD40L.

In some embodiments, the fusion protein further comprises an additional insertion, substitution and/or deletion that modulates biological activity of the stefin A protein variant or that gives additional biological functions. For example, the stefin A protein variant can be fused with other polypeptide for binding to and inhibiting CD40L, modulate the circulating half-life, modulate the therapeutic half-life, modulate the stability of the stefin A protein variant, modulate cleavage by proteases, modulate dose, modulate release or bioavailability, facilitate purification, decrease deamidation, improve shelf-life, or improve or alter a particular route of administration. For Example, the fusion protein may further comprise protease cleavage sequences, reactive groups, antibody binding domainsm, fusion domains for protein expression and purification (e.g. FLAG, poly-His, GST, c-myc etc.), linked molecules(e.g. biotin etc.),or other therapeutic peptide or protein, but is not limited thereto.

In some embodiments, the fusion protein may be one in which an additional peptide sequence (a fusion domain) is fused to one end and/or the other end of the stefin A protein variant.

As used herein, the term "fusion domain" refers to an additional domain or moiety that may be incorporated directly or indirectly by being fused to the stefin A protein variant specifically binding to CD40L of the present invention.

In some embodiments, the fusion domain, for example, may be fused to confer expression properties such as secretion from the cell, or anchoring to the cell surface, or intracellular localization; to serve as substrate or other recognition sequences for post-translational modifications; to create multimeric structures aggregating through protein-protein interactions; to alter serum half-life; or to alter tissue localization or tissue exclusion and other ADME properties; to add other functional proteins or peptides.

In some embodiments, a signal sequence may be comprised in the fusion protein for transport and secretion of the stefin A protein variant. In some embodiments, when the stefin A protein variant is to be expressed in a membrane-anchored form or on the cell surface, the fusion protein comprises a transmembrane domain or a cell surface retention signal sequence.

In some embodiments, the Signal sequences (also referred to as signal peptides or leader sequences) are located at the N-terminus of the stefin A protein variant. The signal sequences target the stefin A protein variant to the endoplasmic reticulum, and secrete stefin A protein variant. Most signal sequences are cleaved from the protein by a signal peptidase after the proteins are transported to the endoplasmic reticulum. The cleavage of the signal sequence from the polypeptide usually occurs at a specific site in the amino acid sequence and is dependent upon amino acid residues within the signal sequence, but is not limited to.

In some embodiments, the signal peptide is about 5 to about 40 amino acids in length (such as about 5 to about 7, about 7 to about 10, about 10 to about 15, about 15 to about 20, about 20 to about 25, or about 25 to about 30, about 30 to about 35, or about 35 to about 40 amino acids in length).

In some embodiments the signal peptide is a native signal peptide from a human protein. In other embodiments, the signal peptide is a non-native signal peptide. For example, in some embodiments, the non-native signal peptide is a mutant native signal peptide from the corresponding native secreted human protein, and can include at least one substitution, insertions and/or deletions.

In some embodiments, the signal peptide is a signal peptide or mutant thereof from a non-IgSF protein family, such as a signal peptide from an immunoglobulin (such as IgG heavy chain or IgG-kappa light chain), a cytokine (such as interleukin-2 (IL-2), or CD33), a serum albumin protein (e.g. HSA or albumin), a human azurocidin preprotein signal sequence, a luciferase, a trypsinogen (e.g. chymotrypsinogen or trypsinogen) or other signal peptide able to efficiently secrete a protein from a cell, but is not limited to.

Table 5 below lists examples of signal peptides that can be used for secretion of stefin A protein variants or fusion proteins thereof of the present invention, but is not limited thereto.

**Table 5. Exemplary Signal Sequences**

| **Native Protein** | **Signal Sequence** | **SEQ ID NO:** |
|---|---|---|
| Human Serum Albumin (HSA) | MKWVTFISLLFLFSSAYS | 486 |
| Ig kappa light chain | MDMRAPAGIFGFLLVLFPGYRS | 487 |
| Human azurocidin preprotein | MTRLTVLALLAGLLASSRA | 488 |
| IgG heavy chain | MELGLSWIFLLAILKGVQC | 489 |
| IgG heavy chain | MELGLRWVFLVAILEGVQC | 490 |
| IgG heavy chain | MKHLWFFLLLVAAPRWVLS | 491 |
| IgG heavy chain | MDWTWRILFLVAAATGAHS | 492 |
| IgG heavy chain | MDWTWRFLFVVAAATGVQS | 493 |
| IgG heavy chain | MEFGLSWLFLVAILKGVQC | 494 |
| IgG heavy chain | ME FGLSWVFLVAL FRGVQC | 495 |
| IgG heavy chain | MDLLHKNMKHLWFFLLLVAAPRWVLS | 496 |
| IgG Kappa light | MDMRVPAQLLGLLLLWLSGARC | 497 |
| IgG Kappa light | MKYLLPTAAAGLLLLAAQPAMA | 498 |
| Gaussia luciferase | MGVKVLFALICIAVAEA | 499 |
| Human chymotrypsinogen | MAFLWLLSCWALLGTTFG | 500 |
| sHuman interleukin-2 | MQLLSCIALILALV | 501 |
| Human trypsinogen-2 | MNLLLILTFVAAAVA | 502 |
| Human CD33 | MPLLLLLPLLWAGALA | 503 |
| Prolactin | MDSKGSSQKGSRLLLLLVVSNLLLCQGVVS | 504 |
| Human tPA | MDAMKRGLCCVLLLCGAVFVSPS | 505 |
| Synthetic/Consensus | MLLLLLLLLLLALALA | 506 |
| Synthetic/Consensus | MWWRLWWLLLLLLLLWPMVWA | 507 |

For another example, some fusion domains are particularly useful for isolation and/or purification of the fusion proteins, such as by affinity chromatography. Well known examples of such fusion domains that facilitate expression or purification include, merely to illustrate, affinity tags such as polyhistidine (e.g., a His₆ tag), Strep II tag, streptavidin-binding peptide (SBP) tag, calmodulin-binding peptide (CBP), glutathione S-transferase (GST), maltose-binding protein (MBP), S-tag, HA tag, c-Myc tag, thioredoxin, protein A and protein G.

In some embodiments, the fusion proteins may also include at least one linker separating stefin A protein variant or fusion domains.

As used herein, the term "linker" refers to a linker amino acid sequence inserted between a first polypeptide (e.g., the stefin A protein variant specifically binding to CD40L) and a second polypeptide (e.g., another stefin A protein variant or the fusion domain).

In some embodiments, the linker may be generally classified into 3 categories according to their structures: flexible linkers, rigid linkers, and *in vivo* cleavable linkers.

In some embodiments, besides the basic role in linking the fusion domains together, the linker may offer many other advantages for the production of fusion proteins, such as improving biological activity, increasing expression yield, and achieving desirable pharmacokinetic profiles.

In some embodiments, the linkers, Preferably, should not adversely affect the expression, secretion, or activity of each domain. In some embodiments, the linkers, Preferably, should not be antigenic and should not elicit an immune response.

In some embodiments, the linker may preferably be a GS linker (glycine-serin linker) including glycine and serine residues, but is not limited thereto. In another example, the linker may include threonine and alanine, but is not limited to.

In some embodiments, the linker can range in length, for example from 1-50 amino acids in length, 1-22 amino acids in length, 1-10 amino acids in length, 1-5 amino acids in length, or 1-3 amino acids in length.

In some embodiments, the linker may comprise a cleavage site.

In some embodiments, the linker can be characterized as flexible. Flexible linkers are usually applied when the fusion domains require a certain degree of movement or interaction, but inot limited to. Examples of the flexible linker are described in Argos P. (1990) "An investigation of oligopeptides linking domains in protein tertiary structures and possible candidates for general gene fusion" J Mol Biol. 211:943-958. The small size of these amino acids provides flexibility and allows for mobility of the connecting functional domains. The incorporation of Ser or Thr can maintain the stability of the linker in aqueous solutions by forming hydrogen bonds with the water molecules, and therefore reduces the unfavorable interaction between the linker and the protein moieties. The most commonly used flexible linkers have sequences consisting primarily of stretches of Gly and Ser residues ("GS" linker). An example of the most widely used flexible linker has the sequence of (Gly-Gly-Gly-Gly-Ser)n (SEQ ID NO: 508), but is not limited thereto. In SEQ ID NO: 508, n is an integer greater than or equal to 1, and the length of this GS linker can be optimized to achieve appropriate separation of the fusion domains by adjusting the copy number "n". the flexible linker may further include amino acids such as Thr, Ala, Lys, and Glu in addition to Gly and Ser.

In some embodiments, the linker can be characterized as rigid. While flexible linkers have the advantage to connect the functional domains passively and permitting certain degree of movements, the lack of rigidity of these linkers can be a limitation in certain fusion protein embodiments, such as in expression yield or biological activity. The ineffectiveness of flexible linkers in these instances was attributed to an inefficient separation of the protein domains or insufficient reduction of their interference with each other. Under these situations, rigid linkers have been successfully applied to keep a fixed distance between the domains and to maintain their independent functions.

Many natural linkers exhibited α-helical structures. The α-helical structure was rigid and stable, with intra-segment hydrogen bonds and a closely packed backbone. Therefore, the stiff α-helical linkers can act as rigid spacers between protein domains. George et al. (2002) "An analysis of protein domain linkers: their classification and role in protein folding" Protein Eng. 15(11):871-9. In general, rigid linkers exhibit relatively stiff structures by adopting α-helical structures or by containing multiple Pro residues. Under many circumstances, they separate the functional domains more efficiently than the flexible linkers. The length of the linkers can be easily adjusted by changing the copy number to achieve an optimal distance between domains. As a result, rigid linkers are chosen when the spatial separation of the domains is critical to preserve the stability or bioactivity of the fusion proteins. In this regard, alpha helix-forming linkers with the sequence of (EAAAK)n (SEQ ID NO: 509) have been applied to the construction of many recombinant fusion proteins. Another type of rigid linkers has a Pro-rich sequence, (XP)n, with X designating any amino acid, preferably Ala, Lys, or Glu, but is not limited thereto.

In some embodiments, Table 6 lists examples of linkers that can be used for the secretion of the stefin A protein variant or fusion protein thereof of the present invention, but is not limited thereto.

**Table 6. Exemplary Linkers**

| **Type** | **Sequence** | **SEQ ID NO:** |
|---|---|---|
| Flexible | (GGGGS)ₙ (e.g., n = 1-6) | 508 |
| Flexible | (Gly)₈ | 510 |
| Flexible | (Gly)₆ | 511 |
| Flexible | KESGSVSSEQLAQFRSLD | 512 |
| Flexible | EGKSSGSGSESKST | 513 |
| Flexible | GSAGSAAGSGEF | 514 |
| Rigid | (EAAAK)ₙ (e.g., n = 1-6) | 509 |
| Rigid | A(EAAAK)₄ALEA(EAAAK)₄A | 515 |
| Rigid | PAPAP | 516 |
| Rigid | AEAAAKEAAAKA | 517 |
| Rigid | (Ala-Pro)n (10 to 34 aa) | 518 |

Other linkers that may be used in the subject fusion proteins include but are not limited to:
SerGly, GGSG (SEQ ID NO: 519), GSGS (SEQ ID NO: 520), GGGS (SEQ ID NO: 521), S(GGS)n (SEQ ID NO: 522) where n is 1-7, GRA, poly(Gly), poly(Ala), GGGSGGG (SEQ ID NO: 523), ESGGGGVT (SEQ ID NO: 524), LESGGGGVT (SEQ ID NO: 525), GRAQVT (SEQ ID NO: 526), WRAQVT (SEQ ID NO: 527), and ARGRAQVT (SEQ ID NO: 528). The hinge regions of the Fc fusions described below may also be considered linkers.

In some embodiments, when the Fc domain is fused to the Stepin A protein variant, hinge regions can be considered as linkers.

In some embodiments, the fusion protein comprises one or more linker domains selected from the following: SGTTSGTTRLLSGHTCFTLTGLLGTLVTMGLLT (SEQ ID NO: 529)
SGTSPGLSAGATVGIMIGVLVGVALI (SEQ ID NO: 530)
SAPVLSAVATVGITIGVLARVALI (SEQ ID NO: 531)
SSPDLSAGTAVSIMIGVLAGMALI (SEQ ID NO: 532)
TLGGNSASYTFVSLLFSAVTLLLLC (SEQ ID NO: 533)
SGTSPGLSAGATVGIMIGVLVGVALI (SEQ ID NO: 534)

Still other modifications for a flanking polypeptide moiety provided as part of a fusion protein is at least one sequence that is a site for post-translational modification by an enzyme. These can include, but are not limited to, glycosylation, acetylation, acylation, lipid-modification, palmitoylation, palmitate addition, phosphorylation, glycolipid-linkage modification, and the like.

In some embodiments, the fusion protein may be localized to a specific organ or location within the cell.

In some embodiments, the fusion protein is a secretory fusion protein and/or a membrane-anchored fusion protein.

In some embodiments, the fusion protein may further include at least one fusion domain.

In some embodiments, the fusion protein may include a fusion domain selected from the group consisting of an antigen-binding protein (domain), a cytokine, a half-life extension domain, a growth factor, an enzyme, and a cell-penetrating domain, but is not limited thereto.

In some embodiments, the fusion protein may further include a therapeutic peptide or protein.

In some embodiments, the "therapeutic peptide or protein" refers to any peptide or protein having a preventive or therapeutic effect on a specific disease. In some embodiments, the therapeutic peptide or protein may be a stefin A protein variant (which may be the same as or different from the stefin A protein variant specifically binding to CD40L of the present invention). It includes, without limitation, peptides and proteins reported to have preventive or therapeutic effects on a specific disease in the art.

In some embodiments, the fusion protein may include a binding domain (or binding moiety).

In some embodiments, when the fusion protein includes a binding domain, it may have multi specificity capable of binding to at least one target molecule in addition to CD40L.

In some embodiments, the binding domain may be selected from the group consisting of, for example, a stefin A protein variant (which may be the same or different than the stefin A protein variant specifically binding to CD40L of the present invention), an antibody or fragment thereof or other antigen binding polypeptide, a ligand binding portion of a receptor (such as a receptor trap polypeptide), a receptor-binding ligand (such as a cytokine, growth factor or the like), engineered T-cell receptor, an enzyme and catalytic fragment thereof, but is not limited thereto.

In some embodiments, examples of the binding domain may include, but are not limited to, adnectins/monobodies, affilins, affibodies, affitins, anticalin, atrimers, avimers, bicyclic peptides, C7 peptide, centyrin, carbohydrate-binding module (CBM), cys-knots, darpin, el-tandem, fynomers, knottin, Kunitz domains, O bodies, pronectin, scFv, Sac7d, Sso7d, Tn3, and the like.

In some embodiments, the fusion domain fused to the stefin A protein variant may be the same or different stefin A protein variant specifically binding to CD40L, and/or a stefin A protein variant specifically binding to another target.

In some embodiments, when the fusion domain is a stefin A protein variant specifically binding to CD40L, the two stefin A protein variants included in the fusion protein of the present invention may bind to the same or different sites of CD40L. In some embodiments, the fusion protein may bind to two sites (biparatopic) or two or more sites (multiparatopic) of CD40L.

As used herein, the term "antibody" includes not only a complete antibody form that specifically binds to a target (antigen), but also an antigen-binding fragment of the antibody molecule. The complete antibody has a structure having two full-length light chains and two full-length heavy chains, and each light chain is connected to the heavy chain by a disulfide bond. As used herein, the term "heavy chain" refers to a full-length heavy chain including a variable region domain VH including an amino acid sequence having a sufficient variable region sequence to confer specificity to an antigen and three constant region domains CH1, CH2, and CH3, and fragments thereof. In addition, as used herein, the term "light chain" refers to a full-length light chain including a variable region domain VL including an amino acid sequence having a sufficient variable region sequence to confer specificity to an antigen and a constant region domain CL, and fragments thereof. The whole antibody includes subtypes of IgA, IgD, IgE, IgM, and IgG, and in particular, IgG includes IgG1, IgG2, IgG3, and IgG4. The heavy-chain constant region has gamma (γ), mu (µ), alpha (α), delta (δ), and epsilon (ε) types, and subclasses such as gamma 1 (γ1), gamma 2 (γ2), gamma 3 (γ3), gamma 4 (γ4), alpha 1 (α1), and alpha 2 (α2). The constant region of the light chain has kappa (κ) and lambda (λ) types.

The antigen-binding fragment of an antibody or antibody fragment refers to a fragment having an antigen-binding function, and includes Fab, F(ab'), F(ab')2, and Fv. Among the antibody fragments, Fab has a structure having variable regions of light and heavy chains, a constant region of a light chain, and a first constant region (CH1) of a heavy chain, and has one antigen-binding site. Fab' differs from Fab in that Fab' has a hinge region including at least one cysteine residue at the C-terminus of the heavy-chain CH1 domain. F(ab')2 is formed by a disulfide bond between cysteine residues in the hinge region of Fab'.

Fv is a minimal antibody fragment having only a heavy-chain variable region and a light-chain variable region. A two-chain Fv is a fragment in which a heavy-chain variable region and a light-chain variable region are linked by a non-covalent bond, and a single-chain Fv (scFv) is a fragment in which a heavy-chain variable region and a light-chain variable region are generally linked by a covalent bond via a peptide linker therebetween, or are directly linked at the C-terminus, forming a dimeric structure, like the two-chain Fv. Such antibody fragments may be obtained using proteases (for example, Fab may be obtained by restriction-cleaving a whole antibody with papain, and the F(ab')2 fragment may be obtained by restriction-cleaving a whole antibody with pepsin), or may be constructed through genetic recombination technology.

An "Fv" fragment is an antibody fragment that contains a complete antibody recognition and binding site. This region is a dimer in which one heavy-chain variable domain and one light-chain variable domain are joined.

A "Fab" fragment includes variable and constant domains of a light chain and variable and first constant domains (CH1) of a heavy chain. An F(ab')2 antibody fragment generally includes a pair of Fab' fragments covalently linked by cysteines in the hinge region present at the C-terminus of the Fab' fragment.

A "single-chain Fv (scFv)" antibody fragment is a construct composed of a single polypeptide chain including the VH and VL domains of an antibody. scFv may further include a polypeptide linker between the VH domain and the VL domain so as form the desired structure for antigen binding.

Examples of the antibody of the present invention may include, but are not limited to, monoclonal antibodies, multispecific antibodies, human antibodies, humanized antibodies, chimeric antibodies, scFv, Fab fragments, F(ab')2 fragments, disulfide-linked Fvs (sdFv), and anti-idiotype (anti-Id) antibodies, epitope-binding fragments of such antibodies, and the like.

The heavy-chain constant region may be selected from among isotypes such as gamma (γ), mu (µ), alpha (α), delta (δ), and epsilon (ε). For example, the constant region is gamma 1 (IgG1), gamma 2 (IgG2), gamma 3 (IgG3), or gamma 4 (IgG4). The light-chain constant region may be a kappa or lambda type.

A monoclonal antibody is an antibody obtained from a population of substantially homogeneous antibodies, in which the individual antibodies that make up the population are identical, except for possible naturally-occurring mutations that may be present in small amounts. A monoclonal antibody is highly specific and is induced against a single epitope on the antigen. In contrast to typical (polyclonal) antibodies, which typically include different antibodies directed against different determinants (epitopes), each monoclonal antibody is directed against a single determinant on the antigen.

In some embodiments, the fusion protein may include an Fc portion of an immunoglobulin. For example, when the fusion protein includes an Fc portion, it may bind to an Fc receptor to activate Fc receptor-positive cells, thereby initiating or increasing the expression of cytokines and/or costimulatory antigens, and inducing antibody-dependent cytotoxicity (ADCC).

In some embodiments, with respect to the fusion protein comprising a full-length immunoglobulin, the fusion protein may preserve the Fc function of the Fc region of the immunoglobulin. For example, the fusion proteinmay be capable of binding, via its Fc portion, to the Fc receptor of Fc receptor-positive cells. In some further embodiments, the fusion protein t may activate the Fc receptor-positive cell by binding to the Fc receptor-positive cell, thereby initiating or increasing the expression of cytokines and/or co-stimulatory antigens. Furthermore, the fusion protein may transfer at least a second activation signal required for physiological activation of the T cell to the T cell via the co-stimulatory antigens and/or cytokines In some embodiments, the fusion protein has an antibody-dependent cytotoxicity (ADCC) function by binding the Fc region to other cells expressing Fc receptors present on the surface of effector cells of the immune system, such as immune cells, liver cells, and endothelial cells.

In some embodiments, apart from the Fc-mediated cytotoxicity, the Fc portion of the fusion protein may contribute to maintaining the serum levels of the fusion protein, critical for its stability and persistence in the body. For example, when the Fc portion binds to Fc receptors on endothelial cells and on phagocytes, the AFFIMER^{®} agent (fusion protein) may become internalized and recycled back to the blood stream, enhancing its half-life within the body.

Exemplary targets of a multi-specific fusion protein include but are not limited to, another immune checkpoint protein, and immune co-stimulatory receptor, a receptor, a cytokine, a growth factor, or a tumor-associated antigen. In some embodiments, the binding domainmay be a monoclonal antibody against at least one autoimmune target (e.g., TNFR2 or IL6-R). In some embodiments, the fusion protein includes one or more binding domains that bind to a protein upregulated in autoimmune conditions (e.g., TNFR2 or IL6-R).The various forms of the fusion protein of the present invention are described in William BA et al. J. Clin. Med. 2019; 8(8): 1261

In some embodiments, the fusion domain may be an immune checkpoint protein, an immune costimulatory receptor, a receptor (or receptor agonist), a cytokine, a growth factor, or a tumor-associated antigen, but is not limited thereto.

In some embodiments, the cytokine is used as a generic term for secretory proteins that play an important role in signaling between cells. Examples of the cytokines may include, but are not limited to, chemokines, interferons, lymphokines, interleukins, tumor necrosis factors, and the like.

In some embodiments, the growth factor refers to a naturally-occurring material or a variant thereof that may stimulate cell proliferation, wound healing, and/or cell differentiation. Examples of the growth factors may include, but are not limited to, GH, EGF, VEGF, FGF, bFGF, HGF, BMPs, M-CSF, G-CSF, GM-CSF, EPO, GDNF, IGF, KGF, BDNF, NGF, PDGF, TPO, TGF, and the like.

In some embodiments, the enzyme is used as a generic term for proteins that catalyze a biological reaction. Examples of the enzyme may include, but are not limited to, α-chymotrypsin, lysozyme, urate oxidase, acetylcholinesterase, Thermomyces lanuginosus lipase, glucose oxidase, superoxie dismutase, caspase, β-glucosidase, Trametes versicolor laccase, alcohol oxidase, Cas9, Cas12, Cas13, Cas14, zinc-finger nuclease, TALLEN, dimethyl sulfoxide, uricase, agalsidase beta, agalsidase alfa, imiglucerase, taliglucerase alfa, velaglucerase alfa, alglucerase, sebelipase alpha, laronidase, idursulfase, elosulfase alpha, galsulfase, alglucosidase alpha, and the like.

In some embodiments, the cell-penetrating peptide is a short peptide that promotes cellular uptake and absorption of various molecules. In some embodiments, when the fusion protein includes a cell-penetrating peptide, the stefin A protein variant may be absorbed into the cell. Examples of the cell-penetrating peptide may include, but are not limited thereto, Tat, penetratin, transporant, Pept1, Pept 2, pVEC, DPV3, DPV6, R8, R9, MPG, MAP, Bip4, C105Y, melittin, and the like.

In some embodiments, the fusion protein may be fixed to the cell membrane of the genetically modified cell or may be expressed on the cell surface.

In some embodiments, when the fusion protein is fixed to a cell membrane or is expressed on a cell surface, it may further include a transmembrane domain. As used herein, the term "transmembrane domain" refers to a protein domain that spans the width of a cell membrane. In some embodiments, the transmembrane domain preferably has an alpha-helical structure, but is not limited thereto.

In some embodiments, the transmembrane domain may be a transmembrane domain derived from, for example, CD3, CD4, CD5, CD8, CD28, CD99, immunoglobulin (e.g. IgG1, IgG4, IgD, etc.), PDGFR, PTGFRN, etc. or a variant thereof, but is not limited thereto.

In some embodiments, the fusion protein may further include a hinge domain in addition to the transmembrane domain. As used herein, the term "hinge domain" refers to a series of amino acid sequences that exist between the extracellular domain and the transmembrane domain of a membrane-anchoring protein. In some embodiments, the hinge domain may be located between the stefin A protein variant specifically binding to CD40L and the transmembrane domain.

In some embodiments, the hinge domain may be a hinge domain derived from, for example, CD3, CD4, CD5, CD8, CD28, CD99, immunoglobulin (e.g. IgG1, IgG4, IgD, etc.), PDGFR, PTGFRN, etc., or a variant thereof, but is not limited thereto.

In some embodiments, the fusion protein may further include a coiled coil domain. As used herein, the term "coiled coil domain" refers to a structural motif of a protein in which two to seven alpha helices are wound like a rope strand. Preferably, the coiled coil domain is configured such that two or three alpha helices are wound.

In some embodiments, the coiled coil domain may be a coiled coil domain derived from a leucine zipper, foldon, cardiac phospholamban, a water-soluble analogue of a membrane phospholamban, COMP (cartilage oligomeric matrix protein), thrombospondin 3, thrombospondin 4, or VASP (vasodilator-stimulated phosphoprotein), or a variant thereof, but is not limited thereto.

In some embodiments, the coiled coil domain may be located between the stefin A protein variant specifically binding to CD40L and the transmembrane domain.

In some embodiments, the fusion protein may further include a virus-derived peptide or protein. In some embodiments, examples of the virus-derived peptide or protein may include, but are not limited to, syncytin-1, syncytin-2, VSVG (vesicular stomatitis virus glycoprotein), F and G proteins of Nipah virus, F and H proteins of measles virus, F and H proteins of Tupaia paramyxovirus, F and G proteins, F and H proteins, or F and HN proteins of paramyxovirus, F and G proteins of Hendra virus, F and G proteins of Henipavirus, F and H proteins of Morbillivirus, F and HN proteins of respirovirus, F and HN proteins of Sendai virus, F and HN proteins of rubulavirus, F and HN proteins of avulavirus, variants thereof, and combinations thereof.

In some embodiments, the fusion protein may further include an immunomodulatory domain or an intracellular signaling domain.

In some embodiments, the immunomodulatory domain or intracellular signaling domain is a domain located in the cytoplasmic direction of a membrane-anchoring protein, and indicates a site that activates or inhibits an immune response when a target antigen is bound to the extracellular domain.

In some embodiments, the immunomodulatory domain or intracellular signaling domain may be an immunomodulatory domain derived from CD3, CD28, CD40L, ICOS, OX40, 4-1BB, TNFR2, etc., but is not limited thereto.

In some embodiments, the fusion protein may be a chimeric antigen receptor (CAR). In some embodiments, when the fusion protein is a chimeric antigen receptor, the stefin A protein variant specifically binding to CD40L may function as an extracellular binding domain.

In some embodiments, when the fusion protein is a chimeric antigen receptor, it may further include the above-described transmembrane domain, hinge domain, and intracellular signaling domain, but the present invention is not limited thereto. The fusion protein may be easily designed and prepared by changing the extracellular antigen-binding domain of various chimeric antigen receptors known in the art or analogues thereof to the stefin A protein variant specifically binding to CD40L of the present invention.

In some embodiments, the fusion protein may further include a localization domain. In some embodiments, when the fusion protein is expressed intracellularly, it is preferable to further include a localization domain. As used herein, the term "localization domain" refers to a peptide or protein sequence that functions to localize a protein to a specific organ within a cell or a specific location within a cell. In some embodiments, the localization domain may be an organ-specific localization domain or an intracellular protein localization domain.

In some embodiments, the localization domain may be a nucleus-specific localization domain derived from VACM-1/CUL5, CXCR4, VP1, 53BP1, ING4, IER5, ERK5, Hrp1, UL79, EWS, PTHrP, Pho4, and rpL23a, a mitochondria-specific localization domain derived from ATP synthase F1b, cytochrome c oxidase polypeptide VIII, SOD2, citrate synthase, Tu translation elongation factor, etc., or a peroxisome localization domain derived from PTS1, PTS2, etc., but is not limited thereto.

In some embodiments, the fusion protein may further include a half-life extension domain. In some embodiments, the half-life extension domain is a domain or moiety that is fused to extend the half-life of the stefin A protein variant of the present invention, and examples of the half-life extension domain may include, but are not limited to, an Fc domain, an Fc-binding protein or peptide, albumin (e.g. HSA), an albumin-binding protein or peptide, transferrin, transferrinbinding protein or peptide, etc.

### Pharmacokinetic and ADME (Absorption, Distribution, Metabolism, Excretion) Characteristics Engineered Fusion Proteins

As used herein, a "half-life" is the amount of time it takes for a substance, such as the stefin A protein varian or the fusion protein of the present inventione, to lose half of its pharmacologic or physiologic activity or concentration. Biological half-life can be affected by elimination, excretion, degradation (e.g., enzymatic) of the substance, or absorption and concentration in certain organs or tissues of the body. In some embodiments, biological half-life can be assessed by determining the time it takes for the blood plasma concentration of the substance to reach half its steady state level.

In some embodiments, the stefin A protein variant or the fusion protein comprising thereof, may not have an adequate half-life and/or pharmacokinetic profile (PK profile). Accordingly, the fusion protein may further comprise a half-life extending domain (or half-life extending moiety) to further improve the half-life or PK profile.

The term "half-life extending domain" refers to a pharmaceutically acceptable moiety, domain, or molecule directly fused or indirectly conjugated or fused through a linker or etc., to the stefin A protein variant of the present invention. In some embodiments, the half-life extending domain may prevent or mitigate in vivo proteolytic degradation or other activity-diminishing modification of the stefin A protein variant, increases half-life, and/or improves or alters other pharmacokinetic or biophysical properties including but not limited to increasing the rate of absorption, reducing toxicity, improving solubility, reducing protein aggregation, increasing biological activity and/or target selectivity, increasing manufacturability, and/or reducing immunogenicity of the stefin A protein variant.

In some embodiments, the half-life extending domain may comprise non-proteinaceous, half-life extending moieties, such as a water soluble polymer such as polyethylene glycol (PEG) or discrete PEG, hydroxyethyl starch (HES), a lipid, a branched or unbranched acyl group, a branched or unbranched C8-C30 acyl group, a branched or unbranched alkyl group, and a branched or unbranched C8-C30 alkyl group; and proteinaceous half-life extending moieties, such as serum albumin, transferrin, adnectins (e.g., albumin-binding or pharmacokinetics extending (PKE) adnectins), Fc domain, and unstructured polypeptide, such as XTEN and PAS polypeptide (e.g. conformationally disordered polypeptide sequences composed of the amino acids Pro, Ala, and/or Ser), and a fragment of any of the foregoing, but are not limited thereto. In some embodiments, the half-life extending domain may extend the half-life of the stefin A protein variant of the present invention circulating in the serum of the subject, compared to the half-life of the fusion protein not comprising it.

In some embodiments, the half-life is extended by greater than or greater than about 1.2-fold, 1.5-fold, 2.0-fold, 3.0-fold, 4.0-fold., 5.0-fold, or 6.0-fold, but is not limited thereto. In some embodiments, the half-life is extended by more than 6 hours, more than 12 hours, more than 24 hours, more than 48 hours, more than 72 hours, more than 96 hours or more than 1 week after in vivo administration compared to the protein without the half-life extending moiety, but is not limited thereto.

In some embodiments, examples of preparation of the fusion protein further comprising the half-life extension domain are as follows, but are not limited thereto:
- Genetic fusion of the stefin A protein variant sequence to a naturally long-half-life protein or protein domain (e.g., Fc fusion, transferrin [Tf] fusion, or albumin fusion. See, for example, Beck et al. (2011) "Therapeutic Fc-fusion proteins and peptides as successful alternatives to antibodies. MAbs. 3:1-2; Czajkowsky et al. (2012) "Fc-fusion proteins: new developments and future perspectives. EMBO Mol Med. 4:1015-28; Huang et al. (2009) "Receptor-Fc fusion therapeutics, traps, and Mimetibody technology" Curr Opin Biotechnol. 2009;20:692-9; Keefe et al. (2013) "Transferrin fusion protein therapies: acetylcholine receptor-transferrin fusion protein as a model. In: Schmidt S, editor. Fusion protein technologies for biopharmaceuticals: applications and challenges. Hoboken: Wiley; p. 345-56; Weimer et al. (2013) "Recombinant albumin fusion proteins. In: Schmidt S, editor. Fusion protein technologies for biopharmaceuticals: applications and challenges. Hoboken: Wiley; 2013. p. 297-323; Walker et al. (2013) "Albumin-binding fusion proteins in the development of novel long-acting therapeutics. In: Schmidt S, editor. Fusion protein technologies for biopharmaceuticals: applications and challenges. Hoboken: Wiley; 2013. p. 325-43.
- Genetic fusion of the stefin A protein variant to an inert polypeptide, e.g., XTEN (also known as recombinant PEG or "rPEG"), a homoamino acid polymer (HAP; HAPylation), a proline-alanine-serine polymer (PAS; PASylation), or an elastin-like peptide (ELP; ELPylation). See, for example, Schellenberger et al. (2009) "A recombinant polypeptide extends the in vivo half-life of peptides and proteins in a tunable manner. Nat Biotechnol. 2009;27:1186-90; Schlapschy et al. Fusion of a recombinant antibody fragment with a homo-amino-acid polymer: effects on biophysical properties and prolonged plasma half-life. Protein Eng Des Sel. 2007;20:273-84; Schlapschy (2013) PASylation: a biological alternative to PEGylation for extending the plasma halflife of pharmaceutically active proteins. Protein Eng Des Sel. 26:489-501. Floss et al. (2012) "Elastin-like polypeptides revolutionize recombinant protein expression and their biomedical application. Trends Biotechnol. 28:37-45. Floss et al. "ELP-fusion technology for biopharmaceuticals. In: Schmidt S, editor. Fusion protein technologies for biopharmaceuticals: application and challenges. Hoboken: Wiley; 2013. p. 372-98.
- Increasing the hydrodynamic radius by chemical conjugation of the pharmacologically active peptide or protein to repeat chemical moieties, e.g., to PEG (PEGylation) or hyaluronic acid. See, for example, Caliceti et al. (2003) "Pharmacokinetic and biodistribution properties of polyethylene glycol)-protein conjugates" Adv Drug Delivery Rev. 55:1261-77; Jevsevar et al. (2010) PEGylation of therapeutic proteins. Biotechnol J 5:113-28; Kontermann (2009) "Strategies to extend plasma half-lives of recombinant antibodies" BioDrugs. 23:93-109; Kang et al. (2009) "Emerging PEGylated drugs" Expert Opin Emerg Drugs. 14:363-80; and Mero et al. (2013) "Conjugation of hyaluronan to proteins" Carb Polymers. 92:2163-70.
- Significantly increasing the negative charge of fusing the pharmacologically active peptide or protein by polysialylation; or, alternatively, (b) fusing a negatively charged, highly sialylated peptide (e.g., carboxy-terminal peptide [CTP; of chorionic gonadotropin (CG) b-chain]), known to extend the half-life of natural proteins such as human CG b-subunit, to the biological drug candidate. See, for example, Gregoriadis et al. (2005) "Improving the therapeutic efficacy of peptides and proteins: a role for polysialic acids" Int J Pharm. 2005; 300: 125-30; Duijkers et al. "Single dose pharmacokinetics and effects on follicular growth and serum hormones of a long-acting recombinant FSH preparation (FSHCTP) in healthy pituitary-suppressed females" (2002) Hum Reprod. 17:1987-93; and Fares et al. "Design of a longacting follitropin agonist by fusing the C-terminal sequence of the chorionic gonadotropin beta subunit to the follitropin beta subunit" (1992) Proc Natl Acad Sci USA. 89:4304-8. 35; and Fares "Half-life extension through O-glycosylation.
- Binding non-covalently, via attachment of a peptide or protein-binding domain to the bioactive protein, to normally long-half-life proteins such as HSA, human IgG, transferrin or fibronectin. See, for example, Andersen et al. (2011) "Extending half-life by indirect targeting of the neonatal Fc receptor (FcRn) using a minimal albumin binding domain" J Biol Chem. 286:5234-41; O'Connor-Semmes et al. (2014) "GSK2374697, a novel albumin-binding domain antibody (albudAb), extends systemic exposure of extendin-4: first study in humans-PK/PD and safety" Clin Pharmacol Ther. 2014;96:704-12. Sockolosky et al. (2014) "Fusion of a short peptide that binds immunoglobulin G to a recombinant protein substantially increases its plasma half-life in mice" PLoS One. 2014;9:e102566.

Classical genetic fusions to long-lived serum proteins offer an alternative method of half-life extension distinct from chemical conjugation to PEG or lipids. Two major proteins have traditionally been used as fusion partners: antibody Fc domains and human serum albumin (HSA). Fc fusions involve the fusion of peptides, proteins or receptor exodomains to the Fc portion of an antibody. Both Fc and albumin fusions achieve extended half-lives not only by increasing the size of the peptide drug, but both also take advantage of the body's natural recycling mechanism: the neonatal Fc receptor, FcRn. The pH-dependent binding of these proteins to FcRn prevents degradation of the fusion protein in the endosome. Fusions based on these proteins can have half-lives in the range of 3-16 days, much longer than typical PEGylated or lipidated peptides. Fusion to antibody Fc domains can improve the solubility and stability of the peptide or protein drug. An example of a peptide Fc fusion is dulaglutide, a GLP-1 receptor agonist currently in late-stage clinical trials. Human serum albumin, the same protein exploited by the fatty acylated peptides is the other popular fusion domain. The main difference between the Fc domain and albumin is that Fc is generally used in dimerization, and HSA is used in monomer structure. The fusion protein of the stefin A protein variant and the Fc domain of the present invention may be used as a dimer, but is not limited thereto.

### Fc domain Fusion

In some embodiments, the fusion protein may include an immunoglobulin Fc domain ("Fc domain"), or a fragment or variant thereof, such as a functional Fc region. In some embodiments, the Fc region is a FcyR null-binding Fc region. In some embodiments, the fusion protein may comprise at least one stefin A protein variant specifically binding to CD40L linked through a peptide backbone either directly or indirectly, to an Fc region of an immunoglobulin. In the present invention, the fusion protein may comprise the Fc region of an antibody, which facilitates effector functions and pharmacokinetics, and the stefin A protein variant specifically binding to CD40L as part of the same peptide. An immunoglobulin Fc region may also be linked indirectly via a linker to at least one Stefin A protein variant specifically binding to CD40L. Various linkers for use in the fusion protein of the present invention are well-known in the art. In the present invention, the fusion protein comprising an Fc domain may be used as a dimer such as a homodimer or a heterodimer.

In some embodiments, When the fusion protein includes an Fc domain, stability may be improved, and antibody-like properties conferred by the Fc region may be used. In some embodiments, When the fusion protein includes an Fc domain, The Fc domain is the salvage neonatal FcRn receptor pathway involving FcRn- mediated recycling of the fusion protein to the cell surface post endocytosis, avoiding lysosomal degradation and resulting in release back into the bloodstream, thus contributing to an extended serum half-life. The fusion of the Fc domain may be usefully used not only to extend the half-life, but also to isolate and purify the fusion protein of the present invention.

In some embodiments, the Fc domain may include the constant region of an antibody excluding the first constant region immunoglobulin domain.

In some embodiments, the Fc domain may comprises the last two constant region immunoglobulin domains of IgA, IgD, and IgG, and the flexible hinge N-terminal to these domains. For IgA and IgM Fc may include the J chain. For IgG, Fc comprises immunoglobulin domains Cγ2 and Cγ3 and the hinge between Cγ1 and Cγ2. Although the boundaries of the Fc domain may vary, the human IgG heavy chain Fc region is usually defined to comprise residues C226 or P230 to its carboxyl-terminus, wherein the numbering is according to the EU index as set forth in Kabat (Kabat et al., Sequences of Proteins of Immunological Interest, 5th Ed. Public Health Service, NIH, Bethesda, Md. (1991)). Fc domain may refer to this region in isolation, or this region in the context of a whole antibody, antibody fragment, or Fc fusion protein. Polymorphisms have been observed at a number of different Fc positions and are also included as fusion domains as used herein.

In some embodiments, The Fc domain isa "functional Fc region". The functional Fc regionrefers to an Fc domain or fragment thereof which retains the ability to bind FcRn. A functional Fc region binds to FcRn but does not possess effector function. The ability of the Fc region or fragment thereof to bind to FcRn can be determined by standard binding assays known in the art. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (e.g., B cell receptor; BCR), etc. Such effector functions can be assessed using various assays known in the art for evaluating such antibody effector functions.

In an exemplary embodiment, the Fc domain is derived from an IgG1 subclass, however, other subclasses (e.g., IgG2, IgG3, and IgG4) may also be used. An exemplary sequence of a human IgG1 immunoglobulin Fc domain which can be used is:

DKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQ
GNVFSCSVMHEALHNHYTQKSLSLSPGK (SEQ ID NO: 535)

In some embodiments, the Fc region used in the fusion protein may comprise the hinge region of an Fc molecule. An exemplary hinge region comprises the core hinge residues spanning positions 1-16 (e.g., DKTHTCPPCPAPELLG ((SEQ ID NO: 536)) of the exemplary human IgG1 immunoglobulin Fc domain sequence provided above. In some embodiments, the fusion protein may adopt a multimeric structure (e.g., dimer) owing, in part, to the cysteine residues at positions 6 and 9 within the hinge region of the exemplary human IgG1 immunoglobulin Fc domain sequence provided above. In other embodiments, the hinge region as used herein, may further include residues derived from the CH1 and CH2 regions that flank the core hinge sequence of the exemplary human IgG1 immunoglobulin Fc domain sequence provided above. In yet other embodiments, the hinge sequence may comprise or consist of GSTHTCPPCPAPELLG (SEQ ID NO: 537) or EPKSCDKTHTCPPCPAPELLG (SEQ ID NO: 538).

In some embodiments, the hinge region may include at least one substitution that confer desirable pharmacokinetic, biophysical, and/or biological properties. Some exemplary hinge sequences comprise or consist of:
EPKSCDKTHTCPPCPAPELLGGPS (SEQ ID NO: 539);
EPKSSDKTHTCPPCPAPELLGGPS (SEQ ID NO: 540);
EPKSSDKTHTCPPCPAPELLGGSS (SEQ ID NO: 541);
EPKSSGSTHTCPPCPAPELLGGSS (SEQ ID NO: 542);
DKTHTCPPCPAPELLGGPS (SEQ ID NO: 543); and
DKTHTCPPCPAPELLGGSS (SEQ ID NO: 544).

In some embodiments, the residue P at position 18 of the exemplary human IgG1 immunoglobulin Fc domain sequence provided above may be replaced with S to ablate Fc effector function; this replacement is exemplified in hinges having the sequences EPKSSDKTHTCPPCPAPELLGGSS (SEQ ID NO: 541), EPKSSGSTHTCPPCPAPELLGGSS (SEQ ID NO: 542), and DKTHTCPPCPAPELLGGSS (SEQ ID NO: 544).

In another embodiment, the residues DK at positions 1-2 of the exemplary human IgG1 immunoglobulin Fc domain sequence provided above may be replaced with GS to remove a potential clip site; this replacement is exemplified in the sequence EPKSSGSTHTCPPCPAPELLGGSS (SEQ ID NO: 542).

In another embodiment, the C at the position 103 of the heavy chain constant region of human IgG1 (e.g., domains CH₁-CH₃), may be replaced with S to prevent improper cysteine bond formation in the absence of a light chain; this replacement is exemplified by EPKSSDKTHTCPPCPAPELLGGPS (SEQ ID NO: 540), EPKSSDKTHTCPPCPAPELLGGSS (SEQ ID NO: 541), and EPKSSGSTHTCPPCPAPELLGGSS (SEQ ID NO: 542).

In some embodiments, the Fc is a mammalian Fc such as a human Fc, including Fc domains derived from IgG1, IgG2, IgG3 or IgG4. The Fc region may possess at least about 80%, 85%, 90%, 95%, 96%, 97%, 98%, or 99% sequence identity with a native Fc region and/or with an Fc region of a parent polypeptide. In some embodiments, the Fc region may have at least about 90% sequence identity with a native Fc region and/or with an Fc region of a parent polypeptide.

In some embodiments, the Fc domain comprises an amino acid sequence selected from SEQ ID NOs: 545-558 or an Fc sequence from the examples provided by SEQ ID NOs: 545-558. It should be understood that the C-terminal lysine of an Fc domain is an optional component of a fusion protein comprising an Fc domain. In some embodiments, the Fc domain comprises an amino acid sequence selected from SEQ ID NOs: 545-558, except that the C-terminal lysine thereof is omitted. In some embodiments, the Fc domain comprises the amino acid sequence selected from SEQ ID NOs: 545-558. In some embodiments, the Fc domain comprises the amino acid sequence selected from SEQ ID NOs: 545-558 except the C-terminal lysine thereof is omitted.

**Table 7. Exemplary Immunoglobulin Sequences**

| **Name** | **Sequence** | **SEQ ID NO:** |
|---|---|---|
| hIgG1a_191 | DKTHTCPPCPAPELLGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTIS | 545 |
| [A subtype] | | |
| | KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | |
| hIgG1a_189 | DKTHTCPPCPAPELLGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSP | 546 |
| [hIgG1a_191 sans "GK" on C term; A subtype] | | |
| hIgG1a_191b | DKTHTCPPCPAPELLGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSRDEMTKNQVSLTCLVKGFYPSD IAVEWESNGQPENNYKTTPPVLDSDGSFFLYSKLTVDKS RWQQGNVFSCSVMHEALHNHYTQKSLSLSPGK | 547 |
| [A/F subtype] | | |
| hIgG1f_1.1_191 | DKTHTCPPCPAPEAEGAPSVFLFPPKPKDTLMISRTPEV TCVVVDVSHEDPEVKFNWYVDGVEVHNAKTKPREEQYNS TYRVVSVLTVLHQDWLNGKEYKCKVSNKALPSSIEKTIS KAKGQPREPQVYTLPPSREEMTKNQVSLTCLVKGFYPSD IAVEWESNGQPENNYKTTPPVLDSDGSFFLYSKLTVDKS RWQQGNVFSCSVMHEALHNHYTQKSLSLSPGK | 548 |
| [Contains five point-mutations to alter ADCC function, F subtype] | | |
| hIgGIf_1.1_186 | EPKSSDKTHTCPPCPAPEAEGAPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPSSI EKTISKAKGQPREPQVYTLPPSREEMTKNQVSLTCLVKG FYPSDIAVEWESNGQPENNYKTTPPVLDSDGSFFLYSKL TVDKSRWQQGNVFSCSVMHEALHNHYTQKSLSLSPGK | 549 |
| [Contains five point-mutations to alter ADCC function and C225S (Edlemen numbering); F subtype] | | |
| hIgG1a_(N297G)_191 | DKTHTCPPCPAPELLGGPSVFLFPPKPKDTLMISRTPEV TCVVVDVSHEDPEVKFNWYVDGVEVHNAKTKPREEQYGS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | 550 |
| [A subtype] | | |
| hIgG1a_190 | DKTHTCPPCPAPELLGGPSVFLFPPKPKDTLMISRTPEV TCVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPG | 551 |
| [hIgG1a_190 sans "K" on C term; A subtype] | | |
| hIgG1a_(N297Q)_191 | DKTHTCPPCPAPELLGGPSVFLFPPKPKDTLMISRTPEV TCVVVDVSHEDPEVKFNWYVDGVEVHNAKTKPREEQYQS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | 552 |
| [A subtype] | | |
| hIgG1a_(N297S)_191 | DKTHTCPPCPAPELLGGPSVFLFPPKPKDTLMISRTPEV TCVVVDVSHEDPEVKFNWYVDGVEVHNAKTKPREEQYSS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | 553 |
| [A subtype] | | |
| hIgG1a_(N297A)_191 | DKTHTCPPCPAPELLGGPSVFLFPPKPKDTLMISRTPEV TCVVVDVSHEDPEVKFNWYVDGVEVHNAKTKPREEQYAS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | 554 |
| [A subtype] | | |
| hIgG1a_(N297H)_191 | DKTHTCPPCPAPELLGGPSVFLFPPKPKDTLMISRTPEV TCVVVDVSHEDPEVKFNWYVDGVEVHNAKTKPREEQYHS TYRVVSVLTVLHQDWLNGKEYKCKVSNKALPAPIEKTIS KAKGQPREPQVYTLPPSRDELTKNQVSLTCLVKGFYPSD IAVEWESNGQPENNYKTTPPVLDSDGSFFLYSKLTVDKS RWQQGNVFSCSVMHEALHNHYTQKSLSLSPGK | 555 |
| [A subtype] | | |
| hIgG4 | DKRVESKYGPPCPSCPAPEFLGGPSVFLFPPKPKDTLMI SRTPEVTCVVVDVSQEDPEVQFNWYVDGVEVHNAKTKPR EEQFNSTYRVVSVLTVLHQDWLNGKEYKCKVSNKGLPSS IEKTISKAKGQPREPQVYTLPPSQEEMTKNQVSLTCLVK GFYPSDIAVEWESNGQPENNYKTTPPVLDSDGSFFLYSR LTVDKSRWQEGNVFSCSVMHEALHNHYTQKSLSLSLGK | 556 |
| hIgG4_(S241P) | DKRVESKYGPPCPPCPAPEFLGGPSVFLFPPKPKDTLMI SRTPEVTCVVVDVSQEDPEVQFNWYVDGVEVHNAKTKPR EEQFNSTYRVVSVLTVLHQDWLNGKEYKCKVSNKGLPSS IEKTISKAKGQPREPQVYTLPPSQEEMTKNQVSLTCLVK GFYPSDIAVEWESNGQPENNYKTTPPVLDSDGSFFLYSR LTVDKSRWQEGNVFSCSVMHEALHNHYTQKSLSLSLGK | 557 |
| hIgG1 (Contain two point-mutations to alter ADCC function L20A, L21A) | SEPKSCDKTHTCPPCPAPEAAGGPSVFLFPPKPKDTLMI SRTPEVTCVVVDVSHEDPEVKFNWYVDGVEVHNAKTKPR EEQYNSTYRVVSVLTVLHQDWLNGKEYKCKVSNKALPAP IEKTISKAKGQPREPQVYTLPPSREEMTKNQVSLTCLVK GFYPSDIAVEWESNGQPENNYKTTPPVLDSDGSFFLYSK LTVDKSRWQQGNVFSCSVMHEALHNHYTQKSLSLSPGK | 558 |

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enables these cytotoxic effector cells to bind specifically to an antigen-bearing target cell and subsequently kill the target cell with cytotoxins.

In some embodiments, the fusion protein includes an Fc domain sequence having no (or reduced) ADCC and/or complement activation or effector functionality. For example, the Fc domain may comprise a naturally disabled constant region of IgG2 or IgG4 isotype or a mutated IgG1 constant region. Examples of suitable modifications are described in EP0307434. One example comprises the substitutions of alanine residues at positions 235 and 237 (EU index numbering).

In other embodiments, the fusion protein includes an Fc domain which retain some or all Fc functionality for example will be capable of one or both of ADCC and CDC activity, as for example if the fusion protein comprises the Fc domain from human IgG1 or IgG3. Levels of effector function can be varied according to known techniques, for example by mutations in the CH2 domain, for example wherein the IgG1 CH2 domain has at least one mutation at positions selected from 239 and 332 and 330, for example the mutations are one or more selected from S239D and I332E and A330L. In some embodiments, the Fc domain is altered the glycosylation profile such that there is a reduction in fucosylation of the Fc region.

### Albumin Fusions

In some embodiments, the fusion protein comprises an albumin sequence or an albumin fragment. In other embodiments, the albumin sequence or an albumin fragment fused or conjugated through chemical linkage other than incorporation into the polypeptide sequence including the stefin A protein variant. In some embodiments, the albumin, albumin variant, or albumin fragment is human serum albumin (HSA), a human serum albumin variant, or a human serum albumin fragment. Albumin serum proteins comparable to HSA are found in, for example, cynomolgus monkeys, cows, dogs, rabbits and rats. Of the non-human species, bovine serum albumin (BSA) is the most structurally similar to HSA. See, e.g., Kosa et al., (2007) J Pharm Sci. 96(11):3117-24. The present disclosure contemplates the use of albumin from non-human species, including, but not limited to, albumin sequence derived from cyno serum albumin or bovine serum albumin.

Mature HSA, a 585 amino acid polypeptide (approx. 67 kDa) having a serum half-life of about 20 days, is primarily responsible for the maintenance of colloidal osmotic blood pressure, blood pH, and transport and distribution of numerous endogenous and exogenous ligands. The protein has three structurally homologous domains (domains I, II and III), is almost entirely in the alpha-helical conformation, and is highly stabilized by 17 disulfide bridges. In some embodiments, the fusion protein can comprise an albumin fusion protein including at least one stefin A protein variant and the sequence for mature human serum albumin (SEQ ID NO: 559) or a variant or fragment thereof which maintains the PK and/or biodistribution properties of mature albumin to the extent desired in the fusion protein.














(SEQ ID NO: 559)

The albumin sequence can be set off from the stefin A protein variant sequence or other flanking sequences in the fusion protein by use of linker sequences as described above.

While unless otherwise indicated, reference herein to "albumin" or to "mature albumin" is meant to refer to HSA. However, it is noted that full-length HSA comprises a signal peptide of 18 amino acids (MKWVTFISLLFLFSSAYS (SEQ ID NO: 486) followed by a pro-domain of 6 amino acids (RGVFRR) (SEQ ID NO: 560); this 24 amino acid residue peptide may be referred to as the pre-pro domain. The fusion proteins comprising stefin A protein variant and HSA can be expressed and secreted using the HSA pre-pro-domain in the recombinant proteins coding sequence. Alternatively, the fusion proteins comprising stefin A protein variant and HSA can be expressed and secreted through inclusion of other secretion signal sequences, such as described above.

In alternative embodiments, the serum albumin, for example, may be covalently coupled to the stefin A protein variant or a fusion protein comprising the same by a bond other than an amide bond such as cross-linked through chemical conjugation between amino acid sidechains on each of the stefin A protein variant or a fusion protein and the albumin.

### Serum binding domains

In some embodiments, the fusion protein can include a serum-binding domain(or moiety). In some embodiments, the serum-binding domain is comprised either as part of a fusion protein (if also a polypeptide) or chemically conjugated through a site other than being part of a contiguous polypeptide chain.

In some embodiments, the serum-binding domain is an albumin binding domain. Albumin contains multiple hydrophobic binding pockets and naturally serves as a transporter of a variety of different ligands such as fatty acids and steroids as well as different drugs. Furthermore, the surface of albumin is negatively charged making it highly water-soluble.

The term "albumin binding domain" as used herein refers to any chemical group capable of binding to albumin, e.g., has albumin binding affinity. Albumin binds to endogenous ligands such as fatty acids; however, it also interacts with exogenous ligands such as warfarin, penicillin and diazepam. As the binding of these drugs to albumin is reversible the albumin-drug complex serves as a drug reservoir that can enhance the drug biodistribution and bioavailability. Incorporation of components that mimic endogenous albumin-binding ligands, such as fatty acids, has been used to potentiate albumin association and increase drug efficacy.

In some embodiments, a chemical modification method that can be applied in the generation of the subject fusion protein to increase protein half-life is lipidation, which involves the covalent binding of fatty acids to peptide side chains. Originally conceived of and developed as a method for extending the half-life of insulin, lipidation shares the same basic mechanism of half-life extension as PEGylation, namely increasing the hydrodynamic radius to reduce renal filtration. However, the lipid moiety is itself relatively small and the effect is mediated indirectly through the non-covalent binding of the lipid moiety to circulating albumin. One consequence of lipidation is that it reduces the water-solubility of the peptide but engineering of the linker between the peptide and the fatty acid can modulate this . Linker engineering and variation of the lipid moeity can affect self-aggregation which can contribute to increased half-life by slowing down biodistribution, independent of albumin. See, for example, Jonassen et al. (2012) Pharm Res. 29(8):2104-14.

In some embodiments, albumin binding moieties may be albumin-binding (PKE2) adnectins (See WO2011140086 "Serum Albumin Binding Molecules", WO2015143199 "Serum albumin-binding Fibronectin Type III Domains" and WO2017053617 "Fast-off rate serum albumin binding fibronectin type iii domains"), the albumin binding domain 3 (ABD3) of protein G of Streptococcus strain G148, and the albumin binding domain antibody GSK2374697 ("AlbudAb") or albumin binding nanobody portion of ATN-103 (Ozoralizumab).

### AFFIMER^{®} XT

In some embodiments, the fusion protein may include a stefin A protein variant capable of binding to serum protein. In some embodiments, the fusion protein includes a stefin A protein variant binding to HSA.

In some embodiments, at least one of the solvent accessible loops of "a stefin A protein variant binding to HSA (HSA AFFIMER^{®})" derived from a wild-type stefin A protein capable of binding to HSA. In some embodiments, the stefin A protein variant binding to HSA binds to HSA with a Kd of 10⁻⁶M or less.

In some embodiments, the stefin A protein variant binding to HSA with a K_{d} of 1×10⁻⁹ M to 1×10⁻⁶ M at pH 7.4 to 7.6. In some embodiments, the stefin A protein variant binding to HSA with a K_{d} of 1×10⁻⁶ M or less at pH 7.4 to 7.6. In some embodiments, the stefin A protein variant binding to HSA with a K_{d} of 1×10⁻⁷ M or less at pH 7.4 to 7.6. In some embodiments, the stefin A protein variant binding to HSAthe stefin A protein variant binding to HSAwith a K_{d} of 1×10⁻⁸ M or less at pH 7.4 to 7.6. In some embodiments, the stefin A protein variant binding to HSAwith a K_{d} of 1×10⁻⁹ M or less at pH 7.4 to 7.6. In some embodiments, the stefin A protein variant binding to HSAwith a K_{d} of 1×10⁻¹⁰ M or less at pH 7.4 to 7.6. In some embodiments, the stefin A protein variant binding to HSAwith a K_{d} of 1×10⁻¹¹ M or less at pH 7.4 to 7.6. In some embodiments, the stefin A protein variant binding to HSAwith a K_{d} of 1×10⁻⁹ M to 1×10⁻⁶ M at pH 7.4. In some embodiments, the stefin A protein variant binding to HSAwith a K_{d} of 1×10⁻⁶ M or less at pH 7.4. In some embodiments, the stefin A protein variant binding to HSAwith a K_{d} of 1×10⁻⁷ M or less at pH 7.4. In some embodiments, the stefin A protein variant binding to HSAwith a K_{d} of 1x10⁻⁸ M or less at pH 7.4. In some embodiments, the stefin A protein variant binding to HSAwith a K_{d} of 1×10⁻⁹ M or less at pH 7.4. In some embodiments, the stefin A protein variant binding to HSAwith a K_{d} of 1×10⁻¹⁰ M or less at pH 7.4. In some embodiments, the stefin A protein variant binding to HSAwith a K_{d} of 1×10⁻¹¹ M or less at pH 7.4.

In some embodiments, the stefin A protein variant binding to HSAmay be the wild-type human Stefin A protein having a backbone sequence and in which one or both of loop 2 (designated (Xaa)ₙ) and loop 4 (designated (Xaa)ₘ) are replaced with alternative loop sequences (Xaa)ₙ and (Xaa)ₘ.

In some embodiments, the stefin A protein variant binding to HSA may comprises an amino acide sequence represented in the general Formula (I):

FR1-(Xaa)n-FR2-(Xaa)m-FR3 (I),

wherein FR1 is an amino acid sequence having at least 70% (e.g., at least 75%, at least 80%, at least 85%, at least 90%, at least 95%, or 100%) identity to MIPGGLSEAK PATPEIQEIV DKVKPQLEEK TNETYGKLEA VQYKTQVLA (SEQ ID NO: 1A); FR2 is an amino acid sequence having at least 70% (e.g., at least 75%, at least 80%, at least 85%, at least 90%, at least 95%, or 100%) identity to GTNYYIKVRA GDNKYMHLKV FKSL (SEQ ID NO: 2); FR3 is an amino acid sequence having at least 70% (e.g., at least 75%, at least 80%, at least 85%, at least 90%, at least 95%, or 100%) identity to EDLVLTGYQV DKNKDDELTG F (SEQ ID NO: 3); Xaa, individually for each occurrence, is an amino acid; and n is an integer from 3 to 20, and m is an integer from 3 to 20.

In some embodiments, FR1 is a polypeptide sequence having 80%-98%, 82%-98%, 84%-98%, 86%-98%, 88%-98%, 90%-98%, 92%-98%, 94%-98%, or 96%-98% homology with SEQ ID NO: 1A. In some embodiments, FR1 is a polypeptide sequence having 80%, 82%, 84%, 86%, 88%, 90%, 92%, 94%, 96%, or 95% homology with SEQ ID NO: 1A. In some embodiments, FR1 is the polypeptide sequence of SEQ ID NO: 1A. In some embodiments, FR2 is a polypeptide sequence having at least 80%-96%, 84%-96%, 88%-96%, or 92%-96% homology with SEQ ID NO: 2. In some embodiments, FR2 is a polypeptide sequence having at least 80%, 84%, 88%, 92%, or 96% homology with SEQ ID NO: 2. In some embodiments, FR2 is a polypeptide sequence having at least 80%, 85%, 90%, 95% or even 98% identity with SEQ ID NO: 2. In some embodiments, FR2 is the polypeptide sequence of SEQ ID NO: 2. In some embodiments, FR3 is a polypeptide sequence having at least 80%-95%, 85%-95%, or 90%-95% homology with SEQ ID NO: 3. In some embodiments, FR3 is a polypeptide sequence having at least 80%, 85%, 90%, or 95% homology with SEQ ID NO: 3. In some embodiments, FR3 is the polypeptide sequence of SEQ ID NO: 3.

In some embodiments, the stefin A protein variant binding to HSA comprises an amino acid sequence represented in the general Formula (II) (SEQ ID NO: 4): 
MIP-Xaa1-GLSEAKPATPEIQEIVDKVKPQLEEKTGETYGKLEAVQYKTQVV-
(Xaa)n-Xaa2-TNYYIKVRAGDNKYMHLKVF-Xaa3-Xaa4-Xaa5-(Xaa)m-Xaa6-D-Xaa7-
VLTGYQVDKNKDDELTGF(SEQ ID NO: 4)
wherein Xaa, individually for each occurrence, is an amino acid residue, and
n and m are each, independently, an integer from 3-20.

In some embodiments, the stefin A protein variant binding to HSA comprises the amino acid sequence represented in general Formula (III) (SEQ ID NO: 5): 
MIPGGLSEAKPATPEIQEIVDKVKPQLEEKTGETYGKLEAVQYKTQVD-(Xaa)n-
GTNYYIKVRAGDNKYMHLKVFKSL-(Xaa)m-
EDLVLTGYQVDKNKDDELTGF(SEQ ID NO: 5),
wherein Xaa, individually for each occurrence, is an amino acid residue, and
n and m are each, independently, an integer from 3-20.

In some embodiments, Xaa1 is Gly, Ala, Val, Arg, Lys, Asp, or Glu; Xaa2 is Gly, Ala, Val, Ser or Thr; Xaa3 is Arg, Lys, Asn, Gln, Ser, Thr; Xaa4 is Gly, Ala, Val, Ser or Thr; Xaa5 is Ala, Val, Ile, Leu, Gly or Pro; Xaa6 is Gly, Ala, Val, Asp or Glu; and Xaa7 is Ala, Val, Ile, Leu, Arg or Lys.

In some embodiments, Xaa1 is Gly, Ala, Arg or Lys. In some embodiments, Xaa1 is Gly or Arg. In some embodiments, Xaa2 is Gly, Ala, Val, Ser or Thr. In some embodiments, Xaa2 is Gly or Ser. In some embodiments, Xaa3 is Arg, Lys, Asn, Gln, Ser, Thr. In some embodiments, Xaa3 is Arg, Lys, Asn or Gln. In some embodiments, Xaa3 is Lys or Asn. In some embodiments, Xaa4 is Gly, Ala, Val, Ser or Thr. In some embodiments, Xaa4 is Gly or Ser. In some embodiments, Xaa5 is Ala, Val, Ile, Leu, Gly or Pro. In some embodiments, Xaa5 is Ile, Leu or Pro. In some embodiments, Xaa5 is Leu or Pro. In some embodiments, Xaa6 is Gly, Ala, Val, Asp or Glu. In some embodiments, Xaa6 is Ala, Val, Asp or Glu. In some embodiments, Xaa6 is Ala or Glu. In some embodiments, Xaa7 is Ala, Val, Ile, Leu, Arg or Lys. In some embodiments, Xaa7 is Ile, Leu or Arg. In some embodiments, Xaa7 is Leu or Arg.

In some embodiments, n is 3, 4, 5, 6, 7, 8, 9, 10, 11, 12, 13, 14, 15, 16, 17, 18, 19, or 20. In some embodiments, n is 8 to 10, 7 to 11, 6 to 12, 5 to 13, 4 to 14, or 3 to 15. In some embodiments, m is 3, 4, 5, 6, 7, 8, 9, 10, 11, 12, 13, 14, 15, 16, 17, 18, 19, or 20. In some embodiments, m is 8 to 10, 7 to 11, 6 to 12, 5 to 13, 4 to 14, or 3 to 15.

In some embodiments, (Xaa)ₙ is represented by Formula (IV):

aa1-aa2-aa3-aa4-aa5-aa6-aa7-aa8-aa9 (IV),

wherein aa1 is an amino acid with a neutral polar hydrophilic side chain; aa2 is an amino acid with a neutral nonpolar hydrophobic side chain; aa3 is an amino acid with a neutral nonpolar hydrophobic side chain; aa4 is an amino acid with a neutral polar hydrophilic side chain; aa5 is an amino acid with a positively charged polar hydrophilic side chain; aa6 is an amino acid with a positively charged polar hydrophilic side chain; aa7 is an amino acid with a neutral nonpolar hydrophobic side chain; aa8 is an amino acid with a neutral nonpolar hydrophobic side chain; and aa9 is an amino acid with a neutral nonpolar hydrophilic side chain.

In some embodiments, (Xaa)ₘ is represented by formula (V):

aa1-aa2-aa3-aa4-aa5-aa6-aa7-aa8-aa9 (V),

wherein aa1 is an amino acid with a neutral nonpolar hydrophobic side chain; aa2 is an amino acid with a positively charged polar hydrophilic side chain; aa3 is an amino acid with a neutral nonpolar hydrophobic side chain; aa4 is an amino acid with a positively charged polar hydrophilic side chain; aa5 is an amino acid with a neutral polar hydrophilic side chain; aa6 is an amino acid with a neutral polar hydrophilic side chain; aa7 is an amino acid with a negatively charged polar hydrophilic side chain; aa8 is an amino acid with a positively charged polar hydrophilic side chain; and aa9 is an amino acid with a neutral nonpolar hydrophilic side chain.

Examples of amino acids with a neutral nonpolar hydrophilic side chain include cysteine (Cys) and glycine (Gly). In some embodiments, the amino acid with a neutral nonpolar hydrophilic side chain is Cys. In some embodiments, the amino acid with a neutral nonpolar hydrophilic side chain is Gly.

Examples of amino acids with a neutral nonpolar hydrophobic side chain include alanine (Ala), isoleucine (Ile), leucine (Leu), methionine (Met), phenylalanine (Phe), proline (Pro), tryptophan (Trp), and valine (Val). In some embodiments, the amino acid with a neutral nonpolar hydrophobic side chain is Ala. In some embodiments, the amino acid with a neutral nonpolar hydrophobic side chain is Ile. In some embodiments, the amino acid with a neutral nonpolar hydrophobic side chain is Leu. In some embodiments, the amino acid with a neutral nonpolar hydrophobic side chain is Met. In some embodiments, the amino acid with a neutral nonpolar hydrophobic side chain is Phe. In some embodiments, the amino acid with a neutral nonpolar hydrophobic side chain is Pro. In some embodiments, the amino acid with a neutral nonpolar hydrophobic side chain is Trp. In some embodiments, the amino acid with a neutral nonpolar hydrophobic side chain is Val.

Examples of amino acids with a neutral polar hydrophilic side chain include asparagine (Asn), glutamine (Gln), serine (Ser), threonine (Thr), and tyrosine (Tyr). In some embodiments, the amino acid with a neutral polar hydrophilic side chain is Asn. In some embodiments, the amino acid with a neutral polar hydrophilic side chain is Gln. In some embodiments, the amino acid with a neutral polar hydrophilic side chain is Ser. In some embodiments, the amino acid with a neutral polar hydrophilic side chain is Thr. In some embodiments, the amino acid with a neutral polar hydrophilic side chain is Tyr.

Examples of amino acids with a positively charged polar hydrophilic side chain include arginine (Arg), histidine (His), and lysine (Lys). In some embodiments, the amino acid with a positively charged polar hydrophilic side is Arg. In some embodiments, the amino acid with a positively charged polar hydrophilic side is His. In some embodiments, the amino acid with a positively charged polar hydrophilic side is Lys.

Examples of amino acids with a negatively charged polar hydrophilic side chain include aspartate (Asp) and glutamate (Glu). In some embodiments, the amino acid with a negatively charged polar hydrophilic side chain is Asp. In some embodiments, the amino acid with a negatively charged polar hydrophilic side chain is Glu.

In some embodiments, (Xaa)ₙ is represented by Formula (IV):

aa1-aa2-aa3-aa4-aa5-aa6-aa7-aa8-aa9 (IV),

wherein aa1 is an amino acid selected from Asp, Gly, Asn, and Val; aa2 is an amino acid selected from Trp, Tyr, His, and Phe; aa3 is an amino acid selected from Trp, Tyr, Gly, Trp, and Phe; aa4 is an amino acid selected from Gln, Ala, and Pro; aa5 is an amino acid selected from Ala, Gln, Glu, Arg, and Ser; aa6 is an amino acid selected from Lys, Arg, and Tyr; aa7 is an amino acid selected from Trp and Gln; aa8 is an amino acid selected from Pro and His; and/or aa9 is an amino acid selected from His, Gly, and Gln. In some embodiments, aa1 is Asp. In some embodiments, aa1 is Gly. In some embodiments, aa1 is Asn. In some embodiments, aa2 is Trp. In some embodiments, aa2 is Tyr. In some embodiments, aa2 is His. In some embodiments, aa2 is Phe. In some embodiments, aa3 is Trp. In some embodiments, aa3 is Tyr. In some embodiments, aa3 is Gly. In some embodiments, aa3 is Trp. In some embodiments, aa3 is Phe. In some embodiments, aa4 is Gln. In some embodiments, aa4 is Ala. In some embodiments, aa4 is Pro. In some embodiments, aa5 is Ala. In some embodiments, aa5 is Gln. In some embodiments, aa5 is Glu. In some embodiments, aa5 is Arg. In some embodiments, aa5 is Ser. In some embodiments, aa6 is Lys. In some embodiments, aa6 is Arg. In some embodiments, aa6 is Tyr. In some embodiments, aa7 is Trp. In some embodiments, aa7 is Gln. In some embodiments, aa8 is Pro. In some embodiments, aa8 is His. In some embodiments, aa9 is His. In some embodiments, aa9 is Gly. In some embodiments, aa9 is Gln.

In some embodiments, (Xaa)ₘ is represented by Formula (IV):

aa1-aa2-aa3-aa4-aa5-aa6-aa7-aa8-aa9 (IV),

wherein aa1 is an amino acid selected from Tyr, Phe, Trp, and Asn; aa2 is an amino acid selected from Lys, Pro, His, Ala, and Thr; aa3 is an amino acid selected from Val, Asn, Gly, Gln, Ala, and Phe; aa4 is an amino acid selected from His, Thr, Lys, Trp, Lys, Val, and Arg; aa5 is an amino acid selected from Gln, Ser, Gly, Pro, and Asn; aa6 is an amino acid selected from Ser, Tyr, Glu, Leu, Lys, and Thr; aa7 is an amino acid selected from Ser, Asp, Val, and Lys; aa8 is an amino acid selected from Gly, Leu, Ser, Pro, His, Asp, and Arg; and/or aa9 is an amino acid selected from Gly, Gln, Glu, and Ala. In some embodiments, aa1 is Tyr. In some embodiments, aa1 is Phe. In some embodiments, aa1 is Trp. In some embodiments, aa1 is Asn. In some embodiments, aa2 is Lys. In some embodiments, aa2 is Pro. In some embodiments, aa2 is His. In some embodiments, aa2 is Ala. In some embodiments, aa2 is Thr. In some embodiments, aa3 is Val. In some embodiments, aa3 is Asn. In some embodiments, aa3 is Gly. In some embodiments, aa3 is Gln. In some embodiments, aa3 is Ala. In some embodiments, aa3 is Phe. In some embodiments, aa4 is His. In some embodiments, aa4 is Thr. In some embodiments, aa4 is Lys. In some embodiments, aa4 is Trp. In some embodiments, aa4 is Lys. In some embodiments, aa4 is Val. In some embodiments, aa4 is Arg. In some embodiments, aa5 is Gln. In some embodiments, aa5 is Ser. In some embodiments, aa5 is Gly. In some embodiments, aa5 is Pro. In some embodiments, aa5 is Asn. In some embodiments, aa6 is Ser. In some embodiments, aa6 is Tyr. In some embodiments, aa6 is Glu. In some embodiments, aa6 is Leu. In some embodiments, aa6 is Lys. In some embodiments, aa6 is Thr. In some embodiments, aa7 is Ser. In some embodiments, aa7 is Asp. In some embodiments, aa7 is Val. In some embodiments, aa7 is Lys. In some embodiments, aa8 is Gly. In some embodiments, aa8 is Leu. In some embodiments, aa8 is Ser. In some embodiments, aa8 is Pro. In some embodiments, aa8 is His. In some embodiments, aa8 is Asp. In some embodiments, aa8 is Arg. In some embodiments, aa9 is Gly. In some embodiments, aa9 is Gln. In some embodiments, aa9 is Glu. In some embodiments, aa9 is Ala.

In some embodiments, (Xaa)ₙ is represented by Formula (V):

Asn-aa1-aa2-Gln-Gln-Arg-Arg-Trp-Pro-Gly (V),

wherein aa1 is an amino acid selected from Trp and Phe; and aa2 is an amino acid selected from Tyr and Phe. In some embodiments, aa1 is Trp. In some embodiments, aa1 is Phe. In some embodiments, aa2 is Tyr. In some embodiments, aa2 it Phe.

In some embodiments, (Xaa)ₙ is represented by Formula (VI):

aa1-aa2-Trp-aa3-aa4-Lys-Trp-Pro-aa5 (VI),

wherein aa1 is an amino acid selected from Asp and Gly; aa2 is an amino acid selected from Trp, Tyr, and Phe; aa3 is an amino acid selected from Gln and Ala; aa4 is an amino acid selected from Ala and Ser; and aa5 is an amino acid selected from His and Gly. In some embodiments, aa1 is Asp. In some embodiments, aa1 is Gly. In some embodiments, aa2 is Trp. In some embodiments, aa2 is Tyr. In some embodiments, aa2 is Phe. In some embodiments, aa3 is Gln. In some embodiments, aa3 is Ala. In some embodiments, aa4 is Ala. In some embodiments, aa4 is Ser. In some embodiments, aa5 is His. In some embodiments, aa5 is Gly.

In some embodiments, (Xaa)ₙ is represented by Formula (VII):

aa1-aa2-aa3-aa4-aa5-aa6-Trp-Pro-Gly (VII),

wherein aa1 is an amino acid selected from Gly and Asn; aa2 is an amino acid selected from Tyr, Phe, Trp, and His; aa3 is an amino acid selected from Trp, Tyr, and Phe; aa4 is an amino acid selected from Ala and Gln; aa5 is an amino acid selected from Ala, Ser, Gln, and Arg; and aa6 is an amino acid selected from Lys, Arg, and Tyr. In some embodiments, aa1 is Gly. In some embodiments, aa1 is Asn. In some embodiments, aa2 is Tyr. In some embodiments, aa2 is Phe. In some embodiments, aa2 is Trp. In some embodiments, aa2 is His. In some embodiments, aa3 is Trp. In some embodiments, aa3 is Tyr. In some embodiments, aa3 is Phe. In some embodiments, aa4 is Ala. In some embodiments, aa4 is Gln. In some embodiments, aa5 is Ala. In some embodiments, aa5 is Ser. In some embodiments, aa5 is Gln. In some embodiments, aa5 is Arg. In some embodiments, aa6 is Lys. In some embodiments, aa6 is Arg. In some embodiments, aa6 is Tyr.

In some embodiments, (Xaa)ₙ is represented by Formula (VIII):
Gly-aa1-aa2-Ala-aa3-aa4-Trp-Pro-Gly (VIII) (SEQ ID NO: 561),
wherein aa1 is an amino acid selected from Tyr, Phe, and His; aa2 is an amino acid selected from Trp and Tyr; aa3 is an amino acid selected from Ala, Ser, and Arg; and aa4 is an amino acid selected from Lys and Tyr. In some embodiments, aa1 is Tyr. In some embodiments, aa1 is Phe His. In some embodiments, aa1 is His. In some embodiments, aa2 is Trp. In some embodiments, aa2 is Tyr. In some embodiments, aa3 is Ala. In some embodiments, aa3 is Ser. In some embodiments, aa3 is Arg. In some embodiments, aa4 is Lys. In some embodiments, aa4 is Tyr.

In some embodiments, (Xaa)ₙ is represented by Formula (IX):

aa1-aa2-aa3-Gln-aa4-aa5-Trp-Pro-aa6 (IX),

wherein aa1 is an amino acid selected from Asp and Asn; aa2 is an amino acid selected from Trp and Phe; aa3 is an amino acid selected from Trp, Tyr, and Phe; aa4 is an amino acid selected from Ala, Gln, and Arg; aa5 is an amino acid selected from Lys and Arg; and aa6 is an amino acid selected from His and Gly. In some embodiments, aa1 is Asp. In some embodiments, aa1 is Asn. In some embodiments, aa2 is Trp. In some embodiments, aa2 is Phe. In some embodiments, aa3 is Trp. In some embodiments, aa3 is Tyr. In some embodiments, aa3 is Phe. In some embodiments, aa4 is Ala. In some embodiments, aa4 is Gln. In some embodiments, aa4 is Arg. In some embodiments, aa5 is Lys. In some embodiments, aa5 is Arg. In some embodiments, aa6 is His. In some embodiments, aa6 is Gly.

In some embodiments, the stefin A protein variant binding to HSA comprises a loop 2 amino acid sequence selected from any one of SEQ ID NOs: 562 to 614 **(Table 8).** In some embodiments, the stefin A protein variant binding to HSA comprises a loop 4 amino acid sequence selected from any one of SEQ ID NOs: 615 to 667 **(Table 8).**

**Table 8. Exemplary Loop Sequences of the stefin A protein variant binding to HSA**

| **Name** | **Loop 2** | **SEQ ID NO:** | **Loop 4** | **SEQ ID NO:** |
|---|---|---|---|---|
| HSA-00 | WTQPKNEHH | 562 | RFKYFAHYQ | 615 |
| HSA1 | HLKHTDAQP | 563 | FHDFWHRRW | 616 |
| HS-A2 | HDQDVLHAW | 564 | DWYHYWWEV | 617 |
| HSA-03 | KFHRQEWAD | 565 | STRSIHVTT | 618 |
| HSA-04 | PEDFWDPEH | 566 | KQHHHYLDK | 619 |
| HSA-05 | VVRTTGHVV | 567 | HSAQDREIP | 620 |
| HSA-06 | YWWFCTGQS | 568 | WVQSGYNSQ | 621 |
| HSA-07 | IHHRQARSL | 569 | AVFWGKWSD | 622 |
| HSA-08 | SHRRRAYIW | 570 | QSFDKPWTT | 623 |
| HSA-09 | WDSHHWRAP | 571 | HYPLKYSFE | 624 |
| HSA-10 | DKRVKYGQ | 572 | WHHPWHRNR | 625 |
| HSA-11 | SDWVYALQL | 573 | DPWWAWVVW | 626 |
| HSA-12 | FWWFWY | 574 | FDNQDLIQY | 627 |
| HSA-13 | VRDWPWNTF | 575 | EKKNWYKWD | 628 |
| HSA-14 | QKKRDEDYI | 576 | DRHKSRWGI | 629 |
| HSA-15 | GVHEEPRKL | 577 | LNPFTPSVT | 630 |
| HSA-16 | EWWQKHWPS | 578 | YKGALLNHD | 631 |
| HSA-17 | NFFQRRWPG | 579 | WKFRNTERG | 632 |
| HSA-18 | DWWQAKWPH | 580 | YKVHQSSGG | 633 |
| HSA-19 | GIWQSRWPG | 581 | FHPIAGRPW | 634 |
| HSA-20 | GYWAAKWPG | 582 | FPNTSYDLQ | 635 |
| HSA-21 | GFYADHWPG | 583 | FAHYNLKSG | 636 |
| HSA-22 | NWYQQRWPG | 584 | WHNYGESSG | 637 |
| HSA-23 | GFYARHWPG | 585 | KFYYADHQW | 638 |
| HSA-24 | DFWKAHWPG | 586 | YTHADPHSQ | 639 |
| HSA-25 | DFYSVRWPG | 587 | FGVPQLGAG | 640 |
| HSA-26 | YWAANHASK | 588 | YSGFPFAGF | 641 |
| HSA-27 | IKRLEHWEY | 589 | WFSWPYTPL | 642 |
| HSA-28 | EWDSPWSEN | 590 | YYHPSIQST | 643 |
| HSA-29 | KHKNLRWPF | 591 | FLGWKDTVV | 644 |
| HSA-30 | RHFPKQTNW | 592 | DWWKWWWAK | 645 |
| HSA-31 | VWGPEYQHQ | 593 | NAGWPL VPE | 646 |
| HSA-32 | TWKNNGQDV | 594 | YALDPFGGK | 647 |
| HSA-33 | ATWLNYYLP | 595 | GYKFWGVSD | 648 |
| HSA-34 | DQESLFLNN | 596 | QGKQYILLR | 649 |
| HSA-35 | GFYAQHWPD | 597 | YKRHSAHDY | 650 |
| HSA-36 | GHYARYWPG | 598 | WAQKSKVHQ | 651 |
| HSA-37 | GFWASKWPG | 599 | FTAVSKKDA | 652 |
| HSA-38 | GFWQRKWPN | 600 | WGDKENIWF | 653 |
| HSA-39 | VWPADNDLK | 601 | WSGHPWVQK | 654 |
| HSA-40 | HWAWTSPGY | 602 | YADYPL SPK | 655 |
| HSA-41 | NFFQRRWPG | 603 | WKFRNTDRG | 656 |
| HSA-42 | HHSHRLKGQ | 604 | QTVATHYHY | 657 |
| HSA-43 | YQNTIFLSI | 605 | WHAKHLLSH | 658 |
| HSA-44 | FQDQFTWSQ | 606 | SGIKKADSV | 659 |
| HSA-45 | GEPHWPWQA | 607 | KANLINVKS | 660 |
| HSA-46 | ADPRHPWVE | 608 | WKSHVEVRS | 661 |
| HSA-47 | FHKRFQSQG | 609 | WVTQKYIIQ | 662 |
| HSA-48 | EWWQNRWPN | 610 | WEHAKDWPT | 663 |
| HSA-49 | EWYQTRWPG | 611 | FHSKVLDKA | 664 |
| HSA-50 | EFWQRHWPG | 612 | YGAQKQAVW | 665 |
| HSA-51 | KFYERHWPG | 613 | FSASHFTSQ | 666 |
| Consensus | GWWQRRWPG | 614 | X₁X₂AX₃KX₄DX₅Q | 667 |

In some embodiments, (Xaa)ₙ comprises an amino acid sequence having at least 80% or at least 90% identity to the amino acid sequence of any one of SEQ ID NOs: 562 to 614. In some embodiments, (Xaa)ₙ comprises an amino acid sequence having 80% to 90% identity to the amino acid sequence of any one of SEQ ID NOs: 562 to 614. In some embodiments, (Xaa)ₙ comprises the amino acid sequence of any one SEQ ID NOs: 562 to 614.

In some embodiments, (Xaa)ₘ comprises an amino acid sequence having at least 80% or at least 90% identity to the amino acid sequence of any one of SEQ ID NOs: 615 to 667. In some embodiments, (Xaa)ₘ comprises an amino acid sequence having 80% to 90% identity to the amino acid sequence of any one of SEQ ID NOs: 615 to 667. In some embodiments, (Xaa)ₘ comprises the amino acid sequence of any one of SEQ ID NOs: 615 to 667.

In some embodiments, the stefin A protein variant binding to HSA comprises an amino acid sequence selected from any one of SEQ ID NOs: 668 to 674 **(Table 9).**

**Table 9. Exemplary Stefin A protein variant binding to HSA**

| **Sequence** | **SEQ ID NO:** |
|---|---|
| MIPRGLSEAKPATPEIQEIVDKVKPQLEEKTNETYGKLEAVQYKTQVLADWWQAKWP HSTNYYIKVRAGDNKYMHLKVFNGPYKVHQSSGGADRVLTGYQVDKNKDDELTGF | 668 |
| MIPRGLSEAKPATPEIQEIVDKVKPQLEEKTNETYGKLEAVQYKTQVLAGYWAAKWP GSTNYYIKVRAGDNKYMHLKVFNGPFPNTSYDLQADRVLTGYQVDKNKDDELTGF | 669 |
| MIPRGLSEAKPATPEIQEIVDKVKPQLEEKTNETYGKLEAVQYKTQVLANWYQQRWP GSTNYYIKVRAGDNKYMHLKVFNGPWHNYGESSGADRVLTGYQVDKNKDDELTGF | 670 |
| MIPRGLSEAKPATPEIQEIVDKVKPQLEEKTNETYGKLEAVQYKTQVLAVWGPEYQHQ STNYYIKVRAGDNKYMHLKVFNGPNAGWPLVPEADRVLTGYQVDKNKDDELTGF | 671 |
| MIPRGLSEAKPATPEIQEIVDKVKPQLEEKTNETYGKLEAVQYKTQVLAGHYARYWPG STNYYIKVRAGDNKYMHLKVFNGPWAQKSKVHQADRVLTGYQVDKNKDDELTGF | 672 |
| MIPRGLSEAKPATPEIQEIVDKVKPQLEEKTNETYGKLEAVQYKTQVLAGFWASKWPG STNYYIKVRAGDNKYMHLKVFNGPFTAVSKKDAADRVLTGYQVDKNKDDELTGF | 673 |
| MIPRGLSEAKPATPEIQEIVDKVKPQLEEKTNETYGKLEAVQYKTQVLANFFQRRWPG STNYYIKVRAGDNKYMHLKVFNGPWKFRNTDRGADRVLTGYQVDKNKDDELTGF | 674 |

In some embodiments, the stefin A protein variant binding to HSA comprises an amino acid sequence having at least 80% or at least 90% identity to the amino acid sequence of any one of SEQ ID NOs: 668 to 674.

In some embodiments, the stefin A protein variant binding to HSA comprises an amino acid sequence having 80% to 90% identity to the amino acid sequence of any one of SEQ ID NOs: 668 to 674.

The stefin A protein variant binding to HSA provided herein, in some embodiments, is linked to another molecule and extend the half-life of that molecule (*e.g*., a therapeutic polypeptide). Provided herein is a range of stefin A protein variant binding to HSA, with a range of binding affinities, for example, that cross-react with other species such as mouse and cynomolgus (cyno) monkey. These stefin A protein variant binding to HSA, in some embodiments, make up what is referred to as the AFFIMER XT^{™} platform. In some embodiments, the Stefin A protein variant binding to HSA may extend the half-life of another Stefin A protein variant or a fusion protein comprising the same, conjugated thereto in a single gene fusion manner, in a controlled manner in an in vivo pharmacokinetic (PK) study. In some embodiments, the Stefin A protein variant binding to HSA (AFFIMER XT^{™}) may be used to extend the half-life of other peptide or protein therapeutics.

In some embodiments, the stefin A protein variant binding to HSA extends the serum half-life of a molecule (*e.g*., a therapeutic polypeptide) *in vivo.* For example the stefin A protein variant binding to HSA may extend the half-life of a molecule by at least 2-fold, relative to the half-life of the molecule not linked to the stefin A protein variant binding to HSA. In some embodiments, the stefin A protein variant binding to HSA extends the half-life of a molecule by at least 3-fold, at least 4-fold, at least 5-fold, at least 6-fold, at least 7-fold, at least 8-fold, at least 9-fold, at least 10-fold, at least 20-fold, or at least 30-fold, relative to the half-life of the molecule not linked to the stefin A protein variant binding to HSA. In some embodiments, the stefin A protein variant binding to HSA extends the half-life of a molecule by 2-fold to 5-fold, 2-fold to 10-fold, 3-fold to 5-fold, 3-fold to 10-fold, 15-fold to 5-fold, 4-fold to 10-fold, or 5-fold to 10-fold, relative to the half-life of the molecule not linked to the stefin A protein variant binding to HSA. In some embodiments, the stefin A protein variant binding to HSA extends the half-life of a molecule by at least 6 hours, at least 12 hours, at least 24 hours, at least 48 hours, at least 72 hours, at least 96 hours, for example, at least 1 week after *in vivo* administration, relative to the half-life of the molecule not linked to the stefin A protein variant binding to HSA.

In some embodiments, the fusion protein may have an increased serum half-life compared to the Stefin A protein variant that specifically binds to CD40L, and may comprise an amino acid sequence that is at least 70%, 75% 80%, 85%, 90%, 95% or 98% identical to any one of the sequences of SEQ ID NOs: 667 to 702 (Table 10). In the present invention, the fusion protein may have an increased serum half-life compared to the Stefin A protein variant that specifically binds to CD40L, and may comprise an amino acid sequence that is at least 70%, 75% 80%, 85%, 90%, 95% or 98% identical to any one of the sequences of SEQ ID NOs: 703 to 728 (Table 11).

### PEGylation, XTEN, PAS and Other Polymers

A wide variety of macromolecular polymers and other molecules can be linked to the stefin A protein variant specifically biding to CD40Lof the present disclosure to modulate biological properties of the resulting fusion protein, and/or provide new biological properties to fusion protein. These macromolecular polymers can be linked to the stefin A protein variant specifically binding to CD40L via a naturally encoded amino acid, via a non-naturally encoded amino acid, or any functional substituent of a natural or non-natural amino acid, or any substituent or functional group added to a natural or non-natural amino acid. The molecular weight of the polymer may be of a wide range, including but not limited to, between about 100 Da and about 100,000 Da or more. The molecular weight of the polymer may be between about 100 Da and about 100,000 Da, including but not limited to, 100,000 Da, 95,000 Da, 90,000 Da, 85,000 Da, 80,000 Da, 75,000 Da, 70,000 Da, 65,000 Da, 60,000 Da, 55,000 Da, 50,000 Da, 45,000 Da, 40,000 Da, 35,000 Da, 30,000 Da, 25,000 Da, 20,000 Da, 15,000 Da, 10,000 Da, 9,000 Da, 8,000 Da, 7,000 Da, 6,000 Da, 5,000 Da, 4,000 Da, 3,000 Da, 2,000 Da, 1,000 Da, 900 Da, 800 Da, 700 Da, 600 Da, 500 Da, 400 Da, 300 Da, 200 Da, and 100 Da. In some embodiments, the molecular weight of the polymer is between about 100 Da and about 50,000 Da. In some embodiments, the molecular weight of the polymer is between about 100 Da and about 40,000 Da. In some embodiments, the molecular weight of the polymer is between about 1,000 Da and about 40,000 Da. In some embodiments, the molecular weight of the polymer is between about 5,000 Da and about 40,000 Da. In some embodiments, the molecular weight of the polymer is between about 10,000 Da and about 40,000 Da.

For this purpose, various methods including pegylation, polysialylation, HESylation, glycosylation, or recombinant PEG analogue fused to flexible and hydrophilic amino acid chain (500 to 600 amino acids) have been developed (See Chapman, (2002) Adv Drug Deliv Rev. 54. 531-545; Schlapschy et al., (2007) Prot Eng Des Sel. 20, 273-283; Contermann (2011) Curr Op Biotechnol. 22, 868-876; Jevsevar et al., (2012) Methods Mol Biol. 901, 233-246).

Examples of polymers include but are not limited to polyalkyl ethers and alkoxy-capped analogs thereof (e.g., polyoxyethylene glycol, polyoxyethylene/propylene glycol, and methoxy or ethoxy-capped analogs thereof, especially polyoxyethylene glycol, the latter is also known as polyethylene glycol or PEG); discrete PEG (dPEG); polyvinylpyrrolidones; polyvinylalkyl ethers; polyoxazolines, polyalkyl oxazolines and polyhydroxyalkyl oxazolines; polyacrylamides, polyalkyl acrylamides, and polyhydroxyalkyl acrylamides (e.g., polyhydroxypropylmethacrylamide and derivatives thereof); polyhydroxyalkyl acrylates; polysialic acids and analogs thereof; hydrophilic peptide sequences; polysaccharides and their derivatives, including dextran and dextran derivatives, e.g., carboxymethyldextran, dextran sulfates, aminodextran; cellulose and its derivatives, e.g., carboxymethyl cellulose, hydroxyalkyl celluloses; chitin and its derivatives, e.g., chitosan, succinyl chitosan, carboxymethylchitin, carboxymethylchitosan; hyaluronic acid and its derivatives; starches; alginates; chondroitin sulfate; albumin; pullulan and carboxymethyl pullulan; polyaminoacids and derivatives thereof, e.g., polyglutamic acids, polylysines, polyaspartic acids, polyaspartamides; maleic anhydride copolymers such as: styrene maleic anhydride copolymer, divinylethyl ether maleic anhydride copolymer; polyvinyl alcohols; copolymers thereof; terpolymers thereof; mixtures thereof; and derivatives of the foregoing.

The polymer selected may be water soluble so that the fusion ptotein to which it is attached does not precipitate in an aqueous environment, such as a physiological environment. The water-soluble polymer may be any structural form including but not limited to linear, forked or branched. Typically, the water-soluble polymer is a poly(alkylene glycol), such as poly(ethylene glycol) (PEG), but other water-soluble polymers can also be employed. By way of example, PEG is used to describe some embodiments of this disclosure. In some embodiments, the polymer may be pharmaceutically acceptable.

The term "PEG" is used broadly to encompass any polyethylene glycol molecule, without regard to size or to modification at an end of the PEG, and can be represented as linked to the stefin A protein variant of the present invention by the formula:

XO-(CH₂CH₂O)ₙ-CH₂CH₂-

or

XO-(CH₂CH₂O)ₙ-

where n is 2 to 10,000 and X is H or a terminal modification, including but not limited to, a C1-4 alkyl, a protecting group, or a terminal functional group. In some cases, a PEG used in the polypeptides of the disclosure terminates on one end with hydroxy or methoxy, e.g., X is H or CH₃ ("methoxy PEG").

It is noted that the other end of the PEG, which is shown in the above formulas by a terminal "-", may attach to the stefin A protein variant via a naturally-occurring or non-naturally encoded amino acid. For instance, the attachment may be through an amide, carbamate or urea linkage to an amine group (including but not limited to, the epsilon amine of lysine or the N-terminus) of the polypeptide. Alternatively, the polymer is linked by a maleimide linkage to a thiol group (including but not limited to, the thiol group of cysteine) - which in the case of attachment to the stefin A protein variantsequence per se requires altering a residue in the stefin A protein variant sequence to a cysteine.

The number of water-soluble polymers linked to the stefin A protein variant or the fusion protein of the present invention(e.g., the extent of PEGylation or glycosylation) can be adjusted to provide an altered (including but not limited to, increased or decreased) pharmacologic, pharmacokinetic or pharmacodynamic characteristic such as *in vivo* half-life in the resulting fusion protein. In some embodiments, the half-life of the resulting fusion proteinis increased at least about 10, 20, 30, 40, 50, 60, 70, 80, 90 percent, 2-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, 20-fold, 25-fold, 30-fold, 35-fold, 40-fold, 50-fold, or at least about 100-fold over an unmodified polypeptide.

Another variation of polymer system useful to modify the PK or other biological properties of the resulting fusion proteinare the use of unstructured, hydrophilic amino acid polymers that are functional analogs of PEG, particularly as part of a fusion protein with the stefin A protein variant sequence. The inherent biodegradability of the polypeptide platform makes it attractive as a potentially more benign alternative to PEG. Another advantage is the precise molecular structure of the recombinant molecule in contrast to the polydispersity of PEG. Unlike HSA and Fc peptide fusions, in which the three-dimensional folding of the fusion partner needs to be maintained, the recombinant fusions to unstructured partners can, in many cases, be subjected to higher temperatures or harsh conditions such as HPLC purification.

One of the more advanced of this class of polypeptides is termed XTEN (Amunix) and is 864 amino acids long and comprised of six amino acids (A, E, G, P, S and T). See Schellenberger et al. "A recombinant polypeptide extends the in vivo half-life of peptides and proteins in a tunable manner" 2009 Nat Biotechnol. 27(12):1186-90. Enabled by the biodegradable nature of the polymer, this is much larger than the 40 KDa PEGs typically used and confers a concomitantly greater half-life extension. The fusion of XTEN to the stefin A protein variant should result in halflife extension of the final fusion protein by 60- to 130-fold over the unmodified polypeptide.

A second polymer based on similar conceptual considerations is PAS (XL-Protein GmbH). Schlapschy et al. "PASYlation: a biological alternative to PEGylation for extending the plasma half-life of pharmaceutically active proteins" 2013 Protein Eng Des Sel. 26(8):489-501. A random coil polymer comprised of an even more restricted set of only three small uncharged amino acids, proline, alanine and serine. As with Fc, HSA and XTEN, the PAS modification can be genetically encoded with the Stefin A protein variant or the fusion protein to produce an inline fusion protein when expressed.

### In-Line Fusion

In some embodiments, the fusion protein may be selected from any one of the polypeptides provided in **Table 10**.

**Table 10. Exemplary Encoded Half-Life Extension In-Line Fusion Stefin A protein variant**

| **Clone #** | **Amino Acid** | **SEQ ID NO:** |
|---|---|---|
| 230 DI | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSGGGGSGGGGSGGGGSMIPGG LSEAKPATPEIQEIVDKVKPQLEEKTGETYGKLEAVQYKTQVVHYYVHYN DQGTNYYIKVRAGDNKYMHLKVFKSLWGENLFAKWEDLVLTGYQVDK NKDDELTGF | 677 |
| 230 DJ | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSGGGGSGGGGSGGGGSMIPGG LSEAKPATPEIQEIVDKVKPQLEEKTGETYGKLEAVQYKTQVVHYYVHYN DQGTNYYIKVRAGDNKYMHLKVFKSLWGENLFAKWEDLVLTGYQVDK NKDDELTGFGGGGSGGGGSGGGGSGGGGSGGGGSGGGGSMIPGGLSEAK PATPEIQEIVDKVKPQLEEKTGETYGKLEAVQYKTQVVHYYVHYNDQGT NYYIKVRAGDNKYMHLKVFKSLWGENLFAKWEDLVLTGYQVDKNKDD ELTGF | 678 |
| 247 DI | IPGGLSEAKPATPEIQEIVDKVKPQLEEKTGETYGKLEAVQYKTQVVHHA YAAVYVGTNYYIKVRAGDNKYMHLKVFKSLPYWYRKYPDEDLVLTGYQ VDKNKDDELTGFGGGGSGGGGSGGGGSGGGGSGGGGSGGGGSMIPGGL SEAKPATPEIQEIVDKVKPQLEEKTGETYGKLEAVQYKTQVVHHAYAAV YVGTNYYIKVRAGDNKYMHLKVFKSLPYWYRKYPDEDLVLTGYQVDKN KDDELTGF | 679 |
| 247 DJ | IPGGLSEAKPATPEIQEIVDKVKPQLEEKTGETYGKLEAVQYKTQVVHHA YAAVYVGTNYYIKVRAGDNKYMHLKVFKSLPYWYRKYPDEDLVLTGYQ VDKNKDDELTGFGGGGSGGGGSGGGGSGGGGSGGGGSGGGGSMIPGGL SEAKPATPEIQEIVDKVKPQLEEKTGETYGKLEAVQYKTQVVHHAYAAV YVGTNYYIKVRAGDNKYMHLKVFKSLPYWYRKYPDEDLVLTGYQVDKN KDDELTGFGGGGSGGGGSGGGGSGGGGSGGGGSGGGGSMIPGGLSEAKP ATPEIQEIVDKVKPQLEEKTGETYGKLEAVQYKTQVVHHAYAAVYVGTN YYIKVRAGDNKYMHLKVFKSLPYWYRKYPDEDLVLTGYQVDKNKDDEL TGF | 680 |
| 230 DT | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKEAAAKEAAAKMIPG GLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVD | 681 |
| | KNKDDELTGFAEAAAKEAAAKEAAAKEAAAKEAAAKEAAAKMIPGGLS EAKPATPEIQEIVDKVKPQLEEKTGETYGKLEAVQYKTQVVHYYVHYND QGTNYYIKVRAGDNKYMHLKVFKSLWGENLFAKWEDLVLTGYQVDKN KDDELTGF | |
| 243 DT | IPGGLSEAKPATPEIQEIVDKVKPQLEEKTGETYGKLEAVQYKTQVVFTYQ ISYTVGTNYYIKVRAGDNKYMHLKVFKSLRWRYPARKPEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVFTYQISYTV GTNYYIKVRAGDNKYMHLKVFKSLRWRYPARKPEDLVLTGYQVDKNKD DELTGFAEAAAKEAAAKEAAAKEAAAKEAAAKEAAAKMIPGGLSEAKP ATPEIQEIVDKVKPQLEEKTGETYGKLEAVQYKTQVVFTYQISYTVGTNY YIKVRAGDNKYMHLKVFKSLRWRYPARKPEDLVLTGYQVDKNKDDELT GF | 682 |
| 246 DT | IPGGLSEAKPATPEIQEIVDKVKPQLEEKTGETYGKLEAVQYKTQVVHIPA LKVHAGTNYYIKVRAGDNKYMHLKVFKSLPYWYRHDQEEDLVLTGYQV DKNKDDELTGFAEAAAKEAAAKEAAAKEAAAKEAAAKEAAAKMIPGGL SEAKPATPEIQEIVDKVKPQLEEKTGETYGKLEAVQYKTQVVHIPALKVH AGTNYYIKVRAGDNKYMHLKVFKSLPYWYRHDQEEDLVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVHIPALKVHAGT NYYIKVRAGDNKYMHLKVFKSLPYWYRHDQEEDLVLTGYQVDKNKDD ELTGF | 683 |
| 247 DT | IPGGLSEAKPATPEIQEIVDKVKPQLEEKTGETYGKLEAVQYKTQVVHHA YAAVYVGTNYYIKVRAGDNKYMHLKVFKSLPYWYRKYPDEDLVLTGYQ VDKNKDDELTGFAEAAAKEAAAKEAAAKEAAAKEAAAKEAAAKMIPGG LSEAKPATPEIQEIVDKVKPQLEEKTGETYGKLEAVQYKTQVVHHAYAAV YVGTNYYIKVRAGDNKYMHLKVFKSLPYWYRKYPDEDLVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVHHAYAAVYVG TNYYIKVRAGDNKYMHLKVFKSLPYWYRKYPDEDLVLTGYQVDKNKDD ELTGF | 684 |
| 248 DT | IPGGLSEAKPATPEIQEIVDKVKPQLEEKTGETYGKLEAVQYKTQVVYDEL YHEKAGTNYYIKVRAGDNKYMHLKVFKSLEYKFRNLEEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVYDELYHEKA GTNYYIKVRAGDNKYMHLKVFKSLEYKFRNLEEDLVLTGYQVDKNKDD ELTGFAEAAAKEAAAKEAAAKEAAAKEAAAKEAAAKMIPGGLSEAKPA TPEIQEIVDKVKPQLEEKTGETYGKLEAVQYKTQVVYDELYHEKAGTNY YIKVRAGDNKYMHLKVFKSLEYKFRNLEEDLVLTGYQVDKNKDDELTGF | 685 |
| 261 DT | IPGGLSEAKPATPEIQEIVDKVKPQLEEKTGETYGKLEAVQYKTQVVVDQ VWSENAGTNYYIKVRAGDNKYMHLKVFKSLPYLWRKHAVEDLVLTGYQ VDKNKDDELTGFAEAAAKEAAAKEAAAKEAAAKEAAAKEAAAKMIPGG LSEAKPATPEIQEIVDKVKPQLEEKTGETYGKLEAVQYKTQVVVDQVWSE NAGTNYYIKVRAGDNKYMHLKVFKSLPYLWRKHAVEDLVLTGYQVDK NKDDELTGFAEAAAKEAAAKEAAAKEAAAKEAAAKEAAAKMIPGGLSE AKPATPEIQEIVDKVKPQLEEKTGETYGKLEAVQYKTQVVVDQVWSENA GTNYYIKVRAGDNKYMHLKVFKSLPYLWRKHAVEDLVLTGYQVDKNK DDELTGF | 686 |
| 268 DT | IPGGLSEAKPATPEIQEIVDKVKPQLEEKTGETYGKLEAVQYKTQVVGDSY WEYVSGTNYYIKVRAGDNKYMHLKVFKSLPYRWRPDHAEDLVLTGYQV DKNKDDELTGFAEAAAKEAAAKEAAAKEAAAKEAAAKEAAAKMIPGGL SEAKPATPEIQEIVDKVKPQLEEKTGETYGKLEAVQYKTQVVGDSYWEY VSGTNYYIKVRAGDNKYMHLKVFKSLPYRWRPDHAEDLVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVGDSYWEYVSG TNYYIKVRAGDNKYMHLKVFKSLPYRWRPDHAEDLVLTGYQVDKNKDD ELTGF | 687 |
| 270 DT | IPGGLSEAKPATPEIQEIVDKVKPQLEEKTGETYGKLEAVQYKTQVVLDGL YHVNSGTNYYIKVRAGDNKYMHLKVFKSLFYYYRSEPTEDLVLTGYQVD KNKDDELTGFAEAAAKEAAAKEAAAKEAAAKEAAAKEAAAKMIPGGLS | 688 |
| | EAKPATPEIQEIVDKVKPQLEEKTGETYGKLEAVQYKTQVVLDGLYHVNS GTNYYIKVRAGDNKYMHLKVFKSLFYYYRSEPTEDLVLTGYQVDKNKD DELTGFAEAAAKEAAAKEAAAKEAAAKEAAAKEAAAKMIPGGLSEAKP ATPEIQEIVDKVKPQLEEKTGETYGKLEAVQYKTQVVLDGLYHVNSGTN YYIKVRAGDNKYMHLKVFKSLFYYYRSEPTEDLVLTGYQVDKNKDDELT GF | |
| 330 DT | IPGGLSEAKPATPEIQEIVDKVKPQLEEKTGETYGKLEAVQYKTQVVKTW EDTVVAGTNYYIKVRAGDNKYMHLKVFKSLEYLYRPLNPEDLVLTGYQV DKNKDDELTGFAEAAAKEAAAKEAAAKEAAAKEAAAKEAAAKMIPGGL SEAKPATPEIQEIVDKVKPQLEEKTGETYGKLEAVQYKTQVVKTWEDTVV AGTNYYIKVRAGDNKYMHLKVFKSLEYLYRPLNPEDLVLTGYQVDKNK DDELTGFAEAAAKEAAAKEAAAKEAAAKEAAAKEAAAKMIPGGLSEAK PATPEIQEIVDKVKPQLEEKTGETYGKLEAVQYKTQVVKTWEDTVVAGT NYYIKVRAGDNKYMHLKVFKSLEYLYRPLNPEDLVLTGYQVDKNKDDE LTGF | 689 |
| 230 DU | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSGGGGSGGGGSGGGGSMIPGG LSEAKPATPEIQEIVDKVKPQLEEKTGETYGKLEAVQYKTQVVHYYVHYN DQGTNYYIKVRAGDNKYMHLKVFKSLWGENLFAKWEDLVLTGYQVDK NKDDELTGFGGGGSGGGGSGGGGSGGGGSGGGGSGGGGSMIPGGLSEAK PATPEIQEIVDKVKPQLEEKTGETYGKLEAVQYKTQVVHYYVHYNDQGT NYYIKVRAGDNKYMHLKVFKSLWGENLFAKWEDLVLTGYQVDKNKDD ELTGFGGGGSGGGGSGGGGSGGGGSGGGGSGGGGSMIPGGLSEAKPATP EIQEIVDKVKPQLEEKTGETYGKLEAVQYKTQVVHYYVHYNDQGTNYYI KVRAGDNKYMHLKVFKSLWGENLFAKWEDLVLTGYQVDKNKDDELTG F | 690 |
| 230 DS | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVHYYVHYND QGTNYYIKVRAGDNKYMHLKVFKSLWGENLFAKWEDLVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVHYYVHYNDQG TNYYIKVRAGDNKYMHLKVFKSLWGENLFAKWEDLVLTGYQVDKNKD DELTGF | 691 |
| 230 XT73 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSGGGGSGGGGSGGGGSMIPGG LSEAKPATPEIQEIVDKVKPQLEEKTGETYGKLEAVQYKTQVVHYYVHYN DQGTNYYIKVRAGDNKYMHLKVFKSLWGENLFAKWEDLVLTGYQVDK NKDDELTGFGGGGSGGGGSGGGGSGGGGSGGGGSGGGGSMIPGGLSEAK PATPEIQEIVDKVKPQLEEKTGETYGKLEAVQYKTQVVHYYVHYNDQGT NYYIKVRAGDNKYMHLKVFKSLWGENLFAKWEDLVLTGYQVDKNKDD ELTGFGGGGSGGGGSGGGGSGGGGSGGGGSGGGGSMIPRGLSEAKPATP EIQEIVDKVKPQLEEKTGETYGKLEAVQYKTQVLANFFQRRWPGSTNYYI KVRAGDNKYMHLKVFNGPWKFRNTDRGADRVLTGYQVDKNKDDELTG F | 692 |
| 230 XT74 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSGGGGSGGGGSGGGGSMIPGG LSEAKPATPEIQEIVDKVKPQLEEKTGETYGKLEAVQYKTQVVHYYVHYN DQGTNYYIKVRAGDNKYMHLKVFKSLWGENLFAKWEDLVLTGYQVDK NKDDELTGFGGGGSGGGGSGGGGSGGGGSGGGGSGGGGSMIPGGLSEAK PATPEIQEIVDKVKPQLEEKTGETYGKLEAVQYKTQVVHYYVHYNDQGT NYYIKVRAGDNKYMHLKVFKSLWGENLFAKWEDLVLTGYQVDKNKDD ELTGFGGGGSGGGGSGGGGSGGGGSGGGGSGGGGSMIPGGLSEAKPATP | 693 |
| | EIQEIVDKVKPQLEEKTGETYGKLEAVQYKTQVVHYYVHYNDQGTNYYI KVRAGDNKYMHLKVFKSLWGENLFAKWEDLVLTGYQVDKNKDDELTG FGGGGSGGGGSGGGGSGGGGSGGGGSGGGGSMIPRGLSEAKPATPEIQEI VDKVKPQLEEKTGETYGKLEAVQYKTQVLANFFQRRWPGSTNYYIKVRA GDNKYMHLKVFNGPWKFRNTDRGADRVLTGYQVDKNKDDELTGF | |
| 230 XT75 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVHYYVHYND QGTNYYIKVRAGDNKYMHLKVFKSLWGENLFAKWEDLVLTGYQVDKN KDDELTGFAEAAAKEAAAKEAAAKEAAAKEAAAKEAAAKMIPRGLSEA KPATPEIQEIVDKVKPQLEEKTGETYGKLEAVQYKTQVLANFFQRRWPGS TNYYIKVRAGDNKYMHLKVFNGPWKFRNTDRGADRVLTGYQVDKNKD DELTGF | 694 |
| 230 XT76 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVHYYVHYND QGTNYYIKVRAGDNKYMHLKVFKSLWGENLFAKWEDLVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVHYYVHYNDQG TNYYIKVRAGDNKYMHLKVFKSLWGENLFAKWEDLVLTGYQVDKNKD DELTGFAEAAAKEAAAKEAAAKEAAAKEAAAKEAAAKMIPRGLSEAKP ATPEIQEIVDKVKPQLEEKTGETYGKLEAVQYKTQVLANFFQRRWPGSTN YYIKVRAGDNKYMHLKVFNGPWKFRNTDRGADRVLTGYQVDKNKDDE LTGF | 695 |
| 230 XT83 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVHYYVHY NDQGTNYYIKVRAGDNKYMHLKVFKSLWGENLFAKWEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVHYYVHYND QGTNYYIKVRAGDNKYMHLKVFKSLWGENLFAKWEDLVLTGYQVDKN KDDELTGFGGGGSGGGGSGGGGSGGGGSGGGGSGGGGSGGGGSGGGGS GGGGSGGGGSGGGGSGGGGSMIPRGLSEAKPATPEIQEIVDKVKPQLEEK TGETYGKLEAVQYKTQVLANFFQRRWPGSTNYYIKVRAGDNKYMHLKV FNGPWKFRNTDRGADRVLTGYQVDKNKDDELTGF | 696 |
| 230 XT84 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSMIPGGLSEAKPATPEIQEIVDK VKPQLEEKTGETYGKLEAVQYKTQVVHYYVHYNDQGTNYYIKVRAGDN KYMHLKVFKSLWGENLFAKWEDLVLTGYQVDKNKDDELTGFGGGGSG GGGSGGGGSMIPGGLSEAKPATPEIQEIVDKVKPQLEEKTGETYGKLEAV QYKTQVVHYYVHYNDQGTNYYIKVRAGDNKYMHLKVFKSLWGENLFA KWEDLVLTGYQVDKNKDDELTGFGGGGSGGGGSGGGGSGGGGSGGGGS GGGGSGGGGSGGGGSGGGGSGGGGSGGGGSGGGGSMIPRGLSEAKPATP EIQEIVDKVKPQLEEKTGETYGKLEAVQYKTQVLANFFQRRWPGSTNYYI KVRAGDNKYMHLKVFNGPWKFRNTDRGADRVLTGYQVDKNKDDELTG F | 697 |
| 230 XT85 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFGGGGSGGGGSGGGGSMIPGGLSEAKPATPEIQEIVDK VKPQLEEKTGETYGKLEAVQYKTQVVHYYVHYNDQGTNYYIKVRAGDN | 698 |
| | KYMHLKVFKSLWGENLFAKWEDLVLTGYQVDKNKDDELTGFGGGGSG GGGSGGGGSMIPGGLSEAKPATPEIQEIVDKVKPQLEEKTGETYGKLEAV QYKTQVVHYYVHYNDQGTNYYIKVRAGDNKYMHLKVFKSLWGENLFA KWEDLVLTGYQVDKNKDDELTGFAEAAAKEAAAKEAAAKEAAAKEAA AKEAAAKEAAAKEAAAKEAAAKEAAAKEAAAKEAAAKMIPRGLSEAKP ATPEIQEIVDKVKPQLEEKTGETYGKLEAVQYKTQVLANFFQRRWPGSTN YYIKVRAGDNKYMHLKVFNGPWKFRNTDRGADRVLTGYQVDKNKDDE LTGF | |
| 230 XT86 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKMIPGGLSEAKPATPEIQEIVD KVKPQLEEKTGETYGKLEAVQYKTQVVHYYVHYNDQGTNYYIKVRAGD NKYMHLKVFKSLWGENLFAKWEDLVLTGYQVDKNKDDELTGFAEAAA KEAAAKEAAAKMIPGGLSEAKPATPEIQEIVDKVKPQLEEKTGETYGKLE AVQYKTQVVHYYVHYNDQGTNYYIKVRAGDNKYMHLKVFKSLWGENL FAKWEDLVLTGYQVDKNKDDELTGFGGGGSGGGGSGGGGSGGGGSGGG GSGGGGSGGGGSGGGGSGGGGSGGGGSGGGGSGGGGSMIPRGLSEAKPA TPEIQEIVDKVKPQLEEKTGETYGKLEAVQYKTQVLANFFQRRWPGSTNY YIKVRAGDNKYMHLKVFNGPWKFRNTDRGADRVLTGYQVDKNKDDEL TGF | 699 |
| 230 XT87 | IPGGLSEAKPATPEIQEIVDKVKPQLEEKTGETYGKLEAVQYKTQVVHYY VHYNDQGTNYYIKVRAGDNKYMHLKVFKSLWGENLFAKWEDLVLTGY QVDKNKDDELTGFAEAAAKEAAAKEAAAKMIPGGLSEAKPATPEIQEIVD KVKPQLEEKTGETYGKLEAVQYKTQVVHYYVHYNDQGTNYYIKVRAGD NKYMHLKVFKSLWGENLFAKWEDLVLTGYQVDKNKDDELTGFAEAAA KEAAAKEAAAKMIPGGLSEAKPATPEIQEIVDKVKPQLEEKTGETYGKLE AVQYKTQVVHYYVHYNDQGTNYYIKVRAGDNKYMHLKVFKSLWGENL FAKWEDLVLTGYQVDKNKDDELTGFAEAAAKEAAAKEAAAKEAAAKE AAAKEAAAKEAAAKEAAAKEAAAKEAAAKEAAAKEAAAKMIPRGLSEA KPATPEIQEIVDKVKPQLEEKTGETYGKLEAVQYKTQVLANFFQRRWPGS TNYYIKVRAGDNKYMHLKVFNGPWKFRNTDRGADRVLTGYQVDKNKD DELTGF | 700 |
| 310 gly XT58 | IPGGLSEAKPATPEIQEIVDKVKPQLEEKTGETYGKLEAVQYKTQVVGGG GGGGGGGTNYYIKVRAGDNKYMHLKVFKSLGGGGGGGGGEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVGGGGGG GGGGTNYYIKVRAGDNKYMHLKVFKSLGGGGGGGGGEDLVLTGYQVD KNKDDELTGFAEAAAKEAAAKEAAAKEAAAKEAAAKEAAAKMIPGGLS EAKPATPEIQEIVDKVKPQLEEKTGETYGKLEAVQYKTQVVGGGGGGGG GGTNYYIKVRAGDNKYMHLKVFKSLGGGGGGGGGEDLVLTGYQVDKN KDDELTGFAEAAAKEAAAKEAAAKEAAAKEAAAKEAAAKMIPRGLSEA KPATPEIQEIVDKVKPQLEEKTGETYGKLEAVQYKTQVLANFFQRRWPGS TNYYIKVRAGDNKYMHLKVFNGPWKFRNTDRGADRVLTGYQVDKNKD DELTGF | 701 |
| 310 gly XT59 | IPGGLSEAKPATPEIQEIVDKVKPQLEEKTGETYGKLEAVQYKTQVVGGG GGGGGGGTNYYIKVRAGDNKYMHLKVFKSLGGGGGGGGGEDLVLTGY QVDKNKDDELTGFAEAAAKEAAAKEAAAKEAAAKEAAAKEAAAKMIPG GLSEAKPATPEIQEIVDKVKPQLEEKTGETYGKLEAVQYKTQVVGGGGGG GGGGTNYYIKVRAGDNKYMHLKVFKSLGGGGGGGGGEDLVLTGYQVD KNKDDELTGFAEAAAKEAAAKEAAAKEAAAKEAAAKEAAAKMIPRGLS EAKPATPEIQEIVDKVKPQLEEKTGETYGKLEAVQYKTQVLANFFQRRWP GSTNYYIKVRAGDNKYMHLKVFNGPWKFRNTDRGADRVLTGYQVDKN KDDELTGFAEAAAKEAAAKEAAAKEAAAKEAAAKEAAAKMIPGGLSEA KPATPEIQEIVDKVKPQLEEKTGETYGKLEAVQYKTQVVGGGGGGGGGG TNYYIKVRAGDNKYMHLKVFKSLGGGGGGGGGEDLVLTGYQVDKNKD DELTGFAEAAAKEAAAKEAAAKEAAAKEAAAKEAAAKMIPGGLSEAKP ATPEIQEIVDKVKPQLEEKTGETYGKLEAVQYKTQVVGGGGGGGGGGTN YYIKVRAGDNKYMHLKVFKSLGGGGGGGGGEDLVLTGYQVDKNKDDE LTGF | 702 |

In some embodiments, a nucleic acid encoding the fusion protein may be selected from the nucleic acids provided in Table 11, but is not limited thereto. It can be clearly understood that nucleic acid sequences encoding fusion proteins can be designed and predicted, based on the amino acid sequence of the Stepin A protein that specifically binds to CD40L of the present invention and the nucleic acid sequence encoding the same, as well as the polypeptide sequence of the fusion domain included in the fusion protein of the present invention and the nucleic acid sequence encoding the same.

**Table 11. Exemplary Encoded Half-Life Extension In-Line Fusion Anti-CD40L AFFIMER^{®} Polynucleotide Sequences**

| **Clone #** | **Nucleic Acid** | **SEQ ID NO:** |
|---|---|---|
| 230 DI | ATCCCTGGCGGACTGTCGGAAGCCAAGCCGGCCACTCCGGAAATCCAG GAGATTGTGGATAAGGTCAAGCCTCAGCTGGAGGAAAAGACGGGCGA AACTTACGGGAAGCTCGAGGCCGTGCAATACAAGACCCAGGTCGTGC ACTATTACGTGCATTACAACGACCAGGGGACCAACTACTACATTAAGG TCCGCGCTGGAGACAACAAGTATATGCACCTGAAAGTGTTCAAGTCGC TGTGGGGGGAGAACCTCTTCGCGAAGTGGGAGGACCTTGTGCTCACCG GGTACCAAGTGGACAAGAACAAGGACGACGAGCTGACAGGCTTCGGC GGAGGAGGTTCCGGAGGAGGCGGATCCGGTGGAGGCGGTTCAGGCGG TGGAGGAAGCGGAGGCGGAGGATCCGGAGGCGGTGGTAGCATGATTC CCGGAGGGTTGTCCGAAGCGAAACCAGCCACCCCCGAGATCCAGGAA ATCGTGGACAAGGTCAAACCGCAGCTGGAAGAGAAAACCGGCGAAAC CTACGGAAAGCTGGAAGCGGTGCAGTACAAGACTCAAGTGGTGCACT ACTACGTGCACTACAACGATCAGGGCACCAATTACTACATCAAAGTCC GGGCCGGCGATAACAAGTACATGCACCTCAAGGTCTTTAAGTCCCTCT GGGGCGAAAACCTGTTCGCCAAATGGGAGGATCTGGTGCTGACTGGCT ACCAAGTCGACAAGAATAAGGATGACGAGTTGACCGGGTTC | 703 |
| 230 DJ | ATTCCCGGCGGACTGTCAGAAGCCAAACCGGCCACTCCGGAGATCCAG GAGATCGTGGACAAGGTCAAGCCACAGCTGGAGGAAAAGACCGGCGA AACCTACGGGAAGTTGGAGGCTGTGCAGTACAAGACCCAGGTTGTGCA CTATTACGTCCACTATAACGACCAAGGCACCAACTACTATATCAAGGT CCGAGCGGGCGATAACAAATACATGCACCTGAAGGTCTTTAAGTCCCT CTGGGGAGAAAACCTTTTTGCGAAGTGGGAGGACCTCGTGCTCACCGG ATACCAAGTGGACAAGAATAAGGACGACGAACTGACTGGATTCGGAGG GGGCGGTTCAGGAGGAGGGGGATCAGGGGGAGGAGGAAGCGGCGG AGGAGGATCCGGAGGAGGCGGAAGCGGTGGTGGCGGATCCATGATTC CGGGAGGCCTTTCCGAGGCCAAGCCTGCCACCCCCGAAATCCAAGAGA TTGTGGATAAGGTCAAACCTCAGCTGGAAGAAAAGACGGGGGAGACT TACGGCAAACTGGAAGCCGTGCAGTATAAGACCCAAGTGGTGCACTAC TACGTGCACTACAACGATCAGGGGACCAATTACTACATTAAGGTCCGC GCTGGAGACAACAAGTACATGCATCTCAAGGTATTCAAGTCGCTGTGG GGCGAAAACCTCTTCGCGAAATGGGAGGACTTGGTGCTGACCGGGTAC CAAGTCGATAAGAACAAGGACGATGAACTTACTGGGTTCGGAGGCGG AGGTTCGGGTGGAGGCGGTTCCGGTGGAGGAGGGTCCGGTGGTGGAG GATCGGGTGGCGGTGGATCGGGAGGCGGTGGTTCCATGATCCCTGGAG GCCTGTCCGAAGCAAAGCCGGCCACCCCAGAAATCCAGGAAATCGTG GATAAAGTCAAGCCCCAGTTGGAGGAGAAAACCGGAGAGACATACGG GAAGCTGGAGGCGGTGCAATACAAGACTCAAGTGGTCCATTATTACGT GCATTACAACGACCAGGGCACTAACTACTACATCAAAGTCCGGGCCGG GGATAACAAGTATATGCACCTCAAGGTGTTCAAGAGCCTCTGGGGGGA GAACCTGTTCGCCAAATGGGAAGATCTGGTGTTGACCGGCTACCAGGT CGACAAGAACAAAGACGACGAGCTGACGGGGTTC | 704 |
| 247 DI | ATCCCTGGTGGCCTGTCCGAAGCCAAGCCTGCTACGCCCGAGATCCAG GAGATTGTGGACAAAGTCAAGCCGCAGCTCGAGGAAAAGACCGGCGA AACCTACGGCAAACTCGAAGCCGTGCAGTACAAGACTCAAGTGGTGC ATCACGCGTACGCCGCTGTGTACGTGGGCACAAACTACTACATCAAAG TCCGGGCCGGCGATAACAAATACATGCATCTGAAAGTGTTCAAGTCGC TGCCGTACTGGTACAGAAAGTACCCAGACGAGGACTTGGTGCTGACTG GGTACCAGGTCGACAAGAACAAGGATGACGAGCTTACCGGCTTCGGT GGTGGAGGATCCGGCGGAGGAGGATCGGGAGGAGGCGGTTCCGGAGG AGGAGGCAGCGGTGGAGGAGGAAGCGGAGGGGGTGGAAGCATGATTC CTGGGGGGCTCTCAGAAGCAAAGCCTGCCACCCCGGAAATTCAGGAG ATCGTGGATAAGGTCAAGCCCCAGCTGGAGGAGAAAACTGGCGAAAC TTACGGGAAGCTGGAAGCGGTGCAATACAAGACCCAGGTCGTGCACC ATGCCTACGCCGCCGTCTACGTGGGGACCAACTATTACATCAAGGTCC GCGCGGGAGATAACAAGTACATGCACCTGAAGGTCTTTAAGTCCCTCC CCTACTGGTACCGGAAGTATCCCGATGAGGACCTGGTGCTGACCGGCT ATCAAGTGGACAAGAATAAGGACGACGAGCTGACCGGTTTC | 705 |
| 247 DJ | ATTCCCGGAGGACTCTCCGAGGCCAAACCGGCCACCCCGGAAATCCAG GAGATCGTGGATAAGGTCAAGCCCCAGCTGGAGGAAAAGACCGGAGA GACTTACGGGAAGCTTGAGGCCGTCCAGTACAAGACCCAGGTGGTGCA CCATGCGTACGCCGCGGTCTACGTCGGGACCAACTACTACATTAAGGT CCGCGCTGGCGATAACAAGTATATGCACTTAAAGGTGTTCAAATCCCT GCCATATTGGTACAGGAAGTACCCCGACGAAGATCTGGTGCTCACCGG CTACCAGGTCGATAAGAATAAGGACGACGAACTGACCGGCTTCGGAG GAGGTGGAAGCGGCGGTGGAGGATCCGGCGGAGGAGGAAGCGGAGG CGGTGGATCCGGTGGAGGAGGGTCTGGTGGAGGGGGCTCCATGATTCC TGGAGGCCTGTCCGAAGCCAAGCCCGCCACTCCCGAAATTCAAGAAAT CGTGGACAAGGTCAAACCGCAGCTGGAAGAAAAGACTGGAGAAACCT ACGGAAAGCTCGAGGCGGTGCAGTACAAAACCCAAGTGGTGCATCAT GCCTACGCCGCCGTCTACGTGGGCACGAACTACTATATCAAAGTCAGA GCCGGCGACAACAAGTACATGCACCTGAAAGTGTTCAAGTCACTGCCG TACTGGTACAGAAAGTACCCGGACGAGGACCTGGTGCTGACCGGGTAC CAGGTGGACAAAAACAAGGACGATGAACTCACCGGGTTTGGAGGAGG CGGAAGCGGTGGTGGTGGCTCCGGAGGAGGAGGCTCAGGAGGCGGAG GATCGGGAGGGGGAGGTTCCGGAGGGGGTGGATCGATGATCCCTGGC GGTCTGTCCGAGGCTAAGCCAGCCACCCCTGAGATCCAGGAAATTGTG GACAAAGTCAAGCCGCAGCTCGAGGAGAAAACCGGCGAAACTTACGG AAAGTTGGAGGCCGTGCAATACAAGACTCAGGTCGTGCACCACGCATA CGCGGCTGTGTACGTGGGGACTAACTATTACATCAAGGTTCGCGCCGG AGACAACAAATACATGCATCTGAAGGTCTTTAAGTCCCTCCCCTACTG GTACCGGAAGTACCCTGACGAGGACTTGGTGCTGACTGGGTACCAAGT GGATAAGAACAAGGATGACGAGCTGACAGGGTTC | 706 |
| 230 DT | ATCCCTGGAGGGCTGTCCGAGGCCAAGCCTGCTACGCCGGAAATTCAA GAAATCGTCGATAAGGTTAAGCCACAGCTCGAAGAGAAAACTGGGGA AACATACGGCAAACTTGAGGCGGTTCAGTACAAAACTCAGGTCGTGCA CTACTACGTGCACTACAACGATCAAGGGACCAACTACTATATCAAGGT CCGCGCGGGAGACAACAAATACATGCATCTCAAAGTGTTCAAATCGCT GTGGGGCGAGAACCTTTTCGCGAAGTGGGAGGATTTGGTGCTGACTGG ATATCAGGTCGATAAAAACAAGGATGACGAGCTTACTGGCTTTGCCGA GGCCGCTGCTAAGGAAGCAGCCGCGAAAGAAGCCGCCGCCAAGGAAG CTGCAGCCAAGGAGGCGGCAGCAAAGGAGGCCGCAGCCAAGATGATT CCCGGAGGCCTCTCAGAAGCCAAACCAGCCACTCCTGAGATTCAGGAA ATTGTCGACAAGGTCAAGCCGCAGCTGGAAGAGAAGACTGGCGAAAC CTACGGGAAGCTAGAGGCGGTCCAGTACAAGACCCAAGTCGTCCATTA CTATGTACATTACAACGACCAGGGAACCAATTACTACATTAAAGTGCG GGCCGGAGATAATAAGTATATGCATCTTAAGGTGTTTAAGTCGTTGTG GGGCGAAAACCTGTTTGCCAAGTGGGAAGATCTTGTGCTCACCGGTTA CCAAGTGGACAAGAACAAGGACGACGAGCTGACCGGGTTCGCTGAAG CTGCCGCGAAGGAAGCCGCTGCGAAGGAGGCTGCCGCAAAGGAAGCG GCCGCTAAGGAGGCAGCCGCCAAAGAGGCCGCCGCGAAGATGATACC TGGCGGACTGTCGGAGGCGAAACCGGCCACCCCTGAAATCCAGGAGA | 707 |
| | TTGTGGACAAGGTGAAACCCCAACTCGAGGAAAAAACCGGCGAAACG TACGGTAAACTCGAAGCCGTGCAGTATAAGACCCAGGTCGTACACTAC TATGTGCACTATAACGATCAGGGAACGAACTACTACATCAAGGTCAGA GCTGGAGACAATAAGTACATGCACCTCAAGGTTTTCAAGTCGTTATGG GGAGAAAACCTCTTCGCGAAATGGGAGGATCTCGTGCTGACCGGCTAC CAGGTGGACAAAAATAAGGATGATGAACTCACCGGTTTC | |
| 243 DT | ATCCCTGGCGGACTGAGCGAGGCCAAGCCTGCCACTCCGGAGATCCAG GAAATCGTGGACAAAGTCAAGCCCCAGCTGGAGGAAAAGACTGGCGA AACTTACGGAAAGCTGGAAGCCGTGCAATACAAGACCCAAGTGGTGTT CACCTATCAAATTTCGTACACCGTCGGGACCAATTACTACATCAAAGT GCGGGCCGGGGATAACAAATACATGCACTTGAAAGTGTTTAAGTCGCT GCGGTGGAGGTACCCTGCCCGCAAGCCTGAAGATCTGGTGCTCACCGG TTACCAAGTCGACAAGAACAAGGATGACGAGCTCACCGGCTTCGCCGA AGCAGCCGCTAAAGAGGCCGCCGCCAAAGAAGCCGCCGCTAAGGAAG CAGCAGCGAAGGAGGCTGCCGCGAAAGAGGCGGCCGCCAAGATGATT CCCGGCGGATTGTCCGAAGCCAAGCCGGCTACCCCCGAAATCCAGGAG ATCGTCGACAAGGTCAAGCCACAGCTCGAAGAGAAAACGGGAGAGAC TTACGGGAAGCTGGAGGCAGTCCAGTATAAGACTCAGGTCGTGTTCAC GTACCAAATCTCCTACACTGTGGGGACTAACTACTATATCAAAGTCCG AGCCGGCGACAACAAGTACATGCACCTCAAGGTGTTCAAGAGCTTGCG CTGGCGGTACCCTGCTCGGAAGCCAGAAGATCTTGTGCTGACTGGGTA CCAGGTCGATAAGAACAAAGACGACGAGTTGACCGGTTTCGCCGAGG CAGCTGCAAAGGAAGCTGCCGCCAAGGAGGCAGCGGCCAAGGAAGCC GCTGCGAAGGAAGCGGCGGCAAAGGAGGCCGCGGCAAAGATGATCCC GGGAGGCCTGTCAGAAGCCAAACCCGCGACCCCTGAGATTCAGGAAA TTGTGGATAAGGTCAAACCGCAGCTGGAAGAAAAGACCGGCGAAACC TACGGAAAGTTGGAGGCGGTGCAGTACAAGACACAGGTTGTGTTTACC TACCAAATTTCCTACACCGTGGGAACCAACTATTACATCAAGGTCCGC GCGGGAGATAACAAGTATATGCACCTTAAAGTGTTCAAGTCCCTGAGA TGGAGATACCCAGCCAGAAAGCCGGAGGACCTGGTGCTGACCGGCTA CCAAGTGGACAAAAACAAGGACGACGAGCTGACTGGTTTC | 708 |
| 246 DT | ATCCCGGGAGGACTGTCGGAAGCGAAGCCGGCCACTCCAGAGATCCA AGAAATTGTGGACAAGGTCAAGCCCCAGCTCGAGGAAAAGACTGGGG AAACCTACGGAAAGCTCGAGGCTGTGCAGTATAAGACCCAAGTGGTG CATATTCCGGCGCTGAAGGTGCACGCCGGAACCAATTACTACATCAAA GTCCGGGCCGGAGACAATAAGTACATGCATCTCAAAGTGTTTAAGTCC CTGCCATACTGGTACCGGCACGACCAGGAGGAGGATCTCGTGCTGACC GGCTATCAGGTCGATAAGAACAAGGACGACGAACTGACTGGTTTCGCT GAGGCTGCGGCAAAGGAGGCCGCCGCTAAGGAGGCGGCAGCGAAGGA AGCTGCGGCCAAGGAGGCAGCCGCGAAGGAGGCTGCCGCGAAGATGA TTCCCGGTGGACTGTCAGAAGCCAAGCCAGCCACTCCCGAAATCCAGG AAATCGTGGATAAGGTCAAACCCCAGCTGGAAGAAAAGACCGGAGAG ACTTACGGGAAGTTGGAGGCCGTGCAGTACAAGACCCAGGTCGTGCAC ATTCCGGCCCTGAAAGTGCACGCGGGTACCAACTACTACATTAAGGTT CGCGCTGGAGACAACAAGTATATGCATCTGAAAGTGTTCAAGTCCCTC CCCTACTGGTATCGCCACGACCAAGAGGAAGATCTGGTGCTGACTGGC TACCAAGTGGACAAAAACAAGGATGATGAACTGACCGGCTTCGCCGA AGCCGCAGCAAAGGAAGCCGCCGCCAAAGAGGCCGCTGCTAAGGAAG CGGCGGCCAAAGAAGCGGCCGCCAAGGAAGCAGCGGCCAAGATGATC CCCGGGGGATTGAGCGAGGCCAAGCCTGCCACCCCTGAGATCCAGGA GATCGTGGACAAAGTCAAGCCGCAGCTTGAAGAGAAAACCGGCGAAA CATACGGGAAGCTGGAGGCCGTCCAGTACAAAACCCAGGTGGTGCAC ATCCCGGCGCTCAAGGTCCACGCCGGCACTAACTACTATATCAAGGTC CGCGCCGGAGATAACAAATACATGCACCTGAAGGTGTTCAAGAGCCTC CCGTACTGGTACAGACACGATCAGGAAGAGGACCTCGTGCTTACCGGG TACCAGGTCGACAAGAACAAAGACGACGAGCTGACGGGCTTC | 709 |
| 247 DT | ATCCCTGGAGGCCTGAGCGAAGCCAAGCCTGCCACTCCGGAGATTCAG GAGATCGTGGACAAAGTCAAGCCCCAGCTGGAGGAAAAGACAGGAGA GACTTATGGAAAGCTGGAAGCCGTGCAGTATAAGACCCAAGTGGTGC ACCATGCCTACGCCGCCGTCTACGTGGGAACCAACTACTACATCAAGG | 710 |
| | TTCGCGCCGGGGATAACAAGTACATGCACCTCAAGGTGTTCAAGTCCC TGCCATACTGGTACAGAAAGTACCCTGATGAGGACCTGGTGCTGACCG GGTACCAGGTCGACAAGAATAAGGACGATGAGCTGACTGGCTTTGCCG AAGCGGCAGCCAAGGAGGCTGCGGCGAAGGAAGCTGCCGCGAAAGAG GCGGCCGCTAAGGAAGCAGCTGCAAAGGAAGCGGCCGCAAAGATGAT TCCCGGTGGACTCTCCGAAGCGAAGCCTGCTACCCCGGAGATCCAGGA GATTGTGGACAAGGTCAAACCGCAGCTCGAAGAAAAGACCGGGGAAA CTTACGGGAAGCTGGAGGCCGTGCAATACAAAACCCAGGTGGTACAC CACGCGTACGCCGCTGTGTACGTCGGCACCAACTATTACATTAAGGTC CGCGCAGGAGACAACAAGTATATGCACCTGAAGGTCTTTAAGAGCCTG CCGTACTGGTACCGGAAGTACCCGGACGAGGACCTCGTGCTCACCGGC TACCAAGTGGATAAGAACAAAGATGACGAGCTCACGGGTTTCGCTGA AGCAGCCGCCAAGGAAGCCGCCGCCAAAGAGGCCGCCGCAAAGGAGG CGGCGGCCAAAGAAGCCGCGGCTAAGGAGGCCGCAGCCAAGATGATC CCTGGTGGCCTGTCGGAGGCCAAACCCGCCACTCCCGAAATCCAGGAA ATCGTGGATAAGGTCAAGCCGCAGCTGGAAGAGAAAACCGGCGAAAC CTACGGAAAGCTCGAAGCGGTGCAGTACAAGACCCAGGTCGTGCACC ACGCCTACGCTGCGGTGTACGTGGGGACCAATTACTATATCAAAGTCC GGGCCGGCGATAACAAATACATGCATCTGAAAGTGTTCAAATCCCTTC CATATTGGTACAGGAAGTACCCCGACGAGGACTTGGTGCTTACCGGCT ATCAAGTGGACAAGAACAAGGACGACGAACTGACTGGATTC | |
| 248 DT | ATCCCCGGTGGACTGTCCGAAGCAAAGCCAGCGACTCCCGAAATCCAG GAAATTGTGGATAAGGTTAAGCCCCAGCTGGAAGAAAAGACTGGAGA AACCTACGGAAAGCTCGAGGCCGTGCAGTACAAAACCCAGGTGGTGT ACGATGAACTGTACCACGAGAAAGCCGGTACTAATTACTACATTAAGG TCCGCGCAGGAGACAACAAGTATATGCACCTCAAAGTGTTTAAGAGCC TGGAGTATAAGTTCCGCAACCTGGAAGAGGACTTGGTGCTTACTGGAT ACCAAGTCGACAAAAACAAGGACGACGAACTGACCGGGTTCGCCGAA GCAGCGGCTAAGGAGGCCGCAGCTAAGGAAGCCGCCGCCAAAGAAGC CGCGGCGAAAGAAGCGGCCGCGAAGGAAGCTGCCGCGAAGATGATTC CGGGCGGTCTGTCAGAAGCCAAGCCGGCTACCCCGGAAATTCAGGAA ATCGTGGACAAGGTCAAGCCGCAGCTCGAGGAAAAGACCGGAGAGAC TTACGGGAAGCTGGAGGCCGTCCAGTACAAGACCCAAGTGGTGTATGA CGAACTCTACCACGAAAAGGCCGGGACGAACTACTACATCAAAGTCA GGGCCGGAGATAACAAGTACATGCACCTGAAGGTGTTCAAGTCCCTGG AGTACAAGTTCCGGAACCTTGAGGAGGACCTGGTGCTGACCGGCTACC AAGTGGACAAGAACAAGGATGATGAGCTGACAGGGTTTGCCGAGGCT GCTGCGAAGGAGGCGGCTGCCAAGGAAGCGGCGGCAAAAGAGGCCGC CGCAAAGGAGGCAGCGGCCAAAGAGGCAGCCGCCAAGATGATCCCCG GCGGATTGTCCGAGGCCAAGCCAGCCACCCCTGAGATCCAGGAGATTG TGGACAAAGTCAAGCCTCAGCTCGAAGAGAAAACCGGCGAAACTTAC GGAAAGTTGGAAGCTGTGCAGTATAAGACCCAGGTCGTGTACGACGA GCTGTACCATGAGAAGGCCGGCACCAACTACTATATCAAAGTCCGGGC CGGCGATAACAAATACATGCATCTGAAAGTGTTCAAATCGCTCGAATA CAAGTTCAGAAACCTTGAAGAGGACCTTGTGCTCACCGGGTACCAGGT CGACAAGAATAAGGATGACGAATTGACTGGATTC | 711 |
| 261 DT | ATTCCTGGAGGCCTGAGCGAAGCCAAGCCCGCCACTCCTGAAATCCAA GAAATCGTGGACAAGGTCAAGCCACAGCTGGAAGAGAAAACCGGGGA AACTTACGGGAAGCTGGAGGCCGTCCAGTACAAGACACAAGTCGTGG TCGACCAAGTCTGGTCCGAGAATGCCGGAACCAACTACTACATCAAGG TCCGGGCCGGAGATAACAAGTATATGCACCTGAAAGTGTTCAAGTCAC TCCCGTACCTTTGGAGGAAGCACGCGGTCGAGGACCTGGTGCTGACCG GGTACCAGGTCGATAAGAACAAAGACGACGAACTCACCGGATTCGCG GAAGCAGCCGCTAAGGAAGCTGCAGCGAAAGAAGCGGCCGCGAAGGA AGCAGCGGCCAAAGAGGCCGCCGCCAAAGAAGCCGCGGCGAAGATGA TCCCGGGAGGACTGTCGGAAGCGAAGCCAGCCACCCCCGAAATCCAG GAGATCGTGGATAAGGTCAAACCGCAGCTGGAGGAGAAAACTGGCGA AACCTACGGAAAGTTGGAGGCCGTGCAGTATAAGACCCAGGTCGTGGT GGACCAAGTGTGGTCCGAAAACGCCGGCACTAACTATTACATCAAAGT CAGAGCCGGCGACAACAAGTACATGCATCTGAAGGTGTTCAAGAGCCT | 712 |
| | CCCCTACTTGTGGCGCAAGCACGCCGTAGAGGATCTCGTGCTGACGGG CTACCAAGTGGACAAGAACAAGGACGACGAGCTTACCGGCTTCGCCG AAGCCGCAGCAAAGGAGGCGGCCGCAAAGGAAGCGGCAGCCAAGGA GGCCGCTGCTAAGGAGGCTGCCGCCAAGGAAGCCGCTGCCAAGATGA TTCCCGGTGGACTGTCCGAGGCCAAGCCTGCGACCCCTGAGATTCAGG AAATCGTCGACAAAGTCAAGCCCCAGTTGGAAGAAAAGACTGGAGAG ACTTACGGAAAGCTCGAGGCGGTGCAGTACAAAACCCAAGTGGTGGT GGATCAAGTCTGGAGCGAGAACGCGGGCACCAATTACTACATTAAGGT CCGCGCAGGAGACAACAAATACATGCACCTCAAGGTCTTTAAGTCCCT GCCGTACCTGTGGCGGAAGCATGCCGTGGAGGACCTCGTGTTGACCGG TTACCAGGTTGACAAAAACAAGGATGATGAGCTGACTGGATTC | |
| 268 DT | ATTCCGGGCGGACTCTCTGAGGCCAAGCCCGCCACTCCTGAGATTCAG GAGATTGTGGATAAGGTCAAACCGCAGCTCGAAGAAAAAACCGGAGA GACTTACGGAAAACTGGAGGCGGTCCAGTACAAAACCCAAGTCGTGG GCGACTCCTATTGGGAATATGTGTCGGGGACCAACTACTACATCAAAG TCCGCGCCGGAGATAACAAATACATGCACCTCAAAGTGTTCAAGAGCT TGCCTTACCGCTGGAGGCCTGACCACGCGGAGGATCTGGTGCTCACCG GGTACCAGGTCGACAAAAACAAGGACGACGAGCTGACTGGCTTTGCC GAAGCTGCCGCAAAAGAGGCTGCCGCTAAGGAGGCGGCCGCGAAAGA GGCAGCAGCCAAGGAGGCCGCTGCCAAAGAGGCCGCAGCCAAGATGA TCCCGGGTGGACTGTCCGAGGCCAAACCCGCGACCCCTGAGATCCAGG AAATCGTGGACAAGGTCAAGCCTCAGCTGGAAGAAAAGACTGGCGAA ACCTACGGAAAGCTGGAAGCTGTGCAGTACAAGACCCAGGTCGTGGG AGACTCATACTGGGAATACGTGTCCGGTACCAACTATTACATTAAGGT CCGCGCGGGAGATAATAAGTACATGCATCTGAAGGTGTTCAAGTCCCT GCCGTACCGGTGGAGGCCGGATCATGCCGAGGACCTGGTCCTCACCGG ATACCAAGTGGATAAGAACAAGGATGACGAACTGACCGGCTTCGCCG AAGCAGCCGCAAAGGAAGCAGCGGCTAAGGAAGCTGCGGCCAAAGAA GCCGCCGCGAAGGAAGCGGCCGCCAAGGAAGCCGCGGCGAAGATGAT TCCTGGCGGTCTGTCCGAAGCTAAGCCCGCTACCCCGGAAATCCAAGA GATCGTGGATAAAGTCAAGCCCCAACTCGAGGAAAAGACCGGAGAAA CTTACGGGAAGCTCGAAGCCGTGCAGTATAAGACTCAAGTGGTCGGGG ACTCGTACTGGGAGTACGTCAGCGGCACTAACTACTATATCAAAGTTC GGGCCGGCGACAACAAGTATATGCACCTTAAAGTGTTTAAGAGCCTGC CCTACCGATGGCGGCCCGATCATGCGGAGGACTTGGTGCTGACGGGAT ACCAGGTGGACAAGAATAAGGACGATGAGCTGACCGGATTC | 713 |
| 270 DT | ATCCCTGGTGGCCTGTCCGAAGCCAAGCCTGCGACCCCTGAGATCCAA GAGATTGTGGACAAAGTCAAGCCGCAGCTGGAAGAAAAGACCGGGGA AACCTACGGCAAACTGGAGGCCGTACAGTACAAGACCCAGGTCGTGCT TGACGGCCTGTACCATGTCAACTCCGGTACAAACTACTACATCAAGGT CCGAGCCGGAGACAACAAATACATGCATCTCAAAGTGTTCAAGAGCCT GTTCTACTATTACCGGTCCGAACCGACTGAGGACCTGGTGCTCACGGG TTACCAGGTCGACAAAAACAAGGACGACGAACTGACCGGTTTTGCCGA AGCCGCAGCCAAGGAAGCTGCGGCCAAAGAAGCCGCCGCAAAGGAAG CAGCCGCCAAGGAGGCTGCCGCGAAGGAAGCCGCGGCGAAGATGATC CCGGGAGGACTCTCAGAGGCCAAACCCGCCACCCCGGAAATCCAGGA GATCGTGGACAAGGTCAAGCCACAGCTGGAGGAAAAGACTGGAGAGA CTTACGGAAAGCTCGAGGCCGTGCAGTACAAAACCCAAGTGGTGCTGG ACGGCCTTTATCACGTGAACAGCGGCACCAACTACTATATCAAGGTTC GCGCGGGAGACAATAAGTACATGCACCTGAAGGTGTTCAAGTCCCTGT TCTATTACTACCGCTCGGAGCCAACCGAGGATTTGGTGCTGACCGGGT ATCAAGTGGATAAGAACAAGGATGACGAGCTGACCGGCTTCGCTGAG GCCGCAGCAAAGGAGGCCGCCGCCAAAGAGGCCGCGGCTAAGGAGGC AGCCGCTAAGGAAGCGGCCGCGAAAGAAGCGGCGGCCAAGATGATTC CCGGCGGACTGTCAGAAGCTAAGCCGGCCACCCCTGAAATTCAGGAA ATCGTGGATAAGGTCAAACCCCAGTTGGAGGAGAAAACCGGCGAAAC TTACGGGAAGCTCGAAGCCGTGCAATACAAGACTCAGGTCGTCCTGGA CGGGCTGTACCACGTGAACTCCGGCACCAATTACTACATTAAGGTCCG CGCCGGAGATAACAAGTATATGCATCTGAAAGTGTTTAAGTCCCTCTT | 714 |
| | CTACTACTACCGGTCGGAGCCTACTGAGGATCTGGTGCTGACTGGGTA CCAAGTGGACAAGAACAAAGACGACGAGCTCACCGGATTC | |
| 330 DT | ATTCCCGGAGGCCTCTCAGAGGCCAAGCCTGCCACCCCCGAAATTCAG GAAATCGTGGATAAGGTCAAGCCGCAGCTGGAGGAGAAAACCGGGGA AACCTACGGAAAGCTGGAAGCGGTGCAATACAAAACCCAGGTCGTCA AGACTTGGGAGGATACTGTGGTCGCCGGTACCAATTACTATATCAAGG TCCGCGCTGGCGATAACAAATATATGCACCTGAAGGTGTTCAAGTCCC TCGAGTACCTGTATCGGCCTCTGAACCCCGAGGACCTGGTGCTGACTG GATACCAGGTCGACAAGAACAAGGACGATGAACTGACGGGGTTCGCT GAAGCTGCCGCCAAGGAGGCTGCGGCAAAGGAAGCAGCCGCGAAGGA GGCAGCGGCCAAGGAAGCTGCGGCCAAAGAGGCCGCGGCGAAGATGA TCCCGGGTGGACTCTCCGAAGCCAAGCCAGCCACCCCGGAAATCCAAG AGATCGTGGACAAGGTCAAACCGCAGCTTGAGGAAAAGACCGGCGAA ACTTACGGGAAGTTGGAAGCCGTGCAGTACAAGACCCAAGTCGTGAA AACCTGGGAAGATACAGTGGTGGCAGGCACCAACTACTACATCAAAG TCCGCGCCGGAGACAACAAATACATGCATCTGAAAGTGTTCAAGAGCC TGGAATACCTTTACCGGCCGCTGAATCCCGAGGATTTGGTGCTCACTG GCTACCAAGTGGACAAGAATAAGGACGACGAACTCACCGGCTTCGCC GAAGCCGCTGCTAAGGAAGCGGCTGCGAAGGAAGCCGCCGCAAAGGA GGCGGCCGCTAAGGAGGCCGCCGCCAAAGAAGCGGCCGCCAAGATGA TTCCTGGTGGCCTGTCGGAGGCGAAACCGGCCACCCCTGAGATTCAGG AGATCGTCGACAAAGTGAAGCCACAGCTGGAAGAAAAGACTGGCGAA ACCTATGGGAAGCTTGAGGCAGTGCAGTATAAGACTCAAGTGGTCAAG ACCTGGGAGGACACTGTGGTGGCCGGAACTAATTACTACATTAAGGTC CGGGCCGGAGATAACAAGTACATGCACCTCAAAGTGTTTAAGTCCCTG GAATACTTGTACAGACCACTGAACCCTGAGGACCTCGTGCTGACCGGG TACCAGGTTGATAAGAACAAAGACGACGAGCTTACTGGATTC | 715 |
| 230 DU | ATCCCTGGGGGACTCAGCGAGGCTAAGCCTGCGACTCCCGAAATCCAA GAGATCGTGGACAAAGTGAAGCCGCAACTGGAAGAAAAGACCGGAGA AACTTACGGGAAACTGGAGGCAGTGCAATACAAGACACAAGTGGTCC ACTATTACGTCCACTACAATGATCAGGGCACCAATTATTACATTAAGG TGCGAGCCGGCGATAACAAATATATGCACCTGAAGGTCTTTAAGTCCC TGTGGGGAGAGAATCTGTTCGCAAAGTGGGAGGACCTCGTGCTTACTG GTTACCAGGTAGACAAGAATAAGGACGATGAGCTGACTGGTTTCGGA GGCGGTGGTAGCGGAGGGGGTGGAAGCGGAGGAGGGGGAAGTGGTG GCGGTGGATCAGGCGGAGGGGGATCAGGGGGAGGAGGGTCCATGATC CCTGGAGGGCTGTCCGAGGCCAAGCCTGCTACGCCGGAAATTCAAGAA ATCGTCGATAAGGTTAAGCCACAGCTCGAAGAGAAAACTGGGGAAAC ATACGGCAAACTTGAGGCGGTTCAGTACAAAACTCAGGTCGTGCACTA CTACGTGCACTACAACGATCAAGGGACCAACTACTATATCAAGGTCCG CGCGGGAGACAACAAATACATGCATCTCAAAGTGTTCAAATCGCTGTG GGGCGAGAACCTTTTCGCGAAGTGGGAGGATTTGGTGCTGACTGGATA TCAGGTCGATAAAAACAAGGATGACGAGCTTACTGGCTTTGGCGGAGG CGGATCAGGAGGAGGTGGCAGCGGTGGAGGAGGTTCCGGTGGAGGCG GTTCCGGAGGAGGAGGCTCAGGAGGGGGAGGCTCCATGATTCCCGGA GGCCTCTCAGAAGCCAAACCAGCCACTCCTGAGATTCAGGAAATTGTC GACAAGGTCAAGCCGCAGCTGGAAGAGAAGACTGGCGAAACCTACGG GAAGCTAGAGGCGGTCCAGTACAAGACCCAAGTCGTCCATTACTATGT ACATTACAACGACCAGGGAACCAATTACTACATTAAAGTGCGGGCCGG AGATAATAAGTATATGCATCTTAAGGTGTTTAAGTCGTTGTGGGGCGA AAACCTGTTTGCCAAGTGGGAAGATCTTGTGCTCACCGGTTACCAAGT GGACAAGAACAAGGACGACGAGCTGACCGGGTTCGGAGGAGGCGGAA GCGGTGGTGGAGGATCGGGAGGCGGAGGATCCGGTGGTGGCGGATCT GGCGGTGGAGGTTCTGGGGGGGGAGGATCCATGATACCTGGCGGACT GTCGGAGGCGAAACCGGCCACCCCTGAAATCCAGGAGATTGTGGACA AGGTGAAACCCCAACTCGAGGAAAAAACCGGCGAAACGTACGGTAAA CTCGAAGCCGTGCAGTATAAGACCCAGGTCGTACACTACTATGTGCAC TATAACGATCAGGGAACGAACTACTACATCAAGGTCAGAGCTGGAGA CAATAAGTACATGCACCTCAAGGTTTTCAAGTCGTTATGGGGAGAAAA | 716 |
| | CCTCTTCGCGAAATGGGAGGATCTCGTGCTGACCGGCTACCAGGTGGA CAAAAATAAGGATGATGAACTCACCGGTTTC | |
| 230 DS | ATCCCTGGGGGACTCAGCGAGGCTAAGCCTGCGACTCCCGAAATCCAA GAGATCGTGGACAAAGTGAAGCCGCAACTGGAAGAAAAGACCGGAGA AACTTACGGGAAACTGGAGGCAGTGCAATACAAGACACAAGTGGTCC ACTATTACGTCCACTACAATGATCAGGGCACCAATTATTACATTAAGG TGCGAGCCGGCGATAACAAATATATGCACCTGAAGGTCTTTAAGTCCC TGTGGGGAGAGAATCTGTTCGCAAAGTGGGAGGACCTCGTGCTTACTG GTTACCAGGTAGACAAGAATAAGGACGATGAGCTGACTGGTTTCGCCG AAGCCGCAGCAAAAGAGGCCGCCGCGAAAGAAGCCGCTGCTAAGGAG GCGGCCGCAAAGGAGGCCGCGGCAAAAGAAGCGGCGGCCAAAATGAT CCCTGGAGGGCTGTCCGAGGCCAAGCCTGCTACGCCGGAAATTCAAGA AATCGTCGATAAGGTTAAGCCACAGCTCGAAGAGAAAACTGGGGAAA CATACGGCAAACTTGAGGCGGTTCAGTACAAAACTCAGGTCGTGCACT ACTACGTGCACTACAACGATCAAGGGACCAACTACTATATCAAGGTCC GCGCGGGAGACAACAAATACATGCATCTCAAAGTGTTCAAATCGCTGT GGGGCGAGAACCTTTTCGCGAAGTGGGAGGATTTGGTGCTGACTGGAT ATCAGGTCGATAAAAACAAGGATGACGAGCTTACTGGCTTTGCTGAGG CTGCCGCCAAGGAGGCTGCGGCGAAGGAAGCAGCAGCGAAAGAGGCA GCCGCCAAAGAAGCTGCAGCGAAGGAGGCAGCAGCCAAGATGATTCC CGGAGGCCTCTCAGAAGCCAAACCAGCCACTCCTGAGATTCAGGAAAT TGTCGACAAGGTCAAGCCGCAGCTGGAAGAGAAGACTGGCGAAACCT ACGGGAAGCTAGAGGCGGTCCAGTACAAGACCCAAGTCGTCCATTACT ATGTACATTACAACGACCAGGGAACCAATTACTACATTAAAGTGCGGG CCGGAGATAATAAGTATATGCATCTTAAGGTGTTTAAGTCGTTGTGGG GCGAAAACCTGTTTGCCAAGTGGGAAGATCTTGTGCTCACCGGTTACC AAGTGGACAAGAACAAGGACGACGAGCTGACCGGGTTCGCTGAAGCT GCGGCCAAAGAGGCGGCGGCAAAGGAAGCGGCCGCTAAAGAAGCAG CGGCCAAGGAAGCTGCCGCTAAGGAAGCCGCGGCGAAGATGATACCT GGCGGACTGTCGGAGGCGAAACCGGCCACCCCTGAAATCCAGGAGAT TGTGGACAAGGTGAAACCCCAACTCGAGGAAAAAACCGGCGAAACGT ACGGTAAACTCGAAGCCGTGCAGTATAAGACCCAGGTCGTACACTACT ATGTGCACTATAACGATCAGGGAACGAACTACTACATCAAGGTCAGAG CTGGAGACAATAAGTACATGCACCTCAAGGTTTTCAAGTCGTTATGGG GAGAAAACCTCTTCGCGAAATGGGAGGATCTCGTGCTGACCGGCTACC AGGTGGACAAAAATAAGGATGATGAACTCACCGGTTTC | 717 |
| 230 XT73 | ATCCCTGGAGGGCTGTCCGAGGCCAAGCCTGCTACGCCGGAAATTCAA GAAATCGTCGATAAGGTTAAGCCACAGCTCGAAGAGAAAACTGGGGA AACATACGGCAAACTTGAGGCGGTTCAGTACAAAACTCAGGTCGTGCA CTACTACGTGCACTACAACGATCAAGGGACCAACTACTATATCAAGGT CCGCGCGGGAGACAACAAATACATGCATCTCAAAGTGTTCAAATCGCT GTGGGGCGAGAACCTTTTCGCGAAGTGGGAGGATTTGGTGCTGACTGG ATATCAGGTCGATAAAAACAAGGATGACGAGCTTACTGGCTTTGGAGG AGGAGGGTCCGGTGGAGGAGGCTCTGGTGGAGGCGGAAGCGGCGGAG GAGGATCGGGTGGCGGAGGAAGCGGTGGTGGAGGCTCCATGATTCCC GGAGGCCTCTCAGAAGCCAAACCAGCCACTCCTGAGATTCAGGAAATT GTCGACAAGGTCAAGCCGCAGCTGGAAGAGAAGACTGGCGAAACCTA CGGGAAGCTAGAGGCGGTCCAGTACAAGACCCAAGTCGTCCATTACTA TGTACATTACAACGACCAGGGAACCAATTACTACATTAAAGTGCGGGC CGGAGATAATAAGTATATGCATCTTAAGGTGTTTAAGTCGTTGTGGGG CGAAAACCTGTTTGCCAAGTGGGAAGATCTTGTGCTCACCGGTTACCA AGTGGACAAGAACAAGGACGACGAGCTGACCGGGTTCGGAGGAGGCG GATCGGGAGGTGGAGGATCCGGTGGCGGTGGAAGCGGAGGTGGCGGA TCTGGGGGAGGGGGATCAGGAGGCGGCGGATCCATGATACCTGGCGG ACTGTCGGAGGCGAAACCGGCCACCCCTGAAATCCAGGAGATTGTGG ACAAGGTGAAACCCCAACTCGAGGAAAAAACCGGCGAAACGTACGGT AAACTCGAAGCCGTGCAGTATAAGACCCAGGTCGTACACTACTATGTG CACTATAACGATCAGGGAACGAACTACTACATCAAGGTCAGAGCTGG AGACAATAAGTACATGCACCTCAAGGTTTTCAAGTCGTTATGGGGAGA AAACCTCTTCGCGAAATGGGAGGATCTCGTGCTGACCGGCTACCAGGT | 718 |
| | GGACAAAAATAAGGATGATGAACTCACCGGTTTCGGAGGCGGAGGAT CAGGCGGAGGGGGTTCAGGAGGGGGTGGCTCAGGAGGAGGGGGTAGT GGAGGCGGTGGATCCGGAGGAGGTGGCAGCATGATCCCACGGGGTCT GTCCGAAGCCAAGCCAGCCACCCCAGAGATCCAAGAAATAGTGGATA AGGTGAAGCCCCAGCTTGAGGAAAAGACTGGAGAGACTTACGGAAAG CTCGAGGCTGTCCAATACAAAACACAGGTGCTGGCGAACTTCTTCCAA AGGAGATGGCCAGGCAGTACTAACTATTACATCAAAGTGCGCGCAGG AGATAACAAATATATGCACTTGAAGGTGTTCAATGGACCGTGGAAGTT CCGGAACACCGACAGAGGCGCCGACAGGGTCTTGACCGGGTACCAAG TCGATAAGAACAAAGACGACGAACTGACCGGTTTC | |
| 230 XT74 | ATCCCTGGGGGACTCAGCGAGGCTAAGCCTGCGACTCCCGAAATCCAA GAGATCGTGGACAAAGTGAAGCCGCAACTGGAAGAAAAGACCGGAGA AACTTACGGGAAACTGGAGGCAGTGCAATACAAGACACAAGTGGTCC ACTATTACGTCCACTACAATGATCAGGGCACCAATTATTACATTAAGG TGCGAGCCGGCGATAACAAGTATATGCACCTGAAGGTCTTTAAGTCCC TGTGGGGAGAGAATCTGTTCGCAAAGTGGGAGGACCTCGTGCTTACTG GTTACCAGGTAGACAAGAATAAGGACGATGAGCTGACTGGTTTCGGG GGCGGAGGATCTGGGGGTGGAGGAAGCGGTGGTGGAGGCTCGGGTGG AGGAGGGTCTGGGGGAGGAGGTTCAGGCGGCGGAGGTTCCATGATCC CTGGAGGGCTGTCCGAGGCCAAGCCTGCTACGCCGGAAATTCAAGAA ATCGTCGATAAGGTTAAGCCACAGCTCGAAGAGAAAACTGGGGAAAC ATACGGCAAACTTGAGGCGGTTCAGTACAAAACTCAGGTCGTGCACTA CTACGTGCACTACAACGATCAAGGGACCAACTACTATATCAAGGTCCG CGCGGGAGACAACAAATACATGCATCTCAAAGTGTTCAAATCGCTGTG GGGCGAGAACCTTTTCGCGAAGTGGGAGGATTTGGTGCTGACTGGATA TCAGGTCGATAAAAACAAGGATGACGAGCTTACTGGCTTTGGAGGCGG TGGATCGGGAGGGGGTGGTTCGGGAGGAGGAGGCTCAGGCGGAGGAG GATCAGGAGGCGGCGGAAGTGGAGGCGGAGGCTCCATGATTCCCGGA GGCCTCTCAGAAGCCAAACCAGCCACTCCTGAGATTCAGGAAATTGTC GACAAGGTCAAGCCGCAGCTGGAAGAGAAGACTGGCGAAACCTACGG GAAGCTAGAGGCGGTCCAGTACAAGACCCAAGTCGTCCATTACTATGT ACATTACAACGACCAGGGAACCAATTACTACATTAAAGTGCGGGCCGG AGATAATAAGTATATGCATCTTAAGGTGTTTAAGTCGTTGTGGGGCGA AAACCTGTTTGCCAAGTGGGAAGATCTTGTGCTCACCGGTTACCAAGT GGACAAGAACAAGGACGACGAGCTGACCGGGTTCGGAGGAGGCGGAT CAGGGGGTGGTGGTTCCGGCGGAGGTGGATCCGGAGGAGGTGGAAGC GGGGGAGGTGGTTCAGGAGGGGGCGGATCCATGATACCTGGCGGACT GTCGGAGGCGAAACCGGCCACCCCTGAAATCCAGGAGATTGTGGACA AGGTGAAACCCCAACTCGAGGAAAAAACCGGCGAAACGTACGGTAAA CTCGAAGCCGTGCAGTATAAGACCCAGGTCGTACACTACTATGTGCAC TATAACGATCAGGGAACGAACTACTACATCAAGGTCAGAGCTGGAGA CAATAAGTACATGCACCTCAAGGTTTTCAAGTCGTTATGGGGAGAAAA CCTCTTCGCGAAATGGGAGGATCTCGTGCTGACCGGCTACCAGGTGGA CAAAAATAAGGATGATGAACTCACCGGTTTCGGCGGTGGAGGATCGG GCGGTGGTGGTAGCGGAGGGGGAGGATCCGGTGGAGGGGGTAGCGGT GGAGGCGGTTCCGGTGGTGGCGGAAGCATGATCCCACGGGGTCTGTCC GAAGCCAAGCCAGCCACCCCAGAGATCCAAGAAATAGTGGATAAGGT GAAGCCCCAGCTTGAGGAAAAGACTGGAGAGACTTACGGAAAGCTCG AGGCTGTCCAATACAAAACACAGGTGCTGGCGAACTTCTTCCAAAGGA GATGGCCAGGCAGTACTAACTATTACATCAAAGTGCGCGCAGGAGATA ACAAATATATGCACTTGAAGGTGTTCAATGGACCGTGGAAGTTCCGGA ACACCGACAGAGGCGCCGACAGGGTCTTGACCGGGTACCAAGTCGAT AAGAACAAAGACGACGAACTGACCGGTTTC | 719 |
| 230 XT75 | ATCCCTGGAGGGCTGTCCGAGGCCAAGCCTGCTACGCCGGAAATTCAA GAAATCGTCGATAAGGTTAAGCCACAGCTCGAAGAGAAAACTGGGGA AACATACGGCAAACTTGAGGCGGTTCAGTACAAAACTCAGGTCGTGCA CTACTACGTGCACTACAACGATCAAGGGACCAACTACTATATCAAGGT CCGCGCGGGAGACAACAAATACATGCATCTCAAAGTGTTCAAATCGCT GTGGGGCGAGAACCTTTTCGCGAAGTGGGAGGATTTGGTGCTGACTGG ATATCAGGTCGATAAAAACAAGGATGACGAGCTTACTGGCTTTGCCGA | 720 |
| | GGCTGCAGCCAAAGAAGCCGCGGCGAAGGAAGCGGCGGCAAAGGAG GCAGCCGCAAAGGAAGCTGCCGCTAAAGAGGCCGCCGCAAAGATGAT TCCCGGAGGCCTCTCAGAAGCCAAACCAGCCACTCCTGAGATTCAGGA AATTGTCGACAAGGTCAAGCCGCAGCTGGAAGAGAAGACTGGCGAAA CCTACGGGAAGCTAGAGGCGGTCCAGTACAAGACCCAAGTCGTCCATT ACTATGTACATTACAACGACCAGGGAACCAATTACTACATTAAAGTGC GGGCCGGAGATAATAAGTATATGCATCTTAAGGTGTTTAAGTCGTTGT GGGGCGAAAACCTGTTTGCCAAGTGGGAAGATCTTGTGCTCACCGGTT ACCAAGTGGACAAGAACAAGGACGACGAGCTGACCGGGTTCGCTGAG GCTGCCGCCAAGGAAGCCGCAGCGAAGGAGGCCGCTGCGAAAGAAGC GGCAGCCAAGGAGGCGGCTGCCAAAGAGGCGGCCGCGAAGATGATAC CTGGCGGACTGTCGGAGGCGAAACCGGCCACCCCTGAAATCCAGGAG ATTGTGGACAAGGTGAAACCCCAACTCGAGGAAAAAACCGGCGAAAC GTACGGTAAACTCGAAGCCGTGCAGTATAAGACCCAGGTCGTACACTA CTATGTGCACTATAACGATCAGGGAACGAACTACTACATCAAGGTCAG AGCTGGAGACAATAAGTACATGCACCTCAAGGTTTTCAAGTCGTTATG GGGAGAAAACCTCTTCGCGAAATGGGAGGATCTCGTGCTGACCGGCTA CCAGGTGGACAAAAATAAGGATGATGAACTCACCGGTTTCGCTGAAGC AGCGGCGAAAGAGGCAGCGGCCAAGGAAGCCGCTGCCAAGGAAGCCG CCGCTAAGGAGGCTGCGGCTAAGGAAGCAGCCGCCAAGATGATCCCA CGGGGTCTGTCCGAAGCCAAGCCAGCCACCCCAGAGATCCAAGAAAT AGTGGATAAGGTGAAGCCCCAGCTTGAGGAAAAGACTGGAGAGACTT ACGGAAAGCTCGAGGCTGTCCAATACAAAACACAGGTGCTGGCGAAC TTCTTCCAAAGGAGATGGCCAGGCAGTACTAACTATTACATCAAAGTG CGCGCAGGAGATAACAAATATATGCACTTGAAGGTGTTCAATGGACCG TGGAAGTTCCGGAACACCGACAGAGGCGCCGACAGGGTCTTGACCGG GTACCAAGTCGATAAGAACAAAGACGACGAACTGACCGGATTC | |
| 230 XT76 | ATCCCTGGGGGACTCAGCGAGGCTAAGCCTGCGACTCCCGAAATCCAA GAGATCGTGGACAAAGTGAAGCCGCAACTGGAAGAAAAGACCGGAGA AACTTACGGGAAACTGGAGGCAGTGCAATACAAGACACAAGTGGTCC ACTATTACGTCCACTACAATGATCAGGGCACCAATTATTACATTAAGG TGCGAGCCGGCGATAACAAGTATATGCACCTGAAGGTCTTTAAGTCCC TGTGGGGAGAGAATCTGTTCGCAAAGTGGGAGGACCTCGTGCTTACTG GTTACCAGGTAGACAAGAATAAGGACGATGAGCTGACTGGTTTCGCCG AAGCTGCAGCGAAAGAGGCAGCTGCGAAAGAAGCGGCAGCCAAAGA AGCCGCTGCCAAGGAGGCTGCCGCCAAAGAGGCGGCCGCCAAAATGA TCCCTGGAGGGCTGTCCGAGGCCAAGCCTGCTACGCCGGAAATTCAAG AAATCGTCGATAAGGTTAAGCCACAGCTCGAAGAGAAAACTGGGGAA ACATACGGCAAACTTGAGGCGGTTCAGTACAAAACTCAGGTCGTGCAC TACTACGTGCACTACAACGATCAAGGGACCAACTACTATATCAAGGTC CGCGCGGGAGACAACAAATACATGCATCTCAAAGTGTTCAAATCGCTG TGGGGCGAGAACCTTTTCGCGAAGTGGGAGGATTTGGTGCTGACTGGA TATCAGGTCGATAAAAACAAGGATGACGAGCTTACTGGCTTTGCCGAA GCCGCAGCGAAGGAAGCAGCCGCGAAGGAGGCAGCGGCAAAAGAGG CCGCGGCTAAAGAAGCTGCCGCAAAGGAGGCCGCCGCAAAGATGATT CCCGGAGGCCTCTCAGAAGCCAAACCAGCCACTCCTGAGATTCAGGAA ATTGTCGACAAGGTCAAGCCGCAGCTGGAAGAGAAGACTGGCGAAAC CTACGGGAAGCTAGAGGCGGTCCAGTACAAGACCCAAGTCGTCCATTA CTATGTACATTACAACGACCAGGGAACCAATTACTACATTAAAGTGCG GGCCGGAGATAATAAGTATATGCATCTTAAGGTGTTTAAGTCGTTGTG GGGCGAAAACCTGTTTGCCAAGTGGGAAGATCTTGTGCTCACCGGTTA CCAAGTGGACAAGAACAAGGACGACGAGCTGACCGGGTTCGCTGAGG CAGCCGCAAAAGAAGCAGCAGCAAAGGAGGCCGCCGCCAAGGAAGC GGCGGCAAAGGAAGCTGCTGCTAAGGAGGCGGCTGCCAAGATGATAC CTGGCGGACTGTCGGAGGCGAAACCGGCCACCCCTGAAATCCAGGAG ATTGTGGACAAGGTGAAACCCCAACTCGAGGAAAAAACCGGCGAAAC GTACGGTAAACTCGAAGCCGTGCAGTATAAGACCCAGGTCGTACACTA CTATGTGCACTATAACGATCAGGGAACGAACTACTACATCAAGGTCAG AGCTGGAGACAATAAGTACATGCACCTCAAGGTTTTCAAGTCGTTATG GGGAGAAAACCTCTTCGCGAAATGGGAGGATCTCGTGCTGACCGGCTA | 721 |
| | CCAGGTGGACAAAAATAAGGATGATGAACTCACCGGTTTCGCTGAAGC GGCCGCTAAAGAGGCTGCGGCTAAGGAAGCCGCGGCGAAGGAAGCAG CGGCCAAGGAGGCTGCTGCAAAGGAGGCCGCCGCTAAGATGATCCCA CGGGGTCTGTCCGAAGCCAAGCCAGCCACCCCAGAGATCCAAGAAAT AGTGGATAAGGTGAAGCCCCAGCTTGAGGAAAAGACTGGAGAGACTT ACGGAAAGCTCGAGGCTGTCCAATACAAAACACAGGTGCTGGCGAAC TTCTTCCAAAGGAGATGGCCAGGCAGTACTAACTATTACATCAAAGTG CGCGCAGGAGATAACAAATATATGCACTTGAAGGTGTTCAATGGACCG TGGAAGTTCCGGAACACCGACAGAGGCGCCGACAGGGTCTTGACCGG GTACCAAGTCGATAAGAACAAAGACGACGAACTGACCGGATTC | |
| 230 XT83 | ATACCTGGCGGTCTGAGCGAAGCAAAGCCGGCCACTCCTGAAATTCAG GAAATCGTGGACAAGGTTAAGCCCCAGCTCGAGGAAAAGACCGGCGA AACCTATGGCAAACTGGAAGCGGTGCAGTACAAGACTCAGGTCGTGC ACTATTACGTGCACTATAACGACCAAGGCACTAACTATTACATCAAGG TCCGAGCGGGAGACAACAAGTACATGCACCTGAAAGTGTTTAAGAGC CTGTGGGGGGAAAACCTCTTCGCCAAATGGGAAGATCTGGTGCTCACC GGGTACCAGGTCGACAAGAACAAAGACGACGAGCTGACCGGGTTCGC TGAGGCAGCGGCAAAAGAAGCCGCGGCTAAAGAGGCTGCCGCCAAGG AAGCCGCCGCAAAGGAGGCCGCTGCCAAAGAGGCCGCCGCCAAGATG ATTCCCGGGGGACTTTCGGAAGCCAAGCCTGCCACACCGGAGATCCAA GAGATTGTGGACAAAGTGAAGCCTCAGCTCGAAGAAAAGACTGGAGA GACTTATGGAAAGCTGGAGGCCGTCCAGTACAAAACCCAAGTCGTGCA TTACTACGTGCATTACAACGATCAGGGGACTAACTACTATATCAAAGT GCGCGCCGGGGATAACAAATATATGCATCTTAAGGTGTTTAAGTCCCT CTGGGGCGAAAACTTGTTCGCGAAATGGGAGGACTTGGTGCTTACTGG ATACCAAGTCGACAAAAACAAGGATGATGAACTGACGGGATTCGCCG AAGCCGCTGCTAAGGAGGCAGCCGCGAAAGAAGCGGCCGCTAAGGAA GCGGCGGCCAAAGAAGCTGCGGCGAAGGAAGCTGCCGCGAAGATGAT CCCGGGAGGACTGAGCGAGGCCAAGCCAGCAACCCCGGAGATTCAAG AAATCGTCGACAAGGTCAAGCCCCAGTTGGAAGAGAAAACCGGGGAA ACCTACGGCAAACTCGAAGCCGTGCAGTATAAGACTCAAGTGGTGCAC TACTACGTCCACTACAACGACCAGGGCACCAACTACTACATTAAGGTC CGCGCTGGTGACAACAAATACATGCATCTGAAGGTCTTTAAGTCGCTG TGGGGAGAGAACCTGTTCGCGAAGTGGGAGGACCTGGTGCTGACCGG CTACCAAGTGGACAAGAATAAGGATGACGAGCTCACCGGCTTCGGAG GAGGAGGGTCCGGTGGTGGAGGTTCGGGAGGGGGTGGTTCCGGAGGG GGAGGTTCCGGTGGAGGCGGAAGTGGCGGAGGAGGATCCGGCGGTGG TGGATCCGGTGGCGGTGGATCAGGAGGCGGAGGCTCAGGAGGTGGCG GAAGCGGAGGAGGGGGTTCAGGAGGAGGCGGCTCCATGATTCCGAGG GGTCTGTCAGAGGCTAAGCCCGCGACCCCTGAAATCCAGGAGATCGTG GATAAGGTCAAACCGCAGCTGGAGGAGAAAACTGGGGAGACTTACGG GAAGCTCGAGGCCGTGCAATACAAGACCCAAGTGCTCGCCAACTTCTT CCAGCGGAGATGGCCCGGAAGCACGAATTACTACATCAAAGTCCGGG CCGGAGATAATAAGTATATGCACTTGAAGGTGTTCAACGGGCCCTGGA AGTTCCGGAACACCGATAGAGGCGCAGATCGCGTGCTGACTGGCTACC AGGTGGACAAAAATAAGGACGACGAACTGACCGGTTTC | 722 |
| 230 XT84 | ATCCCTGGAGGCCTGAGCGAGGCCAAGCCAGCTACCCCGGAGATTCAG GAAATCGTGGACAAGGTCAAACCGCAGCTCGAGGAAAAGACTGGAGA AACCTATGGCAAACTCGAAGCGGTCCAGTACAAGACTCAAGTGGTGCA CTACTACGTGCATTACAATGACCAGGGCACGAACTACTACATCAAAGT CCGGGCTGGCGACAACAAGTATATGCATCTAAAGGTCTTTAAGTCCCT CTGGGGCGAAAACCTGTTCGCCAAATGGGAAGATCTGGTGCTCACTGG GTACCAAGTCGATAAGAACAAAGACGACGAACTCACCGGGTTCGGAG GAGGGGGTTCCGGTGGAGGTGGCAGCGGAGGTGGTGGCTCAATGATT CCGGGAGGACTGTCGGAAGCTAAGCCGGCCACTCCCGAAATTCAAGA GATCGTGGATAAGGTTAAACCCCAGCTCGAAGAAAAGACCGGGGAAA CTTATGGAAAGCTCGAAGCCGTGCAGTACAAAACCCAAGTCGTGCATT ACTACGTCCACTATAACGATCAGGGGACCAACTACTATATCAAGGTCC GCGCCGGAGATAATAAGTACATGCATCTGAAGGTGTTCAAGTCGCTCT GGGGGGAAAACCTTTTCGCGAAATGGGAGGATCTCGTGCTGACCGGGT | 723 |
| | ACCAGGTCGACAAGAACAAGGATGACGAGCTGACCGGCTTCGGTGGA GGAGGAAGCGGCGGAGGTGGAAGCGGTGGTGGCGGATCCATGATCCC CGGAGGCCTTTCCGAAGCGAAGCCTGCCACCCCTGAGATCCAAGAGAT TGTGGACAAAGTCAAGCCACAGCTGGAGGAGAAAACCGGAGAAACTT ACGGAAAGCTGGAGGCCGTGCAATATAAGACCCAGGTCGTGCACTATT ACGTGCACTACAACGACCAGGGAACTAACTATTACATCAAGGTCAGAG CGGGAGATAACAAGTACATGCACCTGAAAGTGTTTAAGTCGCTGTGGG GAGAGAATCTGTTCGCTAAGTGGGAGGACCTGGTGCTGACTGGCTACC AGGTGGACAAGAATAAGGACGATGAGCTTACCGGATTCGGAGGGGGA GGCTCTGGAGGAGGCGGCTCAGGAGGTGGAGGATCGGGGGGTGGAGG TTCCGGCGGAGGAGGATCAGGGGGCGGAGGAAGTGGTGGAGGCGGTT CGGGAGGAGGTGGATCTGGCGGAGGCGGATCAGGAGGAGGAGGGAGT GGCGGTGGTGGATCAGGCGGTGGAGGGTCCATGATTCCTCGGGGACTG AGCGAAGCCAAGCCCGCGACTCCGGAAATCCAGGAGATCGTCGATAA GGTCAAGCCCCAGCTTGAAGAAAAAACCGGCGAAACCTACGGGAAGC TCGAGGCCGTCCAATACAAGACACAAGTGCTGGCCAACTTCTTCCAAC GGCGCTGGCCAGGATCCACGAATTACTACATTAAGGTCCGAGCCGGCG ATAACAAATACATGCACTTGAAAGTGTTCAACGGACCTTGGAAGTTCA GGAACACTGACCGGGGCGCAGACAGAGTGCTTACCGGCTACCAAGTG GACAAAAACAAGGACGACGAGTTGACTGGATTC | |
| 230 XT85 | ATTCCGGGAGGCCTTAGCGAAGCGAAGCCTGCGACGCCAGAGATCCA GGAAATTGTGGACAAAGTGAAGCCCCAACTGGAGGAGAAAACCGGAG AAACCTATGGAAAGCTGGAAGCGGTGCAGTACAAGACCCAAGTGGTG CATTACTACGTGCACTATAACGATCAGGGGACTAATTACTACATTAAG GTCCGGGCTGGGGATAACAAGTATATGCACCTTAAGGTATTCAAGTCA CTGTGGGGGGAGAACCTGTTTGCTAAGTGGGAGGACCTGGTGCTGACC GGTTACCAGGTGGACAAAAATAAGGACGACGAACTGACTGGGTTCGG TGGCGGAGGTTCGGGAGGGGGAGGAAGCGGAGGCGGAGGATCCATGA TTCCGGGTGGACTCTCCGAGGCCAAGCCTGCCACTCCCGAAATCCAGG AGATCGTGGACAAGGTCAAGCCTCAGCTGGAGGAAAAGACTGGAGAA ACATACGGGAAACTGGAAGCCGTGCAGTATAAGACCCAGGTGGTGCA CTATTACGTACACTACAACGACCAGGGCACCAACTACTACATCAAAGT CCGGGCCGGCGATAATAAGTACATGCACCTGAAGGTGTTCAAGTCCCT CTGGGGCGAAAACCTGTTCGCGAAGTGGGAAGATCTCGTGCTCACCGG GTACCAAGTGGACAAGAACAAGGATGACGAGCTGACCGGCTTTGGCG GAGGAGGCTCAGGAGGGGGTGGTAGCGGAGGAGGGGGTTCCATGATC CCCGGAGGTCTGAGCGAAGCCAAGCCCGCAACACCGGAGATCCAAGA GATCGTCGACAAGGTTAAGCCGCAGCTGGAAGAAAAGACCGGCGAAA CCTACGGCAAACTGGAGGCCGTGCAATACAAAACCCAGGTCGTGCACT ACTACGTCCACTACAATGACCAAGGCACTAATTATTACATCAAGGTCC GCGCTGGCGATAACAAATATATGCATCTGAAAGTGTTTAAGTCGCTGT GGGGAGAGAACCTCTTCGCCAAATGGGAGGACTTGGTGTTGACCGGCT ACCAGGTCGACAAAAACAAGGACGATGAGCTTACCGGCTTTGCCGAA GCCGCGGCAAAGGAGGCCGCCGCGAAAGAAGCGGCGGCCAAAGAAG CCGCCGCTAAGGAGGCGGCCGCTAAAGAGGCGGCAGCCAAAGAGGCT GCCGCCAAGGAAGCTGCGGCTAAGGAAGCAGCGGCGAAGGAGGCTGC TGCCAAGGAGGCAGCCGCGAAGGAAGCGGCCGCCAAGATGATCCCTA GGGGCCTGTCCGAAGCTAAGCCAGCCACCCCTGAGATTCAGGAAATCG TGGATAAAGTCAAGCCACAACTGGAAGAGAAAACTGGGGAAACTTAC GGGAAGCTCGAAGCCGTCCAGTACAAAACTCAAGTGCTGGCCAACTTC TTCCAACGGAGATGGCCGGGATCCACCAACTATTACATAAAAGTCCGC GCCGGAGACAACAAATACATGCATCTCAAGGTCTTTAACGGGCCGTGG AAGTTCCGCAACACTGACCGGGGAGCAGACAGAGTGCTGACTGGTTAC CAAGTCGACAAGAATAAGGATGATGAGCTGACGGGATTC | 724 |
| 230 XT86 | ATCCCTGGAGGGCTCAGCGAGGCCAAACCCGCCACTCCTGAGATTCAG GAGATCGTGGATAAGGTCAAACCGCAGCTCGAGGAGAAAACGGGCGA AACTTACGGGAAGCTGGAAGCCGTGCAGTATAAGACCCAAGTGGTGC ATTACTACGTCCATTACAACGATCAGGGCACCAATTACTACATTAAGG TCCGGGCTGGTGACAACAAATACATGCATCTGAAGGTCTTTAAGTCGC TCTGGGGGGAAAACCTCTTCGCGAAGTGGGAAGATCTGGTCCTTACCG | 725 |
| | GATACCAAGTGGACAAGAACAAAGATGACGAGTTGACCGGGTTTGCC GAGGCGGCAGCTAAGGAGGCAGCCGCGAAGGAAGCGGCCGCGAAGAT GATCCCTGGCGGACTGTCCGAAGCCAAGCCTGCGACCCCTGAAATTCA GGAAATCGTGGACAAAGTCAAGCCCCAGTTGGAAGAAAAGACCGGCG AAACATACGGAAAGCTAGAGGCAGTGCAGTACAAGACCCAGGTCGTG CACTACTATGTGCACTACAACGACCAGGGTACTAACTACTATATCAAG GTTCGCGCGGGAGACAACAAGTATATGCATCTCAAAGTGTTCAAGAGC CTGTGGGGCGAAAACCTGTTCGCCAAATGGGAGGACCTGGTGCTGACC GGCTACCAGGTGGATAAGAACAAGGACGACGAGCTGACCGGGTTCGC TGAAGCCGCAGCCAAGGAGGCTGCCGCCAAGGAAGCCGCCGCAAAGA TGATTCCGGGAGGATTGTCCGAAGCGAAGCCGGCCACTCCAGAGATCC AAGAAATTGTGGACAAGGTTAAACCGCAACTGGAGGAAAAGACTGGC GAAACCTATGGAAAGCTCGAGGCCGTCCAGTACAAAACTCAGGTCGTA CACTACTACGTGCACTATAACGACCAAGGGACCAATTATTACATCAAG GTCCGCGCCGGAGATAACAAATATATGCACCTCAAGGTGTTCAAGTCG CTGTGGGGGGAGAACCTGTTTGCCAAGTGGGAGGATCTTGTGCTGACG GGGTACCAGGTTGACAAGAATAAGGACGATGAACTTACCGGCTTTGGA GGGGGTGGTAGCGGAGGCGGTGGTTCCGGTGGTGGCGGAAGCGGAGG AGGAGGCTCCGGTGGAGGAGGATCAGGAGGCGGAGGATCGGGTGGCG GAGGTTCTGGCGGAGGAGGTTCCGGAGGGGGAGGAAGTGGCGGTGGA GGTTCGGGCGGTGGTGGATCAGGGGGAGGCGGATCCATGATCCCCCG AGGACTGAGCGAGGCTAAGCCAGCGACCCCGGAAATCCAAGAGATTG TCGACAAGGTCAAGCCGCAGCTGGAAGAGAAAACCGGGGAAACCTAC GGCAAACTGGAAGCGGTGCAATACAAGACTCAGGTGCTCGCAAACTTC TTCCAACGGCGCTGGCCTGGATCCACCAACTACTACATCAAAGTCCGG GCCGGGGATAACAAGTACATGCACCTGAAAGTGTTTAACGGCCCGTGG AAGTTCCGGAACACCGACAGAGGAGCCGACAGAGTGCTGACTGGATA CCAGGTCGACAAAAACAAAGACGACGAACTGACTGGCTTC | |
| 230 XT87 | ATTCCGGGTGGACTGTCCGAGGCCAAGCCAGCCACCCCCGAAATCCAG GAAATTGTGGACAAGGTCAAGCCACAGCTGGAGGAAAAGACCGGCGA AACCTATGGAAAGCTCGAGGCAGTGCAGTATAAGACCCAGGTGGTCC ACTACTATGTGCACTACAATGACCAAGGGACCAACTATTACATCAAAG TCCGCGCCGGAGACAACAAGTACATGCATCTGAAGGTTTTCAAGTCCC TTTGGGGCGAAAACCTGTTTGCCAAATGGGAGGACTTGGTGCTGACGG GGTACCAAGTGGATAAGAACAAAGATGACGAACTCACTGGGTTCGCC GAAGCAGCCGCTAAGGAGGCGGCCGCCAAAGAGGCGGCTGCCAAGAT GATCCCGGGGGGTCTTTCGGAAGCGAAGCCTGCAACCCCCGAGATCCA AGAGATCGTGGACAAAGTCAAGCCCCAACTGGAAGAGAAAACTGGAG AGACTTACGGGAAGCTCGAAGCCGTGCAGTACAAGACTCAGGTCGTGC ACTACTACGTCCACTACAACGATCAGGGCACCAATTACTACATCAAGG TCCGGGCCGGAGATAATAAGTATATGCATCTCAAGGTGTTCAAGTCGT TGTGGGGAGAGAACCTCTTCGCGAAGTGGGAGGATCTCGTGCTGACTG GTTATCAAGTGGACAAGAACAAGGATGACGAGCTGACCGGATTCGCT GAAGCGGCAGCCAAAGAAGCCGCGGCGAAGGAAGCCGCCGCGAAGAT GATTCCCGGAGGCCTGTCAGAGGCTAAGCCTGCCACTCCGGAAATTCA GGAGATCGTCGACAAGGTTAAGCCGCAGCTGGAAGAAAAAACCGGGG AAACCTACGGAAAGCTGGAAGCGGTGCAATACAAGACCCAAGTGGTG CATTACTACGTGCACTATAACGACCAGGGTACAAACTACTATATCAAA GTCAGAGCGGGAGATAACAAATACATGCACCTTAAGGTCTTTAAGTCA CTGTGGGGGGAGAACCTGTTCGCTAAGTGGGAAGATCTGGTGCTCACC GGGTACCAGGTCGACAAAAACAAGGACGACGAGCTCACCGGCTTTGC CGAAGCCGCTGCAAAGGAAGCCGCAGCCAAGGAGGCTGCGGCAAAAG AAGCAGCGGCGAAAGAGGCTGCCGCAAAAGAGGCCGCGGCAAAGGA GGCAGCGGCTAAAGAAGCTGCCGCCAAGGAAGCAGCAGCTAAGGAAG CTGCAGCGAAGGAGGCCGCTGCGAAAGAAGCGGCCGCGAAAATGATT CCGAGAGGCCTGAGCGAAGCCAAGCCGGCCACTCCTGAGATTCAGGA AATCGTGGATAAAGTGAAGCCTCAGTTGGAAGAAAAGACTGGCGAAA CTTACGGAAAGTTGGAAGCCGTACAGTACAAAACCCAGGTCCTGGCCA ACTTCTTTCAACGCCGCTGGCCTGGATCCACCAACTACTACATAAAAG TGCGGGCCGGCGATAACAAGTATATGCACCTGAAAGTGTTCAACGGAC | 726 |
| | CGTGGAAGTTCCGGAACACTGACAGGGGTGCCGACAGAGTCCTGACC GGATACCAGGTGGATAAAAACAAAGACGACGAACTGACTGGATTC | |
| 3t0 gly XT58 | ATCCCTGGGGGACTCTCCGAAGCGAAGCCTGCCACCCCCGAAATCCAG GAAATCGTGGACAAAGTCAAGCCACAGTTGGAGGAGAAAACTGGAGA GACTTACGGCAAACTGGAAGCCGTGCAGTATAAGACCCAGGTCGTGG GTGGAGGGGGAGGTGGAGGAGGGGGTGGAACCAATTACTACATCAAA GTGCGGGCCGGCGATAACAAGTACATGCATTTGAAGGTGTTCAAGTCA CTAGGCGGAGGCGGAGGAGGAGGTGGTGGAGAAGATCTGGTGCTCAC GGGATACCAGGTGGACAAGAATAAGGACGACGAGCTTACTGGATTCG CGGAAGCTGCTGCAAAGGAAGCTGCAGCGAAGGAAGCCGCCGCGAAG GAGGCTGCTGCCAAAGAGGCTGCCGCTAAGGAGGCAGCGGCCAAGAT GATCCCTGGAGGCCTCTCCGAGGCTAAGCCGGCTACTCCAGAGATTCA AGAGATCGTCGACAAAGTGAAGCCCCAGCTGGAAGAAAAGACTGGGG AAACTTACGGGAAGCTGGAGGCCGTGCAATACAAAACTCAAGTCGTTG GGGGGGGAGGAGGTGGTGGGGGAGGGACCAACTACTACATAAAG GTCCGCGCAGGAGATAATAAGTACATGCACCTCAAAGTGTTTAAGTCA CTGGGTGGCGGTGGTGGAGGAGGCGGTGGAGAGGACCTCGTGCTGAC GGGCTACCAGGTCGATAAGAACAAAGACGACGAACTGACTGGCTTCG CCGAAGCTGCGGCAAAGGAGGCCGCAGCCAAGGAGGCGGCAGCTAAA GAGGCCGCTGCAAAAGAAGCCGCGGCCAAAGAAGCGGCGGCGAAGAT GATTCCTGGGGGGCTCTCAGAGGCCAAGCCGGCAACCCCTGAGATTCA GGAAATTGTGGATAAGGTCAAACCGCAGCTGGAGGAAAAGACCGGGG AAACCTACGGGAAGTTGGAGGCAGTGCAGTACAAAACACAAGTGGTC GGTGGAGGTGGGGGTGGAGGCGGGGGAGGTACTAATTACTATATCAA GGTCCGAGCTGGCGACAACAAGTATATGCACCTGAAGGTCTTTAAGTC CTTGGGCGGTGGAGGAGGTGGTGGCGGAGGAGAGGACTTGGTGTTGA CTGGGTACCAAGTGGATAAAAACAAGGATGACGAGCTGACTGGTTTCG CTGAGGCAGCCGCAAAGGAAGCTGCCGCAAAAGAGGCGGCTGCCAAG GAAGCGGCCGCGAAAGAAGCAGCGGCTAAGGAAGCAGCCGCCAAAAT GATCCCGAGAGGGCTGTCCGAAGCCAAGCCCGCGACTCCTGAGATCCA GGAGATTGTGGACAAGGTCAAGCCTCAGCTTGAAGAGAAAACCGGGG AGACTTATGGGAAGCTCGAGGCCGTCCAGTACAAGACCCAAGTGCTCG CCAACTTCTTCCAACGGAGATGGCCCGGCTCGACCAACTATTACATTA AGGTCAGAGCCGGAGACAACAAATACATGCATCTGAAAGTGTTCAAC GGCCCGTGGAAGTTCCGGAACACCGACCGCGGAGCAGACAGGGTCCT GACCGGATATCAGGTCGACAAGAACAAGGACGATGAGCTCACCGGAT TC | 727 |
| 310 gly XT59 | ATCCCTGGAGGCCTCTCCGAGGCTAAGCCGGCCACTCCAGAGATTCAA GAGATCGTCGACAAAGTCAAGCCCCAGCTGGAAGAAAAGACTGGGGA AACTTACGGGAAGCTGGAGGCGGTGCAATACAAAACTCAGGTCGTCG GAGGCGGAGGAGGAGGTGGTGGCGGAGGGACCAACTACTATATAAAG GTCCGCGCGGGAGATAATAAGTACATGCACCTCAAAGTGTTTAAGTCA CTGGGTGGCGGTGGTGGAGGAGGCGGTGGAGAGGACCTCGTGCTGAC GGGATACCAGGTCGATAAGAACAAAGATGACGAACTGACTGGATTCG CCGAAGCTGCGGCAAAGGAGGCCGCAGCCAAGGAGGCAGCGGCTAAG GAAGCAGCTGCAAAGGAGGCCGCAGCCAAGGAAGCGGCCGCGAAGAT GATTCCTGGGGGGCTCTCAGAGGCAAAGCCGGCGACCCCTGAAATTCA GGAAATTGTGGATAAGGTCAAACCTCAGCTGGAGGAAAAAACCGGGG AAACCTATGGGAAGTTGGAAGCCGTGCAGTACAAAACACAGGTGGTC GGGGGAGGTGGAGGTGGAGGCGGCGGTGGAACTAATTACTATATCAA GGTCCGAGCTGGCGACAACAAGTATATGCACCTGAAGGTTTTCAAGAG CTTGGGCGGTGGAGGAGGTGGTGGCGGAGGAGAGGACTTAGTGCTGA CTGGGTACCAAGTCGATAAAAACAAGGATGACGAGCTGACTGGTTTCG CTGAGGCAGCCGCAAAGGAAGCTGCCGCGAAAGAGGCGGCTGCCAAG GAAGCTGCCGCAAAAGAAGCAGCGGCCAAGGAAGCAGCCGCTAAGAT GATACCCAGAGGCCTGTCCGAAGCCAAGCCCGCCACTCCTGAGATCCA GGAGATTGTGGACAAGGTCAAGCCTCAACTTGAAGAGAAAACCGGAG AGACTTATGGGAAGCTCGAGGCCGTCCAGTACAAGACACAAGTCCTGG CCAACTTCTTCCAACGGAGATGGCCCGGATCGACCAATTACTACATCA AAGTCCGCGCCGGAGACAACAAATACATGCATCTGAAAGTGTTCAACG | 728 |
| | GCCCGTGGAAGTTCCGCAACACCGACCGGGGAGCAGACAGGGTGCTC ACTGGCTACCAAGTGGACAAGAATAAGGATGATGAACTGACCGGCTTT GCTGAGGCTGCGGCGAAGGAAGCTGCCGCTAAAGAAGCCGCCGCCAA GGAAGCCGCTGCGAAGGAGGCTGCCGCCAAAGAAGCGGCGGCCAAGA TGATCCCTGGCGGACTGTCCGAGGCCAAGCCTGCAACCCCCGAGATTC AGGAGATCGTGGACAAAGTGAAGCCGCAGCTCGAGGAAAAGACCGGA GAAACTTATGGAAAGCTGGAAGCCGTACAGTATAAGACCCAGGTCGT GGGTGGCGGCGGAGGTGGAGGAGGGGGTGGCACCAACTATTACATCA AGGTTCGCGCCGGCGATAACAAATATATGCATTTGAAGGTCTTTAAGT CGTTGGGAGGTGGCGGGGGTGGAGGAGGAGGGGAGGACCTGGTGCTG ACCGGCTACCAGGTGGACAAAAACAAAGACGACGAACTCACCGGGTT CGCTGAAGCGGCTGCAAAAGAGGCCGCGGCTAAAGAGGCAGCTGCCA AAGAGGCTGCAGCGAAGGAGGCTGCTGCTAAGGAGGCGGCCGCCAAA ATGATCCCGGGAGGCCTGTCAGAAGCGAAGCCAGCCACCCCGGAAAT TCAAGAAATCGTGGATAAAGTTAAGCCTCAGCTCGAAGAAAAAACGG GCGAAACCTACGGCAAACTTGAAGCCGTCCAATACAAGACCCAAGTG GTCGGAGGTGGGGGTGGTGGTGGGGGAGGAACGAACTACTACAT TAAGGTCCGGGCCGGAGATAACAAGTACATGCACTTGAAGGTGTTCAA GTCCCTTGGTGGAGGGGGAGGAGGAGGAGGCGGAGAAGATCTCGTCC TGACCGGATATCAGGTCGACAAGAACAAGGACGATGAGCTGACCGGA TTC | |

### Stefin A protein variant-Conjugate

In another aspect, the present invention relates to a conjugate comprising a Stefin A protein variant specifically binding to the CD40L.

In another aspect, the present invention relates to the use of a conjugate of the Stefin A protein variant specifically binding to the CD40L.

As used herein, the term "Conjugate" or "conjugation" refers the joining or linking together of two or more compounds resulting in the formation of another compound, by any joining or linking methods known in the art. It can also refer to a compound that is generated by the joining or linking together two or more compounds. For example, the stefin A protein variant specifically binding to the CD40L linked directly or indirectly to at least one chemical moiety or polypeptide is an exemplary conjugate. Such conjugates include fusion proteins, those produced by chemical conjugates and those produced by any other methods. In some embodiments, a stefin A protein variant-conjugate can be broadly understood as including a fusion protein. For a more specific example, the conjugate refers to a conjugates comprising at least one moiety conjugated thereto through a chemical conjugation other than through the formation of a contiguous peptide bond through the C-terminus or N-terminus of the polypeptide portion of the stefin A protein variant specifically binding to the CD40L or the fusion protein of the present invention. The conjugate may be, for example, a stefin A protein variant-drug conjugate, and the stefin A protein variant-drug conjugate may include at least one pharmacologically active moiety.

In some embodiments, the conjugate may also include at least one functional moiety such as a wide variety of functional groups, substituents or moieties. In some embodiments, the Functional Moieties, for example, include but not limited to a label; a dye; an immunoadhesion molecule; a radionuclide; a cytotoxic compound; a drug; an affinity label; a photoaffinity label; a reactive compound; a resin; a second protein or polypeptide or polypeptide analog; an antibody or antibody fragment; a metal chelator; a cofactor; a fatty acid; a carbohydrate; a polynucleotide; a DNA; a RNA; an antisense polynucleotide; a saccharide; a water-soluble dendrimer; a cyclodextrin; an inhibitory ribonucleic acid; a biomaterial; a nanoparticle; a spin label; a fluorophore, a metal-containing moiety; a radioactive moiety; a novel functional group; a group that covalently or noncovalently interacts with other molecules; a photocaged moiety; an actinic radiation excitable moiety; a photoisomerizable moiety; biotin; a derivative of biotin; a biotin analogue; a moiety incorporating a heavy atom; a chemically cleavable group; a photocleavable group; an elongated side chain; a carbon-linked sugar; a redox-active agent; an amino thioacid; a toxic moiety; an isotopically labeled moiety; a biophysical probe; a phosphorescent group; a chemiluminescent group; an electron dense group; a magnetic group; an intercalating group; a chromophore; an energy transfer agent; a biologically active agent; a detectable label; a small molecule; a quantum dot; a nanotransmitter; a radionucleotide; a radiotransmitter; a neutron-capture agent; or any combination of the above, or any other desirable compound or substance.

### - Labels and Detectable Moieties

In some embodiments, the conjugate comprises a functional moiety. In some embodiments, the Functional Moiety is a detectable label. For example, the detectable label, can be a fluorescent label, radioactive label, enzymatic label or any other label known to the skilled person. In some embodiments, the Functional Moiety is a detectable label that can be included as part of a conjugate to form certain conjugate suitable for medical imaging. As used herein, the term "medical imaging" is meant any technique used to visualize an internal region of the human or animal body, for the purposes of diagnosis, research or therapeutic treatment. For instance, the AFFIMER^{®} agent can be detected (and quantitated) by radioscintigraphy, magnetic resonance imaging (MRI), computed tomography (CT scan), nuclear imaging, positron emission comprising a metal tomography (PET) contrast agent, optical imaging (such as fluorescence imaging including near-infrared fluorescence (NIRF) imaging), bioluminescence imaging, or combinations thereof. The Functional Moiety is optionally a contrast agent for X-ray imaging. Agents useful in enhancing such techniques are those materials that enable visualization of a particular locus, organ or disease site within the body, and/or that lead to some improvement in the quality of the images generated by the imaging techniques, providing improved or easier interpretation of those images. Such agents are referred to herein as contrast agents, the use of which facilitates the differentiation of different parts of the image, by increasing the "contrast" between those different regions of the image. The term "contrast agents" thus encompasses agents that are used to enhance the quality of an image that may nonetheless be generated in the absence of such an agent (as is the case, for instance, in MRI), as well as agents that are prerequisites for the generation of an image (as is the case, for instance, in nuclear imaging).

In some embodiments, the detectable label includes a chelate moiety for chelating a metal, e.g., a chelator for a radiometal or paramagnetic ion. In some embodiments, the detectable label is a chelator for a radionuclide useful for radiotherapy or imaging procedures. Radionuclides useful within the present disclosure include gamma-emitters, positron-emitters, Auger electron-emitters, X-ray emitters and fluorescence-emitters, with beta- or alpha-emitters for therapeutic use. Examples of radionuclides useful as toxins in radiation therapy include: ⁴³K, ⁴⁷Sc, ⁵¹Cr, ⁵⁷Co, ⁵⁸Co, ⁵⁹Fe , ⁶⁴Cu, ⁶⁷Ga, ⁶⁷Cu, ⁶⁸Ga, ⁷¹Ge, ⁷⁵Br, ⁷⁶Br , ⁷⁷Br, ⁷⁷As, ⁸¹Rb, ⁹⁰Y, ⁹⁷Ru, ^{99m}Tc, ¹⁰⁰Pd, ¹⁰¹Rh, ¹⁰³Pb, ¹⁰⁵Rh, ¹⁰⁹Pd, ¹¹¹Ag, ¹¹¹In, ¹¹³In, ¹¹⁹Sb ¹²¹Sn, ¹²³I, ¹²⁵I, ¹²⁷Cs, ¹²⁸Ba, ¹²⁹Cs, ¹³¹I, ¹³¹Cs, ¹⁴³Pr, ¹⁵³Sm, ¹⁶¹Tb, ¹⁶⁶HO, ¹⁶⁹Eu, ¹⁷⁷Lu, ¹⁸⁶Re, ¹⁸⁸Re, ¹⁸⁹Re, ¹⁹¹Os, ¹⁹³Pt, ¹⁹⁴Ir, ¹⁹⁷Hg, ¹⁹⁹Au, ²⁰³Pb, ²¹¹At, ²¹²Pb, ²¹²Bi and ²¹³Bi. Conditions under which a chelator will coordinate a metal are described, for example, by Gansow et al., U.S. Pat. NOS: 4,831,175, 4,454,106 and 4,472,509. Examples of chelators includes, merely to illustrate, 1,4,7-triazacyclononane-N,N',N"-triacetic acid (NOTA) 1,4,7,10-tetraazacyclododecane-N,N',N",N‴-tetraacetic acid (DOTA) 1 ,4,8,11-tetraazacyclotetradecane-N,N',N",N‴-tetraacetic acid (TETA).

In some embodiments, other detectable isotopes that can be incorporated directly into the amino acid residues of the AFFIMER^{®} polypeptide or which otherwise do not require a chelator, include ³H, ¹⁴C, ³²P, ³⁵S and ³⁶Cl.

Paramagnetic ions, useful for diagnostic procedures, may also be administered. Examples of paramagnetic ions include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), erbium (III), or combinations of these paramagnetic ions.

Examples of fluorescent labels include, but are not restricted to, organic dyes (e.g., cyanine, fluorescein, rhodamine, Alexa Fluors, Dylight fluors, ATTO Dyes, BODIPY Dyes, etc.), biological fluorophores (e.g., green fluorescent protein (GFP), R-Phycoerythrin, etc.), and quantum dots.

Non-limiting fluorescent compound that may be used in the present disclosure include, Cy5, Cy5.5 (also known as Cy5++), Cy2, fluorescein isothiocyanate (FITC), tetramethylrhodamine isothiocyanate (TRITC), phycoerythrin, Cy7, fluorescein (FAM), Cy3, Cy3.5 (also known as Cy3++), Texas Red, LightCycler-Red 640, LightCycler Red 705, tetramethylrhodamine (TMR), rhodamine, rhodamine derivative (ROX), hexachlorofluorescein (HEX), rhodamine 6G (R6G), the rhodamine derivative JA133, Alexa Fluorescent Dyes (such as Alexa Fluor 488, Alexa Fluor 546, Alexa Fluor 633, Alexa Fluor 555, and Alexa Fluor 647), 4',6-diamidino-2-phenylindole (DAPI), Propidium iodide, AMCA, Spectrum Green, Spectrum Orange, Spectrum Aqua, Lissamine, and fluorescent transition metal complexes, such as europium. Fluorescent compound that can be used also include fluorescent proteins, such as GFP (green fluorescent protein), enhanced GFP (EGFP), blue fluorescent protein and derivatives (BFP, EBFP, EBFP2, Azurite, mKalama1), cyan fluorescent protein and derivatives (CFP, ECFP, Cerulean, CyPet) and yellow fluorescent protein and derivatives (YFP, Citrine, Venus, YPet). WO2008142571, WO2009056282, WO9922026.

Examples of enzymatic labels include, but are not restricted to, horseradish peroxidase (HRP), alkaline phosphatase (AP), glucose oxidase and β-galactosidase.

In some embodiments, the label is biotin. Biotin labels are typically composed of the biotinyl group, a spacer arm and a reactive group that is responsible for attachment to target functional groups on proteins. Biotin can be useful for attaching the labelled protein to other moieties which comprise an avidin moiety.

### - Stefin A protein variant-Drug Conjugates

In some embodiments, the conjugate includes at least one therapeutic agent, e.g., to form an Stefin A protein variant-drug conjugate. As used herein, the term "therapeutic agent" refers to a substance that may be used in the cure, mitigation, treatment, or prevention of disease in a human or another animal. Such therapeutic agents include substances recognized in the official United States Pharmacopeia, official Homeopathic Pharmacopeia of the United States, official National Formulary, or any supplement thereof, and include but are not limited to small molecules, nucleotides, oligopeptides, polypeptides, etc. Therapeutic agents that may be attached to Stefin A protein variantinclude but are not limited to, cytotoxic agents, anti-metabolites, alkylating agents, antibiotics, growth factor, cytokines, anti-angiogenic agents, anti-mitotic agents, toxins, apoptotic agents or the like, such as DNA alkylating agents, topoisomerase inhibitors, microtubule inhibitors (e.g., DM1, DM4, MMAF and MMAE), endoplasmic reticulum stress inducing agents, platinum compounds, antimetabolites, vincalkaloids, taxanes, epothilones, enzyme inhibitors, receptor antagonists, therapeutic antibodies, tyrosine kinase inhibitors, radiosensitizers, and chemotherapeutic combination therapies, such as illustrations.
Any method known in the art for conjugating to antibodies and other proteins may be employed in generating the conjugates of the present disclosure, including those methods described by Hunter, et al., (1962) Nature 144:945; David, et al., (1974) Biochemistry 13:1014; Pain, et al., (1981) J. Immunol. Meth. 40:219; and Nygren, J., (1982) Histochem. and Cytochem. 30:407. Methods for conjugating peptide, polypeptide and organic and inorganic moieties to antibodies and other proteins are conventional and very well known in the art and readily adapted for generating those versions of the conjugate. Where the conjugated moiety is a peptide or polypeptide, that moiety can be chemically cross-linked to the Stefin A protein variantor can be included as part of a fusion protein with the Stefin A protein variant. And illustrative example would be a diptheria toxin-AFFIMER^{®} fusion protein. In the case of non-peptide entities, the addition to the Stefin A protein variant will generally be by way of chemical conjugation to the Stefin A protein variant- such as through a functional group on an amino acid side chain or the carboxyl group at the C-terminal or amino group at the N-terminal end of the polypeptide. In some embodiment, whether as a fusion protein or chemically cross-linked moiety, the conjugated moiety will include at least one site that is sensitive to an environmental condition (such as pH) that permits the conjugated moiety to be released from the Stefin A protein variant, such as in a diseased tissue or tissue for protect.

### - Spacers

In some embodiments, the conjugate comprises a spacer or bond (L¹) between the half-life extension moiety and the substrate recognition sequence (SRS) cleavable by the enzyme, e.g., present in an inflammatory microenvironment.

The spacer may be any molecule, for example, one or more nucleotides, amino acids, chemical functional groups. In some embodiments, the spacer is a peptide linker (e.g., two or more amino acids). Spacers should not adversely affect the expression, secretion, or bioactivity of the polypeptides. In some embodiments, spacers are not antigenic and do not elicit an immune response. An immune response includes a response from the innate immune system and/or the adaptive immune system. Thus, an immune response may be a cell-mediate response and/or a humoral immune response. The immune response may be, for example, a T cell response, a B cell response, a natural killer (NK) cell response, a monocyte response, and/or a macrophage response. Other cell responses are contemplated herein. In some embodiments, linkers are non-protein-coding.

In some embodiments, L¹ is a hydrocarbon (straight chain or cyclic) such as 6-maleimidocaproyl, maleimidopropanoyl and maleimidom ethyl cyclohexane- 1-carboxylate, or L¹ is N-Succinimidyl 4-(2-pyridylthio) pentanoate, N- Succinimidyl 4-(N- maleimidomethyl) cyclohexane-1 carboxylate, N-Succinimidyl (4-iodo-acetyl) aminobenzoate.

In some embodiments, L¹ is a polyether such as a poly (ethylene glycol) or other hydrophilic linker. For instance, where the CBM includes a thiol (such as a cysteine residue), L¹ can be a polyethylene glycol) coupled to the thiol group through a maleimide moiety.

Non-limiting examples of linkers for use in accordance with the present disclosure are described in International Publication No. WO 2019/236567, published December 12, 2019, incorporated by reference herein. In some embodiments, L1 may be represented by the following formula, for example: 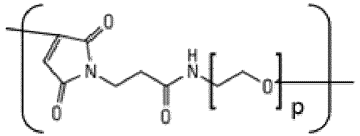 wherein, p represents an integer from 1 to 100, preferably 6 to 50, more preferably 6 to 12.

In some embodiments, wherein the region or residue to which L¹ is coupled includes a thiol and L¹ is a hydrocarbon moiety coupled to the thiol group through a maleimide moiety, L¹ may be represented by the following the formula:
wherein, p represents an integer from 1 to 20, preferably 1 to 4.

### Self-Immolative Linkers

In some embodiments, the conjugate comprises a self-immolative linker (L²) between the substrate recognition sequence (SRS) for the enzyme and the drug moiety.

In some embodiments, aself-immolative moiety may be defined as a bifunctional chemical group that is capable of covalently linking together two spaced chemical moieties into a normally stable molecule, releasing one of the spaced chemical moieties from the molecule by means of enzymatic cleavage; and following enzymatic cleavage, spontaneously cleaving from the remainder of the bifunctional chemical group to release the other of said spaced chemical moieties. Therefore, in some embodiments, the self-immolative moiety is covalently linked at one of its ends, directly or indirectly through a spacer unit, to the ligand by an amide bond and covalently linked at its other end to a chemical reactive site (functional group) pending from the drug moiety. The derivatization of the drug moiety with the self-immolative moiety may render the drug less pharmacologically active (e.g. less toxic) or not active at all until the drug is cleaved.

In some embodiments, the stefin A protein variant-drug conjugate is generally stable in circulation, or at least that should be the case in the absence of an enzyme capable of cleaving the amide bond between the substrate recognition sequence (enzyme-cleavable linker) and the self-immolative moiety. Upon exposure of an stefin A protein variant-drug conjugate to a suitable enzyme, the amide bond is cleaved initiating a spontaneous self-immolative reaction resulting in the cleavage of the bond covalently linking the self-immolative moiety to the drug moiety, to thereby effect release of the free drug moiety in its underivatized or pharmacologically active form. The self-immolative moiety in conjugates either incorporate one or more heteroatoms and thereby provides improved solubility, improves the rate of cleavage and decreases propensity for aggregation of the conjugate.

In some embodiments, L² is a benzyl oxy carbonyl group. In other embodiments, the self-immolative linker L² is- NH- (CH₂)₄ -C(=O)- or - NH-(CH₂)₃-C(=O)-. In yet other embodiments, the self-immolative linker L² is p-aminobenzyloxycarbonyl (PABC). In still other embodiments, the self-immolative linker L² is 2,4-bis(hydroxymethyl)aniline.

The stefin A protein variant-drug conjugate of the present disclosure can employ a heterocyclic self-immolative moiety covalently linked to the therapeutic moiety and the cleavable substrate recognition sequence.

In some embodiments, L² is a benzyloxycarbonyl group.

In some embodiments, L² is the following the formula: 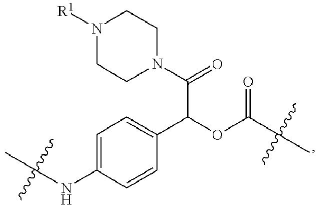 wherein R¹ is hydrogen, unsubstituted or substituted C₁₋₃ alkyl, or unsubstituted or substituted heterocyclyl. In some embodiments, R¹ is hydrogen. In some instances, R¹ is methyl.In some embodiments, L² is selected from

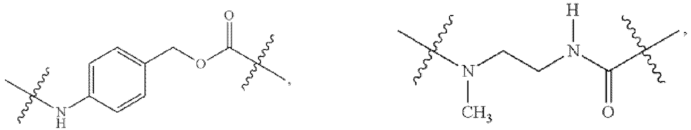

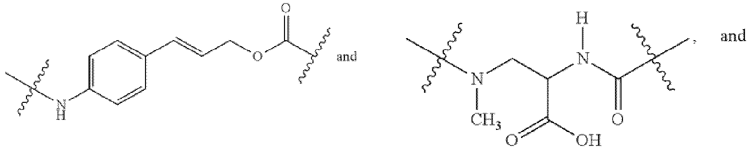

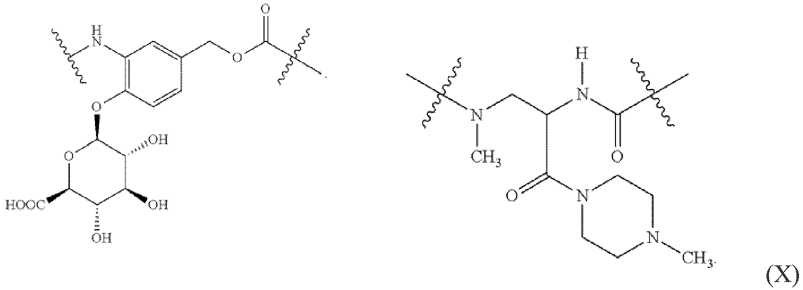

In some embodiments, the self-immolative moiety L₂ is selected from 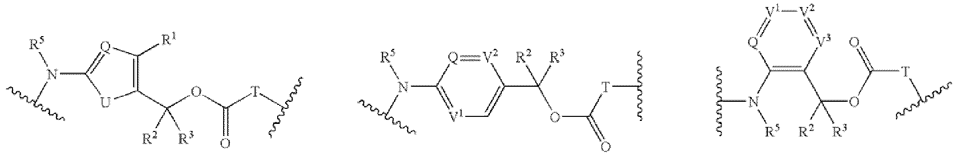 wherein
U is O, S or NR⁶;
Q is CR⁴ or N;
V¹, V² and V³ are independently CR⁴ or N provided that for formula (X) and (XI) at least one of Q, V¹ and V² is N;
T is NH, NR⁶, O or S pending from said therapeutic moiety;
R¹, R², R³ and R⁴ are independently selected from H, F, Cl, Br, I, OH, -N(R⁵)₂, - N(R⁵)₃ ⁺, C₁-C₈ alkylhalide, carboxylate, sulfate, sulfamate, sulfonate, -SO₂R⁵, - S(=O)R⁵, -SR⁵, -SO₂N(R⁵)₂, -C(=O)R⁵, -CO₂R⁵, -C(=O)N(R⁵)₂, -CN, -N₃, - NO₂, C₁-C₈ alkoxy, C₁-C₈ halosubstituted alkyl, polyethyleneoxy, phosphonate, phosphate, C₁-C₈ alkyl, C₁-C₈ substituted alkyl, C₂-C₈ alkenyl, C₂-C₈ substituted alkenyl, C₂-C₈ alkynyl, C₂-C₈ substituted alkynyl, C₆-C₂₀ aryl, C₆-C₂₀ substituted aryl, C₁-C₂₀ heterocycle, and C₁-C₂₀ substituted heterocycle; or when taken together, R² and R³ form a carbonyl (=O), or spiro carbocyclic ring of 3 to 7 carbon atoms; and
R⁵ and R⁶ are independently selected from H, C₁-C₈ alkyl, C₁-C₈substituted alkyl, C₂-C₈ alkenyl, C₂-C₈ substituted alkenyl, C₂-C₈alkynyl, C₂-C₈ substituted alkynyl, C₆-C₂₀ aryl, C₆-C₂₀ substituted aryl, C₁-C₂₀ heterocycle, and C₁-C₂₀ substituted heterocycle;
where C₁-C₈ substituted alkyl, C₂-C₈ substituted alkenyl, C₂-C₈substituted alkynyl, C₆-C₂₀ substituted aryl, and C₂-C₂₀ substituted heterocycle are independently substituted with one or more substituents selected from F, Cl, Br, I, OH, -N(R⁵)₂, -N(R⁵)₃⁺, C₁-C₈ alkylhalide, carboxylate, sulfate, sulfamate, sulfonate, C₁-C₈ alkyl sulfonate, C₁-C₈ alkylamino, 4-dialkylaminopyridinium, C₁-C₈ alkylhydroxyl, C₁-C₈ alkylthiol, - SO₂R⁵, -S(=O)R⁵, -SR⁵, -SO₂N(R⁵)₂, -C(=O)R⁵, -CO₂R⁵, -C(=O)N(R⁵)₂, -CN, -N₃, -NO₂, C₁-C₈ alkoxy, C₁-C₈ trifluoroalkyl, C₁-C₈ alkyl, C₃-C₁₂ carbocycle, C₆-C₂₀ aryl, C₂-C₂₀ heterocycle, polyethyleneoxy, phosphonate, and phosphate.

It will be understood that when T is NH, it is derived from a primary amine (-NH2) pending from the therapeutic moiety (prior to coupling to the self-immolative moiety) and when T is N, it is derived from a secondary amine (-NH-) from the therapeutic moiety (prior to coupling to the self-immolative moiety). Similarly, when T is O or S, it is derived from a hydroxyl (-OH) or sulfhydryl (-SH) group respectively pending from the therapeutic moiety prior to coupling to the self-immolative moiety.

In some embodiments, the self-immolative linker L² is -NH-(CH₂)₄-C(=O)- or - NH-(CH₂)₃-C(=O)-.

In some embodiments, the self-immolative linker L² is p-aminobenzyloxycarbonyl (PABC).

In some embodiments, the self-immolative linker L² is 2,4-bis(hydroxymethyl)aniline.

Other examples of self-immolative linkers that are readily adapted for use in AFFIMER^{®} polypeptide-drug conjugates described herein are taught in, for example, US Patent 7,754,681; WO 2012/074693A1; US 9,089,614; EP 1,732,607; WO 2015/038426A1 (all of which are incorporated by reference); Walther et al. "Prodrugs in medicinal chemistry and enzyme prodrug therapies" Adv Drug Deliv Rev. 2017 Sep 1; 118:65-77; and Tranoy-Opalinski et al."Design of self- immolative linkers for tumor-activated prodrug therapy", Anticancer Agents Med Chem. 2008 Aug;8(6):618-37; the teachings of each of which are incorporated by reference herein.

Yet other non-limiting examples of self-immolative linkers for use in accordance with the present disclosure are described in International Publication No. WO 2019/236567, published December 12, 2019, incorporated by reference herein.

In another aspect, the present invention relates to nucleic acids encoding the stefin A protein variant specifically binding to CD40L, or the fusion protein or the conjugate comprising the same.

In another aspect, the present invention relates to a vector comprising the nucleic acids.

In another aspect, the present invention relates to a delivery vehicle comprising the nucleic acids.

In the present invention, the delivery vehicle may be used for *in vivo* delivery.

In another aspect, the present invention relates to a genetically engineered cell into which the nucleic acids or vector is introduced.

In the present invention, the nucleic acid may be delivered *in vivo.*

### Neucleic Acids encoding the stefin A protein variant specifically binding to CD40L, or the fusion protein or the conjugate comprising the same for In vivo Delivery

An alternative approach to the delivery of the stefin A protein variant specifically binding to CD40L, or the fusion protein or the conjugate comprising the same would be to leave the production of the therapeutic polypeptide to the body itself. A multitude of clinical studies have illustrated the utility of *in vivo* gene transfer into cells using a variety of different delivery systems. *In vivo* gene transfer seeks to administer to patients the encoded polynucleotide, rather than the stefin A protein variant. This allows the patient's body to produce the stefin A protein variant specifically binding to CD40L, or the fusion protein or the conjugate comprising the same of interest for a prolonged period of time, and secrete it either systemically or locally, depending on the production site. Gene-based encoded nucleic acids can present a labor- and cost-effective alternative to the conventional production, purification and administration of the protein. A number of antibody expression platforms have been pursued *in vivo* to which delivery of encoded neucleic acids can be adapted: these include viral vectors, naked DNA and RNA. encoded neucleic acids transfer can not only enable cost-savings by reducing the cost of goods and of production but may also be able to reduce the frequency of drug administration. Overall, a prolonged *in vivo* production of the stefin A protein variant specifically binding to CD40L, or the fusion protein or the conjugate comprising the same by expression of the encoded neucleic acids can contribute to (i) a broader therapeutic or prophylactic application in price-sensitive conditions, (ii) an improved accessibility to therapy in both developed and developing countries, and (iii) more effective and affordable treatment modalities. In addition to *in vivo* gene transfer, cells can be harvested from the host (or a donor), engineered with encoded neucleic acids to produce the genetically modified cell therapeutics and re-administered to patients.

Intramuscular antibody gene administration has been most widely evaluated (reviewed in Deal et al. (2015) "Engineering humoral immunity as prophylaxis or therapy" Curr Opin Immunol. 35:113-22.), and also carries the highest clinical translatability and may be applied as an administration method for in vivo delivery of the nucleic acid of the present invention.. Indeed, the inherent anatomical, cellular and physiological properties of skeletal muscle make it a stable environment for long-term neucleic acids expression and systemic circulation. Skeletal muscle is easily accessible, allowing multiple or repeated administrations. The abundant blood vascular supply provides an efficient transport system for secreted therapeutic the stefin A protein variant specifically binding to CD40L, or the fusion protein or the conjugate comprising the sameinto the circulation. The syncytial nature of muscle fibers allows dispersal of nucleotides from a limited site of penetration to a large number of neighboring nuclei within the fiber. Skeletal muscle fibers are also terminally differentiated cells, and nuclei within the fibers are post-mitotic. Consequently, integration in the host genome is not a prerequisite to attain prolonged monoclonal antibody (mAb) expression. The liver is another site often used for pre-clinical antibody gene transfer and is typically transfected via intravenous (i.v.) injection and can also be a site of gene transfer for encoded nucleic acids either for local delivery of the stefin A protein variant specifically binding to CD40L, or the fusion protein or the conjugate comprising the same (such as in the treatment of inflammatory or autoimmune disease) or for the generation of the stefin A protein variant specifically binding to CD40L, or the fusion protein or the conjugate comprising the samethat are secreted into the vascular for systemic circulation. This organ has various physiological functions, including the synthesis of plasma proteins. This organ can be particularly well suited for *in vivo* encoded the stefin A protein variant specifically binding to CD40L, or the fusion protein expression.

The success of gene therapy has largely been driven by improvements in nonviral and viral gene transfer vectors. An array of physical and chemical nonviral methods have been used to transfer DNA and mRNA to mammalian cells and a substantial number of these have been developed as clinical stage technologies for gene therapy, both ex vivo and *in vivo,* and are readily adapted for delivery of the nucleic acids of the present disclosure. To illustrate, cationic liposome technology can be employed, which is based on the ability of amphipathic lipids, possessing a positively charged head group and a hydrophobic lipid tail, to bind to negatively charged DNA or RNA and form particles that generally enter cells by endocytosis. Some cationic liposomes also contain a neutral co-lipid, thought to enhance liposome uptake by mammalian cells. See, for example, Felgner et al. (1987) Lipofection: a highly efficient, lipid-mediated DNA-transfection procedure. MNAS 84:7413-7417; San et al. (1983) "Safety and short-term toxicity of a novel cationic lipid formulation for human gene therapy" Hum. Gene Ther. 4:781-788; Xu et al. (1996) "Mechanism of DNA release from cationic liposome/DNA complexes used in cell transfection" Biochemistry 35:5616-5623; and Legendre et al. (1992) "Delivery of plasmid DNA into mammalian cell lines using pH-sensitive liposomes: comparison with cationic liposomes" Pharm. Res. 9, 1235-1242.

Similarly, other polycations, such as poly-l-lysine and polyethylene-imine, can be used to deliver the nucleic acids of the present invention. These polycations complex with nucleic acids via charge interaction and aid in the condensation of DNA or RNA into nanoparticles, which are then substrates for endosome-mediated uptake. Several of these cationic nucleic acid complex technologies have been developed as potential clinical products, including complexes with plasmid DNA, oligodeoxynucleotides, and various forms of synthetic RNA. Modified (and unmodified or "naked") DNA and RNA have also been shown to mediate successful gene transfer in a number of circumstances and can also be used as systems for delivery of encoded nucleic acids. These include the use of plasmid DNA by direct intramuscular injection. See, for example, Rodrigo et al. (2012) "De novo automated design of small RNA circuits for engineering synthetic riboregulation in living cells" PNAS 109:15271-15276; Oishi et al. (2005) "Smart polyion complex micelles for targeted intracellular delivery of PEGylated antisense oligonucleotides containing acid-labile linkages" Chembiochem. 6:718-725; Bhatt et al. (2015) "Microbeads mediated oral plasmid DNA delivery using polymethacrylate vectors: an effectual groundwork for colorectal cancer" Drug Deliv. 22:849-861; Ulmer et al. (1994) Protective immunity by intramuscular injection of low doses of influenza virus DNA vaccines" Vaccine 12: 1541-1544; and Heinzerling et al. (2005).

Viral vectors are currently used as a delivery vehicle in the vast majority of pre-clinical and clinical gene therapy trials and in the first to be approved directed gene therapy. See Gene Therapy Clinical Trials Worldwide 2017 (abedia.com/wiley/). The main driver thereto is their exceptional gene delivery efficiency, which reflects a natural evolutionary development; viral vector systems are attractive for gene delivery, because viruses have evolved the ability to cross through cellular membranes by infection, thereby delivering nucleic acids such as encoded nucleic acids to target cells. Pioneered by adenoviral systems, the field of viral vector-mediated antibody gene transfer made significant strides in the past decades. The myriad of successfully evaluated administration routes, pre-clinical models and disease indications puts the capabilities of antibody gene transfer at full display through which the skilled artisan would readily be able to identify and adapt antibody gene transfer systems and techniques for *in vivo* delivery of encoded nucleic acids. Muscle has emerged as the administration site of choice for prolonged mAb expression and would similarly be a suitable target tissue for prolonged expression.

*In vivo* gene transfer of encoded nucleic acids of the present invention can also be accomplished by use of nonviral vectors, such as expression plasmids. Nonviral vectors are easily produced and do not seem to induce specific immune responses. Muscle tissue is most often used as target tissue for transfection because muscle tissue is well vascularized and easily accessible, and myocytes are long-lived cells. Intramuscular injection of naked plasmid DNA results in transfection of a certain percentage of myocytes. Using this approach, plasmid DNA encoding cytokines and cytokine/IgG1 chimeric proteins has been introduced *in vivo* and has positively influenced (autoimmune) disease outcome.

In some instances, in order to increase transfection efficiency via so-called intravascular delivery in which increased gene delivery and expression levels are achieved by inducing a short-lived transient high pressure in the veins. Special blood-pressure cuffs that may facilitate localized uptake by temporarily increasing vascular pressure and can be adapted for use in human patients for this type of gene delivery. See, for example, Zhang et al. (2001) "Efficient expression of naked DNA delivered intraarterially to limb muscles of nonhuman primates" Hum. Gene Ther., 12:427-438

Increased efficiency can also be gained through other techniques, such as in which delivery of the nucleic acid is improved by use of chemical carriers-cationic polymers or lipids-or via a physical approach-gene gun delivery or electroporation. See Tranchant et al. (2004) "Physicochemical optimization of plasmid delivery by cationic lipids" J. Gene Med., 6 (Suppl. 1):S24-S35; and Niidome et al. (2002) "Gene therapy progress and prospects: nonviral vectors" Gene Ther., 9:1647-1652. Electroporation is especially regarded as an interesting technique for nonviral gene delivery. Somiari, et al. (2000) "Theory and in vivo application of electroporative gene delivery" Mol. Ther. 2:178-187; and Jaroszeski et al. (1999) "In vivo gene delivery by electroporation" Adv. Drug Delivery Rev., 35:131-137. With electroporation, pulsed electrical currents are applied to a local tissue area to enhance cell permeability, resulting in gene transfer across the membrane. Research has shown that *in vivo* gene delivery can be at least 10-100 times more efficient with electroporation than without. See, for example, Aihara et al. (1998) "Gene transfer into muscle by electroporation in vivo" Nat. Biotechnol. 16:867-870; Mir, et al. (1999) "High-efficiency gene transfer into skeletal muscle mediated by electric pulses" PNAS 96:4262-4267; Rizzuto, et al. (1999) "Efficient and regulated erythropoietin production by naked DNA injection and muscle electroporation" PNAS 96: 6417-6422; and Mathiesen (1999) "Electropermeabilization of skeletal muscle enhances gene transfer in vivo" Gene Ther., 6:508-514.

Encoded nucleic acids can be delivered by a wide range of gene delivery system commonly used for gene therapy including viral, non-viral, or physical. See, for example, Rosenberg et al., Science, 242:1575-1578, 1988, and Wolff et al., Proc. Natl. Acad. Sci. USA 86:9011-9014 (1989). Discussion of methods and compositions for use in gene therapy include Eck et al., in Goodman & Gilman's The Pharmacological Basis of Therapeutics, Ninth Edition, Hardman et al., eds., McGraw-Hill, New York, (1996), Chapter 5, pp. 77-101; Wilson, Clin. Exp. Immunol. 107 (Suppl. 1):31-32, 1997; Wivel et al., Hematology/Oncology Clinics of North America, Gene Therapy, S. L. Eck, ed., 12(3):483-501, 1998; Romano et al., Stem Cells, 18:19-39, 2000, and the references cited therein. U.S. Pat. No. 6,080,728 also provides a discussion of a wide variety of gene delivery methods and compositions.

The routes of delivery include, for example, systemic administration and administration in situ. An effective encoded nucleic acids transfer approach must be directed to the specific tissues/cells where it is needed, and the resulting transgene expression should be at a level that is appropriate to the specific application. Promoters are a major cis-acting element within the vector genome design that can dictate the overall strength of expression as well as cell-specificity.

**Table 12. Exemplary Ubiquitous and Cell-specific Promoters.**

| **Promoter** | **Specificity** | **Relative Strength** | **Size (bps)** | **Reference(s)** |
|---|---|---|---|---|
| CMV | Ubiquitous | +++ | 750-800 | Xu et al. Gene Ther. 2001 8:1323-1332; Gray et al., Hum Gene Ther. 2011 22:1143-1153 |
| CBA (including derivatives: CAG, CBh, etc.) | Ubiquitous | +++ | 248-1,600 | Klein et al. Exp Neurol. 2002 176(1):66-74; Ohlfest et al. Blood. 2005 105:2691-2698; and Gray et al. Hum Gene Ther. 2011 22:1143-1153. |
| EF-1α | Ubiquitous | ++ | 2,500 | Gill et al. Gene Ther. 2001 8(20):1539-1546; Xu et al. Gene Ther. 2001 8:1323-1332; and Gilham et al. J Gene Med. 2010 12(2):129-136. |
| PGK | Ubiquitous | ++ | 426 | Gilham et al. J Gene Med. 2010 12(2):129-136. |
| UBC | Ubiquitous | + | 403 | Gill et al. Gene Ther. 2001 8(20):1539-1546; Qin et al. PLoS One. 2010 5(5):e10611. |
| GUSB (hGBp) | Ubiquitous | + | 378 | Husain et al. Gene Ther. 2009 16:927-932. |
| UCOE (Promoter of HNRPA2B1-CBX3) | Ubiquitous | ++ | 600-2,500 | Antoniou et al. Hum Gene Ther. 2013 24(4):363-374. |
| hAAT | Liver | ++ | 347-1,500 | Van Linthout et al. Hum Gene Ther. 2002 13(7):829-840; Cunningham et al. Mol Ther. 2008 16(6):1081-1088 |
| TBG | Liver | ++ | 400 | Yan et al. Gene. 2012 506(2):289-294. |
| Desmin | Skeletal muscle | +++ | 1,700 | Talbot et al. Mol Ther. 2010 18:601-608. |
| MCK | Skeletal muscle | ++ | 595-1,089 | Talbot et al. Mol Ther. 2010 18:601-608; Wang et al. Gene Ther. 2008 15:1489-1499; Katwal et al. Gene Ther. 2013 20(9):930-938. |
| C5-12 | Skeletal, cardiac, and diaphragm | ++ | 312 | Wang et al. Gene Ther. 2008 15:1489-1499 |
| NSE | Neuron | +++ | 300-2,200 | Xu et al. Gene Ther. 2001 8:1323-1332 |
| Synapsin | Neuron | + | 470 | Kiigler et al. Virology. 2003 311:89-95; Hioki et al. Gene Ther. 2007 14:872-882; Kuroda et al. J Gene Med. 2008 10:1163-1175. |
| PDGF | Neuron | +++ | 1,400 | Patterna et al. Gene Ther. 2000 7(15):1304-1311; Hioki et al. Gene Ther. 2007 14:872-882 |
| MecP2 | Neuron | + | 229 | Rastegar et al. LoS One. 2009 4:e6810; Gray et al., Hum Gene Ther. 2011 22:1143-1153 |
| CaMKII | Neuron | ++ | 364-2,300 | Hioki et al. Gene Ther. 2007 14:872-882; Kuroda et al. J Gene Med. 2008 10:1163-1175 |
| mGluR2 | Neuron | + | 1,400 | Brené et al. Eur J Neurosci. 2000 12:1525-1533; Kuroda et al. J Gene Med. 2008 10:1163-1175 |
| NFL | Neuron | + | 650 | Xu et al. Gene Ther. 2001 8:1323-1332 |
| NFH | Neuron | + | 920 | Xu et al. Gene Ther. 2001 8:1323-1332 |
| nβ2 | Neuron | + | 650 | Xu et al. Gene Ther. 2001 8:1323-1332 |
| PPE | Neuron | + | 2700 | Xu et al. Gene Ther. 2001 8:1323-1332 |
| Enk | Neuron | + | 412 | Xu et al. Gene Ther. 2001 8:1323-1332 |
| EAAT2 | Neuron and astrocyte | ++ | 966 | Su et al. Proc Natl Acad Sci USA. 2003 100:1955-1960; Kuroda et al. J Gene Med. 2008 10:1163-1175 |
| GFAP | Astrocyte | ++ | 681-2,200 | Brenner et al. J Neurosci. 1994 14:1030-1037; Xu et al. Gene Ther. 2001 8:1323-1332; Lee et al. Glia. 2008 56:481-493; Dirren et al. Hum Gene Ther. 2014 25:109-120 |
| MBP | Oligodendrocytes | ++ | 1,900 | Chen et al. Gene Ther. 1998 5(1):50-58 |

In some embodiments, ubiquitous expression of the encoded AFFIMER^{®} polynucleotide in all cell types is desired. Constitutive promoters such as the human elongation factor 1α-subunit (EF1α), immediate-early cytomegalovirus (CMV), chicken β-actin (CBA) and its derivative CAG, the β glucuronidase (GUSB), or ubiquitin C (UBC) can be used to promote expression of the encoded nucleic acids in most tissues. Generally, CBA and CAG promote the larger expression among the constitutive promoters; however, their size of ~1.7 kbs in comparison to CMV (~0.8 kbs) or EF1α (~1.2 kbs) may limit use in vectors with packaging constraints such as AAV, particularly where AFFIMER^{®} agent produced by expression of the encoded nucleic acids is large. The GUSB or UBC promoters can provide ubiquitous gene expression with a smaller size of 378 bps and 403 bps, respectively, but they are considerably weaker than the CMV or CBA promoter. Thus, modifications to constitutive promoters in order to reduce the size without affecting its expression have been pursued and examples such as the CBh (-800 bps) and the miniCBA (-800 bps) can promote expression comparable and even higher in selected tissues (Gray et al., Hum Gene Ther. 2011 22:1143-1153).

When expression of the encoded nucleic acids should be restricted to certain cell types within an organ, promoters can be used to mediate this specificity. For example, within the nervous system promoters have been used to restrict expression to neurons, astrocytes, or oligodendrocytes. In neurons, the neuron-specific enolase (NSE) promoter drives stronger expression than ubiquitous promoters. Additionally, the platelet-derived growth factor B-chain (PDGF-β), the synapsin (Syn), and the methyl-CpG binding protein 2 (MeCP2) promoters can drive neuron-specific expression at lower levels than NSE. In astrocytes, the 680 bps-long shortened version [gfaABC(1)D] of the glial fibrillary acidic protein (GFAP, 2.2 kbs) promoter can confer higher levels of expression with the same astrocyte-specificity as the GFAP promoter. Targeting oligodendrocytes can also be accomplished by the selection of the myelin basic protein (MBP) promoter, whose expression is restricted to this glial cell; however, its size of 1.9 kbs and low expression levels limit its use.

In the case of expressing the encoded nucleic acids in skeletal muscle cells, exemplary promoters based on muscle creatine kinase (MCK) and desmin (1.7 kbs) have showed a high rate of specificity (with minimal expression in the liver if desired). The promoter of the α-myosin heavy chain (α-MHC; 1.2 kbs) has shown significant cardiac specificity in comparison with other muscle promoters (Lee et al., 2011 J Cardiol. 57(1):115-22). In hematopoietic stem cells the synthetic MND promoter (Li et al., 2010 J Neurosci Methods. 189(1):56-64) and the promoter contained in the 2AUCOE (ubiquitous chromatin opening element) have shown to drive a higher transgene expression in all cell lineages when compared to the EF1α and CMV promoters, respectively (Zhang et al., 2007 Blood. 110(5):1448-57; Koldej 2013 Hum Gene Ther Clin Dev. 24(2):77-85; Dighe et al., 2014 PLoS One. 9(8):e104805.). Conversely, using promoters to restrict expression to only liver hepatocytes after vector-mediated gene transfer has been shown to reduce transgene-specific immune responses in systems where that is a risk, and to even induce immune tolerance to the expressed protein (Zhang et al., 2012 Hum Gene Ther. 23(5):460-72). The α1-antitrypsin (hAAT; 347 bps) and the thyroxine binding globulin (TBG; ~400 bps) promoters drive gene expression restricted to the liver with minimal invasion to other tissues (Yan et al., 2012 Gene. 506(2):289-94; Cunningham et al., 2008 Mol Ther. 16(6):1081-8).

In some embodiments, a mechanism to control the duration and amount of *in vivo* encoded nucleic acids expression will typically be desired. There are a variety of inducible promoters which can be adapted for use with viral vectored- and plasmid DNA-based encoded AFFIMER^{®} gene transfer. See Fang et al. (2007) Mol Ther. 5(6):1153-9; and Perez et al. (2004) Genet Vaccines Ther. 2(1):2. An exemplary a regulatable mechanism currently under clinical evaluation is an ecdysone-based gene switch activated by a small molecule ligand. Cai et al. (2016) Clin Pharmacol Drug Dev. 2016.

In some embodiments of an encoded nucleic acids, viral post-transcriptional regulatory elements (PREs) may be used for in vivo delivery and expression; these cis-acting elements are required for nuclear export of intronless viral RNA (Huang and Yen, 1994 J Virol. 68(5):3193-9; and 1995 Mol Cell Biol. 15(7):3864-9). Examples include HPRE (Hepatitis B Virus PRE, 533 bps) and WPRE (Woodchuck Hepatitis Virus PRE, 600 bps), which can increase the level of transgene expression by almost 10-fold in certain instances (Donello et al., 1998 J Virol. 72(6):5085-92). To further illustrate, using lentiviral and AAV vectors, WPRE was found to increase CMV promoter driven transgene expression, as well as increase PPE, PDGF and NSE promoter-driven transgene expression. Another effect of the WPRE can be to protect encoded AFFIMER^{®} polynucleotides transgenes from silencing (Paterna et al., 2000 Gene Ther. 7(15):1304-11; Xia et al., 2007 Stem Cells Dev. 2007 Feb; 16(1):167-76).

The polyadenylation of a transcriptome of the encoded nucleic acids can also be important for nuclear export, translation, and mRNA stability. Therefore, in some embodiments, the encoded nucleic acids will include a polyadenylation signal sequence. A variety of studies are available that have determined the effects of different polyA signals on gene expression and mRNA stability. Exemplary polyadenylation signal sequences include SV40 late or bovine growth hormone polyA (bGHpA) signal sequences, as well as minimal synthetic polyA (SPA) signal (Levitt et al., 1989 Genes Dev. 3(7):1019-25; Yew et al., 1997 Hum Gene Ther. 1997 8(5):575-84). The efficiency of polyadenylation is increased by the SV40 late polyA signal upstream enhancer (USE) placed upstream of other polyA signals (Schek et al., 1992 Mol Cell Biol. 12(12):5386-93). In some embodiments, for examples, the encoded nucleic acidswill include an SV40 late + 2xUSE polyA signal.

**Table 13. Exemplary Polyadenylation Signals**

| **PolyA Signal and USE** | **Relative Strength** | **Size (bps)** | **Source** | **Reference(s)** |
|---|---|---|---|---|
| hGH | + | 624 | Human growth hormone | Ostedgaard et al. Proc Natl Acad Sci U S A. 2005 102(8):2952-2957 |
| SV40 late | +++ | 135 | Simian virus 40 | Choi et al. Mol Brain. 2014 7:17 |
| SPA (synthetic polyA) | + | 49 | Rabbit β-globin | Levitt et al. Genes Dev. 3(7):1019-1025; Yew et al. Hum Gene Ther. 1997 8(5):575-584; Ostedgaard et al. Proc Natl Acad Sci U S A. 2005 102(8):2952-2957; Choi et al. Mol Brain. 2014 7:17 |
| bGH | ++ | 250 | Bovine growth hormone | Yew et al. Hum Gene Ther. 1997 8(5):575-584; Xu et al. Gene Ther. 2001 8:1323-1332; Wu et al. Mol Ther. 2008 16(2):280-289; Gray et al., Hum Gene Ther. 2011 22:1143-1153; Choi et al. Mol Brain. 2014 7:17 |
| SV40 late 2xUSE | ++ | 100 | Simian virus 40 | Schambach et al. Mol Ther. 2007 15(6):1167-1173; Choi et al. Mol Brain. 2014 7:17 |
| HIV-1 USE | + | 35 | Human immunodeficiency virus 1 | Schambach et al. Mol Ther. 2007 15(6):1167-1173 |
| GHV USE | + | 39 | Ground squirrel hepatitis virus | Schambach et al. Mol Ther. 2007 15(6):1167-1173 |
| Adenovirus (L3) USE | + | 21 | Adenovirus | Schambach et al. Mol Ther. 2007 15(6):1167-1173 |
| hTHGB USE | + | 21 | Human prothrombin | Schambach et al. Mol Ther. 2007 15(6):1167-1173 |
| hC2 USE | + | 53 | Human C2 complement gene | Schambach et al. Mol Ther. 2007 15(6):1167-1173 |

In some embodiments, the encoded nucleic acids preferably further include at least one regulatory enhancer, in addition to any promoter sequences. The CMV enhancer is upstream of the CMV promoter at -598 to -68 (Boshart et al., 1985 Cell. 41(2):521-30) (-600 bps) and contains transcription binding sites. In some embodiments, a CMV enhancer can be included in the construct to increase tissue-specific promoter-driven transgene expression, such as using the ANF (atrial natriuretic factor) promoter, the CC10 (club cell 10) promoter, SP-C (surfactant protein C) promoter, or the PDGF-β (platelet-derived growth factor-β) promoter (merely as examples). Altogether, the CMV enhancer increases transgene expression under different cell-specific promoters and different cell types making it a broadly applicable tool to increase transgene expression levels. In muscle, for example, in AAV expression systems transgene expression using the CMV enhancer with a muscle-specific promoter can increase expression levels of the protein encoded by the transgene, so would be particularly useful in the current disclosure for expressing the nucleic acids int muscle cells. The encoded nucleic acids may also include at least one intronic sequence. The presence of an intron or intervening sequence in mRNA was first described, in vitro, to be important for mRNA processing and increased transgene expression (Huang and Gorman, 1990 Mol Cell Biol. 10(4): 1805-10; Niwa et al., 1990 Genes Dev. 4(9): 1552-9). The intron(s) can be placed within the coding sequence for the stefin A protein variant or the fusion protein and/or can be placed between the promoter and transgene. A variety of introns **(Table 14)** placed between the promoter and transgene were compared, in mice using AAV2, for liver transgene expression (Wu et al., 2008). The MVM (minute virus of mice) intron increased transgene expression more than any other intron tested and more than 80-fold over no intron (Wu et al., 2008). However, in cultured neurons using AAV expression cassettes, transgene expression was less under a CaMPKII promoter with a chimeric intron (human β-globin donor and immunoglobulin heavy chain acceptor) between the transgene and polyA signal compared to a WPRE (Choi et al., 2014). Together, an intron can be a valuable element to include in an expression cassette to increase transgene expression.

**Table 14. Exemplary Introns**

| **Itron** | **Relative Strength** | **Size (bps)** | **Source** | **Reference(s)** |
|---|---|---|---|---|
| MVM | +++ | 67-97 | Minute virus of mice | Wu et al. Mol Ther. 2008 16(2):280-289 |
| F.IX truncated intron 1 | + | 300 | Human factor IX | Wu et al. Mol Ther. 2008 16(2):280-289; Kurachi et al. J Biol Chem. 1995 270(10):5276-5281 |
| β-globin SD / immunoglobin heavy chain SA | + | 250 | Human, pZac2.1 | Wu et al. Mol Ther. 2008 16(2):280-289; Choi et al. Mol Brain. 2014;7:17 |
| Adenovirus SD^{#} / immunoglobulin SA* | ++ | 500 | pAdβ | Wong et al. Chromosoma. 1985 92(2):124-135; Yew et al. Hum Gene Ther. 1997 8(5):575-584 |
| SV40 late SD^{#} / SA* (19S/16S) | + | 180 | pCMVβ | Yew et al. Hum Gene Ther. 1997 8(5):575-584 |
| Hybrid adenovirus SD^{#} / IgG SA* | +++ | 230 | Adenovirus | Choi et al. Mol Brain. 2014;7:17; Huang et al. Mol Cell Biol. 1990 10(4):1805-1810 |

In some embodiments, the vector is episomal vector. In the case of episomal vectors, the encoded AFFIMER^{®} polynucleotides may also include at least one origin of replication, minichromosome maintenance elements (MME) and/or nuclear localization elements. Episomal vectors of the disclosure comprise a portion of a virus genomic DNA that encodes an origin of replication (ori) , which is required for such vectors to be self- replicating and, thus, to persist in a host cell over several generations. In addition, an episomal vector of the disclosure also may contain at least one gene encoding at least one viral protein required for replication, e.g., replicator protein (s). Optionally, the replicator protein(s) which help initiate replication may be expressed in trans on another DNA molecule, such as on another vector or on the host genomic DNA, in the host cell containing a self-replicating episomal expression vector of this disclosure. Preferred self-replicating episomal LCR-containing expression vectors of the disclosure do not contain viral sequences that are not required for long-term stable maintenance in a eukaryotic host cell such as regions of a viral genome DNA encoding core or capsid proteins that would produce infectious viral particles or viral oncogenic sequences which may be present in the full-length viral genomic DNA molecule. The term "stable maintenance" herein, refers to the ability of a self-replicating episomal expression vector of this disclosure to persist or be maintained in non-dividing cells or in progeny cells of dividing cells in the absence of continuous selection without a significant loss (e.g., >50%) in copy number of the vector for two, three, four, or five or more generations. In some embodiments, the vectors will be maintained over 10-15 or more cell generations. In contrast, "transient" or "short-term" persistence of a plasmid in a host cell refers to the inability of a vector to replicate and segregate in a host cell in a stable manner; that is, the vector will be lost after one or two generations or will undergo a loss of >51% of its copy number between successive generations.

Several representative self-replicating, LCR-containing, episomal vectors useful in the context of the present disclosure are described further below. The self-replicating function may alternatively be provided by at least one mammalian sequence such as described by Wohlgeuth et al., 1996, Gene Therapy 3:503; Vos et al., 1995, Jour. Cell. Biol., Supp. 21A, 433; and Sun et al., 1994, Nature Genetics 8:33, optionally in combination with at least one sequence that may be required for nuclear retention. The advantage of using mammalian, especially human sequences for providing the self- replicating function is that no extraneous activation factors are required which could have toxic or oncogenic properties. It will be understood by one of skill in the art that the disclosure is not limited to any one origin of replication or any one episomal vector but encompasses the combination of the tissue-restricted control of an LCR in an episomal vector. See also WO1998007876 and US Patent 7790446.

In some embodiments, the episomal vector is Epstein-Barr Virus-Based Self-Replicating Episomal Expression Vectors(Yates et. al., Proc . Natl . Acad . Sci . USA 81:3806-3810 (1984); Yates et al., Nature 313:812-815 (1985); Krysan et al., Mol . Cell . Biol . 9:1026-1033 (1989); James et al. Gene 86: 233-239 (1990), Peterson and Legerski, Gene 107:279-284 (1991); and Pan et al., Som . Cell Molec. Genet . 18:163-177 (1992)); Papilloma Virus-Based, Self-Replicating, Episomal Expression Vectors (Ustav et al., EMBO J. 10: 449-457 (1991); Ustavet al., EMBO J . 10:4231-4329, (1991); Ustav et al., Proc . Natl . Acad . Sci . USA 90: 898-902 (1993)); or Papovavirus-Based, Self-Replicating, Episomal Expression Vectors (De Benedetti and Rhoads, Nucleic Acids Res. 19:1925 (1991), as can a 3.2 kb fragment of the BK virus (Cooper and Miron, Human Gene Therapy 4:557 (1993)), but not limited thereto.

The nucleic acids encoding the stefin A protein variant specifically binding to CD40L or the fusion protein of the present disclosure can be provided as circular or linear nucleic acids. The circular and linear nucleic acids are capable of directing expression of the coding sequence in an appropriate subject cell. The at least one nucleic acid system for expressing may be chimeric, meaning that at least one of its components is heterologous with respect to at least one of its other components.

### Method of introducing the nucleic acids (or vector)

As used herein, the term "introduction" refers to allowing the host cell to receive a foreign gene (nucleic acid) that the host cell does not have.

As used herein, the term "introduction" refers to allowing the host cell to receive a foreign gene (nucleic acid) that the host cell does not have.

In some embodiments, the nucleic acid encoding the stefin A protein variant specifically binding to CD40L or the fusion protein or the conjugate including the same may be introduced into a host cell using a vector including the same.

As used herein, a "vector" is a means for expressing a target gene in a host cell, and may be viral vector or non-viral vector. In some embodiments, the viral vector is, for example, may include, but are not limited to, viral vectors such as adenoviral vector, retroviral vector, adeno-associated viral vector, and vectors derived from viruses such as vaccinia virus (Puhlmann M. et al., Human Gene Therapy, 10:649-657 (1999); Ridgeway, 467-492 (1988); Baichwal and Sugden, In: Kucherlapati R., ed. Gene transfer. New York: Plenum Press, 117-148 (1986) and Coupar et al., Gene, 68:1-10(1988)), lentivirus (Wang G. et al., J. Clin. Invest., 104(11):R55-62(1999)), herpes simplex virus (Chamber R., et al., Proc. Natl. Acad. Sci USA, 92:1411-1415 (1995)), poxvirus (GCE, NJL, Krupa M., Esteban M., Curr. Gene Ther. 8(2):97-120 (2008)), reovirus, measles virus, Semliki Forest virus, and poliovirus, and non-viral vectors such as plasmid vectors (Sambrook et al., 1989) and mini circles (Yew et al. 2000 Mol. Ther. 1(3), 255-62).

In some embodiments, the viral vector may include a viral vector such as naked plasmid DNA (pDNA), linear nucleic acid or linear expression cassette (LEC); plasmid vectors (Sambrook et al., 1989) and minicircles (Yew et al. 2000 Mol Ther 1(3), 255-62), but not limited thereto.

The vector may typically include at least one component selected from a signal sequence, an origin of replication, at least one antibiotic resistance marker gene, an enhancer element, a promoter, and a transcription termination sequence, but is not limited thereto. The nucleic acids encoding the stefin A protein variant, or the fusion protein or the conjugate comprising the same of the present invention be operably linked with a promoter and a transcription termination sequence.

As used herein, the term "operably linked" means a functional linkage between a nucleic acid expression control sequence (e.g. a promoter, a signal sequence, or an array of transcriptional regulator binding sites) and a different nucleic acid sequence, whereby the control sequence serves to control the transcription and/or translation of the different nucleic acid sequence.

When a prokaryotic cell is used as a host, a strong promoter capable of promoting transcription (e.g. a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence are generally included. In addition, for example, when a eukaryotic cell is used as a host, a promoter derived from the genome of a mammalian cell (e.g. a metallothionine promoter, β-actin promoter, human hemoglobin promoter or human muscle creatine promoter) or a promoter derived from a mammalian virus (e.g. an adenovirus late promoter, vaccinia virus 7.5k promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), or promoter of Rous sarcoma virus (RSV)) may be used, and a polyadenylation sequence is generally used as a transcription termination sequence

In some embodiments, the promoter may be a eukaryotic promoter, is preferably selected from among a cytomegalovirus (CMV) promoter, a PGK promoter, an EF1α promoter, an EFS promoter, a CBh promoter, an MSCV promoter, an SFFV promoter, and a UbC promoter, and is most preferably selected from among a CMV promoter, an EF1α promoter, and a CBh promoter, but is not limited thereto.

In some embodiments, the promoter may further include an enhancer sequence, but is not limited thereto.

In some cases, the vector may be fused with another sequence in order to facilitate purification of the antibody expressed therefrom. Examples of the sequence that is fused therewith include glutathione S-transferase (Pharmacia, USA), maltose-binding protein (NEB, USA), FLAG (IBI, USA), and 6x His (hexa-histidine; Qiagen, USA)).

The vector may include, as a selective marker, an antibiotic resistance gene that is commonly used in the art, for example, a gene conferring resistance to ampicillin, gentamicin, carbenicillin, chloramphenicol, streptomycin, kanamycin, puromycin, blasticidin, hygromycin, geneticin, neomycin, and tetracycline, but is not limited thereto.

In some embodiments, a nucleic acid encoding the stefin A protein variant specifically binding to CD40L or the fusion protein including the same may be incorporated and introduced into the gene of the host cell.

In some embodiments, the nucleic acid encoding the stefin A protein variant specifically binding to CD40L or the fusion protein including the same may be constructed through chemical synthesis using an oligonucleotide synthesizer. Oligonucleotides may be designed based on the amino acid sequence of the desired polypeptide and by selecting codons that are favored in the host cell in which the recombinant polypeptide of interest will be produced. A polynucleotide sequence encoding the isolated polypeptide of interest may be synthesized using standard methods. For example, a reverse-translated gene may be constructed using a complete amino acid sequence. In addition, a DNA oligomer containing a nucleotide sequence encoding a particular isolated polypeptide may be synthesized. For example, several small oligonucleotides encoding portions of a desired polypeptide may be synthesized and then ligated. Individual oligonucleotides generally contain 5' or 3' overhangs for complementary assembly.

In some embodiments, when the nucleic acid sequence encoding the stefin A protein variant specifically binding to CD40L or the fusion protein including the same is obtained, a vector including the same may be produced through recombinant DNA technology using a technique well known in the art. An expression vector containing a sequence encoding the stefin A protein variant of the present invention or the fusion protein including the same and appropriate transcriptional and translational control signals may be constructed using methods well known to those skilled in the art. Examples of such methods may include in-vitro recombinant DNA techniques, synthesis techniques, and in-vivo genetic recombination (e.g. Sambrook et al., 1990, MOLECULAR CLONING, A LABORATORY MANUAL, 2d Ed., Cold Spring Harbor Laboratory, Cold Spring Harbor, N.Y. and Ausubel et al. eds., 1998, CURRENT PROTOCOLS IN Molecular Biology, John Wiley & Sons, NY).

In some embodiments, introduction of the nucleic acid or the vector into a host cell may be performed through various methods known to those skilled in the art.

In some embodiments, the nucleic acid encoding the stefin A protein variant specifically binding to CD40L or the fusion protein including the same or the non-viral expression vector including the same may be delivered to the host cell using typical techniques (e.g. electroporation, RNA mediated gene transfer, liposome transfection, and calcium phosphate precipitation).

In some embodiments, when the gene delivery system of the present invention is constructed based on viral vector construction, delivery can be performed by conventional infection methods known in the art. For example, electroporation (Neumann, E. et al., EMBO J., 1:841 (1982); and Tur-Kaspa et al., Mol. Cell Biol., 6:716-718 (1986), gene bombardment in which DNA is loaded onto particles (eg, gold) and penetrated into cells (gene bombardment, Yang et al., Proc. Natl. Acad. Sci., 87:9568-9572 (1990), sonoporation, magnetofection, hydrodynamic delivery, etc., but are not limited thereto.

In some embodiments, the nucleic acid or vector may be introduced into the host cell through RNA-mediated gene transfer (see mRNA-based therapeutics: developing a new class of drugs" Nat Rev Drug Discov. 13(10):759-80.; Pardi et al. 2015 J Control Release 217:345-51; WO2017/162266; Stadler et al. (2017) Nature Medicine 23:815-817; WO/2017/036889; WO2015/034928; WO2015/034925; WO2013/151666 etc.). In some embodiments, exemplary mRNAs and other nucleic acids are included by reference in the specification and drawings described in WO2017/049275, WO2016/118724, WO2016/118725, WO2016/011226, WO2015/196128, WO/2015/196130, WO/2015/196118, WO/2015/089511, and WO2015/105926.

In some embodiments, the nucleic acid or vector may be introduced into the host cell through electroporation (Kim et al. Cancer Gene Ther, (2016) 23(10): 341-347). In some embodiments, the electroporation includes, but is not limited to, methods described in U.S. Patent No. 7,245,963; 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359; International Publication WO/2017/106795, WO/2016/161201, WO/2016/154473, WO/2016/112359 and WO/2014/066655.

In some embodiments, a transfection enhancing formulations may be used for delivery of the nucleic acids. In some embodiments, the nucleic acids of the present invention can also be encapsulated in liposomes, preferably cationic liposomes (Wong, T. K. et al., Gene, 10:87(1980); Nicolau and Sene, Biochim. Biophys. Acta, 721:185-190 (1982); and Nicolau et al., Methods Enzymol., 149:157-176 (1987)) or polymersomes (synthetic liposomes) which can interact with the cell membrane and fuse or undergo endocytosis to effect nucleic acid transfer into the cell. The DNA also can be formed into complexes with polymers (polyplexes) or with dendrimers which can directly release their load into the cytoplasm of a cell.

Illustrative carriers useful in this regard include microparticles of poly(lactide-co-glycolide), polyacrylate, latex, starch, cellulose, dextran and the like. Other illustrative carriers include supramolecular biovectors, which comprise a non-liquid hydrophilic core (e.g., a crosslinked polysaccharide or oligosaccharide) and, optionally, an external layer comprising an amphiphilic compound, such as a phospholipid (see e.g., U.S. Pat. No. 5,151,254 and PCT applications WO 94/20078, WO/94/23701 and WO 96/06638). The amount of active agent contained within a sustained release formulation depends upon the site of implantation, the rate and expected duration of release and the nature of the condition to be treated or prevented. Biodegradable microspheres (e.g., polylactate polyglycolate) may be employed as carriers for compositions. Suitable biodegradable microspheres are disclosed, for example, in U.S. Pat. NOS: 4,897,268; 5,075,109; 5,928,647; 5,811,128; 5,820,883; 5,853,763; 5,814,344, 5,407,609 and 5,942,252. Modified hepatitis B core protein carrier systems such as described in WO/99 40934, and references cited therein, will also be useful for many applications. Another illustrative carrier/delivery system employs a carrier comprising particulate-protein complexes, such as those described in U.S. Pat. No. 5,928,647, which can have the added benefit when used to deliver the nucleic acids of the present invention. Biodegradable polymeric nanoparticles facilitate nonviral nucleic acid transfer to cells. Small (approximately 200 nm), positively charged (approximately 10 mV) particles are formed by the self-assembly of cationic, hydrolytically degradable poly (beta-amino esters) and plasmid DNA.

The nucleic acids of the present invention may also be introduced into cells by direct microinjection, temporary cell permeabilizations (e.g., co-administration of repressor and/or activator with a cell permeabilizing agent), fusion to membrane translocating peptides, and the like.

Lipid-mediated nucleic acid delivery and expression of foreign nucleic acids, including mRNA, in vitro and in vivo has been very successful. Lipid based non-viral formulations provide an alternative to viral gene therapies. Current in vivo lipid delivery methods use subcutaneous, intradermal, intratumoral, or intracranial injection. Advances in lipid formulations have improved the efficiency of gene transfer in vivo (see PCT Application WO 98/07408). For instance, a lipid formulation composed of an equimolar ratio of 1,2-bis(oleoyloxy)-3-(trimethyl ammonio)propane (DOTAP) and cholesterol can significantly enhance systemic in vivo gene transfer. The DOTAP : cholesterol lipid formulation forms unique structure termed a "sandwich liposome". This formulation is reported to "sandwich" DNA between an invaginated bi-layer or 'vase' structure. Beneficial characteristics of these lipid structures include a positive p, colloidal stabilization by cholesterol, two-dimensional nucleic acid packing and increased serum stability. Beneficial characteristics of these lipid structures include a positive p, colloidal stabilization by cholesterol, two-dimensional nucleic acid packing and increased serum stability.

Cationic liposome technology is based on the ability of amphipathic lipids, possessing a positively charged head group and a hydrophobic lipid tail, to bind to negatively charged DNA or RNA and form particles that generally enter cells by endocytosis. Some cationic liposomes also contain a neutral co-lipid, thought to enhance liposome uptake by mammalian cells. Similarly, other polycations, such as poly-l-lysine and polyethylene-imine, complex with nucleic acids via charge interaction and aid in the condensation of DNA or RNA into nanoparticles, which are then substrates for endosome-mediated uptake. Several of these cationic-nucleic acid complex technologies have been developed as potential clinical products, including complexes with plasmid DNA (pDNA), oligodeoxynucleotides, and various forms of synthetic RNA, and can be used as part of the delivery system for the encoded nucleic acids.

The encoded nucleic acids of the present invention may be associated with polycationic molecules that serve to enhance uptake into cells. Complexing the nucleic acid construct with polycationic molecules also helps in packaging the construct such their size is reduced, which is believed to assist with cellular uptake. Once in the endosome, the complex dissociates due to the lower pH, and the polycationic molecules can disrupt the endosome's membrane to facilitate DNA escape into the cytoplasm before it can be degraded. Preliminary data shows that the nucleic acid construct embodiments had enhanced uptake into SCs over DCs when complexed with the polycationic molecules polylysine or polyethyleneimine. One example of polycationic molecules useful for complexing with nucleic acid constructs includes cell penetrating peptides (CPP), examples include polylysine (described above), polyarginine and Tat peptides. Cell penetrating peptides (CPP) are small peptides which can bind to DNA and once released penetrate cell membranes to facilitate escape of the DNA from the endosome to the cytoplasm. Another example of a CPP pertains to a 27-residue chimeric peptide, termed MPG, was shown some time ago to bind ss- and ds-oligonucleotides in a stable manner, resulting in a non-covalent complex that protected the nucleic acids from degradation by DNase and effectively delivered oligonucleotides to cells in vitro (Mahapatro A, et al., J Nanobiotechnol, 2011, 9:55). The non-covalent complex formed small particles of approximately 150 nm to 1 um when different peptide:DNA ratios were examined, and the 10:1 and 5: 1ratios (150 nm and 1 um respectively). Another CPP pertains to a modified tetrapeptide [tetralysine containing guanidinocarbonylpyrrole (GCP) groups (TL-GCP)], which was reported to bind with high affinity to a 6.2 kb plasmid DNA resulting in a positive charged aggregate of 700-900 nm (Li et al., Agnew Chem Int Ed Enl 2015; 54(10):2941-4). RNA can also be complexed by such polycationic molecules for in vivo delivery. Other examples of polycationic molecules that may be complexed with the nucleic acid constructs described herein include polycationic polymers commercially available as JETPRIME^{®} and In vivo JET (Polypus-transfection, S.A., Illkirch, France).

In some embodiments, the present disclosure contemplates a method of delivering an mRNA (or other polynucleotide) encoding the stefin A protein variant specifically binding to CD40L or the fusion protein or the conjugate to a patient's cells by administering a nanoparticle composition comprising (i) a lipid component comprising a compound of formula (I), a phospholipid, a structural lipid, and a PEG lipid; and (ii) an mRNA (or other polynucleotide), said administering comprising contacting said mammalian cell with said nanoparticle composition, whereby said mRNA (or other polynucleotide) is delivered to said cell.

In exemplary embodiments, the PEG lipid is selected from the group consisting of a PEG-modified phosphatidylethanolamine, a PEG-modified phosphatide acid, a PEG-modified ceramide, a PEG-modified dialkylamine, a PEG-modified diacylglycerol and a PEG-modified dialkylglycerol. In exemplary embodiments, the structural lipid is selected from the group consisting of cholesterol, fecosterol, sitosterol, ergosterol, campesterol, stigmasterol, brassicasterol, tomatidine, ursolic acid, and alphatocopherol. In some embodiments, the structural lipid is cholesterol.

In exemplary embodiments, the phospholipid includes a moiety selected from the group consisting of phosphatidyl choline, phosphatidyl ethanolamine, phosphatidyl glycerol, phosphatidyl serine, phosphatidic acid, 2-lysophosphatidyl choline, and a sphingomyelin. In some embodiments, the phospholipid includes at least one fatty acid moiety selected from the group consisting of lauric acid, myristic acid, myristoleic acid, palmitic acid, palmitoleic acid, stearic acid, oleic acid, linoleic acid, alpha-linolenic acid, erucic acid, arachidic acid, arachidonic acid, phytanoic acid, eicosapentaenoic acid, behenic acid, docosapentaenoic acid, and docosahexaenoic acid. In some embodiments, the phospholipid is selected from the group consisting of 1 ,2-dilinoleoyl-sn-glycero-3-phosphocholine (DLPC), 1 ,2-dimyristoyl-sn-glycero-phosphocholine (DMPC), 1 ,2-dioleoyl-sn-glycero-3-phosphocholine (DOPC), 1 ,2-dipalmitoyl-sn-glycero-3-phosphocholine (DPPC), 1 ,2-distearoyl-sn-glycero-3-phosphocholine (DSPC), 1 ,2-diundecanoyl-sn-glycero-phosphocholine (DUPC), 1 -palmitoyl-2-oleoyl-sn-glycero-3-phosphocholine (POPC), 1 ,2-di-0-octadecenyl-sn-glycero-3-phosphocholine (1 8:0 Diether PC), 1 -oleoyl-2-cholesterylhemisuccinoyl-sn-glycero-3-phosphocholine (OChemsPC), 1 -hexadecyl-sn-glycero-3-phosphocholine (C16 Lyso PC), 1 ,2-dilinolenoyl-sn-glycero-3-phosphocholine, 1 ,2-diarachidonoyl-sn-glycero-3-phosphocholine, 1 ,2-didocosahexaenoyl-sn-glycero-3-phosphocholine, 1 ,2-dioleoyl-sn-glycero-3-phosphoethanola mine (DOPE), 1 ,2-diphytanoyl-sn-glycero-3-phosphoethanolamine (ME 1 6.0 PE), 1 ,2-distearoyl-sn-glycero-3-phosphoethanolamine, 1 ,2-dilinoleoyl-sn-glycero-3-phosphoethanolamine, 1 ,2-dilinolenoyl-sn-glycero-3-phosphoethanolamine, 1 ,2-diarachidonoyl-sn-glycero-3-phosphoethanolamine, 1 ,2-didocosahexaenoyl-sn-glycero-3-phosphoethanolamine, 1 ,2-dioleoyl-sn-glycero-3-phospho-rac-(1 -glycerol) sodium salt (DOPG), and sphingomyelin In some embodiments, the phospholipid is DOPE or DSPC.

To further examples, the phospholipid can be DOPE and said the lipid component can comprise about 35 mol % to about 45 mol % said compounds, about 1 0 mol % to about 20 mol % DOPE, about 38.5 mol % to about 48.5 mol % structural lipid, and about 1 .5 mol % PEG lipid. The lipid component can be about 40 mol % said compounds, about 15 mol % phospholipid, about 43.5 mol % structural lipid, and about 1 .5 mol % PEG lipid.

In some embodiments, the wt/wt ratio of lipid component to the stefin A protein variant specifically binding to CD40L or the fusion protein or the conjugate encoding mRNA (or other polynucleotide) is from about 5:1 to about 50:1, or about 10:1 to about 40:1

In some embodiments, the mean size of said nanoparticle composition is from about 50 nm to about 150 nm, or from about 80 nm to about 120 nm.

In some embodiments, the polydispersity index of said nanoparticle composition is from about 0 to about 0.18, or from about 0.13 to about 0.17.

In some embodiments, the nanoparticle composition has a zeta potential of about -10 to about +20 mV.

In some embodiments, the nanoparticle composition further comprises a cationic and/or ionizable lipid selected from the group consisting of 3-(didodecylamino)-N1 ,N 1 ,4-tridodecyl-1 -piperazineethanamine (KL1 0), 14,25-ditridecyl-1 5, 1 8,21 ,24-tetraaza-octatriacontane (KL25), 1,2-dilinoleyloxy-N,N-dimethylaminopropane (DLin-DMA), 2,2-dilinoleyl-4-dimethylaminomethyl-[1 ,3]-dioxolane (DLin-K-DMA), heptatriaconta-6, 9,28,31 -tetraen-1 9-yl 4-(dimethylamino)butanoate (DLin-MC3-DMA), 2,2-dilinoleyl-4-(2-dimethylaminoethyl)-[1 ,3]-dioxolane (DLin-KC2-DMA), 1 ,2-dioleyloxy-N,N-dimethylaminopropane (DODMA), and (2R)-2-({8-[(3P)-cholest-5-en-3-yloxy]octyl}oxy)-N,N-dimethyl-3-[(9Z,1 2Z)-octadeca-9, 12-dien-1 - yl oxy]propan-1 -amine (Octyl-CLinDMA (2R)).

The nucleic acids or the vector of the present invention may be introduced into the host cell through a method such as transduction or transfection. As used herein, the term "transduction" refers to introduction of DNA into a host such that the DNA becomes replicable either as an extrachromosomal factor or through chromosomal integration. As used herein, the term "transfection" means that an expression vector is accommodated by the host cell, regardless of whether or not any coding sequence is actually expressed. In order to introduce the vector, a variety of techniques commonly used to introduce exogenous nucleic acids (DNA or RNA) into prokaryotic or eukaryotic host cells, for example, electrophoresis, calcium phosphate precipitation, DEAE-dextran transfection, or lipofection may be used, but the present invention is not limited thereto.

It is to be understood that not all vectors and expression control sequences function equally in expressing the DNA sequence of the present invention. Likewise, not all hosts function equally for the same expression system. However, those skilled in the art will be able to make an appropriate selection from among various vectors, expression control sequences, and hosts without undue experimentation and without departing from the scope of the present invention. For example, a vector may be selected in consideration of the host. This is because the vector has to be able to replicate in the host. Also, the number of copies of a vector, ability to control the number of copies, and expression of another protein encoded by the vector, for example, an antibiotic marker, have to be taken into consideration. In selecting the expression control sequence, various factors have to be considered. For example, the relative strength of the sequences, controllability thereof, compatibility with the DNA sequences of the present invention, etc., should be taken into account, particularly with regard to possible secondary structures. The single-celled host should be selected in consideration of factors such as the selected vector, the toxicity and secretory properties of the product encoded by the DNA sequence of the invention, the ability to correctly fold the protein, culture and fermentation requirements, ease of purification of the product encoded by the DNA sequence of the present invention from the host, and the like. Within the scope of these parameters, those skilled in the art may select various vector/expression control sequence/host combinations capable of expressing the DNA sequence of the present invention in fermentation or large-scale animal culture. Examples of a screening method of cloning cDNA by expression cloning may include a binding method, a panning method, a film emulsion method, etc.

In some embodiments, a "transformation enhancer" may be additionally used to increase the efficiency of transformation. The "transformation enhancer" is generally preferably a cationic polymer, and facilitates incorporation of negatively charged nucleic acids or genes into host cells.

In some embodiments, the transduction enhancer may be selected from among, for example, polybrene, protamine sulfate, and LentiBOOST from Sirion, and is most preferably polybrene, but is not limited thereto.

### Expression Methods and Systems

The stefin A protein variant specifically binding to CD40L or the fusion protein or the conjugate of the present invention can be produced by any suitable method known in the art. Such methods range from direct protein synthesis methods to constructing a DNA sequence encoding polypeptide sequences and expressing those sequences in a suitable host. For stefin A protein variant including further modifications, such as a chemical modifications or conjugation, thestefin A protein variant can be further manipulated chemically or enzymatically after isolation form the host cell or chemical synthesis.

Therefore, in another aspect, the present invention relates to a method for producing the stefin A protein variant specifically binding to CD40L or the fusion protein or the conjugate comprising the step of culturing the genetically engineered cell.

In some embodiments, when the conjugate includes various modifications such as chemical modification or conjugation, it may be prepared by further manipulation chemically or enzymatically after separation from the host cell or chemical synthesis.

Methods for introducing and expressing a sequence encoding a protein or polypeptide into a host cell for production are described in, for example, WO 04/041862, WO 2006/122786, WO 2008/020079, WO 2008/142164 and WO 2009/068627. Reference may be made to what has been described, but is not limited thereto.

In some embodiments, the nucleic acid encoding producing the stefin A protein variant specifically binding to CD40L or the fusion protein or the conjugate may be constructed by chemical synthesis using an oligonucleotide synthesizer. Oligonucleotides can be designed based on the amino acid sequence of the desired polypeptide and selecting those codons that are favored in the host cell in which the recombinant polypeptide of interest will be produced. Standard methods can be applied to synthesize a polynucleotide sequence encoding an isolated polypeptide of interest. For example, a complete amino acid sequence can be used to construct a back-translated gene. Further, a DNA oligomer containing a nucleotide sequence coding for the particular isolated polypeptide can be synthesized. For example, several small oligonucleotides coding for portions of the desired polypeptide can be synthesized and then ligated. The individual oligonucleotides typically contain 5' or 3' overhangs for complementary assembly.

In some embodiments, the nucleic acid encoding a stefin A protein variant that specifically binds to CD40L of the present invention may be, for example, any one of those listed in Table 4, but is not limited thereto.

Based on the nucleic acid encoding the stefin A protein variant that specifically binds to CD40L of the present invention, an expression vector containing may be constructed using recombinant DNA technology known in the art. Methods which are well known to those skilled in the art can be used to construct expression vectors containing the stefin A protein variant specifically binding to CD40L or the fusion protein or the conjugate coding sequences and appropriate transcriptional and translational control signals. These methods include, for example, in vitro recombinant DNA techniques, synthetic techniques, and *in vivo* genetic recombination, but not limited thereto (See, for example, the techniques described in Sambrook et al, 1990, MOLECULAR CLONING, A LABORATORY MANUAL, 2d Ed., Cold Spring Harbor Laboratory, Cold Spring Harbor, N.Y. and Ausubel et al. eds., 1998, CURRENT PROTOCOLS IN MOLECULAR BIOLOGY, John Wiley & Sons, NY).

In some embodiments, the expression vector comprising the nucleic acids of the present invention can be transferred to a host cell by conventional techniques (e.g., electroporation, liposomal transfection, and calcium phosphate precipitation) and the transfected cells are then cultured by conventional techniques to produce the stefin A protein variant specifically binding to CD40L or the fusion protein or the conjugate of the disclosure. In specific embodiments, the expression of protein is regulated by a constitutive, an inducible or a tissue, specific promoter.

In some embodiments, the expression vector may include an origin of replication, such as may be selected based upon the type of host cell being used for expression. By way of example, the origin of replication from the plasmid pBR322 (Product No. 303-3s, New England Biolabs, Beverly, Mass.) is useful for most Gram- negative bacteria while various origins from SV40, polyoma, adenovirus, vesicular stomatitus virus (VSV) or papillomaviruses (such as HPV or BPV) are useful for cloning vectors in mammalian cells. Generally, the origin of replication component is not needed for mammalian expression vectors (for example, the SV40 origin is often used because it contains the early promoter).

In some embodiments, the vector may include at least one selectable marker gene, e.g., genetic elements that encode a protein necessary for the survival and growth of a host cell grown in a selective culture medium. Typical selection marker genes encode proteins that (a) confer resistance to antibiotics or other toxins, e.g., ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells. Other selection genes may be used to amplify the gene which will be expressed. Amplification is a process where genes which are in greater demand for the production of a protein critical for growth are reiterated in tandem within the chromosomes of successive generations of recombinant cells. Examples of selectable markers for mammalian cells include dihydrofolate reductase (DHFR) and thymidine kinase. The mammalian cell transformants are placed under selection pressure which only the transformants are uniquely adapted to survive by virtue of the marker present in the vector. Selection pressure is imposed by culturing the transformed cells under conditions in which the concentration of selection agent in the medium is successively changed, thereby leading to amplification of both the selection gene and the DNA that encodes the stefin A protein variant specifically binding to CD40L or the fusion protein or conjugate. As a result, increased quantities of proteinare synthesized from the amplified DNA.

In some embodiments, the vector may also include at least one ribosome binding site, which will be transcribed into the mRNA including the coding sequence for the stefin A protein variant specifically binding to CD40L or the fusion protein or conjugate. For example, such a site is characterized by a Shine-Dalgarno sequence (prokaryotes) or a Kozak sequence (eukaryotes). The element is typically located 3' to the promoter and 5' to the coding sequence of the polypeptide to be expressed. The Shine-Dalgarno sequence is varied but is typically a polypurine (having a high A-G content). Many Shine-Dalgarno sequences have been identified, each of which can be readily synthesized using methods set forth above and used in a prokaryotic vector.

In some embodiments, the expression vectors will typically contain a promoter that is recognized by the host organism and operably linked to a nucleic acid molecule encoding the stefin A protein variant specifically binding to CD40L or the fusion protein or conjugate. Either a native or heterologous promoter may be used depending on the host cell used for expression and the yield desired.

In some embodiments, the promoters for use with prokaryotic hosts include the beta-lactamase and lactose promoter systems; alkaline phosphatase, a tryptophan (trp) promoter system; and hybrid promoters such as the tac promoter. Other known bacterial promoters are also suitable. Their sequences have been published, and they can be ligated to a desired nucleic acid sequence(s), using linkers or adapters as desired to supply restriction sites.

Promoters for use with yeast hosts are also known in the art. Yeast enhancers are advantageously used with yeast promoters. Suitable promoters for use with mammalian host cells are well known and include those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40). Other suitable mammalian promoters include heterologous mammalian promoters, e.g., heat-shock promoters and the actin promoter.

Additional promoters which may be used for expressing the selective binding agents of the disclosure include but are not limited to: the SV40 early promoter region (Bernoist and Chambon, Nature, 290:304-310, 1981); the CMV promoter; the promoter contained in the 3' long terminal repeat of Rous sarcoma virus (Yamamoto et al. (1980), Cell 22: 787-97); the herpes thymidine kinase promoter (Wagner et al. (1981), Proc. Natl. Acad. Sci. U.S.A. 78: 1444-5); the regulatory sequences of the metallothionine gene (Brinster et al, Nature, 296; 39-42, 1982); prokaryotic expression vectors such as the beta- lactamase promoter (Villa-Kamaroff, et al., Proc. Natl. Acad. Sci. U.S.A., 75; 3727-3731, 1978); or the tac promoter (DeBoer, et al. (1983), Proc. Natl. Acad. Sci. U.S.A., 80: 21-5). Also of interest are the following animal transcriptional control regions, which exhibit tissue specificity and have been utilized in transgenic animals: the elastase I gene control region which is active in pancreatic acinar cells (Swift et al. (1984), Cell 38: 639-46; Omitz et al. (1986), Cold Spring Harbor Symp. Quant. Biol. 50: 399-409; MacDonald (1987), Hepatology 7: 425-515); the insulin gene control region which is active in pancreatic beta cells (Hanahan (1985), Nature 315: 115-22); the immunoglobulin gene control region which is active in lymphoid cells (Grosschedl et al. (1984), Cell 38; 647-58; Adames et al. (1985), Nature 318; 533-8; Alexander et al. (1987), Mol. Cell. Biol. 7: 1436-44); the mouse mammary tumor virus control region which is active in testicular, breast, lymphoid and mast cells (Leder et al. (1986), Cell 45: 485-95), albumin gene control region which is active in liver (Pinkert et al. (1987), Genes and Devel. 1: 268-76); the alphafetoprotein gene control region which is active in liver (Krumlauf et al. (1985), MoI. Cell. Biol. 5: 1639-48; Hammer et al. (1987), Science, 235: 53-8); the alpha 1-antitrypsin gene control region which is active in the liver (Kelsey et al. (1987), Genes and Devel. 1: 161-71); the beta-globin gene control region which is active in myeloid cells (Mogram et al., Nature, 315 338-340, 1985; Kollias et al. (1986), Cell 46: 89-94); the myelin basic protein gene control region which is active in oligodendrocyte cells in the brain (Readhead et al. (1987), Cell, 48: 703-12); the myosin light chain-2 gene control region which is active in skeletal muscle (Sani (1985), Nature, 314: 283-6); and the gonadotropic releasing hormone gene control region which is active in the hypothalamus (Mason et al. (1986), Science 234: 1372-8).

An enhancer sequence may be inserted into the vector to increase transcription in eukaryotic host cells. Several enhancer sequences available from mammalian genes are known (e.g., globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters.

While an enhancer may be spliced into the vector at a position 5' or 3' to the polypeptide coding region, it is typically located at a site 5' from the promoter.

Vectors for expressing nucleic acids include those which are compatible with bacterial, insect, and mammalian host cells. Such vectors include, inter alia, pCRII, pCR3, and pcDNA3.1 (Invitrogen Company, San Diego, Calif.), pBSII (Stratagene Company, La Jolla, Calif.), pET15 (Novagen, Madison, Wis.), pGEX (Pharmacia Biotech, Piscataway, N.J.), pEGFP-N2 (Clontech, Palo Alto, Calif.), pETL (BlueBacII; Invitrogen), pDSR- alpha (PCT Publication No. WO90/14363) and pFastBacDual (Gibco/BRL, Grand Island, N.Y.).

In some embodiments, additional possible vectors include but are not limited to, cosmids, plasmids or modified viruses, but the vector system must be compatible with the selected host cell. Such vectors include but are not limited to plasmids such as Bluescript^{®} plasmid derivatives (a high copy number ColEl-based phagemid, Stratagene Cloning Systems Inc., La Jolla Calif.), PCR cloning plasmids designed for cloning Taq-amplified PCR products (e.g., TOPO^{™}. TA Cloning^{®} Kit, PCR2.1 plasmid derivatives, Invitrogen, Carlsbad, Calif.), and mammalian, yeast or virus vectors such as a baculovirus expression system (pBacPAK plasmid derivatives, Clontech, Palo Alto, Calif.). The recombinant molecules can be introduced into host cells via transformation, transfection, infection, electroporation, or other known techniques

Eukaryotic and prokaryotic host cells, including mammalian cells as hosts for expression of the stefin A protein variant specifically binding to CD40L or the fusion protein or conjugatedisclosed herein are well known in the art and include many immortalized cell lines available from the American Type Culture Collection (ATCC). These include, inter alia, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, HEK-293 cells and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. Fungal cells include yeast and filamentous fungus cells including, for example, Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens and Neurospora crassa. Pichia sp., any Saccharomyces sp., Hansenula polymorpha, any Kluyveromyces sp., Candida albicans, any Aspergillus sp., Trichoderma reesei, Chrysosporium lucknowense, any Fusarium sp., Yarrowia lipolytica, and Neurospora crassa.

A variety of host-expression vector systems may be utilized to express the stefin A protein variant or the fusion protein or conjugate of the present invention of the disclosure. Such host-expression systems represent vehicles by which the coding sequences of the stefin A protein variant or the fusion protein or conjugate of the present inventionmay be produced and subsequently purified, but also represent cells which may, when transformed or transfected with the appropriate nucleotide coding sequences, express the stefin A protein variant or the fusion protein or conjugate of the present inventionin situ. These include but are not limited to, microorganisms such as bacteria (e.g., E. coli and B. subtilis) transformed with recombinant bacteriophage DNA, plasmid DNA or cosmid DNA expression vectors containing the nucleic acids sequences of the present invention; yeast (e.g., Saccharomyces pichia) transformed with recombinant yeast expression vectors containing the nucleic acids of the present invention; insect cell systems infected with recombinant virus expression vectors (e.g., baculovirus) containing the nucleic acids of the present invention; plant cell systems infected with recombinant virus expression vectors (e.g., cauliflower mosaic virus (CµMV) and tobacco mosaic virus (TMV)) or transformed with recombinant plasmid expression vectors (e.g., Ti plasmid) containing the nucleic acids of the present invention; or mammalian cell systems (e.g., COS, CHO, BHK, 293, 293T, 3T3 cells, lymphotic cells (see U.S. Pat. No. 5,807,715), Per C.6 cells (rat retinal cells developed by Crucell)) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the protein being expressed. For example, when a large quantity of such a protein is to be produced, for the generation of pharmaceutical compositions of the protein, vectors which direct the expression of high levels of fusion protein products that are readily purified may be desirable. Such vectors include but are not limited, to the E. coli expression vector pUR278 (Ruther et al. (1983) "Easy Identification Of cDNA Clones," EMBO J. 2:1791-1794), in which the stefin A protein variant coding sequence may be ligated individually into the vector in frame with the lac Z coding region so that a fusion protein is produced; pIN vectors (Inouye et al. (1985) "Up-Promoter Mutations In The Lpp Gene Of Escherichia coli," Nucleic Acids Res. 13:3101-3110; Van Heeke et al. (1989) "Expression Of Human Asparagine Synthetase In Escherichia coli," J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in Spodoptera frugiperda cells, the stefin A protein variant or the fusion protein or conjugate of the present inventioncoding sequence may be cloned individually into non-essential regions (e.g., the polyhedrin gene) of the virus and placed under control of an AcNPV promoter (e.g., the polyhedrin promoter).

In mammalian host cells, a number of viral-based expression systems may be utilized. In cases where an adenovirus is used as an expression vector, the protein coding sequence of interest may be ligated to an adenovirus transcription/translation control complex, e.g., the late promoter and tripartite leader sequence. This chimeric gene may then be inserted in the adenovirus genome by in vitro or *in vivo* recombination. Insertion in a non-essential region of the viral genome (e.g., region E1 or E3) will result in a recombinant virus that is viable and capable of expressing the immunoglobulin molecule in infected hosts. (see e.g., see Logan et al. (1984) "Adenovirus Tripartite Leader Sequence Enhances Translation Of mRNAs Late After Infection," Proc. Natl. Acad. Sci. (U.S.A.) 81:3655-3659). Specific initiation signals may also be required for efficient translation of inserted the nucleic acids. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bitter et al. (1987) "Expression and Secretion Vectors For Yeast," Methods in Enzymol. 153:516-544).

In addition, a host cell strain may be chosen which modulates the expression of the inserted sequences or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 293T, 3T3, WI38, BT483, Hs578T, HTB2, BT20 and T47D, CRL7030 and Hs578Bst.

For long-term, high-yield production of recombinant proteins, stable expression is contemplated. For example, cell lines which stably express an antibody of the disclosure may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the stefin A protein variant or the fusion protein or conjugate of the present invention. Such engineered cell lines may be particularly useful in screening and evaluation of compounds that interact directly or indirectly with the stefin A protein variant or the fusion protein or conjugate of the present invention.

In some embodiments, anumber of selection systems may be used, including but not limited to the herpes simplex virus thymidine kinase (Wigler et al. (1977) "Transfer of Purified Herpes Virus Thymidine Kinase Gene to Cultured Mouse Cells," Cell 11:223-232), hypoxanthine-guanine phosphoribosyltransferase (Szybalska et al. (1962) "Genetics of Human Cess Line. IV. DNA-Mediated Heritable Transformation of a Biochemical Trait," Proc. Natl. Acad. Sci. (U.S.A.) 48:2026-2034), and adenine phosphoribosyltransferase (Lowy et al. (1980) "Isolation of Transforming DNA: Cloning The Hamster Aprt Gene," Cell 22:817-823) genes can be employed in tk-, hgprt- or aprt- cells, respectively. Also, antimetabolite resistance can be used as the basis of selection for the following genes: *dhfr,* which confers resistance to methotrexate (Wigler et al. (1980) "Transformation Of Mammalian Cells With An Amplfiable Dominant-Acting Gene," Proc. Natl. Acad. Sci. (U.S.A.) 77:3567-3570; O'Hare et al. (1981) "Transformation Of Mouse Fibroblasts To Methotrexate Resistance By A Recombinant Plasmid Expressing A Prokaryotic Dihydrofolate Reductase," Proc. Natl. Acad. Sci. (U.S.A.) 78:1527-1531); *gpt,* which confers resistance to mycophenolic acid (Mulligan et al. (1981) "Selection For Animal Cells That Express The Escherichia coli Gene Coding For Xanthine-Guanine Phosphoribosyltransferase," Proc. Natl. Acad. Sci. (U.S.A.) 78:2072-2076); *neo,* which confers resistance to the aminoglycoside G-418 (Tachibana et al. (1991) "Altered Reactivity Of Immunoglobutin Produced By Human-Human Hybridoma Cells Transfected By pSV.2-Neo Gene," Cytotechnology 6(3):219-226; Tolstoshev (1993) "Gene Therapy, Concepts, Current Trials And Future Directions," Ann. Rev. Pharmacol. Toxicol. 32:573-596; Mulligan (1993) "The Basic Science of Gene Therapy," Science 260:926-932; and Morgan et al. (1993) "Human gene therapy," Ann. Rev. Biochem. 62:191-217). Methods commonly known in the art of recombinant DNA technology which can be used are described in Ausubel et al. (eds.), 1993, CURRENT PROTOCOLS IN MOLECULAR BIOLOGY, John Wiley & Sons, NY; Kriegler, 1990, GENE TRANSFER AND EXPRESSION, A LABORATORY MANUAL, Stockton Press, NY; and in Chapters 12 and 13, Dracopoli et al. (eds), 1994, CURRENT PROTOCOLS IN HUMAN GENETICS, John Wiley & Sons, NY.; Colbere-Garapin et al. (1981) "A New Dominant Hybrid Selective Marker For Higher Eukaryotic Cells," J. Mol. Biol. 150:1-14; and hygro, which confers resistance to hygromycin (Santerre et al. (1984) "Expression Of Prokaryotic Genes For Hygromycin B And G418 Resistance As Dominant-Selection Markers In Mouse L Cells," Gene 30:147-156).

The expression levels of the stefin A protein variant or the fusion protein or conjugate of the present inventioncan be increased by vector amplification (for a review, see Bebbington and Hentschel, "The Use of Vectors Based on Gene Amplification For The Expression Of Cloned Genes In Mammaian Cells," in DNA CLONING, Vol. 3. (Academic Press, New York, 1987)). When a marker in the vector system expressing a the stefin A protein variant or the fusion protein or conjugate of the present inventionis amplifiable, increase in the level of inhibitor present in culture of host cell will increase the number of copies of the marker gene. Since the amplified region is associated with the nucleotide sequence of the recombinant AFFIMER^{®} agent protein, production of the stefin A protein variant or the fusion protein or conjugate of the present inventionwill also increase (Crouse et al. (1983) "Expression and Amplification of Engineered Mouse Dihydrofolate Reductase Minigenes," Mol. Cell. Biol. 3:257-266).

Where the fusion protein of the present invention is an antibody fusion or other multiprotein complex, the host cell may be co-transfected with two expression vectors, for instance the first vector encoding a heavy chain and the second vector encoding a light chain derived polypeptide, one or both of which includes the stefin A protein variant coding sequence. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot (1986) "Expression and Amplification of Engineered Mouse Dihydrofolate Reductase Minigenes," Nature 322:562-565; Kohler (1980) "Immunoglobulin Chain Loss In Hybridoma Lines," Proc. Natl. Acad. Sci. (U.S.A.) 77:2197-2199). The coding sequences for the heavy and light chains may comprise cDNA or genomic DNA.

In general, glycoproteins produced in a particular cell line or transgenic animal will have a glycosylation pattern that is characteristic for glycoproteins produced in the cell line or transgenic animal. Therefore, the particular glycosylation pattern of the stefin A protein variant or the fusion protein or conjugate of the present invention will depend on the particular cell line or transgenic animal used to produce the protein. In some embodiments of stefin A protein variant/antibody fusions, a glycosylation pattern comprising only non-fucosylated N-glycans may be advantageous, because in the case of antibodies this has been shown to typically exhibit more potent efficacy than fucosylated counterparts both in vitro and *in vivo* (See for example, Shinkawa et al., J. Biol. Chem. 278: 3466-3473 (2003); U.S. Pat. NOS: 6,946,292 and 7,214,775).

Further, expression of the stefin A protein variant or the fusion protein or conjugate of the present inventionfrom production cell lines can be enhanced using a number of known techniques. For example, the glutamine synthetase gene expression system (the GS system) is a common approach for enhancing expression under certain conditions. The GS system is discussed in whole or part in connection with European Patent NOS: 0216846, 0256055, and 0323997 and European Patent Application No. 89303964.4. Thus, in some embodiments of the disclosure, the mammalian host cells (e.g., CHO) lack a glutamine synthetase gene and are grown in the absence of glutamine in the medium wherein, however, the polynucleotide encoding the immunoglobulin chain comprises a glutamine synthetase gene which complements the lack of the gene in the host cell. Such host cells containing the binder or polynucleotide or vector as discussed herein as well as expression methods, as discussed herein, for making the binder using such a host cell are part of the present disclosure.

Expression of recombinant proteins in insect cell culture systems (e.g., baculovirus) also offers a robust method for producing correctly folded and biologically functional proteins. Baculovirus systems for production of heterologous proteins in insect cells are well-known to those of skill in the art.

The stefin A protein variant or the fusion protein or conjugate of the present invention produced by a transformed host can be purified according to any suitable method. Standard methods include chromatography (e.g., ion exchange, affinity, and sizing column chromatography), centrifugation, differential solubility, or by any other standard technique for protein purification. Affinity tags such as hexa-histidine, maltose binding domain, influenza coat sequence, and glutathione-S-transferase can be attached to the protein to allow easy purification by passage over an appropriate affinity column. Isolated proteins can also be physically characterized using such techniques as proteolysis, mass spectrometry (MS), nuclear magnetic resonance (NMR), high performance liquid chromatography (HPLC), and x-ray crystallography.

In some embodiments, the stefin A protein variant or the fusion protein or conjugate of the present invention produced in bacterial culture can be isolated, for example, by initial extraction from cell pellets, followed by at least one concentration, salting-out, aqueous ion exchange, or size exclusion chromatography steps. HPLC can be employed for final purification steps. Microbial cells employed in expression of a recombinant protein can be disrupted by any convenient method, including freeze-thaw cycling, sonication, mechanical disruption, or use of cell lysing agents.

### USE

### Use - Medical/Pharmaceutical use (a method, a pharmaceutical composition, etc.)

In particular, it is well known in the art that the activation of T cells and B cells by interaction of CD40L/CD40 acts as a pathogenic factor for autoimmune diseases or inflammatory diseases that have a major influence on pathology. Specifically, it is a pathogenic factor of various diseases such as type 1 diabetes, thyroiditis, psoriasis, lupus (systemic lupus erythematosus (SLE)), rheumatoid arthritis (RA), multiple sclerosis (MS), and the like. Various compounds or antibodies targeting CD40L have been developed for the treatment of these diseases (Semin Immunol. 2009;21(5):293-300; Advanced Drug Delivery Reviews Volume 141, 15 February 2019, Pages 92-103).

In example of the present invention, it has been confirmed that, when the stefin A protein variant specifically binding to CD40L is administered to an animal model, the stefin A protein variant exhibit significant therapeutic effect for graft-versus-host disease (GVHD).

Therefore, in another aspect, the present invention relates to a pharmaceutical composition containing the stefin A protein variant specifically binding to CD40L the fusion protein, the conjugate, the nucleic acid, and/or the delivery vehicle as an active ingredient.

In some embodiments, the pharmaceutical composition can be used for the prevention or treatment of various diseases related to CD40L, in particular, the diseases known in the art that can achieve preventive or therapeutic effects through targeting to CD40L or inhibition of CD40L.

Therefore, from another aspect, the present invention relates to a pharmaceutical composition for preventing or treating immune diseases containing the stefin A protein variant specifically binding to CD40L the fusion protein, the conjugate, the nucleic acid, and/or the delivery vehicle as an active ingredient.

In another aspect, the present invention is related to a method for preventing or treating an immune disease comprising administering the stefin A protein variant specifically binding to CD40L the fusion protein, the conjugate, the nucleic acid, and/or the delivery vehicle to the subj ect.

In another aspect, the present invention relates to a use of the stefin A protein variant specifically binding to CD40L the fusion protein, the conjugate, the nucleic acid, and/or the delivery vehicle for preventing or treating immune diseases.

In another aspect, the present invention relates to a use of the stefin A protein variant specifically binding to CD40L the fusion protein, the conjugate, the nucleic acid, and/or the delivery vehicle for manufacturing the pharmaceutical composition. In some embodiments, the pharmaceutical composition is for preventing or treating immune diseases.

As used herein, the term "prevention" refers to any action that inhibits or delays the onset of an immune disease by administering the pharmaceutical composition provided in the present invention to a subject who is expected to develop an immune disease.

As used herein, the term "treatment" refers to any action that clinically intervenes to alter the natural process of a subject or cell to be treated, and may be performed during the course of or to prevent a clinical pathology. The desired therapeutic effect includes prevention of occurrence or recurrence of disease, alleviation of symptoms, inhibition of all direct or indirect pathological consequences of disease, prevention of metastasis, reduction of disease progression rate, alleviation or temporary alleviation of disease state, and prognosis improvement. For the purpose of the present invention, the treatment may be interpreted as including all actions of ameliorating the symptoms of an autoimmune disease by administering the pharmaceutical composition of the present invention to a patient suffering from an autoimmune disease including psoriasis, but the present invention is not particularly limited thereto.

In some embodiments, the pharmaceutical composition may further include at least one pharmaceutically acceptable carrier.

The pharmaceutically acceptable carrier included in the composition of the present invention is commonly used in formulations, and includes lactose, dextrose, sucrose, sorbitol, mannitol, starch, acacia gum, calcium phosphate, alginate, gelatin, calcium silicate, microcrystalline cellulose, polyvinylpyrrolidone, water, syrup, methyl cellulose, methyl hydroxybenzoate, propyl hydroxybenzoate, talc, magnesium stearate, mineral oil, and the like, but is not limited thereto. The composition of the present invention may further include a lubricant, a wetting agent, a sweetening agent, a flavoring agent, an emulsifying agent, a suspension agent, a preservative, and the like, in addition to the above components.

The pharmaceutical composition of the present invention may be administered orally or parenterally, and parenteral administration may include intravenous injection, subcutaneous injection, intramuscular injection, intraperitoneal injection, endothelial administration, topical administration, intranasal administration, intrapulmonary administration, and intrarectal administration.

When administered orally, the protein or peptide is digestible and therefore oral compositions should be formulated to coat the active agent or to protect the same from degradation in the stomach. In addition, the pharmaceutical composition may be administered by any device capable of transporting the active material to a target cell.

A suitable dosage of the composition according to the present invention may vary depending on factors such as formulation method, administration mode, patient's age, body weight, gender, and pathological condition, food, administration time, administration route, excretion rate, and reaction sensitivity. Here, an effective dosage thereof for the desired treatment or prevention may be easily determined and prescribed by skilled doctors. As used herein, the term "pharmaceutically effective amount" refers to an amount sufficient to prevent or treat an immune disease.

The pharmaceutical composition of the present invention may be formulated into a unit dosage form or placed in a multi-dose container using a pharmaceutically acceptable carrier and/or excipient according to a method that may be easily carried out by a person of ordinary skill in the art to which the present invention belongs. Here, the formulation may be in the form of a solution, suspension, or emulsion in oil or aqueous medium, or may be in the form of an extract, powder, suppository, powder, granule, tablet, or capsule, and may additionally include a dispersant or stabilizer.

The pharmaceutical composition according to the present invention may be administered in combination with a known drug or pharmaceutical composition having an effect of preventing, ameliorating, or eliminating symptoms of an immune disease, and may be co-administered with, for example, one or more other immunotherapeutic agents, chemotherapeutic agents, antibody therapeutic agents, and the like.

Specifically, the pharmaceutical composition of the present invention may be used in combination with a drug selected from the group consisting of disease-modifying antirheumatic drugs (DMARDs), nonsteroidal anti-inflammatory drugs (NSAIDs), corticosteroids, Janus kinase inhibitors, calcineurin inhibitors, mTOR inhibitors, IMDH inhibitors, and biological agents, but the present invention is not limited thereto.

In some embodiments, examples of the disease-modifying antirheumatic drugs (DMARDs) may include, but are not limited to, actarit, auranofin, azathioprine, bucillamine, cyclophosphamide, D-penicillamine, leflunomide, lobenzarit disodium, methotrexate, minocycline hydrochloride, mizoribine, salazosulfapyridine, and the like.

In some embodiments, examples of the nonsteroidal anti-inflammatory drugs (NSAIDs) may include, but are not limited to, celecoxib, diclofenac sodium, ibuprofen, ketoprofen, meloxicam, naproxen, piroxicam, and the like.

In some embodiments, examples of the corticosteroids may include, but are not limited to, prednisone (Deltasone, Orasone), budesonide (Entocort EC), prednisolone (Millipred), methylprednisolone, and the like.

In some embodiments, examples of the Janus kinase inhibitors may include, but are not limited to, approved drugs such as tofacitinib, abrocitinib, baricitinib, delgocitinib, fedratinib, filgotinib, oclacitinib, peficitinib, ruxolitinib, and upadacitinib, drugs in clinical trials such as cerdulatinib, gandotinib, lestaurtinib, momelotinib, pacritinib, and deucravacitinib, and the like.

In some embodiments, examples of the calcineurin inhibitors include, but are not limited to, cyclosporine, tacrolimus, and the like.

In some embodiments, examples of the mTOR inhibitors may include, but are not limited to, sirolimus (Rapamune), everolimus (Afinitor, Zortress), and the like.

In some embodiments, examples of the IMDH inhibitors may include, but are not limited to, azathioprine (Azasan, Imuran), mycophenolate (CellCept, Myfortic), and the like.

In some embodiments, examples of the biological agents includes abatacept, adalimumab, anakinra, certolizumab, etanercept, golimumab, infliximab, ixekizumab, natalizumab, rituximab, secukinumab, tocilizumab, ustekinumab, vedolizumab, basiliximab, daclizumab, and the like.

In some embodiments, the immune disease may be an autoimmune disease or an inflammatory disease.

In some embodiments, the immune disease may be selected from the group consisting of lupus (SLE), lupus nephritis (e.g. drug-induced lupus nephritis), immune thrombocytopenia (ITP), rheumatoid arthritis (RA), multiple sclerosis (MS), inflammatory bowel disease (IBD) (e.g. Crohn's disease and colitis/ulcerative colitis), graft-versus-host disease (GVHD) or allograft rejection, transplantation/solid organ transplantation (SOT), primary biliary cholangitis (PBC), psoriasis, psoriatic arthritis, collagen-induced arthritis, experimental allergic encephalomyelitis (EAE), oophoritis, allergic rhinitis, asthma, Sj ogren's syndrome, atopic eczema, myasthenia gravis, Graves' disease, and/or glomerulosclerosis, but is not limited thereto.

### Lupus

Systemic lupus erythematosus (SLE), also called lupus, is a chronic autoimmune disease that may cause swelling (inflammation) and pain throughout the body. There are several types of lupus, and systemic lupus erythematosus is the most common. Other types of lupus are described below.

Cutaneous lupus erythematosus: This type of lupus affects the skin. "Cutaneous" is a term that means skin. People with cutaneous lupus erythematosus may experience skin problems such as sensitivity to the sun and rashes. Hair loss may also be a symptom of the disease.

Drug-induced lupus: This lupus is caused by certain drugs. People with drug-induced lupus may have many of the same symptoms as systemic lupus erythematosus, but these symptoms are usually temporary.

Neonatal lupus: Neonatal lupus, which is a rare type of lupus, is a disease found in infants at birth. Children born with neonatal lupus inherit antibodies from their mother, and the mother may have had lupus during pregnancy or may develop the disease later. Not all babies born to mothers with lupus will develop lupus.

Examples of therapies that may be used in combination with the pharmaceutical composition of the present invention may include, but are not limited to, steroids (including corticosteroids, prednisone); hydroxychloroquine (Plaquenil^{®}); azathioprine (Imuran^{®}); methotrexate (Rheumatrex^{®}); cyclophosphamide (Cytoxan^{®}) and mycophenolate mofetil (CellCept^{®}); belimumab (Benlysta^{®}); and/or rituximab (Rituxan^{®}).

### Lupus nephritis

Lupus nephritis develops as a complication of lupus. Lupus nephritis occurs when lupus autoantibodies affect the structure of the kidneys, which filter waste. This causes kidney inflammation and may lead to blood in the urine, protein in the urine, high blood pressure, kidney dysfunction, or kidney failure. About half of adults with systemic lupus develop lupus nephritis. In systemic lupus, immune system proteins damage the kidneys, impairing the ability to filter waste.

### Rheumatoid arthritis

Rheumatoid arthritis is a type of chronic (progressive) arthritis that occurs in both joints of the body, such as the hands, wrists, and knees. The short-term goal of drugs for rheumatoid arthritis is to reduce joint pain and swelling and improve joint function. The long-term goal thereof is to slow or stop disease progression, particularly joint damage.

Arthritis is a general term that describes inflammation of the joints. Rheumatoid arthritis is a type of chronic (progressive) arthritis (causing pain and swelling) that usually occurs symmetrically in the joints (on both sides of the body, such as the hands, wrists, and knees). This involvement of multiple joints helps to distinguish rheumatoid arthritis from other types of arthritis.

In addition to affecting the joints, rheumatoid arthritis may sometimes affect the skin, eyes, lungs, heart, blood, nerves, or kidneys.

Therapies that may be used in combination with a pharmaceutical composition of the present invention to treat rheumatoid arthritis may include the following examples.

Pain relievers: These products include nonsteroidal anti-inflammatory drugs (NSAIDs) such as ibuprofen (MOTRIN^{®}), naproxen (ALEVE^{®}), celecoxib, diclofenac sodium, ketoprofen, meloxicam, and piroxicam. A COX-2 inhibitor, which is another type of drug, also falls in this category and relieves the signs and symptoms of rheumatoid arthritis. Celecoxib (CELEBREX^{®}), which is an example of the COX-2 inhibitor, is available in the United States. A COX-2 inhibitor is designed to have fewer side effects of gastrointestinal bleeding.

Disease-modifying antirheumatic drugs (DMARDs): Unlike other NSAIDs, DMARDs may actually slow disease progression by modifying the immune system. Previous DMARDs include methotrexate (TREXALL^{®}), gold salts, penicillamine (CUPRIMINE^{®}), hydroxychloroquine (PLAQUENIL^{®}), sulfasalazine (AZULFIDINE^{®}), cyclosporine (SANDIMMUNE^{®}), cyclophosphamide (CYTOXAN^{®}), and leflunomide (ARAVA^{®}). Currently available examples thereof may include methotrexate, leflunomide, hydroxychloroquine, and sulfasalazine (cyclosporine, cyclophosphamide, gold salt, and penicillamine are no longer commonly used).

Biological agents: In addition to these "traditional" DMARDs, new drugs are being approved. 7 classes of drugs are currently available, and in some cases, there are different types for each class (some of which are anti-TNF series and have been in use since 2000). Collectively, these DMARDs are known by other names: biological agents (or biological materials). Compared to traditional DMARDs, these products target molecules that cause inflammation in rheumatoid arthritis. Inflammatory cells in the joints are involved in the onset of rheumatoid arthritis itself. Biological agents reduce the inflammatory process that ultimately induces joint damage in rheumatoid arthritis. By attacking cells at a specific level rather than inflammation itself, biological agents are considered to be more effective and more specifically targeted. The biological agents include etanercept (ENBREL^{®}), infliximab (REMICADE^{®}), adalimumab (HUMIRA^{®}), anakinra (KINARET^{®}), abatacept (ORENCIA^{®}), rituximab (RITUXAN^{®}), certolizumab pegol (CIMZIA^{®}), golimumab (SYMPON^{®}), tocilizumab (ACTEMRA^{®}), and tofacitinib (XELJANJ^{®}). Some biological agents are used in combination with traditional DMARDs, especially methotrexate.

### Multiple sclerosis

Multiple sclerosis (MS) is an autoimmune disease. Under such conditions, the immune system mistakenly attacks healthy cells. In patients with multiple sclerosis, the immune system attacks myelin cells, which are the protective sheath that surrounds nerves in the brain and spinal cord. When myelin is damaged, nerve signals from the brain to other parts of the body are blocked. Damage may cause symptoms that affect the brain, spinal cord, and eyes.

There are four types of multiple sclerosis.

Clinically isolated syndrome (CIS): When there are the first symptoms of MS, health care providers often classify the same as CIS. Not all cases of CIS progress to multiple sclerosis.

Relapse-remitting MS (RRMS): This is the most common form of multiple sclerosis. People with RRMS have flares of new or worsening symptoms - also called relapses or exacerbations. A period of remission continues (when symptoms stabilize or disappear).

Primary-progressive MS (PPMS): People diagnosed with PPMS have symptoms that gradually worsen over time without relapse or remission.

Secondary-progressive MS (SPMS): In most cases, people originally diagnosed with RRMS eventually progress to SPMS. In secondary-progressive multiple sclerosis, nerve damage continues to accumulate. Symptoms gradually worsen. Such people may experience some relapse or flare-up (when symptoms increase), but there is no longer a period of remission after that (when symptoms stabilize or disappear).

Therapies that may be used in combination with the pharmaceutical composition of the present invention may include the following examples.

Disease-modifying therapy (DMT): Several drugs have received FDA approval for the treatment of long-term MS. These drugs help reduce relapses (also called flares or seizures). They slow the progression of the disease, and may prevent the formation of new lesions in the brain and spinal cord.

Relapse management medication: If there are severe seizures, the neurologist may recommend high-dose corticosteroids. Medications may reduce inflammation quickly, and may slow damage to the myelin sheaths surrounding nerve cells.

Physical rehabilitation: Multiple sclerosis may affect bodily functions. Staying physically healthy and strong will help a patient stay mobile.

Mental health counseling: Coping with chronic illness may be emotionally challenging. MS may sometimes affect mood and memory. Working with a neuropsychologist or getting other emotional support is an essential part of disease management.

### Inflammatory bowel disease

Inflammatory bowel disease (IBD) is a group of disorders that cause chronic inflammation (pain and swelling) of the intestine.

Crohn's disease and ulcerative colitis are the main types of IBD. The types thereof are described below.

Crohn's disease causes pain and swelling in the digestive tract, and may affect everything from the mouth to the anus. It most commonly affects the small intestine and upper part of the large intestine.

Ulcerative colitis causes boils and wounds (ulcers) in the large intestine (colon and rectum).

Microscopic colitis causes intestinal inflammation that may only be detected under a microscope.

Examples of therapies that may be used in combination with the pharmaceutical composition of the present invention may include aminosalicylates (anti-inflammatory agents such as sulfasalazine, mesalamine, or balsalazide) that minimize irritation to the intestines; antibiotics that treat infections and abscesses; biological agents that block signals from the immune system that cause inflammation; corticosteroids, such as prednisone, which suppress the immune system and manage flares; immunomodulators that calm the overactive immune system; antidiarrheal drugs; nonsteroidal anti-inflammatory drugs (NSAIDs); supplements such as vitamins and probiotics.

### Graft-versus-host disease (GVHD)

Graft-versus-host disease (GVHD) is a disease that may occur after allotransplantation. In GVHD, donated bone marrow or peripheral blood stem cells treat the recipient's body as a foreign object and the donated cells/bone marrow attack the body.

GVHD may include acute graft-versus-host disease (aGVHD) and chronic graft-versus-host disease (cGVHD).

### Psoriasis

Psoriasis is a chronic skin disorder meaning a skin disease with no cure. People with psoriasis have thick patches of pink or red skin covered with white or silvery scales. Thick scaly patches are called plaques. Psoriasis usually begins in early adulthood, but it may also start later. In addition to red scaly patches, symptoms of psoriasis include itching, cracking, dry skin, scaly scalp, skin pain, pitted nails, cracked or brittle nails, and joint pain.

Examples of therapies that may be used in combination with the pharmaceutical composition of the present invention may include steroid creams, moisturizers for dry skin, anthralin (drugs that slow the production of skin cells), medicated lotions, shampoos, and bath solutions to ameliorate scalp psoriasis, vitamin D3 ointment, vitamin A or retinoid creams, phototherapy, PUVA (treatment including both a drug called psoralen and UV exposure in a special form), methotrexate, retinoids, cyclosporine, and/or immunotherapy, but are not limited thereto.

### Sjogren's syndrome

Sjogren's syndrome is a lifelong autoimmune disease that reduces the amount of water produced by the sweat glands of the eyes and mouth. This disease is named after Henrik Sjogren, a Swedish ophthalmologist who first described the condition. Dry mouth and dry eyes are the main symptoms, but most people with these problems do not have Sjogren's syndrome. Dry mouth is also called xerostomia.

There are two forms of Sjogren's syndrome: primary Sjogren's syndrome, which occurs without other autoimmune diseases, and secondary Sjogren's syndrome, which occurs in people who already have other autoimmune diseases, such as rheumatoid arthritis, lupus, and psoriatic arthritis.

Examples of therapies that may be used in combination with the pharmaceutical composition of the present invention may include dry eye treatment (e.g. artificial tears, prescription eye drops, punctual plugs, surgery, and autologous serum eye drops), dry mouth treatment (e.g. saliva-forming agents), and treatment for joint or organ problems (e.g. analgesic agents, antirheumatic agents, immunosuppressive agents, steroids, antifungal agents, and vaginal dryness therapeutic agents).

### Myasthenia gravis

Myasthenia gravis (MG) is an autoimmune disease in which the body's immune system inadvertently attacks parts of itself. MG affects signal transmission between nerves and muscles (neuromuscular junctions).

Patients with myasthenia gravis lose the ability to voluntarily control their muscles. They experience muscle weakness and fatigue of varying severity, and may be unable to move the muscles of eyes, face, neck, or limbs. MG is a lifelong neuromuscular disease.

Myasthenia gravis affects about 20 out of 100,000 people. Experts estimate that between 36,000 and 60,000 Americans have this neuromuscular disease. The actual number of people affected may be higher because some people with mild symptoms may not know they have the disease. MG mainly affects women between the ages of 20 and 40 and men between the ages of 50 and 80. About 1 in 10 cases of MG occurs in teens (juvenile MG). The disease may affect people of all ages, but is rare in children.

Autoimmune MG is the most common form of this neuromuscular disease. Autoimmune MG may include the following examples.

Ocular MG: The muscles that move the eyes and eyelids weaken. People with ocular MG have droopy eyelids or may not be able to open their eyes, and may have double vision in some cases. Weakness of vision is often the first sign of MG. Nearly half of people with ocular MG evolve to a systemic form within 2 years after their first symptoms.

Systemic MG: Muscle weakness affects the eyes, face, and other parts of the body, such as the neck, arms, and legs. Patients may have difficulty talking, swallowing, raising their arms above the head, standing from a sitting position, walking long distances, or climbing stairs.

Examples of therapies that may be used in combination with the pharmaceutical composition of the present invention may include drugs, monoclonal antibodies, IV immunoglobulin (IVIG), plasma exchange, and/or surgery.

### Examples

Hereinafter, the present invention will be described in more detail with reference to the following examples. However, it will be obvious to those skilled in the art that the following examples are provided only for illustration of the present invention and should not be construed as limiting the scope of the present invention.

### Example 1: Selection of anti-CD40L stefin A protein variant from Phage Display Library

### Identification of candidate clones

Stefin A protein variant specifically binding to CD40L (Herein after, anti-CD40L stefin A protein variants) of the present invention were identified by selection from a library of stefin A protein variant with two random loop sequences, each loop having a length of about 9 amino acids displayed in a constant stefin A protein variant framework backbone based on the amino acid sequence of Stefin A. Such selection procedures have been described (see, e.g., Tiede et al. Protein Eng Des Sel. 2014. 27(5): 145-155 and Hughes et al. Sci Signal. 2018. 10(505): eaaj2005). According to such procedures, suspensions of phage expressing stefin A protein variant were incubated with human or mouse CD40L as required. In some cases, the CD40L was biotinylated and captured on alternating streptavidin and neutravidin beads, alternatively the CD40L was passively absorbed to a surface. Unbound phage particles were then washed away and, following washing, bound phages were eluted. Elution of bound phage was accomplished by incubating the antigen with low pH solution, followed by high pH solution and exposure to trypsin. Eluted phage particles were then used to infect Escherichia coli (E. coli), and infected bacteria were incubated under conditions suitable for replication of the bacteriophage. Following release of bacteriophage particles from these infected bacteria, the cycle of allowing phage particles to bind to the target antigen, eluting bound phage particles, propagating eluted phage particles in bacteria, and isolating released phage particles from infected bacteria was repeated to enrich the bacteriophage population for phage particles displaying proteins that bind the target antigen. Specific conditions were modified in these cycles, such as increasing the number of wash steps, reducing the amount of available antigen, or adding a blocking reagent, to select for phage particles displaying proteins that bind more tightly or specifically to the target antigen.

Following multiple rounds of phage display library selection and amplification, proteins expressed by phages were expressed and screened by enzyme-linked immunosorbent assay (ELISA). Briefly, stefin A protein variants were overexpressed from phagemid vectors, the bacterial cells were lysed, and lysates were used as substrates in ELISAs. In these ELISAs, human CD40L was immobilized on a plate, lysates were added, and the amount of anti-CD40L stefin A protein variant in each plate was measured using a detector antibody specific to the 6xMyc tag expressed on the candidate Stefin A protein variant. The phagemid vectors encoding the stefin A protein variant with the best human CD40L-binding activity were sequenced to identify DNA sequences of candidate clones for further development. The loop 2 and loop 4 amino acid sequences of each of these candidate clones are shown in Table 1 and Table 2, respectively.

### Example 2: Screening of anti-CD40L stefin A protein variant by Direct ELISA

A binding ELISA was performed to measure the affinity of different monomer DAW01 clones for hCD40L.

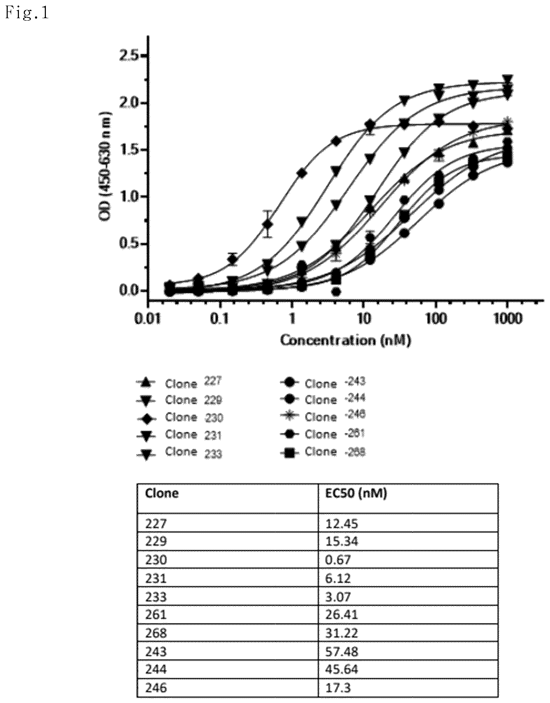

Briefly, plates were coated with hCD40L antigen at 5 ug/ml and incubated overnight at 4°C. Plates were washed 2 times with 150 µl of washing buffer (PBS, Tween 20 0.1%) with a plate washer and saturated with Casein 5% (Sigma) in PBS for 90 minutes at room temperature (25 ±1°C). For binding, each DAW01 clone and rhCD40 Fc were added to the plate, starting from 1µM, 1 in 3 and 300 nM, 1 in 3 respectively. Plates were washed 3 times as described previously. Anti-human CD40 biotinylated polyclonal antibody was then added, and the plate was incubated 90 min. Then, cystatin A biotinylated polyclonal antibody (BAF1407) was diluted to a concentration of 0.05µg/ml in dilution buffer (PBS, 1% casein, 0.01% Tween 20) and the plate was incubated for 90 minutes at room temperature (25 ±1°C). PolyHRP- streptavidin was then diluted in dilution buffer and the plates were incubated for 90 minutes at room temperature (25 ±1°C). The plates were then washed 3 times as described previously, and the substrate (TMB, Pierce Thermo-Scientific) was added to the plates for 10 minutes. The reaction was stopped using an acidic solution, and plates were read at 450 -630 nm. An example of the results is shown in FIG. 1. The EC50 values ranged between 0.67 to 60 nM.

### Example 3: Screening of anti-CD40L stefin A protein variant on hCD40L-HEK293 Cells by Flow Cytometry

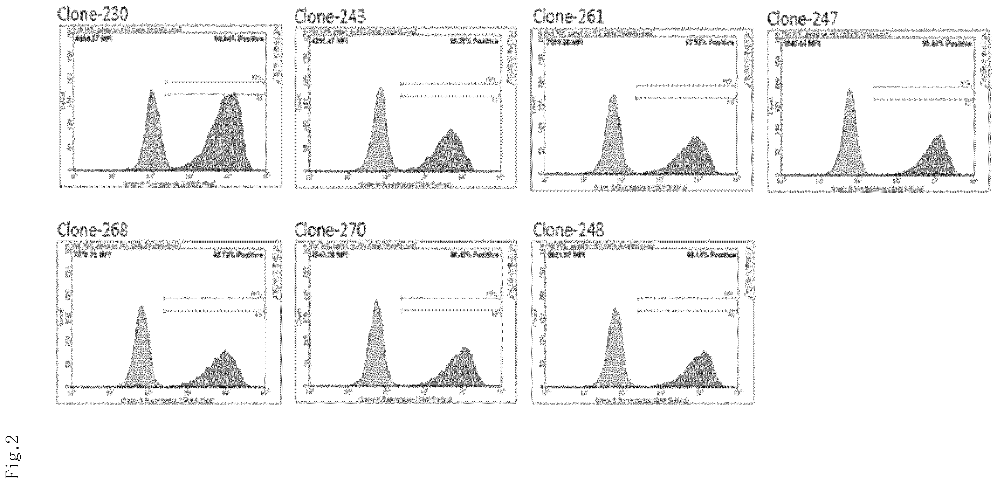

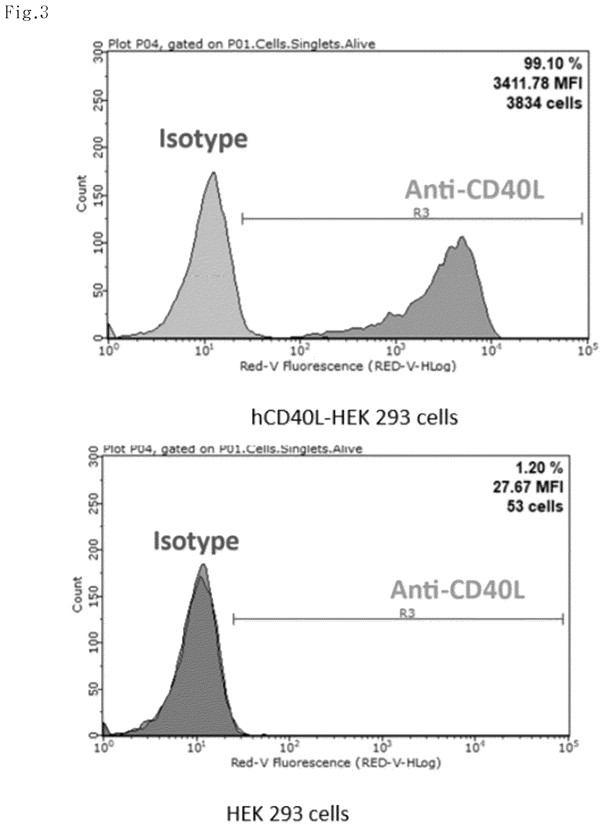

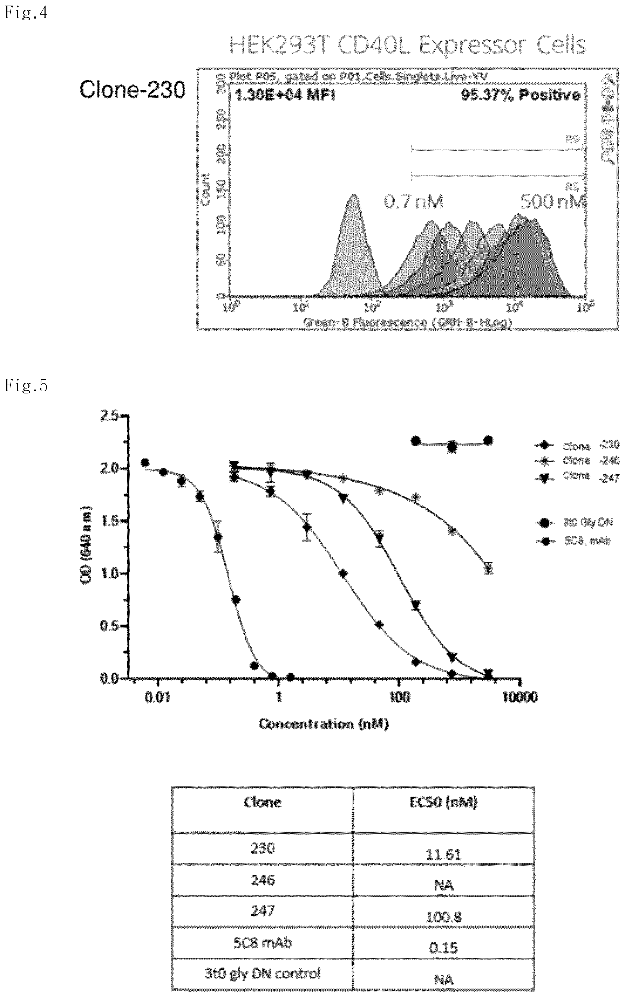

To examine the binding capacity of DAW01 monomer stefin A protein variant for hCD40L expressed on the surface of cells, a flow cytometry cell binding assay was performed. Briefly, hCD40L-HEK-293 cells (Crown Biosciences, C2041) were collected by centrifugation at 300rpm for 5 min. The cells were resuspended in PBS and 200,000 cells per well were dispatched in a round bottom 96 well plate. Cells were washed with PBS. The stefin A protein variant and controls were diluted in staining buffer containing 1 % BSA, 0.01 % Sodium Azide (NaN3), 2 mM EDTA in DPBS in duplicate and added on cells for staining for approximately 60 min at 4 ±1°c. Cells were washed and the secondary anti-Cystatin A (R&D, AF1407) was diluted 0.2mg/ml in staining buffer and added on cells for staining for approximately 45 min at 4 ±1°c. Cells were washed again and the detection antibody A488 anti- goat (ThermoFisher, A21467) was diluted 1:500 in staining buffer and added to the cells for staining (approximately 30 min at 4 ±1°C). Finally, the cells were washed and live and dead cells were stained using LID stain Zombie Yellow (Biolegend, 423103) diluted in staining buffer for 10 min at 4 ±1°C. Cells were washed again and fixation buffer (R&D) was added to each well for 10 min at 4 ±1°C then PBS with EDTA (Lonza) was added prior reading the plate on the flow cytometer (Guava 12 HT, Millipore). Dead cells were excluded, and the fluorescent green channel (488 nm/ 525/30) was acquired. Results were analyzed using Incyte and data were plotted using GraphPad. An example of results at 1 µM is shown in FIG. 2. stefin A protein variant were shown to bind specifically to hCD40L-HEK293 cells (dark gray). HEK-293 negatives cells were also used in the experiment to assess non-specific binding (light gray). The huCD40L HEK293 and HEK293 control cells were assessed for their expression of hCD40L using a BV711 mAb (clone 24-31). The results are shown in FIG. 3. The binding of clone 230 (SEQ ID NO: 249) to hCD40L-HEK293 cells was assessed at different doses ranging from 0.7 to 500 nM. The binding of clone 230 was shown to be dose-dependent between 0.7-55 nM and reached saturation above 55nM as seen in FIG. 4.

### Example 4: Screening of Anti-CD40L stefin A protein variant in a CD40-HEK Blue Reporter Assay

### HEK-Blue CD40 expressor cells (Invivogen), allow detection of bioactive CD40L

through activation of NF-κB following CD40 stimulation. Activation of the NF-κB pathway can be determined by measuring levels of secreted embryonic alkaline phosphatase (SEAP) in the cell medium. The assay was performed according to the manufacturer's instructions.

Briefly, cells were seeded at 20000 cells /well in (100µl) in test medium (DMEM High glucose with Blasticin and Zeocin) in a 96-well flat bottom tissue culture plate and grown overnight at 37 °C, 5% CO2. Cells were then treated by removing 50 µl of test medium and adding 50 µl 4X dilution of test the stefin A protein variant or controls with hCD40L (0.8 nM final concentration). Plates were then incubated for 22h at 37 °C, 5% CO2. The following day, the supernatant was collected and SEAP activity was detected by mixing 30ul of each well with 200 ul of HEK-Blue detection reagent (Invivogen). The mixture was incubated 37 °C and any color change was monitored periodically. Absorbance (640 nm) was measured at 3 hours using Pherastar plate reader. The data were plotted. The IC50 was then calculated using the interpolated non - linear four-parameters curve as OD=f(log concentration). 5C8, a clinical grade monoclonal anti-hCD40L antibody, was used as a positive control in this assay. The calculated IC50 ranged from 11.6 to 100 nM (e.g., clone 230 (SEQ ID NO: 249) and clone 248 (SEQ ID NO: 267)). An example of the results is shown in FIG. 5.

### Example 5: Formatting the stefin A protein variant in Dimeric or Trimeric Configurations to Increase Avidity to hCD40L

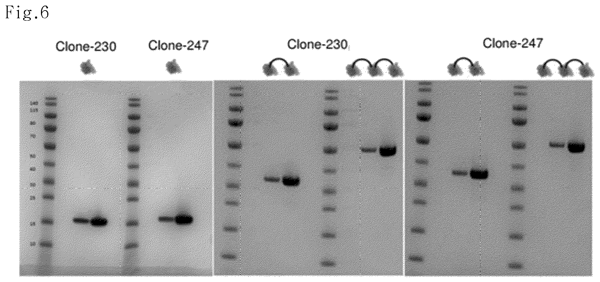

The stefin A protein variant can be engineered to assemble into stable multimeric oligomers to increase avidity. The resulting stefin A protein variant, which are In Line Fusion (ILF) proteins, can be dimers or trimers as pictured in FIG. 6 with various linkers (rigid linkers, see e.g., SEQ ID NOs: 508, 510-514; or flexible linkers, see e.g., SEQ ID NOs: 509, 515-518).

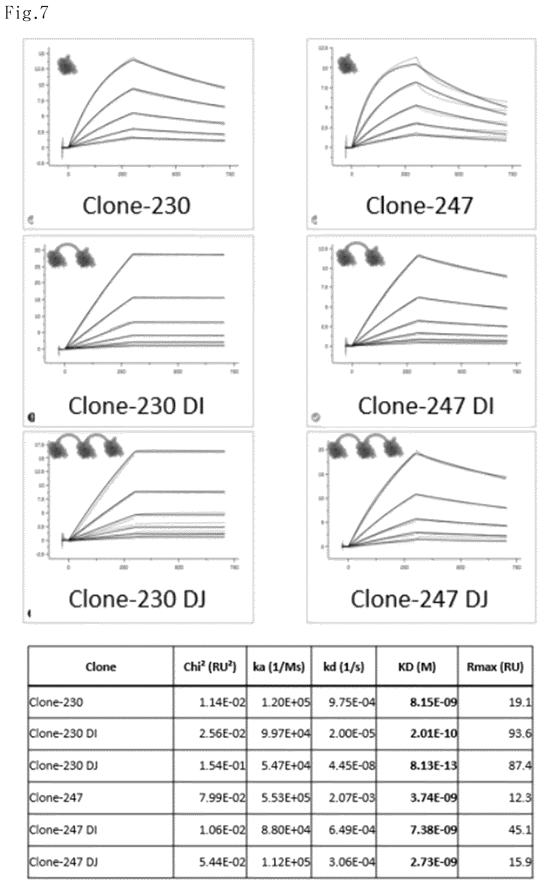

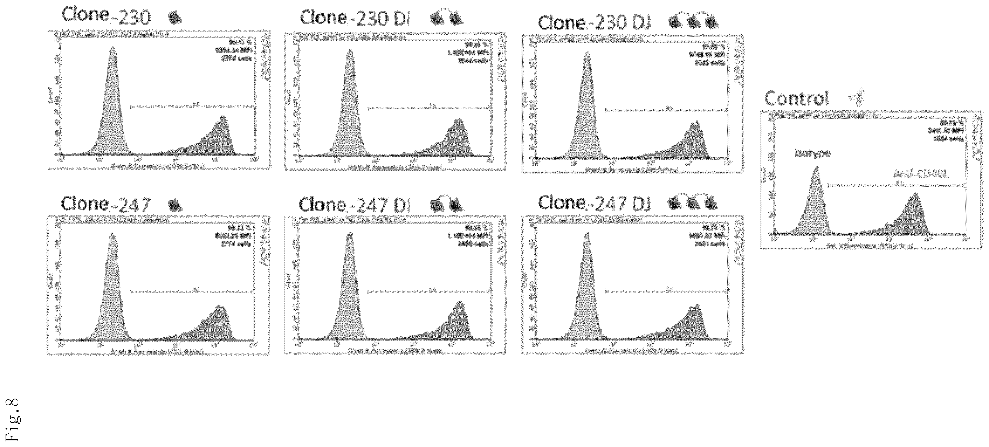

The binding of the different stefin A protein variant (monomeric, dimeric, trimeric) to hCD40L was examined as described above. The results are shown in FIG. 7 and demonstrate that the ILF proteins had comparable or greater binding affinities for the target ligand than the monomeric formulations. The binding was further examined with flow cytometry (FIG. 8), and it was demonstrated that the stefin A protein variant (monomeric, dimeric, and trimeric formats) bound hCD40L at levels comparable to an anti-CD40L antibody.

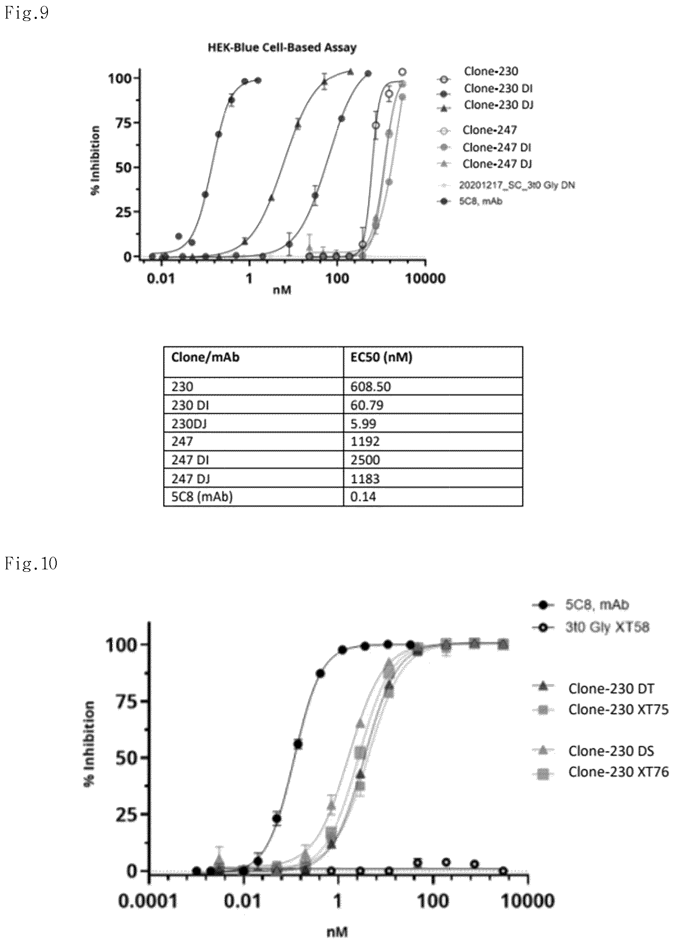

The different formats were also screened using a HEK-Blue cell-based assay, as described above. The results are shown in FIG. 9, and demonstrate that the clone 230 DJ format (trimeric) performed the best, with an EC50 of 5.99 nM.

### Example 6: Characterization of In Line Fusion (ILF) stefin A protein variant in Trimeric or Tetrameric Configurations with HSA binding stefin A protein variants

In a further experiment, stefin A protein variants were engineered to assemble into stable multimeric oligomers. The resulting stefin A protein variants, which are In Line Fusion (ILF) proteins, can be trimers or tetramers with various linkers (rigid linkers, see e.g., SEQ ID NOs: 508, 510-514; or flexible linkers, see e.g., SEQ ID NOs: 509, 515-518). Four stefin A protein variants were tested: clone-230 DT (trimer with a rigid linker), clone-230 XT75 (trimer with a rigid linker and an HSA binding stefin A protein variants), clone-230 DS (tetramer with a rigid linker), and clone-230 XT76 (tetramer with a rigid linker and an HSA binding stefin A protein variants).

To evaluate the avidity of the different formats, a CD40L competitive ELISA was performed. Briefly, the plates were coated with rhCD40 Fc at 1 µg/ml and incubated overnight at 4°C. The plates were washed twice with 150 µl of washing buffer (PBS, Tween 20 0.1%) with a plate washer and saturated with Casein 5% (Sigma) in PBS for 90 minutes at room temperature (25 ±1°C). Human CD40L at 2x EC80 (1 nM) was combined with anti-hCD40L monoclonal antibody starting from 10 nM or with each tested ILF clone. The resulting solution was then mixed and added to the plates. The plates were washed 3 times as described previously. Biotinylated anti-hCD40L polyclonal antibody was then diluted in dilution buffer and added to the plates. The plates were incubated for 90 minutes at room temperature (25 ±1°C). The plates were then washed. PolyHRP-Streptavidin was then diluted in dilution buffer and added to the plates. The plates were incubated an additional 90 minutes at room temperature (25 ±1°C). Plates were washed 3 times as described previously and the substrate (TMB, Pierce Thermo-Scientific) was added in the plate for 10 minutes. The reaction was stopped using an acidic solution and the plates were read at 450 -630 nm and the resulting percent inhibition was calculated. The results are shown in FIG. 10 and demonstrate that the tetrameric formats slightly outcompete the trimeric formats.

The different formats were also screened using a HEK-Blue cell-based assay, as described above. The results are shown in FIG. 11, and demonstrate that the tetrameric formats outperformed the trimeric formats.

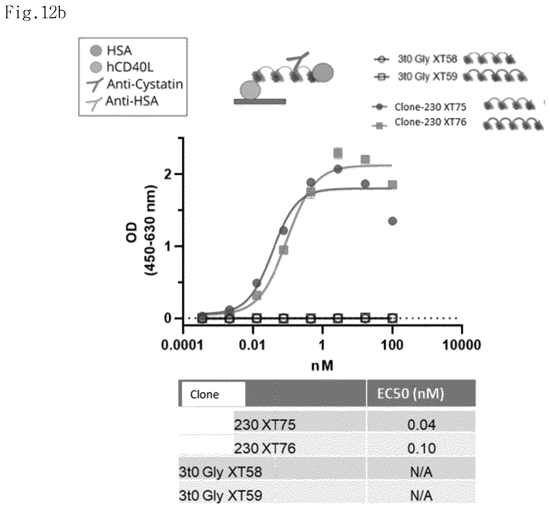

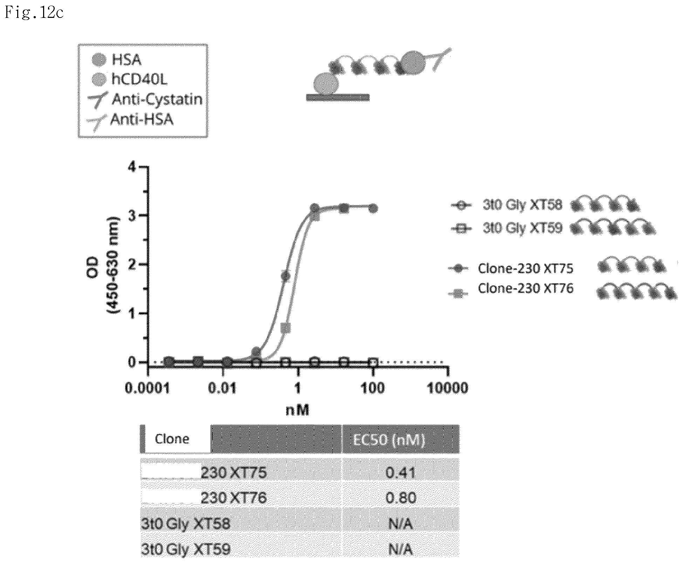

To demonstrate that the different stefin A protein variants ILF proteins were able to engage both targets (human CD40L and HSA) simultaneously, a bridging ELISA was performed. The assay captured the bispecific Stefin A protein variant using hCD40L and detecting the Stefin A protein variant using an anti-HSA antibody, that is, permitting the detection of the HSA binding stefin A protein variants. Briefly, human CD40L was coated on 96 well plates at 0.5 mg/ml in carbonate buffer. After saturation with 5% casein/PBS buffer, the plates were washed and a dilution of Stefin A protein variant or controls was incubated with HSA at a final concentration of 10 µM for 90 minutes. Plates were then washed, and a biotinylated polyclonal antibody, anti-HSA (HRP-conjugated) (Abcam), was added for 90 minutes. After a last washing step, TMB was added for the development of the experiment and the plates were read at 450 nm. The EC50 was then calculated using the interpolated non-linear four-parameters standard curve (FIG. 12C). In control experiments, an anti-cystatin antibody, which binds to the stefin A framework of the stefin A protein variants was added in place of the anti-HSA antibody in the absence (FIG. 12A) or presence (FIG. 12B) of HSA, demonstrating that the Stefin A protein variant bind hCD40L under both conditions. The bridging ELISA data showed that engagement with HSA does not impact CD40L binding for either of the two stefin A protein variantformats tested.

### Example 7: Therapeutic effect of anti-CD40L stefin A protein variant on haploidentical genotype GVHD animal model

### Example 7-1: Experimental method

192 healthy female C57BL/6 mice (6 weeks old) were purchased from Janvier (France) and used for splenocyte extraction for GVHD induction. 12 healthy female B6D2F1 mice (6 weeks old) were purchased from Charles River and used for GVHD induction (syngeneic control). 70 healthy female B6D2F1 mice (6 weeks old) were purchased from Charles River and used as donor mice.

The stefin A protein variant specifically binding to mouse CD40L was expressed in the form of a trimeric in-line fusion (Table 15).

**[Table 15]**

| Stefin A protein variant | Sequence | SEQ ID NO: |
|---|---|---|
| XT54 | IPGGLSEAKPATPEIQEIVDKVKPQLEEKTGETYGKL EAVQYKTQVVFQYVFSDEDGTNYYIKVRAGDNKY MHLKVFKSLFRWQRGNPEDLVLTGYQVDKNKDDE LTGFGGGGSGGGGSGGGGSGGGGSGGGGSGGGGS | 729 |
| | MIPGGLSEAKPATPEIQEIVDKVKPQLEEKTGETYGK LEAVQYKTQVVFQYVFSDEDGTNYYIKVRAGDNKY MHLKVFKSLFRWQRGNPEDLVLTGYQVDKNKDDE LTGFGGGGSGGGGSGGGGSGGGGSGGGGSGGGGS MIPGGLSEAKPATPEIQEIVDKVKPQLEEKTGETYGK LEAVQYKTQVVFQYVFSDEDGTNYYIKVRAGDNKY MHLKVFKSLFRWQRGNPEDLVLTGYQVDKNKDDE LTGFGGGGSGGGGSGGGGSGGGGSGGGGSGGGGS MIPRGLSEAKPATPEIQEIVDKVKPQLEEKTGETYGK LEAVQYKTQVLANFFQRRWPGSTNYYIKVRAGDNK YMHLKVFNGPWKFRNTDRGADRVLTGYQVDKNKD DELTGFAAAGEQKLISEEDLGAAHHHHHH-- | |
| XT55 | IPGGLSEAKPATPEIQEIVDKVKPQLEEKTGETYGKL EAVQYKTQVVFQYVFSDEDGTNYYIKVRAGDNKY MHLKVFKSLFRWQRGNPEDLVLTGYQVDKNKDDE LTGFAEAAAKEAAAKEAAAKEAAAKEAAAKEAAA KMIPGGLSEAKPATPEIQEIVDKVKPQLEEKTGETYG KLEAVQYKTQVVFQYVFSDEDGTNYYIKVRAGDNK YMHLKVFKSLFRWQRGNPEDLVLTGYQVDKNKDD ELTGFAEAAAKEAAAKEAAAKEAAAKEAAAKEAA AKMIPGGLSEAKPATPEIQEIVDKVKPQLEEKTGETY GKLEAVQYKTQVVFQYVFSDEDGTNYYIKVRAGDN KYMHLKVFKSLFRWQRGNPEDLVLTGYQVDKNKD DELTGFAEAAAKEAAAKEAAAKEAAAKEAAAKEA AAKMIPRGLSEAKPATPEIQEIVDKVKPQLEEKTGET YGKLEAVQYKTQVLANFFQRRWPGSTNYYIKVRAG DNKYMHLKVFNGPWKFRNTDRGADRVLTGYQVDK NKDDELTGFAAAGEQKLISEEDLGAAHHHHHH-- | 730 |

The spleens of C57BL/6 and B6D2F1 mice were excised, and splenocytes were extracted therefrom and prepared in an HBSS buffer. Red blood cells contained in the extracted splenocytes were prepared through dissolution in a red blood cell lysate (BD Pharma) and then washing. 64 B6D2F1 mice (G4-G11) were injected intravenously with 6×10⁷ cells/head of splenocytes isolated from C57BL/6 spleens. Three B6D2F1 mice (G3) were injected intravenously with 6×10⁷ cells/head of splenocytes isolated from the spleens of C57BL/6 mice. Three B6D2F1 mice (G2) were injected intravenously with 6×10⁷ cells/head of splenocytes isolated from the spleens of the same B6D2F1 mice (FIG. 13).

The animals for this experiment were randomly grouped depending on the body weight, and analysis of variance (ANOVA) for homogeneity between groups was performed to confirm that there was no statistical significance.

The test material was administered intraperitoneally. For intraperitoneal administration, the pH of the formulation was adjusted to 7.3-7.4, and the volume thereof was set to 20 mL/kg. The type and amount of administration material injected to the test animals are as shown in the following table. The first administration of the test material was carried out 1 hour after GVHD induction, and the administration interval depending on the administration material was set as follows. Administration was carried out six times at 2-day intervals after GVHD induction in the stefin A protein variant administration group except the antibody administration group, and the MR-1 antibody was administered three times at 2-day intervals after GVHD induction (FIG. 14, Table 16). Animal survival and behavior were observed daily and clinical measures were recorded daily. Body weight and clinical score were recorded daily. The clinical scoring of GVHD was evaluated by performing visual observation through the following evaluation formats and recording the sum of the observed scores. For statistical significance between groups, ANOVA was performed using GraphPad Prism. Statistical significance between groups was judged to be significant when p<0.05 or less.

**[Table 16]**

| Group | No. Animals | Cells | Treatment | Dose (mg/kg) | Treatment Schedule |
|---|---|---|---|---|---|
| 1 | 3 | NO | Untreated | - | - |
| 2 | 3 | syngenic control | Untreated | - | - |
| 3 | 3 | C57BL/6 splenocytes | Untreated | - | - |
| 4 | 8 | C57BL/6 splenocytes | MR1 | 10 | Q2DX3 |
| 5 | 8 | C57BL/6 splenocytes | XT54 | 2.5 | Q2DX6 |
| 6 | 8 | C57BL/6 splenocytes | XT54 | 5 | Q2DX6 |
| 7 | a | C57BL/6 splenocytes | XT54 | 10 | Q2DX6 |
| 8 | 8 | C57BL/6 splenocytes | XT55 | 2.5 | Q2DX6 |
| 9 | 8 | C57BL/6 splenocytes | XT55 | 5 | Q2DX6 |
| 10 | 8 | C57BL/6 splenocytes | XT55 | 10 | Q2DX6 |

### Example 7-2: Result

After random grouping, the mean body weight of the animal groups was 21.5 g, the body weight ranging from 18.8 g to 23.8 g. Based on the results of statistical analysis, there was no significant difference between groups. Changes in body weight of experimental animals were monitored throughout the study period. In order to confirm body weight changes, the mean body weight change (MBWC%) was compared based on D18. The mean body weight change in the G1 group was increased by 9.7%, and the mean body weight change in the G2 group was increased by 15.6%. In the G3 group, which is the GVHD control, the mean body weight change was decreased by 24.4%. The mean body weight changes of the drug administration groups, G4 to G11 groups, were as follows.

G4 group: 1.6% increase, G5 group: 4.1% decrease, G6 group: 14.6% decrease, G7 group: 2.8% increase, G8 group: 6.4% decrease, G9 group: 9.0% decrease, G10 group: 4.4% decrease, G11 Group: 21.7% decrease, indicating that the stefin A protein variant administration groups showed good mean body weight changes compared to the G3 group.

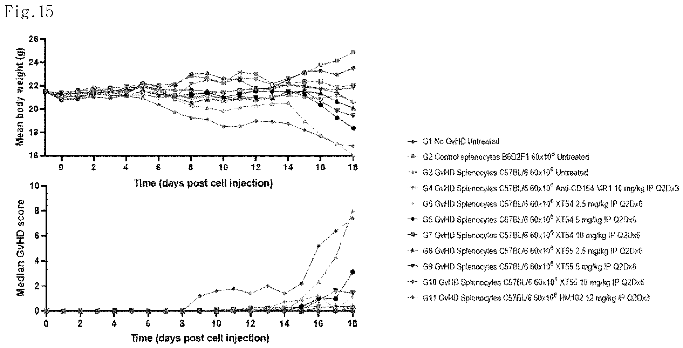

Changes in clinical scores for individual animals were recorded using a scorecard including body, skin, hair, and mobility criteria. The average GVHD scores of the G1 and G2 groups did not increase throughout the study period. The GVHD score of the G3 group started increasing from D15, and recorded an average value of 8.0 on D18. Similar to the G1 and G2 groups, the GVHD score did not increase in the G4 group throughout the study period, and the GVHD scores of the G5 to G7 groups started to increase between 9 and 14 days after GVHD induction, and showed 0.3 to 1.1 on D18, the end of the experiment. The clinical scores of the G8 to G10 groups started to increase between 7 and 10 days after GVHD induction, and showed a GVHD score of 0.3 to 1.4 on D18, the end of the experiment (FIG. 15).

In consideration thereof, it could be confirmed that the therapeutic effect by the stefin A protein variant was statistically significantly inhibited compared to the GVHD control.

### Industrial applicability

The stefin A protein variant specifically binding to CD40L of the present invention, and/or the fusion protein or conjugate comprising thereof, is a non-immunogenic polypeptide that can bind to CD40L with high affinity and specificity, thereby it is useful for targeting a variety of cells expressing CD40L and CD40L. In addition, stefin A protein variant specifically binding to CD40L of the present invention exhibits excellent ability to inhibit CD40L activity and can be usefully used for medical/pharmaceutical purposes for the prevention and treatment of various diseases related to CD40L.

Although specific embodiments of the present invention have been described illustratively, those skilled in the art will appreciate that the present invention may be embodied in other specific forms without changing the technical spirit or essential features thereof. Thus, the embodiments described above should be understood to be non-limiting and illustrative in every way.

## Claims

1. A stefin A protein variant specifically binding to CD40L.

2. The stefin A protein variant of claim 1, wherein the stefin A protein variant specifically binds to CD40L with a K_{d} of 1 × 10⁻⁶M or less.

3. The stefin A protein variant of claim 1, comprising an amino acid sequence having at least 80%, at least 90%, at least 95%, at least 98% or at least 100% identity to the following amino acid sequence: 
MIPGGLSEAKPATPEIQEIVDKVKPQLEEKTGETYGKLEAVQYKTQVV-(Xaa)n-
GTNYYIKVRAGDNKYMHLKVFKSL-(Xaa)m-EDLVLTGYQVDKNKDDELTGF (SEQ ID
NO: 5),
 wherein Xaa, individually for each occurrence, is an amino acid residue; and n and m are each, independently, an integer from 3 to 20.

4. The stefin A protein variant of claim 1, comprising the amino acid sequence selected from the group consisting of SEQ ID NOs: 246 to 365.

5. The stefin A protein variant of claim 3, wherein (Xaa)ₙ comprises an amino acid sequence having at least 75%, at least 85% or at least 95% identity to the amino acid sequence selected from SEQ ID NOs: 6 to 125.

6. The stefin A protein variant of claim 3, wherein (Xaa)ₙ comprises the amino acid sequence selected from the group consisting of SEQ ID NOs: 126 to 245.

7. The stefin A protein variant of claim 3, wherein (Xaa)ₘ comprises an amino acid sequence having at least 75% or at least 85% identity to the amino acid sequence selected from SEQ ID NOs: 126 to 245.

8. The stefin A protein variant of claim 3, wherein (Xaa)ₘ comprises the amino acid sequence selected from SEQ ID NOs: 126 to 245.

9. The stefin A protein variant of claim 1, further comprising signal peptide.

10. A fusion protein comprising the stefin A protein variant of claim 1.

11. The fusion protein of claim 10, wherein the fusion protein comprises an amino acid sequence having at least 90%, at least 95% or 100% identity to the the amino acid sequence of SEQ ID NO: 681, 691, 694 or 695.

12. The fusion protein of claim 10, wherein the fusion protein is a trimer or tetramer of the stefin A protein variant, wherein the trimer or tetramer is one in which the stefin A protein variants are linked by a linker.

13. The fusion protein of claim 12, wherein the linker is a rigid linker or flexible linker.

14. The fusion protein of claim 12, wherein the linker is selected form the amino acid sequence of SEQ ID NO: 508 to 534.

15. The fusion protein of claim 14, wherein the linker is a peptide linker comprising the sequence (EAAAK)ₙ, wherein n is 1-6.

16. The fusion protein of claim 10, wherein the fusion protein further comprises at least one selected from the group consisting of binding domain, cytokines, half-life extension domain, growth factor, enzyme, and cell penetrating domain.

17. The fusion protein of claim 10, wherein the fusion protein further comprises at least one selected from the group consisted of transmembrane domain, hinge domain, coiled coil domain, viral domain, intracellular signaling domain and localization domain.

18. The fusion protein of claim 10, the fusion protein comprises a therapeutic or diagnostic moiety.

19. The fusion protein of claim 18, wherein the therapeutic moiety is a therapeutic protein.

20. The fusion protein of claim 19, wherein the therapeutic protein is antibody.

21. The fusion protein of claim 18, wherein the diagnostic moiety is a fluorescent protein.

22. The fusion protein of claim 18, wherein the therapeutic or diagnostic moiety is linked by a linker.

23. The fusion protein of claim 10, the fusion protein further comprises a half-life extension moiety.

24. The fusion protein of claim 23, wherein the half-life extension moiety is selected from antibody Fc domains, human serum albumin, serum binding proteins.

25. The fusion protein of claim 23, wherein the half-life extension moiety is a Stefin A protein variant that binds specifically to human serum albumin with a K_{d} of 1× 10⁻⁶M or less.

26. A conjugate comprising the stefin A protein variant of claim 1.

27. The conjugate of claim 26, the stefin A protein variant is conjugated with drug.

28. A nucleic acids encoding the stefin A protein variant of claim 1 or the fusion protein of claim 10.

29. The nucleic acids of claim 28, wherein the polynucleotide is a DNA or an RNA.

30. The nucleic acids of claim 29, wherein the RNA is an mRNA.

31. A delivery vehicle comprising the nucleic acids of claim 28.

32. The delivery vehicle of claim 31, which is a viral delivery vehicle or a non viral delivery vehicle.

33. The delivery vehicle of claim 32, wherein the viral delivery vehicle is an adenoviral vector, a retroviral vector, a lentiviral vector, or an adeno-associated viral (AAV) vector.

34. The delivery vehicle of claim 32, wherein the non-viral delivery vehicle is a liposome or a lipid nanoparticle.

35. The delivery vehicle of claim 34, wherein lipid nanoparticle is cationic lipid nanoparticle.

36. An expression vector comprising the nucleic acids of claim 28.

37. The expression vector of claim 36, the expression vector is a plasmid or minicircle.

38. A genetically engineered cell into which the nucleic acids of claim 28 has been introduced.

39. A method for producing a stefin A protein variant or a fusion protein comprising,
culturing the genetically engineered cell of claim 38 to produce a stefin A protein variant or the fusion protein; and
obtaining the produced stefin A protein variant or the fusion protein.

40. A pharmaceutical composition comprising at least one selected from the group consistied of the stefin A protein variant of claim 1, the fusion protein of claim 10, the conjugate of claim 26, a nuecleic acids encoding the stefin A protein variant of claim 1 or the fusion protein of claim 10, and a delivery vehicle comprising the nucleic acids.

41. The pharmaceutical composition of claim 40, the pharmaceutical composition is used for prevention or treatment of immune diseases.

42. The pharmaceutical composition of claim 41, wherein the immune disease is selected from the group consisting of lupus (SLE), lupus nephritis, immune thrombocytopenia (ITP), rheumatoid arthritis (RA), multiple sclerosis (MS), inflammatory bowel disease (IBD), graft-versus-host disease (GVHD) or allograft rejection, transplantation/solid organ transplantation (SOT), primary biliary cholangitis (PBC), psoriasis, psoriatic arthritis, collagen-induced arthritis, oophoritis, allergic rhinitis, asthma, Sjogren's syndrome, atopic eczema, myasthenia gravis, Graves' disease, and glomerulosclerosis.
